(19) Europäisches Patentamt
European Patent Office
Office européen des brevets

(11) EP 1 442 750 B1

(12) **EUROPEAN PATENT SPECIFICATION**

(45) Date of publication and mention of the grant of the patent:
**01.08.2012 Bulletin 2012/31**

(51) Int Cl.:
***A61K 48/00*** *(2006.01)* ***A61P 35/00*** *(2006.01)*

(21) Application number: **03012772.4**

(22) Date of filing: **20.11.1998**

(54) **Treatment of cancer using cytokine-expressing polynucleotides and compositions therefor**

Krebsbehandlung mit Zytokin exprimierenden Polynukleotiden und entsprechende Mittel

Traitement de cancer à l'aide de polynucléotides exprimant des cytokines et préparations correspondantes

(84) Designated Contracting States:
**AT BE CH CY DE DK ES FI FR GB GR IE IT LI LU MC NL PT SE**

(30) Priority: **20.11.1997 US 67087 P**
**30.03.1998 US 79914 P**
**15.09.1998 US 100820 P**

(43) Date of publication of application:
**04.08.2004 Bulletin 2004/32**

(60) Divisional application:
**08005898.5 / 1 987 845**

(62) Document number(s) of the earlier application(s) in accordance with Art. 76 EPC:
**98960333.7 / 1 032 428**

(73) Proprietor: **VICAL INCORPORATED**
**San Diego, California 92121-4343 (US)**

(72) Inventors:
- **Horton, Holly**
  **San Diego, CA 92131 (US)**
- **Parker, Suezanne**
  **San Diego, CA 92131 (US)**
- **Manthorpe, Marston**
  **San Diego, CA 92129 (US)**
- **Felgner, Philip**
  **Rancho Santa Fe, CA 92067 (US)**

(74) Representative: **Walton, Seán Malcolm et al**
**Mewburn Ellis LLP**
**33 Gutter Lane**
**London**
**EC2V 8AS (GB)**

(56) References cited:
**WO-A-97/30089 US-A- 5 268 169**

- **DATABASE EMBASE [Online] ELSEVIER SCIENCE PUBLISHERS, AMSTERDAM, NL; 1997, KINOSHITA Y ET AL: "The possibility of gene immunotherapy for tumor using in vivo lipofection procedure" XP002283031 Database accession no. EMB-1997118387 & BIOTHERAPY 1997 JAPAN, vol. 11, no. 3, 1997, pages 448-450, ISSN: 0914-2223**
- **DATABASE BIOSIS [Online] BIOSCIENCES INFORMATION SERVICE, PHILADELPHIA, PA, US 1995 HOFLAND HANS ET AL: 'Inhibition of human ovarian carcinoma cell proliferation by liposome-plasmid DNA complex' Database accession no. PREV199598174545 & BIOCHEMICAL AND BIOPHYSICAL RESEARCH COMMUNICATIONS, vol. 207, no. 2, 1995, pages 492-496, ISSN: 0006-291X**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



Printed by Jouve, 75001 PARIS (FR)

SEQUENCE LISTING

<110> VICAL
Horton, Holly
Parker, Suezanne
Manthorpe, Marston
Felgner, Philip

<120> Treatment of Cancer Using Cytokine-Expressing Polynucleotides and Compositions Therefor

<130> 1530.006PC03

<140>
<141>

<150> US 60/067,087
<151> 1997-11-20

<150> US 60/079,914
<151> 1998-03-30

<150> US 60/100,820
<151> 1998-09-15

<160> 26

<170> PatentIn Ver. 2.0
<210> 1
<211> 5428
<212> DNA
<213> Homo sapiens

<400> 1

```
tcgcgcgttt cggtgatgac ggtgaaaacc tctgacacat gcagctcccg gagacggtca 60
cagcttgtct gtaagcggat gccgggagca gacaagcccg tcagggcgcg tcagcgggtg 120
ttggcgggtg tcggggctgg cttaactatg cggcatcaga gcagattgta ctgagagtgc 180
accatatgcg gtgtgaaata ccgcacagat gcgtaaggag aaaataccgc atcagattgg 240
ctattggcca ttgcatacgt tgtatccata tcataatatg tacatttata ttggctcatg 300
tccaacatta ccgccatgtt gacattgatt attgactagt tattaatagt aatcaattac 360
```

ggggtcatta gttcatagcc catatatgga gttccgcgtt acataactta cggtaaatgg 420
cccgcctggc tgaccgccca acgacccccg cccattgacg tcaataatga cgtatgttcc 480
catagtaacg ccaataggga ctttccattg acgtcaatgg gtggagtatt tacggtaaac 540
tgcccacttg gcagtacatc aagtgtatca tatgccaagt acgcccccta ttgacgtcaa 600
tgacggtaaa tggcccgcct ggcattatgc ccagtacatg accttatggg actttcctac 660
ttggcagtac atctacgtat tagtcatcgc tattaccatg gtgatgcggt tttggcagta 720
catcaatggg cgtggatagc ggtttgactc acggggattt ccaagtctcc accccattga 780
cgtcaatggg agtttgtttt ggcaccaaaa tcaacgggac tttccaaaat gtcgtaacaa 840
ctccgcccca ttgacgcaaa tgggcggtag gcgtgtacgg tgggaggtct atataagcag 900
agctcgttta gtgaaccgtc agatcgcctg gagacgccat ccacgctgtt ttgacctcca 960
tagaagacac cgggaccgat ccagcctccg cggccgggaa cggtgcattg gaacgcggat 1020
tccccgtgcc aagagtgacg taagtaccgc ctatagactc tataggcaca cccctttggc 1080
tcttatgcat gctatactgt ttttggcttg gggcctatac acccccgctt ccttatgcta 1140
taggtgatgg tatagcttag cctataggtg tgggttattg accattattg accactcccc 1200
tattggtgac gatactttcc attactaatc cataacatgg ctctttgcca caactatctc 1260
tattggctat atgccaatac tctgtccttc agagactgac acggactctg tatttttaca 1320
ggatggggtc ccatttatta tttacaaatt cacatataca acaacgccgt cccccgtgcc 1380
cgcagttttt attaaacata gcgtgggatc tccacgcgaa tctcgggtac gtgttccgga 1440
catgggctct tctccggtag cggcggagct tccacatccg agccctggtc ccatgcctcc 1500
agcggctcat ggtcgctcgg cagctccttg ctcctaacag tggaggccag acttaggcac 1560
agcacaatgc ccaccaccac cagtgtgccg cacaaggccg tggcggtagg gtatgtgtct 1620
gaaaatgagc gtggagattg ggctcgcacg gctgacgcag atggaagact taaggcagcg 1680
gcagaagaag atgcaggcag ctgagttgtt gtattctgat aagagtcaga ggtaactccc 1740
gttgcggtgc tgttaacggt ggagggcagt gtagtctgag cagtactcgt tgctgccgcg 1800
cgcgccacca gacataatag ctgacagact aacagactgt tcctttccat gggtcttttc 1860
tgcagatggc ctcgcccttt gctttactga tggtcctggt ggtgctcagc tgcaagtcaa 1920

gctgctctct gggctgtgat ctccctgaga cccacagcct ggataacagg aggaccttga 1980
tgctcctggc acaaatgagc agaatctctc cttcctcctg tctgatggac agacatgact 2040
ttggatttcc ccaggaggag tttgatggca accagttcca gaaggctcca gccatctctg 2100
tcctccatga gctgatccag cagatcttca acctctttac cacaaagcat tcatctgctg 2160
cttgggatga ggacctccta gacaaattct gcaccgaact ctaccagcag ctgaatgact 2220
tggaagcctg tgtgatgcag gaggagaggg tgggagaaac tcccctgatg aatgcggact 2280
ccatcttggc tgtgaagaaa tacttccgaa gaatcactct ctatctgaca gagaagaaat 2340
acagcccttg tgcctgggag gttgtcagag cagaaatcat gagatccctc tctttatcaa 2400
caaacttgca agaaagatta aggaggaagg aataaggatc cagatctgct gtgccttcta 2460
gttgccagcc atctgttgtt tgcccctccc ccgtgccttc cttgaccctg gaaggtgcca 2520
ctcccactgt cctttcctaa taaaatgagg aaattgcatc gcattgtctg agtaggtgtc 2580
attctattct ggggggtggg gtggggcagc acagcaaggg ggaggattgg gaagacaata 2640
gcaggcatgc tggggatgcg gtgggctcta tgggtaccca ggtgctgaag aattgacccg 2700
gttcctcctg ggccagaaag aagcaggcac atccccttct ctgtgacaca ccctgtccac 2760
gcccctggtt cttagttcca gccccactca taggacactc atagctcagg agggctccgc 2820
cttcaatccc acccgctaaa gtacttggag cggtctctcc ctccctcatc agcccaccaa 2880
accaaaccta gcctccaaga gtgggaagaa attaaagcaa gataggctat taagtgcaga 2940
gggagagaaa atgcctccaa catgtgagga agtaatgaga gaaatcatag aatttcttcc 3000
gcttcctcgc tcactgactc gctgcgctcg gtcgttcggc tgcggcgagc ggtatcagct 3060
cactcaaagg cggtaatacg gttatccaca gaatcagggg ataacgcagg aaagaacatg 3120
tgagcaaaag gccagcaaaa ggccaggaac cgtaaaaagg ccgcgttgct ggcgtttttc 3180
cataggctcc gcccccctga cgagcatcac aaaaatcgac gctcaagtca gaggtggcga 3240
aacccgacag gactataaag ataccaggcg tttccccctg gaagctccct cgtgcgctct 3300
cctgttccga ccctgccgct taccggatac ctgtccgcct ttctcccttc gggaagcgtg 3360
gcgctttctc aatgctcacg ctgtaggtat ctcagttcgg tgtaggtcgt tcgctccaag 3420
ctgggctgtg tgcacgaacc ccccgttcag cccgaccgct gcgccttatc cggtaactat 3480

cgtcttgagt ccaacccggt aagacacgac ttatcgccac tggcagcagc cactggtaac 3540

aggattagca gagcgaggta tgtaggcggt gctacagagt tcttgaagtg gtggcctaac 3600

tacggctaca ctagaaggac agtatttggt atctgcgctc tgctgaagcc agttaccttc 3660

ggaaaaagag ttggtagctc ttgatccggc aaacaaacca ccgctggtag cggtggtttt 3720

tttgtttgca agcagcagat tacgcgcaga aaaaaaggat ctcaagaaga tcctttgatc 3780

ttttctacgg ggtctgacgc tcagtggaac gaaaactcac gttaagggat tttggtcatg 3840

agattatcaa aaaggatctt cacctagatc cttttaaatt aaaaatgaag ttttaaatca 3900

atctaaagta tatatgagta aacttggtct gacagttacc aatgcttaat cagtgaggca 3960

cctatctcag cgatctgtct atttcgttca tccatagttg cctgactccg gggggggggg 4020

gcgctgaggt ctgcctcgtg aagaaggtgt tgctgactca taccaggcct gaatcgcccc 4080

atcatccagc cagaaagtga gggagccacg gttgatgaga gctttgttgt aggtggacca 4140

gttggtgatt ttgaactttt gctttgccac ggaacggtct gcgttgtcgg gaagatgcgt 4200

gatctgatcc ttcaactcag caaaagttcg atttattcaa caaagccgcc gtcccgtcaa 4260

gtcagcgtaa tgctctgcca gtgttacaac caattaacca attctgatta gaaaaactca 4320

tcgagcatca aatgaaactg caatttattc atatcaggat tatcaatacc atatttttga 4380

aaaagccgtt tctgtaatga aggagaaaac tcaccgaggc agttccatag gatggcaaga 4440

tcctggtatc ggtctgcgat tccgactcgt ccaacatcaa tacaacctat taatttcccc 4500

tcgtcaaaaa taaggttatc aagtgagaaa tcaccatgag tgacgactga atccggtgag 4560

aatggcaaaa gcttatgcat ttctttccag acttgttcaa caggccagcc attacgctcg 4620

tcatcaaaat cactcgcatc aaccaaaccg ttattcattc gtgattgcgc ctgagcgaga 4680

cgaaatacgc gatcgctgtt aaaaggacaa ttacaaacag gaatcgaatg caaccggcgc 4740

aggaacactg ccagcgcatc aacaatattt tcacctgaat caggatattc ttctaatacc 4800

tggaatgctg ttttcccggg gatcgcagtg gtgagtaacc atgcatcatc aggagtacgg 4860

ataaaatgct tgatggtcgg aagaggcata aattccgtca gccagtttag tctgaccatc 4920

tcatctgtaa catcattggc aacgctacct ttgccatgtt tcagaaacaa ctctggcgca 4980

tcgggcttcc catacaatcg atagattgtc gcacctgatt gcccgacatt atcgcgagcc 5040

catttatacc catataaatc agcatccatg ttggaattta atcgcggcct cgagcaagac 5100

gtttcccgtt gaatatggct cataacaccc cttgtattac tgtttatgta agcagacagt 5160

tttattgttc atgatgatat atttttatct tgtgcaatgt aacatcagag attttgagac 5220

acaacgtggc tttccccccc cccccattat tgaagcattt atcagggtta ttgtctcatg 5280

agcggataca tatttgaatg tatttagaaa aataaacaaa taggggttcc gcgcacattt 5340

ccccgaaaag tgccacctga cgtctaagaa accattatta tcatgacatt aacctataaa 5400

aataggcgta tcacgaggcc ctttcgtc 5428

<210> 2
<211> 5259
<212> DMA
<213> Homo sapiens

<400> 2

tcgcgcgttt cggtgatgac ggtgaaaacc tctgacacat gcagctcccg gagacggtca 60

cagcttgtct gtaagcggat gccgggagca gacaagcccg tcagggcgcg tcagcgggtg 120

ttggcgggtg tcggggctgg cttaactatg cggcatcaga gcagattgta ctgagagtgc 180

accatatgcg gtgtgaaata ccgcacagat gcgtaaggag aaaataccgc atcagattgg 240

ctattggcca ttgcatacgt tgtatccata tcataatatg tacatttata ttggctcatg 300

tccaacatta ccgccatgtt gacattgatt attgactagt tattaatagt aatcaattac 360

ggggtcatta gttcatagcc catatatgga gttccgcgtt acataactta cggtaaatgg 420

cccgcctggc tgaccgccca acgacccccg cccattgacg tcaataatga cgtatgttcc 480

catagtaacg ccaataggga ctttccattg acgtcaatgg gtggagtatt tacggtaaac 540

tgcccacttg gcagtacatc aagtgtatca tatgccaagt acgcccccta ttgacgtcaa 600

tgacggtaaa tggcccgcct ggcattatgc ccagtacatg accttatggg actttcctac 660

ttggcagtac atctacgtat tagtcatcgc tattaccatg gtgatgcggt tttggcagta 720

catcaatggg cgtggatagc ggtttgactc acggggattt ccaagtctcc accccattga 780

cgtcaatggg agtttgtttt ggcaccaaaa tcaacgggac tttccaaaat gtcgtaacaa 840

ctccgcccca ttgacgcaaa tgggcggtag gcgtgtacgg tgggaggtct atataagcag 900

agctcgttta gtgaaccgtc agatcgcctg gagacgccat ccacgctgtt ttgacctcca 960

tagaagacac cgggaccgat ccagcctccg cggccgggaa cggtgcattg gaacgcggat 1020

tccccgtgcc aagagtgacg taagtaccgc ctatagactc tataggcaca cccctttggc 1080

tcttatgcat gctatactgt ttttggcttg gggcctatac acccccgctt ccttatgcta 1140

taggtgatgg tatagcttag cctataggtg tgggttattg accattattg accactcccc 1200

tattggtgac gatactttcc attactaatc cataacatgg ctctttgcca caactatctc 1260

tattggctat atgccaatac tctgtccttc agagactgac acggactctg tatttttaca 1320

ggatggggtc ccatttatta tttacaaatt cacatataca acaacgccgt cccccgtgcc 1380

cgcagttttt attaaacata gcgtgggatc tccacgcgaa tctcgggtac gtgttccgga 1440

catgggctct tctccggtag cggcggagct tccacatccg agccctggtc ccatgcctcc 1500

agcggctcat ggtcgctcgg cagctccttg ctcctaacag tggaggccag acttaggcac 1560

agcacaatgc ccaccaccac cagtgtgccg cacaaggccg tggcggtagg gtatgtgtct 1620

gaaaatgagc gtggagattg ggctcgcacg gctgacgcag atggaagact taaggcagcg 1680

gcagaagaag atgcaggcag ctgagttgtt gtattctgat aagagtcaga ggtaactccc 1740

gttgcggtgc tgttaacggt ggagggcagt gtagtctgag cagtactcgt tgctgccgcg 1800

cgcgccacca gacataatag ctgacagact aacagactgt tcctttccat gggtcttttc 1860

tgcagatggc ctcgcccttt gctttactga tggtcctggt ggtgctcagc tgcaagtcaa 1920

gctgctctct gggctgtgat ctccctgaga cccacagcct ggataacagg aggaccttga 1980

tgctcctggc acaaatgagc agaatctctc cttcctcctg tctgatggac agacatgact 2040

ttggatttcc ccaggaggag tttgatggca accagttcca gaaggctcca gccatctctg 2100

tcctccatga gctgatccag cagatcttca acctctttac cacaaaagat tcatctgctg 2160

cttgggatga ggacctccta gacaaattct gcaccgaact ctaccagcag ctgaatgact 2220

tggaagcctg tgtgatgcag gaggagaggg tgggagaaac tcccctgatg aatgcggact 2280

ccatcttggc tgtgaagaaa tacttccgaa gaatcactct ctatctgaca gagaagaaat 2340

acagcccttg tgcctgggag gttgtcagag cagaaatcat gagatccctc tctttatcaa 2400

caaacttgca agaaagatta aggaggaagg aataaggatc cagatctact tctggctaat 2460

aaaagatcag agctctagag atctgtgtgt tggttttttg tgtggtaccc aggtgctgaa 2520

gaattgaccc ggttcctcct gggccagaaa gaagcaggca catccccttc tctgtgacac 2580

accctgtcca cgcccctggt tcttagttcc agccccactc ataggacact catagctcag 2640

gagggctccg ccttcaatcc cacccgctaa agtacttgga gcggtctctc cctccctcat 2700

cagcccacca aaccaaacct agcctccaag agtgggaaga aattaaagca agataggcta 2760

ttaagtgcag agggagagaa aatgcctcca acatgtgagg aagtaatgag agaaatcata 2820

gaatttcttc cgcttcctcg ctcactgact cgctgcgctc ggtcgttcgg ctgcggcgag 2880

cggtatcagc tcactcaaag gcggtaatac ggttatccac agaatcaggg gataacgcag 2940

gaaagaacat gtgagcaaaa ggccagcaaa aggccaggaa ccgtaaaaag gccgcgttgc 3000

tggcgttttt ccataggctc cgcccccctg acgagcatca caaaaatcga cgctcaagtc 3060

agaggtggcg aaacccgaca ggactataaa gataccaggc gtttccccct ggaagctccc 3120

tcgtgcgctc tcctgttccg accctgccgc ttaccggata cctgtccgcc tttctccctt 3180

cgggaagcgt ggcgctttct caatgctcac gctgtaggta tctcagttcg gtgtaggtcg 3240

ttcgctccaa gctgggctgt gtgcacgaac cccccgttca gcccgaccgc tgcgccttat 3300

ccggtaacta tcgtcttgag tccaacccgg taagacacga cttatcgcca ctggcagcag 3360

ccactggtaa caggattagc agagcgaggt atgtaggcgg tgctacagag ttcttgaagt 3420

ggtggcctaa ctacggctac actagaagga cagtatttgg tatctgcgct ctgctgaagc 3480

cagttacctt cggaaaaaga gttggtagct cttgatccgg caaacaaacc accgctggta 3540

gcggtggttt ttttgtttgc aagcagcaga ttacgcgcag aaaaaaagga tctcaagaag 3600

atcctttgat cttttctacg gggtctgacg ctcagtggaa cgaaaactca cgttaaggga 3660

ttttggtcat gagattatca aaaaggatct tcacctagat ccttttaaat taaaaatgaa 3720

gttttaaatc aatctaaagt atatatgagt aaacttggtc tgacagttac caatgcttaa 3780

tcagtgaggc acctatctca gcgatctgtc tatttcgttc atccatagtt gcctgactcc 3840

gggggggggg ggcgctgagg tctgcctcgt gaagaaggtg ttgctgactc ataccaggcc 3900

tgaatcgccc catcatccag ccagaaagtg agggagccac ggttgatgag agctttgttg 3960

taggtggacc agttggtgat tttgaacttt tgctttgcca cggaacggtc tgcgttgtcg 4020

ggaagatgcg tgatctgatc cttcaactca gcaaaagttc gatttattca acaaagccgc 4080

cgtcccgtca agtcagcgta atgctctgcc agtgttacaa ccaattaacc aattctgatt 4140

agaaaaactc atcgagcatc aaatgaaact gcaatttatt catatcagga ttatcaatac 4200

catatttttg aaaaagccgt ttctgtaatg aaggagaaaa ctcaccgagg cagttccata 4260

ggatggcaag atcctggtat cggtctgcga ttccgactcg tccaacatca atacaaccta 4320

ttaatttccc ctcgtcaaaa ataaggttat caagtgagaa atcaccatga gtgacgactg 4380

aatccggtga gaatggcaaa agcttatgca tttctttcca gacttgttca acaggccagc 4440

cattacgctc gtcatcaaaa tcactcgcat caaccaaacc gttattcatt cgtgattgcg 4500

cctgagcgag acgaaatacg cgatcgctgt taaaaggaca attacaaaca ggaatcgaat 4560

gcaaccggcg caggaacact gccagcgcat caacaatatt ttcacctgaa tcaggatatt 4620

cttctaatac ctggaatgct gttttcccgg ggatcgcagt ggtgagtaac catgcatcat 4680

caggagtacg gataaaatgc ttgatggtcg gaagaggcat aaattccgtc agccagttta 4740

gtctgaccat ctcatctgta acatcattgg caacgctacc tttgccatgt ttcagaaaca 4800

actctggcgc atcgggcttc ccatacaatc gatagattgt cgcacctgat tgcccgacat 4860

tatcgcgagc ccatttatac ccatataaat cagcatccat gttggaattt aatcgcggcc 4920

tcgagcaaga cgtttcccgt tgaatatggc tcataacacc ccttgtatta ctgtttatgt 4980

aagcagacag ttttattgtt catgatgata tatttttatc ttgtgcaatg taacatcaga 5040

gattttgaga cacaacgtgg ctttcccccc ccccccatta ttgaagcatt tatcagggtt 5100

attgtctcat gagcggatac atatttgaat gtatttagaa aaataaacaa ataggggttc 5160

cgcgcacatt tccccgaaaa gtgccacctg acgtctaaga aaccattatt atcatgacat 5220

taacctataa aaataggcgt atcacgaggc cctttcgtc 5259

<210> 3
<211> 5480
<212> DNA
<213> Homo sapiens

<400> 3

tcgcgcgttt cggtgatgac ggtgaaaacc tctgacacat gcagctcccg gagacggtca 60

cagcttgtct gtaagcggat gccgggagca gacaagcccg tcagggcgcg tcagcgggtg 120

ttggcgggtg tcggggctgg cttaactatg cggcatcaga gcagattgta ctgagagtgc 180
accatatgcg gtgtgaaata ccgcacagat gcgtaaggag aaaataccgc atcagattgg 240
ctattggcca ttgcatacgt tgtatccata tcataatatg tacatttata ttggctcatg 300
tccaacatta ccgccatgtt gacattgatt attgactagt tattaatagt aatcaattac 360
ggggtcatta gttcatagcc catatatgga gttccgcgtt acataactta cggtaaatgg 420
cccgcctggc tgaccgccca acgacccccg cccattgacg tcaataatga cgtatgttcc 480
catagtaacg ccaataggga ctttccattg acgtcaatgg gtggagtatt tacggtaaac 540
tgcccacttg gcagtacatc aagtgtatca tatgccaagt acgcccccta ttgacgtcaa 600
tgacggtaaa tggcccgcct ggcattatgc ccagtacatg accttatggg actttcctac 660
ttggcagtac atctacgtat tagtcatcgc tattaccatg gtgatgcggt tttggcagta 720
catcaatggg cgtggatagc ggtttgactc acggggattt ccaagtctcc accccattga 780
cgtcaatggg agtttgtttt ggcaccaaaa tcaacgggac tttccaaaat gtcgtaacaa 840
ctccgcccca ttgacgcaaa tgggcggtag gcgtgtacgg tgggaggtct atataagcag 900
agctcgttta gtgaaccgtc agatcgcctg gagacgccat ccacgctgtt ttgacctcca 960
tagaagacac cgggaccgat ccagcctccg cggccgggaa cggtgcattg gaacgcggat 1020
tccccgtgcc aagagtgacg taagtaccgc ctatagactc tataggcaca cccctttggc 1080
tcttatgcat gctatactgt ttttggcttg gggcctatac acccccgctt ccttatgcta 1140
taggtgatgg tatagcttag cctataggtg tgggttattg accattattg accactcccc 1200
tattggtgac gatactttcc attactaatc cataacatgg ctctttgcca caactatctc 1260
tattggctat atgccaatac tctgtccttc agagactgac acggactctg tatttttaca 1320
ggatggggtc ccatttatta tttacaaatt cacatataca acaacgccgt cccccgtgcc 1380
cgcagttttt attaaacata gcgtgggatc tccacgcgaa tctcgggtac gtgttccgga 1440
catgggctct tctccggtag cggcggagct tccacatccg agccctggtc ccatgcctcc 1500
agcggctcat ggtcgctcgg cagctccttg ctcctaacag tggaggccag acttaggcac 1560
agcacaatgc ccaccaccac cagtgtgccg cacaaggccg tggcggtagg gtatgtgtct 1620
gaaaatgagc gtggagattg ggctcgcacg gctgacgcag atggaagact taaggcagcg 1680

gcagaagaag atgcaggcag ctgagttgtt gtattctgat aagagtcaga ggtaactccc 1740

gttgcggtgc tgttaacggt ggagggcagt gtagtctgag cagtactcgt tgctgccgcg 1800

cgcgccacca gacataatag ctgacagact aacagactgt tcctttccat gggtcttttc 1860

tgcagtcacc gtcgtcgaca cgtgtgatca gatatcacca tggccctcct gttccctcta 1920

ctggcagccc tagtgatgac cagctatagc cctgttggat ctctgggctg tgatctgcct 1980

cagaaccatg gcctacttag caggaacacc ttggtgcttc tgcaccaaat gaggagaatc 2040

tccccttct tgtgtctcaa ggacagaaga gacttcaggt tcccccagga gatggtaaaa 2100

gggagccagt tgcagaaggc ccatgtcatg tctgtcctcc atgagatgct gcagcagatc 2160

ttcagcctct tccacacaga gcgctcctct gctgcctgga acatgaccct cctagaccaa 2220

ctccacactg gacttcatca gcaactgcaa cacctggaga cctgcttgct gcaggtagtg 2280

ggagaaggag aatctgctgg ggcaattagc agccctgcac tgaccttgag gaggtacttc 2340

cagggaatcc gtgtctacct gaaagagaag aaatacagcg actgtgcctg ggaagttgtc 2400

agaatggaaa tcatgaaatc cttgttctta tcaacaaaca tgcaagaaag actgagaagt 2460

aaagatagag acctgggctc atcttgagga tccagatctg ctgtgccttc tagttgccag 2520

ccatctgttg tttgcccctc ccccgtgcct tccttgaccc tggaaggtgc cactcccact 2580

gtcctttcct aataaaatga ggaaattgca tcgcattgtc tgagtaggtg tcattctatt 2640

ctggggggtg gggtggggca gcacagcaag ggggaggatt gggaagacaa tagcaggcat 2700

gctggggatg cggtgggctc tatgggtacc caggtgctga agaattgacc cggttcctcc 2760

tgggccagaa agaagcaggc acatcccctt ctctgtgaca caccctgtcc acgcccctgg 2820

ttcttagttc cagccccact cataggacac tcatagctca ggagggctcc gccttcaatc 2880

ccacccgcta aagtacttgg agcggtctct ccctccctca tcagcccacc aaaccaaacc 2940

tagcctccaa gagtgggaag aaattaaagc aagataggct attaagtgca gagggagaga 3000

aaatgcctcc aacatgtgag gaagtaatga gagaaatcat agaatttctt ccgcttcctc 3060

gctcactgac tcgctgcgct cggtcgttcg gctgcggcga gcggtatcag ctcactcaaa 3120

ggcggtaata cggttatcca cagaatcagg ggataacgca ggaaagaaca tgtgagcaaa 3180

aggccagcaa aaggccagga accgtaaaaa ggccgcgttg ctggcgtttt tccataggct 3240

ccgcccccct gacgagcatc acaaaaatcg acgctcaagt cagaggtggc gaaacccgac 3300

aggactataa agataccagg cgtttccccc tggaagctcc ctcgtgcgct ctcctgttcc 3360

gaccctgccg cttaccggat acctgtccgc ctttctccct tcgggaagcg tggcgctttc 3420

tcaatgctca cgctgtaggt atctcagttc ggtgtaggtc gttcgctcca agctgggctg 3480

tgtgcacgaa ccccccgttc agcccgaccg ctgcgcctta tccggtaact atcgtcttga 3540

gtccaacccg gtaagacacg acttatcgcc actggcagca gccactggta acaggattag 3600

cagagcgagg tatgtaggcg gtgctacaga gttcttgaag tggtggccta actacggcta 3660

cactagaagg acagtatttg gtatctgcgc tctgctgaag ccagttacct tcggaaaaag 3720

agttggtagc tcttgatccg gcaaacaaac caccgctggt agcggtggtt tttttgtttg 3780

caagcagcag attacgcgca gaaaaaaagg atctcaagaa gatcctttga tcttttctac 3840

ggggtctgac gctcagtgga acgaaaactc acgttaaggg attttggtca tgagattatc 3900

aaaaaggatc ttcacctaga tccttttaaa ttaaaaatga agttttaaat caatctaaag 3960

tatatatgag taaacttggt ctgacagtta ccaatgctta atcagtgagg cacctatctc 4020

agcgatctgt ctatttcgtt catccatagt tgcctgactc cggggggggg gggcgctgag 4080

gtctgcctcg tgaagaaggt gttgctgact cataccaggc ctgaatcgcc ccatcatcca 4140

gccagaaagt gagggagcca cggttgatga gagctttgtt gtaggtggac cagttggtga 4200

ttttgaactt ttgctttgcc acggaacggt ctgcgttgtc gggaagatgc gtgatctgat 4260

ccttcaactc agcaaaagtt cgatttattc aacaaagccg ccgtcccgtc aagtcagcgt 4320

aatgctctgc cagtgttaca accaattaac caattctgat tagaaaaact catcgagcat 4380

caaatgaaac tgcaatttat tcatatcagg attatcaata ccatattttt gaaaaagccg 4440

tttctgtaat gaaggagaaa actcaccgag gcagttccat aggatggcaa gatcctggta 4500

tcggtctgcg attccgactc gtccaacatc aatacaacct attaatttcc cctcgtcaaa 4560

aataaggtta tcaagtgaga aatcaccatg agtgacgact gaatccggtg agaatggcaa 4620

aagcttatgc atttctttcc agacttgttc aacaggccag ccattacgct cgtcatcaaa 4680

atcactcgca tcaaccaaac cgttattcat tcgtgattgc gcctgagcga gacgaaatac 4740

gcgatcgctg ttaaaaggac aattacaaac aggaatcgaa tgcaaccggc gcaggaacac 4800

tgccagcgca tcaacaatat tttcacctga atcaggatat tcttctaata cctggaatgc 4860

tgttttcccg gggatcgcag tggtgagtaa ccatgcatca tcaggagtac ggataaaatg 4920

cttgatggtc ggaagaggca taaattccgt cagccagttt agtctgacca tctcatctgt 4980

aacatcattg gcaacgctac ctttgccatg tttcagaaac aactctggcg catcgggctt 5040

cccatacaat cgatagattg tcgcacctga ttgcccgaca ttatcgcgag cccatttata 5100

cccatataaa tcagcatcca tgttggaatt taatcgcggc ctcgagcaag acgtttcccg 5160

ttgaatatgg ctcataacac cccttgtatt actgtttatg taagcagaca gttttattgt 5220

tcatgatgat atatttttat cttgtgcaat gtaacatcag agattttgag acacaacgtg 5280

gctttccccc cccccccatt attgaagcat ttatcagggt tattgtctca tgagcggata 5340

catatttgaa tgtatttaga aaaataaaca aataggggtt ccgcgcacat ttccccgaaa 5400

agtgccacct gacgtctaag aaaccattat tatcatgaca ttaacctata aaaataggcg 5460

tatcacgagg ccctttcgtc 5480

<210> 4
<211> 5322
<212> DNA
<213> Homo sapiens

<400> 4

tcgcgcgttt cggtgatgac ggtgaaaacc tctgacacat gcagctcccg gagacggtca 60

cagcttgtct gtaagcggat gccgggagca gacaagcccg tcagggcgcg tcagcgggtg 120

ttggcgggtg tcggggctgg cttaactatg cggcatcaga gcagattgta ctgagagtgc 180

accatatgcg gtgtgaaata ccgcacagat gcgtaaggag aaaataccgc atcagattgg 240

ctattggcca ttgcatacgt tgtatccata tcataatatg tacatttata ttggctcatg 300

tccaacatta ccgccatgtt gacattgatt attgactagt tattaatagt aatcaattac 360

ggggtcatta gttcatagcc catatatgga gttccgcgtt acataactta cggtaaatgg 420

cccgcctggc tgaccgccca acgacccccg cccattgacg tcaataatga cgtatgttcc 480

catagtaacg ccaataggga ctttccattg acgtcaatgg gtggagtatt tacggtaaac 540

tgcccacttg gcagtacatc aagtgtatca tatgccaagt acgcccccta ttgacgtcaa 600

tgacggtaaa tggcccgcct ggcattatgc ccagtacatg accttatggg actttcctac 660

ttggcagtac atctacgtat tagtcatcgc tattaccatg gtgatgcggt tttggcagta 720

catcaatggg cgtggatagc ggtttgactc acggggattt ccaagtctcc accccattga 780

cgtcaatggg agtttgtttt ggcaccaaaa tcaacgggac tttccaaaat gtcgtaacaa 840

ctccgcccca ttgacgcaaa tgggcggtag gcgtgtacgg tgggaggtct atataagcag 900

agctcgttta gtgaaccgtc agatcgcctg gagacgccat ccacgctgtt ttgacctcca 960

tagaagacac cgggaccgat ccagcctccg cggccgggaa cggtgcattg gaacgcggat 1020

tccccgtgcc aagagtgacg taagtaccgc ctatagactc tataggcaca cccctttggc 1080

tcttatgcat gctatactgt ttttggcttg gggcctatac acccccgctt ccttatgcta 1140

taggtgatgg tatagcttag cctataggtg tgggttattg accattattg accactcccc 1200

tattggtgac gatactttcc attactaatc cataacatgg ctctttgcca caactatctc 1260

tattggctat atgccaatac tctgtccttc agagactgac acggactctg tatttttaca 1320

ggatggggtc ccatttatta tttacaaatt cacatataca acaacgccgt cccccgtgcc 1380

cgcagttttt attaaacata gcgtgggatc tccacgcgaa tctcgggtac gtgttccgga 1440

catgggctct tctccggtag cggcggagct tccacatccg agccctggtc ccatgcctcc 1500

agcggctcat ggtcgctcgg cagctccttg ctcctaacag tggaggccag acttaggcac 1560

agcacaatgc ccaccaccac cagtgtgccg cacaaggccg tggcggtagg gtatgtgtct 1620

gaaaatgagc gtggagattg ggctcgcacg gctgacgcag atggaagact taaggcagcg 1680

gcagaagaag atgcaggcag ctgagttgtt gtattctgat aagagtcaga ggtaactccc 1740

gttgcggtgc tgttaacggt ggagggcagt gtagtctgag cagtactcgt tgctgccgcg 1800

cgcgccacca gacataatag ctgacagact aacagactgt tcctttccat gggtcttttc 1860

tgcagtcacc gtcgtcgaca cgtgtgatca gatatcgcgg ccgctctaga atggccctcc 1920

tgttccctct actggcagcc ctagtgatga ccagctatag ccctgttgga tctctgggct 1980

gtgatctgcc tcagaaccat ggcctactta gcaggaacac cttggtgctt ctgcaccaaa 2040

tgaggagaat ctcccctttc ttgtgtctca aggacagaag agacttcagg ttcccccagg 2100

agatggtaaa agggagccag ttgcagaagg cccatgtcat gtctgtcctc catgagatgc 2160

tgcagcagat cttcagcctc ttccacacag agcgctcctc tgctgcctgg aacatgaccc 2220

tcctagacca actccacact ggacttcatc agcaactgca acacctggag acctgcttgc 2280

tgcaggtagt gggagaagga gaatctgctg gggcaattag cagccctgca ctgaccttga 2340

ggaggtactt ccagggaatc cgtgtctacc tgaaagagaa gaaatacagc gactgtgcct 2400

gggaagttgt cagaatggaa atcatgaaat ccttgttctt atcaacaaac atgcaagaaa 2460

gactgagaag taaagataga gacctgggct catcttgagg atccagatct acttctggct 2520

aataaaagat cagagctcta gagatctgtg tgttggtttt ttgtgtggta cccaggtgct 2580

gaagaattga cccggttcct cctgggccag aaagaagcag gcacatcccc ttctctgtga 2640

cacaccctgt ccacgcccct ggttcttagt tccagcccca ctcataggac actcatagct 2700

caggagggct ccgccttcaa tcccacccgc taaagtactt ggagcggtct ctccctccct 2760

catcagccca ccaaaccaaa cctagcctcc aagagtggga agaaattaaa gcaagatagg 2820

ctattaagtg cagagggaga gaaaatgcct ccaacatgtg aggaagtaat gagagaaatc 2880

atagaatttc ttccgcttcc tcgctcactg actcgctgcg ctcggtcgtt cggctgcggc 2940

gagcggtatc agctcactca aaggcggtaa tacggttatc cacagaatca ggggataacg 3000

caggaaagaa catgtgagca aaaggccagc aaaaggccag gaaccgtaaa aaggccgcgt 3060

tgctggcgtt tttccatagg ctccgccccc ctgacgagca tcacaaaaat cgacgctcaa 3120

gtcagaggtg gcgaaacccg acaggactat aaagatacca ggcgtttccc cctggaagct 3180

ccctcgtgcg ctctcctgtt ccgaccctgc cgcttaccgg atacctgtcc gcctttctcc 3240

cttcgggaag cgtggcgctt tctcaatgct cacgctgtag gtatctcagt tcggtgtagg 3300

tcgttcgctc caagctgggc tgtgtgcacg aaccccccgt tcagcccgac cgctgcgcct 3360

tatccggtaa ctatcgtctt gagtccaacc cggtaagaca cgacttatcg ccactggcag 3420

cagccactgg taacaggatt agcagagcga ggtatgtagg cggtgctaca gagttcttga 3480

agtggtggcc taactacggc tacactagaa ggacagtatt tggtatctgc gctctgctga 3540

agccagttac cttcggaaaa agagttggta gctcttgatc cggcaaacaa accaccgctg 3600

gtagcggtgg tttttttgtt tgcaagcagc agattacgcg cagaaaaaaa ggatctcaag 3660

aagatccttt gatcttttct acggggtctg acgctcagtg gaacgaaaac tcacgttaag 3720

ggatcttggt catgagatta tcaaaaagga tcttcaccta gatcctttta aattaaaaat 3780

gaagttttaa atcaatctaa agtatatatg agtaaacttg gtctgacagt taccaatgct 3840

taatcagtga ggcacctatc tcagcgatct gtctatttcg ttcatccata gttgcctgac 3900

tccggggggg gggggcgctg aggtctgcct cgtgaagaag gtgttgctga ctcataccag 3960

gcctgaatcg ccccatcatc cagccagaaa gtgagggagc cacggttgat gagagctttg 4020

ttgtaggtgg accagttggt gattttgaac ttttgctttg ccacggaacg gtctgcgttg 4080

tcgggaagat gcgtgatctg atccttcaac tcagcaaaag ttcgatttat tcaacaaagc 4140

cgccgtcccg tcaagtcagc gtaatgctct gccagtgtta caaccaatta accaattctg 4200

attagaaaaa ctcatcgagc atcaaatgaa actgcaattt attcatatca ggattatcaa 4260

taccatattt ttgaaaaagc cgtttctgta atgaaggaga aaactcaccg aggcagttcc 4320

ataggatggc aagatcctgg tatcggtctg cgattccgac tcgtccaaca tcaatacaac 4380

ctattaattt cccctcgtca aaaataaggt tatcaagtga gaaatcacca tgagtgacga 4440

ctgaatccgg tgagaatggc aaaagcttat gcatttcttt ccagacttgt tcaacaggcc 4500

agccattacg ctcgtcatca aaatcactcg catcaaccaa accgttattc attcgtgatt 4560

gcgcctgagc gagacgaaat acgcgatcgc tgttaaaagg acaattacaa acaggaatcg 4620

aatgcaaccg gcgcaggaac actgccagcg catcaacaat attttcacct gaatcaggat 4680

attcttctaa tacctggaat gctgttttcc cggggatcgc agtggtgagt aaccatgcat 4740

catcaggagt acggataaaa tgcttgatgg tcggaagagg cataaattcc gtcagccagt 4800

ttagtctgac catctcatct gtaacatcat tggcaacgct acctttgcca tgtttcagaa 4860

acaactctgg cgcatcgggc ttcccataca atcgatagat tgtcgcacct gattgcccga 4920

cattatcgcg agcccattta tacccatata aatcagcatc catgttggaa tttaatcgcg 4980

gcctcgagca agacgtttcc cgttgaatat ggctcataac accccttgta ttactgttta 5040

tgtaagcaga cagttttatt gttcatgatg atatattttt atcttgtgca atgtaacatc 5100

agagattttg agacacaacg tggctttccc ccccccccca ttattgaagc atttatcagg 5160

gttattgtct catgagcgga tacatatttg aatgtattta gaaaaataaa caaatagggg 5220

ttccgcgcac atttccccga aaagtgccac ctgacgtcta agaaaccatt attatcatga 5280

cattaaccta taaaaatagg cgtatcacga ggccctttcg tc 5322

<210> 5
<211> 5422
<212> DNA
<213> Mus musculus

<400> 5

tcgcgcgttt cggtgatgac ggtgaaaacc tctgacacat gcagctcccg gagacggtca 60
cagcttgtct gtaagcggat gccgggagca gacaagcccg tcagggcgcg tcagcgggtg 120
ttggcgggtg tcggggctgg cttaactatg cggcatcaga gcagattgta ctgagagtgc 180
accatatgcg gtgtgaaata ccgcacagat gcgtaaggag aaaataccgc atcagattgg 240
ctattggcca ttgcatacgt tgtatccata tcataatatg tacatttata ttggctcatg 300
tccaacatta ccgccatgtt gacattgatt attgactagt tattaatagt aatcaattac 360
ggggtcatta gttcatagcc catatatgga gttccgcgtt acataactta cggtaaatgg 420
cccgcctggc tgaccgccca acgacccccg cccattgacg tcaataatga cgtatgttcc 480
catagtaacg ccaataggga ctttccattg acgtcaatgg gtggagtatt tacggtaaac 540
tgcccacttg gcagtacatc aagtgtatca tatgccaagt acgcccccta ttgacgtcaa 600
tgacggtaaa tggcccgcct ggcattatgc ccagtacatg accttatggg actttcctac 660
ttggcagtac atctacgtat tagtcatcgc tattaccatg gtgatgcggt tttggcagta 720
catcaatggg cgtggatagc ggtttgactc acggggattt ccaagtctcc accccattga 780
cgtcaatggg agtttgtttt ggcaccaaaa tcaacgggac tttccaaaat gtcgtaacaa 840
ctccgcccca ttgacgcaaa tgggcggtag gcgtgtacgg tgggaggtct atataagcag 900
agctcgttta gtgaaccgtc agatcgcctg gagacgccat ccacgctgtt ttgacctcca 960
tagaagacac cgggaccgat ccagcctccg cggccgggaa cggtgcattg gaacgcggat 1020
tccccgtgcc aagagtgacg taagtaccgc ctatagactc tataggcaca cccctttggc 1080
tcttatgcat gctatactgt ttttggcttg gggcctatac acccccgctt ccttatgcta 1140
taggtgatgg tatagcttag cctataggtg tgggttattg accattattg accactcccc 1200
tattggtgac gatactttcc attactaatc cataacatgg ctctttgcca caactatctc 1260
tattggctat atgccaatac tctgtccttc agagactgac acggactctg tatttttaca 1320

ggatggggtc ccatttatta tttacaaatt cacatataca acaacgccgt cccccgtgcc 1380

cgcagttttt attaaacata gcgtgggatc tccacgcgaa tctcgggtac gtgttccgga 1440

catgggctct tctccggtag cggcggagct tccacatccg agccctggtc ccatgcctcc 1500

agcggctcat ggtcgctcgg cagctccttg ctcctaacag tggaggccag acttaggcac 1560

agcacaatgc ccaccaccac cagtgtgccg cacaaggccg tggcggtagg gtatgtgtct 1620

gaaaatgagc gtggagattg ggctcgcacg gctgacgcag atggaagact taaggcagcg 1680

gcagaagaag atgcaggcag ctgagttgtt gtattctgat aagagtcaga ggtaactccc 1740

gttgcggtgc tgttaacggt ggagggcagt gtagtctgag cagtactcgt tgctgccgcg 1800

cgcgccacca gacataatag ctgacagact aacagactgt tcctttccat gggtcttttc 1860

tgcagatggc taggctctgt gctttcctga tggtcctggc ggtgatgagc tactggccaa 1920

cctgctctct aggatgtgac ctgcctcaga ctcataacct caggaacaag agagccttga 1980

cactcctggt acaaatgagg agactctccc ctctctcctg cctgaaggac aggaaggact 2040

ttggattccc gcaggagaag gtggatgccc agcagatcaa gaaggctcaa gccatccctg 2100

tcctgagtga gctgacccag cagatcctga acatcttcac atcaaagcac tcatctgctg 2160

cttggaatgc aaccctccta gactcattct gcaatgacct ccaccagcag ctcaatgacc 2220

tgcaaggttg tctgatgcag caggtggggg tgcaggaatt tcccctgacc caggaagatg 2280

ccctgctggc tgtgaggaaa tacttccaca ggatcactgt gtacctgaga gagaagaaac 2340

acagcccctg tgcctgggag gtggtcagag cagaagtctg gagagccctg tcttcctctg 2400

ccaatgtgct gggaagactg agagaagaga aatgaagatc tgctgtgcct tctagttgcc 2460

agccatctgt tgtttgcccc tcccccgtgc cttccttgac cctggaaggt gccactccca 2520

ctgtcctttc ctaataaaat gaggaaattg catcgcattg tctgagtagg tgtcattcta 2580

ttctgggggg tggggtgggg cagcacagca agggggagga ttgggaagac aatagcaggc 2640

atgctgggga tgcggtgggc tctatgggta cccaggtgct gaagaattga cccggttcct 2700

cctgggccag aaagaagcag gcacatcccc ttctctgtga cacaccctgt ccacgcccct 2760

ggttcttagt tccagcccca ctcataggac actcatagct caggagggct ccgccttcaa 2820

tcccacccgc taaagtactt ggagcggtct ctccctccct catcagccca ccaaaccaaa 2880

cctagcctcc aagagtggga agaaattaaa gcaagatagg ctattaacgg cagaggaga 2940

gaaaatgcct ccaacatgtg aggaagtaat gagagaaatc atagaattcc ttccgcttcc 3000

tcgctcactg actcgctgcg ctcggtcgtt cggctgcggc gagcggtatc agctcactca 3060

aaggcggtaa tacggttatc cacagaatca ggggataacg caggaaagaa catgtgagca 3120

aaaggccagc aaaaggccag gaaccgtaaa aaggccgcgt tgctggcgtt tttccatagg 3180

ctccgccccc ctgacgagca tcacaaaaat cgacgctcaa gtcagaggtg gcgaaacccg 3240

acaggactat aaagatacca ggcgtttccc cctggaagct ccctcgtgcg ctctcctgtt 3300

ccgaccctgc cgcttaccgg atacctgtcc gcctttctcc cttcgggaag cgtggcgctt 3360

tctcaatgct cacgctgtag gtatctcagt tcggtgtagg tcgttcgctc caagctgggc 3420

tgtgtgcacg aaccccccgt tcagcccgac cgctgcgcct tatccggtaa ctatcgtctt 3480

gagtccaacc cggtaagaca cgacttatcg ccactggcag cagccactgg taacaggatt 3540

agcagagcga ggtatgtagg cggtgctaca gagttcttga agtggtggcc taactacggc 3600

tacactagaa ggacagtatt tggtatctgc gctctgctga agccagttac cttcggaaaa 3660

agagttggta gctcttgatc cggcaaacaa accaccgctg gtagcggtgg tttttttgtt 3720

tgcaagcagc agattacgcg cagaaaaaaa ggatctcaag aagatccttt gatcttttct 3780

acggggtctg acgctcagtg gaacgaaaac tcacgttaag ggattttggt catgagatta 3840

tcaaaaagga tcttcaccta gatcctttta aattaaaaat gaagttttaa atcaatctaa 3900

agtatatatg agtaaacttg gtctgacagt taccaatgct taatcagtga ggcacctatc 3960

tcagcgatct gtctatttcg ttcatccata gttgcctgac tccggggggg gggggcgctg 4020

aggtctgcct cgtgaagaag gtgttgctga ctcataccag gcctgaatcg ccccatcatc 4080

cagccagaaa gtgagggagc cacggttgat gagagctttg ttgtaggtgg accagttggt 4140

gattttgaac ttttgctttg ccacggaacg gtctgcgttg tcgggaagat gcgtgatctg 4200

atccttcaac tcagcaaaag ttcgatttat tcaacaaagc cgccgtcccg tcaagtcagc 4260

gtaatgctct gccagtgtta caaccaatta accaattctg attagaaaaa ctcatcgagc 4320

atcaaatgaa actgcaattt attcatatca ggattatcaa taccatattt ttgaaaaagc 4380

cgtttctgta atgaaggaga aactcaccga ggcagttcca taggatggca agatcctgg 4440

tatccggtctg cgattccgac tcgtccaaca tcaatacaac ctattaattt cccctcgtca 4500

aaaataaggt tatcaagtga gaaatcacca tgagtgacga ctgaatccgg tgagaatggc 4560

aaaagcttat gcatttcttt ccagacttgt tcaacaggcc agccattacg ctcgtcatca 4620

aaatcactcg catcaaccaa accgttattc attcgtgatt gcgcctgagc gagacgaaat 4680

acgcgatcgc tgttaaaagg acaattacaa acaggaatcg aatgcaaccg gcgcaggaac 4740

actgccagcg catcaacaat attttcacct gaatcaggat attcttctaa tacctggaat 4800

gctgttttcc cggggatcgc agtggtgagt aaccatgcat catcaggagt acggataaaa 4860

tgcttgatgg tcggaagagg cataaattcc gtcagccagt ttagtctgac catctcatct 4920

gtaacatcat tggcaacgct acctttgcca tgtttcagaa acaactctgg cgcatcgggc 4980

ttcccataca atcgatagat tgtcgcacct gattgcccga cattatcgcg agcccattta 5040

tacccatata aatcagcatc catgttggaa tttaatcgcg gcctcgagca agacgtttcc 5100

cgttgaatat ggctcataac accccttgta ttactgttta tgtaagcaga cagttttatt 5160

gttcatgatg atatattttt atcttgtgca atgtaacatc agagattttg agacacaacg 5220

tggctttccc cccccccca ttattgaagc atttatcagg gttattgtct catgagcgga 5280

tacatatttg aatgtattta gaaaaataaa caaatagggg ttccgcgcac atttccccga 5340

aaagtgccac ctgacgtcta agaaaccatt attatcatga cattaaccta taaaaatagg 5400

cgtatcacga ggccctttcg tc 5422

<210> 6
<211> 5259
<212> DNA
<213> Mus musculus

<400> 6

tcgcgcgttt cggtgatgac ggtgaaaacc tctgacacat gcagctcccg gagacggtca 60

cagcttgtct gtaagcggat gccgggagca gacaagcccg tcagggcgcg tcagcgggtg 120

ttggcgggtg tcggggctgg cttaactatg cggcatcaga gcagattgta ctgagagtgc 180

accatatgcg gtgtgaaata ccgcacagat gcgtaaggag aaaataccgc atcagattgg 240

ctattggcca ttgcatacgt tgtatccata tcataatatg tacatttata ttggctcatg 300

tccaacatta ccgccatgtt gacattgatt attgactagt tattaatagt aatcaattac 360

ggggtcatta gttcatagcc catatatgga gttccgcgtt acataactta cggtaaatgg 420

cccgcctggc tgaccgccca acgacccccg cccattgacg tcaataatga cgtatgttcc 480

catagtaacg ccaataggga ctttccattg acgtcaatgg gtggagtatt tacggtaaac 540

tgcccacttg gcagtacatc aagtgtatca tatgccaagt acgcccccta ttgacgtcaa 600

tgacggtaaa tggcccgcct ggcattatgc ccagtacatg accttatggg actttcctac 660

ttggcagtac atctacgtat tagtcatcgc tattaccatg gtgatgcggt tttggcagta 720

catcaatggg cgtggatagc ggtttgactc acggggattt ccaagtctcc accccattga 780

cgtcaatggg agtttgtttt ggcaccaaaa tcaacgggac tttccaaaat gtcgtaacaa 840

ctccgcccca ttgacgcaaa tgggcggtag gcgtgtacgg tgggaggtct atataagcag 900

agctcgttta gtgaaccgtc agatcgcctg gagacgccat ccacgctgtt ttgacctcca 960

tagaagacac cgggaccgat ccagcctccg cggccgggaa cggtgcattg gaacgcggat 1020

tccccgtgcc aagagtgacg taagtaccgc ctatagactc tataggcaca cccctttggc 1080

tcttatgcat gctatactgt ttttggcttg gggcctatac acccccgctt ccttatgcta 1140

taggtgatgg tatagcttag cctataggtg tgggttattg accattattg accactcccc 1200

tattggtgac gatactttcc attactaatc cataacatgg ctctttgcca caactatctc 1260

tattggctat atgccaatac tctgtccttc agagactgac acggactctg tatttttaca 1320

ggatggggtc ccatttatta tttacaaatt cacatataca acaacgccgt cccccgtgcc 1380

cgcagttttt attaaacata gcgtgggatc tccacgcgaa tctcgggtac gtgttccgga 1440

catgggctct tctccggtag cggcggagct tccacatccg agccctggtc ccatgcctcc 1500

agcggctcat ggtcgctcgg cagctccttg ctcctaacag tggaggccag acttaggcac 1560

agcacaatgc ccaccaccac cagtgtgccg cacaaggccg tggcggtagg gtatgtgtct 1620

gaaaatgagc gtggagattg ggctcgcacg gctgacgcag atggaagact taaggcagcg 1680

gcagaagaag atgcaggcag ctgagttgtt gtattctgat aagagtcaga ggtaactccc 1740

gttgcggtgc tgttaacggt ggagggcagt gtagtctgag cagtactcgt tgctgccgcg 1800

cgcgccacca gacataatag ctgacagact aacagactgt tcctttccat gggtcttttc 1860

tgcagatggc taggctctgt gctttcctga tggtcctggc ggtgatgagc tactggccaa 1920

cctgcttcct aggatgtgac ctgcctcaga ctcataacct caggaacaag agagccttga 1980

cactcctggt acaaatgagg agactctccc ctctctcctg cctgaaggac aggaaggact 2040

ttggattccc gcaggagaag gtggatgccc agcagatcaa gaaggctcaa gccatccctg 2100

tcctgagtga gctgacccag cagatcctga acatcttcac atcaaaggac tcatctgctg 2160

cttggaatgc aaccctccta gactcattct gcaatgacct ccaccagcag ctcaatgacc 2220

tgcaaggttg tctgatgcag caggtggggg tgcaggaatt tcccctgacc caggaagatg 2280

ccctggtggc tgtgaggaaa tacttccaca ggatcactgt gtacctgaga gagaagaaac 2340

acagccactg tgcctgggag gtggtcagag cagaagtctg gagagccctg tcttcctctg 2400

ccaatgggct gggaagactg agagaagaga aatgaagatc cagatctact tctggctaat 2460

aaaagatcag agctctagag atctgtgtgt tggtttttg tgtggtaccc aggtgctgaa 2520

gaattgaccc ggttcctcct gggccagaaa gaagcaggca catccccttc tctgtgacac 2580

accctgtcca cgcccctggt tcttagttcc agccccactc ataggacact catagctcag 2640

gagggctccg ccttcaatcc cacccgctaa agtacttgga gcggtctctc cctccctcat 2700

cagcctacca aaccaaacct agcctccaag agtgggaaga aattaagca agataggcta 2760

ttaagtgcag agggagagaa aatgcctcca acatgtgagg aagtaatcag agaaatcata 2820

gaattctttc cgcttcctcg ctcactgact cgctgcgctc ggtcgttcgg ctgcggcgag 2880

cggtatcagc tcactcaaag gcggtaatac ggttatccac agaatcaggg gataacgcag 2940

gaaagaacat gtgagcaaaa ggccagcaaa aggccaggaa ccgtaaaaag gccgcgttgc 3000

tggcgttttt ccataggctc cgcccccctg acgagcatca caaaaatcga cgctcaagtc 3060

agaggtggcg aaacccgaca ggactataaa gataccaggc gtttccccct ggaagctccc 3120

tcgtgcgctc tcctgttccg accctgccgc ttaccggata cctgtccgcc tttctccctt 3180

cgggaagcgt ggcgctttct caatgctcac gctgtaggta tctcagttcg gtgtaggtcg 3240

ttcgctccaa gctgggctgt gtgcacgaac cccccgttca gcccgaccgc tgcgccttat 3300

ccggtaacta tcgtcttgag tccaacccgg taagacacga cttatcgcca ctggcagcag 3360

ccactggtaa caggattagc agagcgaggt atgtaggcgg tgctacagag ttcttgaagt 3420

ggtggcctaa ctacggctac actagaagga cagtatttgg tatctgcgct ctgctgaagc 3480

cagttacctt cggaaaaaga gttggtagct cttgatccgg caaacaaacc accgctggta 3540

gcggtggttt ttttgtttgc aagcagcaga ttacgcgcag aaaaaaagga tctcaagaag 3600

atcctttgat cttttctacg gggtctgacg ctcagtggaa cgaaaactca cgttaaggga 3660

ttttggtcat gagattatca aaaaggatct tcacctagat ccttttaaat taaaaatgaa 3720

gttttaaatc aatctaaagt atatatgagt aaacttggtc tgacagttac caatgcttaa 3780

tcagtgaggc acctatctca gcgatctgtc tatttcgttc atccatagtt gcctgactcc 3840

gggggggggg ggcgctgagg tctgcctcgt gaagaaggtg ttgctgactc ataccaggcc 3900

tgaatcgccc catcatccag ccagaaagtg agggagccac ggttgatgag agctttgttg 3960

taggtggacc agttggtgat tttgaacttt tgctttgcca cggaacggtc tgcgttgtcg 4020

ggaagatgcg tgatctgatc cttcaactca gcaaaagttc gatttattca acaaagccgc 4080

cgtcccgtca agtcagcgta atgctctgcc agtgttacaa ccaattaacc aattctgatt 4140

agaaaaactc atcgagcatc aaatgaaact gcaatttatt catatcagga ttatcaatac 4200

catatttttg aaaaagccgt ttctgtaatg aaggagaaaa ctcaccgagg cagttccata 4260

ggatggcaag atcctggtat cggtctgcga ttccgactcg tccaacatca atacaaccta 4320

ttaatttccc ctcgtcaaaa ataaggttat caagtgagaa atcaccatga gtgacgactg 4380

aatccggtga gaatggcaaa agcttatgca tttctttcca gacttgttca acaggccagc 4440

cattacgctc gtcatcaaaa tcactcgcat caaccaaacc gttattcatt cgtgattgcg 4500

cctgagcgag acgaaatacg cgatcgctgt taaaaggaca attacaaaca ggaatcgaat 4560

gcaaccggcg caggaacact gccagcgcat caacaatatt ttcacctgaa tcaggatatt 4620

cttctaatac ctggaatgct gttttcccgg ggatcgcagt ggtgagtaac catgcatcat 4680

caggagtacg gataaaatgc ttgatggtcg gaagaggcat aaattccgtc agccagttta 4740

gtctgaccat ctcatctgta acatcattgg caacgctacc tttgccatgt ttcagaaaca 4800

actctggcgc atcgggcttc ccatacaatc gatagattgt cgcacctgat tgcccgacat 4860

tatcgcgagc ccatttatac ccatataaat cagcatccat gttggaattt aatcgcggcc 4920

tcgagcaaga cgtttcccgt tgaatatggc tcataacacc ccttgtatta ctgtttatgt 4980

aagcagacag ttttattgtt catgatgata tatttttatc ttgtgcaatg taacatcaga 5040

gattttgaga cacaacgtgg ctttcccccc ccccccatta ttgaagcatt tatcagggtt 5100

attgtctcat gagcggatac atatttgaat gtatttagaa aaataaacaa ataggggttc 5160

cgcgcacatt tccccgaaaa gtgccacctg acgtctaaga aaccattatt atcatgacat 5220

taacctataa aaataggcgt atcacgaggc cctttcgtc 5259

<210> 7
<211> 585
<212> DNA
<213> Homo sapiens

<220>
<221> CDS
<222> (1)..(585)

<220>
<221> sig_peptide
<222> (1)..(69)

<220>
<221> mat_peptide
<222> (70)..(585)

<400> 7

```
atg gcc ctc ctg ttc cct cta ctg gca gcc cta gtg atg acc agc tat      48
Met Ala Leu Leu Phe Pro Leu Leu Ala Ala Leu Val Met Thr Ser Tyr
            -20                 -15                 -10

agc cct gtt gga tct ctg ggc tgt gat ctg cct cag aac cat ggc cta      96
Ser Pro Val Gly Ser Leu Gly Cys Asp Leu Pro Gln Asn His Gly Leu
        -5                  -1   1               5

ctt agc agg aac acc ttg gtg ctt ctg cac caa atg agg aga atc tcc     144
Leu Ser Arg Asn Thr Leu Val Leu Leu His Gln Met Arg Arg Ile Ser
 10                  15                  20                  25

cct ttc ttg tgt ctc aag gac aga aga gac ttc agg ttc ccc cag gag     192
Pro Phe Leu Cys Leu Lys Asp Arg Arg Asp Phe Arg Phe Pro Gln Glu
                 30                  35                  40

atg gta aaa ggg agc cag ttg cag aag gcc cat gtc atg tct gtc ctc     240
Met Val Lys Gly Ser Gln Leu Gln Lys Ala His Val Met Ser Val Leu
             45                  50                  55

cat gag atg ctg cag cag atc ttc agc ctc ttc cac aca gag cgc tcc     288
His Glu Met Leu Gln Gln Ile Phe Ser Leu Phe His Thr Glu Arg Ser
         60                  65                  70
```

```
tct gct gcc tgg aac atg acc ctc cta gac caa ctc cac act gga ctt    336
Ser Ala Ala Trp Asn Met Thr Leu Leu Asp Gln Leu His Thr Gly Leu
     75                 80                  85

cat cag caa ctg caa cac ctg gag acc tgc ttg ctg cag gta gtg gga    384
His Gln Gln Leu Gln His Leu Glu Thr Cys Leu Leu Gln Val Val Gly
 90                 95                 100                 105

gaa gga gaa tct gct ggg gca att agc agc cct gca ctg acc ttg agg    432
Glu Gly Glu Ser Ala Gly Ala Ile Ser Ser Pro Ala Leu Thr Leu Arg
                110                 115                 120

agg tac ttc cag gga atc cgt gtc tac ctg aaa gag aag aaa tac agc    480
Arg Tyr Phe Gln Gly Ile Arg Val Tyr Leu Lys Glu Lys Lys Tyr Ser
            125                 130                 135

gac tgt gcc tgg gaa gtt gtc aga atg gaa atc atg aaa tcc ttg ttc    528
Asp Cys Ala Trp Glu Val Val Arg Met Glu Ile Met Lys Ser Leu Phe
        140                 145                 150

tta tca aca aac atg caa gaa aga ctg aga agt aaa gat aga gac ctg    576
Leu Ser Thr Asn Met Gln Glu Arg Leu Arg Ser Lys Asp Arg Asp Leu
    155                 160                 165

ggc tca tct                                                        585
Gly Ser Ser
170
```

<210> 8
<211> 195
<212> PRT
<213> Homo sapiens

<400> 8

```
Met Ala Leu Leu Phe Pro Leu Leu Ala Ala Leu Val Met Thr Ser Tyr
            -20                 -15                 -10

Ser Pro Val Gly Ser Leu Gly Cys Asp Leu Pro Gln Asn His Gly Leu
        -5                  -1   1                   5

Leu Ser Arg Asn Thr Leu Val Leu Leu His Gln Met Arg Arg Ile Ser
 10                  15                  20                  25

Pro Phe Leu Cys Leu Lys Asp Arg Arg Asp Phe Arg Phe Pro Gln Glu
                 30                  35                  40

Met Val Lys Gly Ser Gln Leu Gln Lys Ala His Val Met Ser Val Leu
            45                  50                  55

His Glu Met Leu Gln Gln Ile Phe Ser Leu Phe His Thr Glu Arg Ser
```

60 65 70

Ser Ala Ala Trp Asn Met Thr Leu Leu Asp Gln Leu His Thr Gly Leu
75 80 85

His Gln Gln Leu Gln His Leu Glu Thr Cys Leu Leu Gln Val Val Gly
90 95 100 105

Glu Gly Glu Ser Ala Gly Ala Ile Ser Ser Pro Ala Leu Thr Leu Arg
110 115 120

Arg Tyr Phe Gln Gly Ile Arg Val Tyr Leu Lys Glu Lys Lys Tyr Ser
125 130 135

Asp Cys Ala Trp Glu Val Val Arg Met Glu Ile Met Lys Ser Leu Phe
140 145 150

Leu Ser Thr Asn Met Gln Glu Arg Leu Arg Ser Lys Asp Arg Asp Leu
155 160 165

Gly Ser Ser
170

<210> 9
<211> 567
<212> DNA
<213> Homo sapiens

<220>
<221> CDS
<222> (1)..(567)

<220>
<221> sig_peptide
<222> (1)...(69)

<220>
<221> mat_peptide
<222> (70)..(567)

<400> 9

atg gcc tcg ccc ttt gct tta ctg atg gtc ctg gtg gtg ctc agc tgc 48
Met Ala Ser Pro Phe Ala Leu Leu Met Val Leu Val Val Leu Ser Cys
-20 -15 -10

aag tca agc tgc tct ctg ggc tgt gat ctc cct gag acc cac agc ctg 96
Lys Ser Ser Cys Ser Leu Gly Cys Asp Leu Pro Glu Thr His Ser Leu
-5 -1 1 5

gat aac agg agg acc ttg atg ctc ctg gca caa atg agc aga atc tct 144
Asp Asn Arg Arg Thr Leu Met Leu Leu Ala Gln Met Ser Arg Ile Ser

```
10                  15                  20                  25

cct tcc tcc tgt ctg atg gac aga cat gac ttt gga ttt ccc cag gag    192
Pro Ser Ser Cys Leu Met Asp Arg His Asp Phe Gly Phe Pro Gln Glu
                30                  35                  40

gag ttt gat ggc aac cag ttc cag aag gct cca gcc atc tct gtc ctc    240
Glu Phe Asp Gly Asn Gln Phe Gln Lys Ala Pro Ala Ile Ser Val Leu
            45                  50                  55

cat gag ctg atc cag cag atc ttc aac ctc ttt acc aca aaa gat tca    288
His Glu Leu Ile Gln Gln Ile Phe Asn Leu Phe Thr Thr Lys Asp Ser
        60                  65                  70

tct gct gct tgg gat gag gac ctc cta gac aaa ttc tgc acc gaa ctc    336
Ser Ala Ala Trp Asp Glu Asp Leu Leu Asp Lys Phe Cys Thr Glu Leu
    75                  80                  85

tac cag cag ctg aat gac ttg gaa gcc tgt gtg atg cag gag gag agg    384
Tyr Gln Gln Leu Asn Asp Leu Glu Ala Cys Val Met Gln Glu Glu Arg
90                  95                  100                 105

gtg gga gaa act ccc ctg atg aat gcg gac tcc atc ttg gct gtg aag    432
Val Gly Glu Thr Pro Leu Met Asn Ala Asp Ser Ile Leu Ala Val Lys
                110                 115                 120

aaa tac ttc cga aga atc act ctc tat ctg aca gag aag aaa tac agc    480
Lys Tyr Phe Arg Arg Ile Thr Leu Tyr Leu Thr Glu Lys Lys Tyr Ser
            125                 130                 135

cct tgt gcc tgg gag gtt gtc aga gca gaa atc atg aga tcc ctc tct    528
Pro Cys Ala Trp Glu Val Val Arg Ala Glu Ile Met Arg Ser Leu Ser
        140                 145                 150

tta tca aca aac ttg caa gaa aga tta agg agg aag gaa                567
Leu Ser Thr Asn Leu Gln Glu Arg Leu Arg Arg Lys Glu
    155                 160                 165
```

<210> 10
<211> 189
<212> PRT
<213> Homo sapiens

<400> 10

```
Met Ala Ser Pro Phe Ala Leu Leu Met Val Leu Val Val Leu Ser Cys
            -20                 -15                 -10

Lys Ser Ser Cys Ser Leu Gly Cys Asp Leu Pro Glu Thr His Ser Leu
        -5                  -1  1               5

Asp Asn Arg Arg Thr Leu Met Leu Leu Ala Gln Met Ser Arg Ile Ser
```

10 15 20 25

Pro Ser Ser Cys Leu Met Asp Arg His Asp Phe Gly Phe Pro Gln Glu
30 35 40

Glu Phe Asp Gly Asn Gln Phe Gln Lys Ala Pro Ala Ile Ser Val Leu
45 50 55

His Glu Leu Ile Gln Gln Ile Phe Asn Leu Phe Thr Thr Lys Asp Ser
60 65 70

Ser Ala Ala Trp Asp Glu Asp Leu Leu Asp Lys Phe Cys Thr Glu Leu
75 80 85

Tyr Gln Gln Leu Asn Asp Leu Glu Ala Cys Val Met Gln Glu Glu Arg
90 95 100 105

Val Gly Glu Thr Pro Leu Met Asn Ala Asp Ser Ile Leu Ala Val Lys
110 115 120

Lys Tyr Phe Arg Arg Ile Thr Leu Tyr Leu Thr Glu Lys Lys Tyr Ser
125 130 135

Pro Cys Ala Trp Glu Val Val Arg Ala Glu Ile Met Arg Ser Leu Ser
140 145 150

Leu Ser Thr Asn Leu Gln Glu Arg Leu Arg Arg Lys Glu
155 160 165

<210> 11
<211> 567
<212 DNA
<213> Mus musculus

<220>
<221> CDS
<222> (1)..(567)

<220>
<221> sig_peptide
<222> (1)..(69)

<220>
<221> mat_peptide
<222> (70)..(567)

<400> 11

atg gct agg ctc tgt gct ttc ctg atg gtc ctg gcg gtg atg agc tac 48
Met Ala Arg Leu Cys Ala Phe Leu Met Val Leu Ala Val Met Ser Tyr
-20 -15 -10

```
tgg cca acc tgc tct cta gga tgt gac ctg cct cag act cat aac ctc    96
Trp Pro Thr Cys Ser Leu Gly Cys Asp Leu Pro Gln Thr His Asn Leu
        -5                  -1   1               5

agg aac aag aga gcc ttg aca ctc ctg gta caa atg agg aga ctc tcc    144
Arg Asn Lys Arg Ala Leu Thr Leu Leu Val Gln Met Arg Arg Leu Ser
 10                  15                  20                  25

cct ctc tcc tgc ctg aag gac agg aag gac ttt gga ttc ccg cag gag    192
Pro Leu Ser Cys Leu Lys Asp Arg Lys Asp Phe Gly Phe Pro Gln Glu
                30                  35                  40

aag gtg gat gcc cag cag atc aag aag gct caa gcc atc cct gtc ctg    240
Lys Val Asp Ala Gln Gln Ile Lys Lys Ala Gln Ala Ile Pro Val Leu
            45                  50                  55

agt gag ctg acc cag cag atc ctg aac atc ttc aca tca aag gac tca    288
Ser Glu Leu Thr Gln Gln Ile Leu Asn Ile Phe Thr Ser Lys Asp Ser
        60                  65                  70

tct gct gct tgg aat gca acc ctc cta gac tca ttc tgc aat gac ctc    336
Ser Ala Ala Trp Asn Ala Thr Leu Leu Asp Ser Phe Cys Asn Asp Leu
    75                  80                  85

cac cag cag ctc aat gac ctg caa ggt tgt ctg atg cag cag gtg ggg    384
His Gln Gln Leu Asn Asp Leu Gln Gly Cys Leu Met Gln Gln Val Gly
 90                  95                 100                 105

gtg cag gaa ttt ccc ctg acc cag gaa gat gcc ctg ctg gct gtg agg    432
Val Gln Glu Phe Pro Leu Thr Gln Glu Asp Ala Leu Leu Ala Val Arg
                110                 115                 120

aaa tac ttc cac agg atc act gtg tac ctg aga gag aag aaa cac agc    480
Lys Tyr Phe His Arg Ile Thr Val Tyr Leu Arg Glu Lys Lys His Ser
            125                 130                 135

ccc tgt gcc tgg gag gtg gtc aga gca gaa gtc tgg aga gcc ctg tct    528
Pro Cys Ala Trp Glu Val Val Arg Ala Glu Val Trp Arg Ala Leu Ser
        140                 145                 150

tcc tct gcc aat gtg ctg gga aga ctg aga gaa gag aaa                567
Ser Ser Ala Asn Val Leu Gly Arg Leu Arg Glu Glu Lys
    155                 160                 165
```

<210 > 12
<211> 189
<212> PRT
<213> Mus musculus

<400> 12

```
Met Ala Arg Leu Cys Ala Phe Leu Met Val Leu Ala Val Met Ser Tyr
```

```
                -20                 -15                 -10

Trp Pro Thr Cys Ser Leu Gly Cys Asp Leu Pro Gln Thr His Asn Leu
        -5              -1   1                   5

Arg Asn Lys Arg Ala Leu Thr Leu Leu Val Gln Met Arg Arg Leu Ser
 10                  15                  20                  25

Pro Leu Ser Cys Leu Lys Asp Arg Lys Asp Phe Gly Phe Pro Gln Glu
                 30                  35                  40

Lys Val Asp Ala Gln Gln Ile Lys Lys Ala Gln Ala Ile Pro Val Leu
             45                  50                  55

Ser Glu Leu Thr Gln Gln Ile Leu Asn Ile Phe Thr Ser Lys Asp Ser
        60                  65                  70

Ser Ala Ala Trp Asn Ala Thr Leu Leu Asp Ser Phe Cys Asn Asp Leu
     75                  80                  85

His Gln Gln Leu Asn Asp Leu Gln Gly Cys Leu Met Gln Gln Val Gly
 90                  95                 100                 105

Val Gln Glu Phe Pro Leu Thr Gln Glu Asp Ala Leu Leu Ala Val Arg
                110                 115                 120

Lys Tyr Phe His Arg Ile Thr Val Tyr Leu Arg Glu Lys Lys His Ser
            125                 130                 135

Pro Cys Ala Trp Glu Val Val Arg Ala Glu Val Trp Arg Ala Leu Ser
        140                 145                 150

Ser Ser Ala Asn Val Leu Gly Arg Leu Arg Glu Glu Lys
    155                 160                 165
```

<210> 13
<211> 459
<212> DNA
<215> Homo sapiens

<220>
<221> CDS
<222> (1)..(459)

<220>
<221> sig_peptide
<222> (1)..(60)

<220>
<221> mat_peptide
<222> (61)..(459)

<400> 13

```
atg tac agg atg caa ctc ctg tct tgc att gca cta agt ctt gca ctt     48
Met Tyr Arg Met Gln Leu Leu Ser Cys Ile Ala Leu Ser Leu Ala Leu
-20                 -15                 -10                 -5

gtc aca aac agt gca cct act tca agt tct aca aag aaa aca cag cta     96
Val Thr Asn Ser Ala Pro Thr Ser Ser Ser Thr Lys Lys Thr Gln Leu
            -1   1               5                   10

caa ctg gag cat tta ctt ctg gat tta cag atg att ttg aat gga att    144
Gln Leu Glu His Leu Leu Leu Asp Leu Gln Met Ile Leu Asn Gly Ile
        15                  20                  25

aat aat tac aag aat ccc aaa ctc acc agg atg ctc aca ttt aag ttt    192
Asn Asn Tyr Lys Asn Pro Lys Leu Thr Arg Met Leu Thr Phe Lys Phe
    30                  35                  40

tac atg ccc aag aag gcc aca gaa ctg aaa cat ctt cag tgt cta gaa    240
Tyr Met Pro Lys Lys Ala Thr Glu Leu Lys His Leu Gln Cys Leu Glu
45                  50                  55                  60

gaa gaa ctc aaa cct ctg gag gaa gtg cta aat tta gct caa agc aaa    288
Glu Glu Leu Lys Pro Leu Glu Glu Val Leu Asn Leu Ala Gln Ser Lys
                65                  70                  75

aac ttt cac tta aga ccc agg gac tta atc agc aat atc aac gta ata    336
Asn Phe His Leu Arg Pro Arg Asp Leu Ile Ser Asn Ile Asn Val Ile
            80                  85                  90

gtt ctg gaa cta aag gga tct gaa aca aca ttc atg tgt gaa tat gct    384
Val Leu Glu Leu Lys Gly Ser Glu Thr Thr Phe Met Cys Glu Tyr Ala
        95                  100                 105

gat gag aca gca acc att gta gaa ttt ctg aac aga tgg att acc ttt    432
Asp Glu Thr Ala Thr Ile Val Glu Phe Leu Asn Arg Trp Ile Thr Phe
    110                 115                 120

tgt caa agc atc atc tca aca ctg act                                459
Cys Gln Ser Ile Ile Ser Thr Leu Thr
125                 130
```

<210> 14
<211> 153
<212> PRT
<213> Homo sapiens

<400> 14

```
Met Tyr Arg Met Gln Leu Leu Ser Cys Ile Ala Leu Ser Leu Ala Leu
-20                 -15                 -10                 -5
```

Val Thr Asn Ser Ala Pro Thr Ser Ser Ser Thr Lys Lys Thr Gln Leu
-1 1 5 10

Gln Leu Glu His Leu Leu Leu Asp Leu Gln Met Ile Leu Asn Gly Ile
15 20 25

Asn Asn Tyr Lys Asn Pro Lys Leu Thr Arg Met Leu Thr Phe Lys Phe
30 35 40

Tyr Met Pro Lys Lys Ala Thr Glu Leu Lys His Leu Gln Cys Leu Glu
45 50 55 60

Glu Glu Leu Lys Pro Leu Glu Glu Val Leu Asn Leu Ala Gln Ser Lys
65 70 75

Asn Phe His Leu Arg Pro Arg Asp Leu Ile Ser Asn Ile Asn Val Ile
80 85 90

Val Leu Glu Leu Lys Gly Ser Glu Thr Thr Phe Met Cys Glu Tyr Ala
95 100 105

Asp Glu Thr Ala Thr Ile Val Glu Phe Leu Asn Arg Trp Ile Thr Phe
110 115 120

Cys Gln Ser Ile Ile Ser Thr Leu Thr
125 130

<210> 15
<211> 26
<212> DNA
<213> Artificial Sequence

<220>
<223> Description of Artificial Sequence:PCR oligo

<400> 15
aactgcagat ggctaggctc tgtgct 26

<210> 16
<211> 26
<212> DNA
<213> Artificial Sequence

<220>
<223> Description of Artificial Sequence:PCR oligo

<400> 16
gaagatcttc atttctcttc tctcag 26

<210> 17
<211> 26
<212> DNA
<213> Artificial Sequence

<220>

<223> Description of Artificial Sequence:PCR oligo

<400> 17
aactgcagat ggcctcgccc tttgct 26

<210> 18
<211> 28
<212> DNA
<213> Artificial Sequence

<220>
<223> Description of Artificial Sequence:PCR oligo

<400> 18
cgggatcctt attccttcct ccttaatc 28

<210> 19
<211> 25
<212> DNA
<213> Artificial Sequence

<220>
<223> Description of Artificial Sequence:PCR oligo

<400> 19
gctctagatg gccctcctgt tccct 25

<210> 20
<211> 26
<212> DNA
<213> Artificial Sequence

<220>
<223> Description of Artificial Sequence:PCR oligo

<400> 20
gcggatcctc aagatgagcc caggtc 26

<210> 21
<211> 36
<212> DNA
<213> Artificial Sequence

<220>
<211> Description of Artificial Sequence: PCR oligo

<400> 21
acgcgtcgac atgtgtcctc agaagctaac catctc 36

<210> 22
<211> 34
<212> DNA
<213> Artificial Sequence

<220>
<223> Description of Artificial Sequence: PCR oligo

<400> 22

gcggatccct aggatcggac cctgcaggga acac 34

<210> 23
<211> 37
<212> DNA
<213> Artificial Sequence

<22C>
<223> Description of Artificial Sequence: PCR oligo

<400> 23
catgccatgg gtcaatcacg ctacctcctc tttttgg 37

<210> 24
<211> 30
<212> DNA
<213> Artificial Sequence

<220>
<223> Description of Artificial Sequence: PCR oligo

<400> 24
gcggatcctc aggcggagct cagatagccc 30

<210> 25
<211> 5469
<212> DNA
<213> Mus musculus

<220>
<221> CDS
<222> (1923)..(2393)

<220>
<221> sig_peptide
<222> (1923)..(1994)

<22C>
<221> mat_peptide
<222> (1995)..(2393)

<400> 25

tcgcgcgttt cggtgatgac ggtgaaaacc tctgacacat gcagctcccg gagacggtca 60

cagcttgtct gtaagcggat gccgggagca gacaagcccg tcagggcgcg tcagcgggtg 120

ttggcgggtg tcggggctgg cttaactatg cggcatcaga gcagattgta ctgagagtgc 180

accatatgcg gtgtgaaata ccgcacagat gcgtaaggag aaaataccgc atcagattgg 240

ctattggcca ttgcatacgt tgtatccata tcataatatg tacatttata ttggctcatg 300

tccaacatta ccgccatgtt gacattgatt attgactagt tattaatagt aatcaattac 360

ggggtcatta gttcatagcc catatatgga gttccgcgtt acataactta cggtaaatgg 420

cccgcctggc tgaccgccca acgacccccg cccattgacg tcaataatga cgtatgttcc 480

catagtaacg ccaataggga ctttccattg acgtcaatgg gtggagtatt tacggtaaac 540

tgcccacttg gcagtacatc aagtgtatca tatgccaagt acgcccccta ttgacgtcaa 600

tgacggtaaa tggcccgcct ggcattatgc ccagtacatg accttatggg actttcctac 660

ttggcagtac atctacgtat tagtcatcgc tattaccatg gtgatgcggt tttggcagta 720

catcaatggg cgtggatagc ggtttgactc acggggattt ccaagtctcc accccattga 780

cgtcaatggg agtttgtttt ggcaccaaaa tcaacgggac tttccaaaat gtcgtaacaa 840

ctccgcccca ttgacgcaaa tgggcggtag gcgtgtacgg tgggaggtct atataagcag 900

agctcgttta gtgaaccgtc agatcgcctg gagacgccat ccacgctgtt ttgacctcca 960

tagaagacac cgggaccgat ccagcctccg cggccgggaa cggtgcattg gaacgcggat 1020

tccccgtgcc aagagtgacg taagtaccgc ctatagagtc tataggccca cccccttggc 1080

ttcttatgca tgctatactg tttttggctt ggggtctata cacccccgct tcctcatgtt 1140

ataggtgatg gtatagctta gcctataggt gtgggttatt gaccattatt gaccactccc 1200

ctattggtga cgatactttc cattactaat ccataacatg gctctttgcc acaactctct 1260

ttattggcta tatgccaata cactgtcctt cagagactga cacggactct gtatttttac 1320

aggatggggt ctcatttatt atttacaaat tcacatatac aacaccaccg tccccagtgc 1380

```
cccgagtttt tattaaacat aacgtgggat ctccacgcga atctcgggta cgtgttccgg 1440

acatgggctc ttctccggta gcggcggagc ttctacatcc gagccctgct cccatgcctc 1500

cagcgactca tggtcgctcg gcagctcctt gctcctaaca gtggaggcca gacttaggca 1560

cagcacgatg cccaccacca ccagtgtgcc gcacaaggcc gtggcggtag ggtatgtgtc 1620

tgaaaatgag ctcggggagc gggcttgcac cgctgacgca tttggaagac ttaaggcagc 1680

ggcagaagaa gatgcaggca gctgagttgt tgtgttctga taagagtcag aggtaactcc 1740

cgttgcggtg ctgttaacgg tggagggcag tgtagtctga gcagtactcg ttgctgccgc 1800

gcgcgccacc agacataata gctgacagac taacagactg ttcctttcca tgggtctttt 1860

ctccagtcac cgtccttaga tctagcctca accctgacta tcttccaggt cattgttcca 1920

ac atg gcc ctg tgg atc gac agg atg caa ctc ctg tct tgc att gca    1967
   Met Ala Leu Trp Ile Asp Arg Met Gln Leu Leu Ser Cys Ile Ala
                   -20                 -15                 -10

cta agt ctt gca ctt gtc aca aac agt gca cct act tca agt tct aca   2015
Leu Ser Leu Ala Leu Val Thr Asn Ser Ala Pro Thr Ser Ser Ser Thr
                -5              -1   1               5

aag aaa aca cag cta caa ctg gag cat tta ctg ctg gat tta cag atg   2063
Lys Lys Thr Gln Leu Gln Leu Glu His Leu Leu Leu Asp Leu Gln Met
        10                  15                  20

att ttg aat gga att aat aat tac aag aat ccc aaa ctc acc agg atg   2111
Ile Leu Asn Gly Ile Asn Asn Tyr Lys Asn Pro Lys Leu Thr Arg Met
    25                  30                  35

ctc aca ttt aag ttt tac atg ccc aag aag gcc aca gaa ctg aaa cat   2159
Leu Thr Phe Lys Phe Tyr Met Pro Lys Lys Ala Thr Glu Leu Lys His
40                  45                  50                  55

ctt cag tgt cta gaa gaa gaa ctc aaa cct ctg gag gaa gtg cta aat   2207
Leu Gln Cys Leu Glu Glu Glu Leu Lys Pro Leu Glu Glu Val Leu Asn
                60                  65                  70

tta gct caa agc aaa aac ttt cac tta aga ccc agg gac tta atc agc   2255
Leu Ala Gln Ser Lys Asn Phe His Leu Arg Pro Arg Asp Leu Ile Ser
            75                  80                  85

aat atc aac gta ata gtt ctg gaa cta aag gga tct gaa aca aca ttc   2303
Asn Ile Asn Val Ile Val Leu Glu Leu Lys Gly Ser Glu Thr Thr Phe
        90                  95                  100

atg tgt gaa tat gct gat gag aca gca acc att gta gaa ttt ctg aac   2351
```

Met Cys Glu Tyr Ala Asp Glu Thr Ala Thr Ile Val Glu Phe Leu Asn
    105                 110                 115

aga tgg att acc ttt tgt caa agc atc atc tca aca cta act 2393
Arg Trp Ile Thr Phe Cys Gln Ser Ile Ile Ser Thr Leu Thr
120                 125                 130

tgataattaa gtgcttccca cttaaaacat atcagggatc tcgactctag aggatcatcg 2453

cgcccgctct agaccaggcg cctggatcca gatctgctgt gccttctagt tgccagccat 2513

ctgttgtttg cccctccccc gtgccttcct tgaccctgga aggtgccact cccactgtcc 2573

tttcctaata aaatgaggaa attgcatcgc attgtctgag taggtgtcat tctattctgg 2633

ggggtggggt ggggcagcac agcaaggggg aggattggga agacaatagc aggcatgctg 2693

gggatgcggt gggctctatg ggtacccagg tgctgaagaa ttgacccggt tcctcctggg 2753

ccagaaagaa gcaggcacat ccccttctct gtgacacacc ctgtccacgc ccctggttct 2813

tagttccagc cccactcata ggacactcat agctcaggag ggctccgcct tcaatcccac 2873

ccgctaaagt acttggagcg gtctctccct ccctcatcag cccaccaaac caaacctagc 2933

ctccaagagt gggaagaaat taaagcaaga taggctatta agtgcagagg gagagaaaat 2993

gcctccaaca tgtgaggaag taatgagaga aatcatagaa tttcttccgc ttcctcgctc 3053

actgactcgc tgcgctcggt cgttcggctg cggcgagcgg tatcagctca ctcaaaggcg 3113

gtaatacggt tatccacaga atcaggggat aacgcaggaa agaacatgtg agcaaaaggc 3173

cagcaaaagg ccaggaaccg taaaaaggcc gcgttgctgg cgtttttcca taggctccgc 3233

ccccctgacg agcatcacaa aaatcgacgc tcaagtcaga ggtggcgaaa cccgacagga 3293

ctataaagat accaggcgtt tccccctgga agctccctcg tgcgctctcc tgttccgacc 3353

ctgccgctta ccggatacct gtccgccttt ctcccttcgg gaagcgtggc gctttctcaa 3413

tgctcacgct gtaggtatct cagttcggtg taggtcgttc gctccaagct gggctgtgtg 3473

cacgaacccc ccgttcagcc cgaccgctgc gccttatccg gtaactatcg tcttgagtcc 3533

aacccggtaa gacacgactt atcgccactg gcagcagcca ctggtaacag gattagcaga 3593

gcgaggtatg taggcggtgc tacagagttc ttgaagtggt ggcctaacta cggctacact 3653

agaaggacag tatttggtat ctgcgctctg ctgaagccag ttaccttcgg aaaaagagtt 3713

ggtagctctt gatccggcaa acaaaccacc gctggtagcg gtggtttttt tgtttgcaag 3773

```
cagcagatta cgcgcagaaa aaaaggatct caagaagatc ctttgatctt ttctacgggg 3833
tctgacgctc agtggaacga aaactcacgt taagggattt tggtcatgag attatcaaaa 3893
aggatcttca cctagatcct tttaaattaa aaatgaagtt ttaaatcaat ctaaagtata 3953
tatgagtaaa cttggtctga cagttaccaa tgcttaatca gtgaggcacc tatctcagcg 4013
atctgtctat ttcgttcatc catagttgcc tgactccggg gggggggggc gctgaggtct 4073
gcctcgtgaa gaaggtgttg ctgactcata ccaggcctga atcgccccat catccagcca 4133
gaaagtgagg gagccacggt tgatgagagc tttgttgtag gtggaccagt tggtgatttt 4193
gaacttttgc tttgccacgg aacggtctgc gttgtcggga agatgcgtga tctgatcctt 4253
caactcagca aaagttcgat ttattcaaca aagccgccgt cccgtcaagt cagcgtaatg 4313
ctctgccagt gttacaacca attaaccaat tctgattaga aaaactcatc gagcatcaaa 4373
tgaaactgca atttattcat atcaggatta tcaataccat atttttgaaa aagccgtttc 4433
tgtaatgaag gagaaaactc accgaggcag ttccatagga tggcaagatc ctggtatcgg 4493
tctgcgattc cgactcgtcc aacatcaata caacctatta atttcccctc gtcaaaaata 4553
aggttatcaa gtgagaaatc accatgagtg acgactgaat ccggtgagaa tggcaaaagc 4613
ttatgcattt ctttccagac ttgttcaaca ggccagccat tacgctcgtc atcaaaatca 4673
ctcgcatcaa ccaaaccgtt attcattcgt gattgcgcct gagcgagacg aaatacgcga 4733
tcgctgttaa aaggacaatt acaaacagga atcgaatgca accggcgcag gaacactgcc 4793
agcgcatcaa caatattttc acctgaatca ggatattctt ctaatacctg gaatgctgtt 4853
ttcccgggga tcgcagtggt gagtaaccat gcatcatcag gagtacggat aaaatgcttg 4913
atggtcggaa gaggcataaa ttccgtcagc cagtttagtc tgaccatctc atctgtaaca 4973
tcattggcaa cgctaccttt gccatgtttc agaaacaact ctggcgcatc gggcttccca 5033
tacaatcgat agattgtcgc acctgattgc ccgacattat cgcgagccca tttataccca 5093
tataaatcag catccatgtt ggaatttaat cgcggcctcg agcaagacgt ttcccgttga 5153
atatggctca taacacccct tgtattactg tttatgtaag cagacagttt tattgttcat 5213
gatgatatat ttttatcttg tgcaatgtaa catcagagat tttgagacac aacgtggctt 5273
tccccccccc cccattattg aagcatttat cagggttatt gtctcatgag cggatacata 5333
```

```
tttgaatgta tttagaaaaa taaacaaata ggggttccgc gcacatttcc ccgaaaagtg 5393

ccacctgacg tctaagaaac cattattatc atgacattaa cctataaaaa taggcgtatc 5453

acgaggccct ttcgtc                                                 5469
```

<210> 26
<211> 157
<212> PRT
<213> Mus musculus

<400> 26

```
Met Ala Leu Trp Ile Asp Arg Met Gln Leu Leu Ser Cys Ile Ala Leu
                -20                 -15                 -10

Ser Leu Ala Leu Val Thr Asn Ser Ala Pro Thr Ser Ser Ser Thr Lys
            -5                  -1  1               5

Lys Thr Gln Leu Gln Leu Glu His Leu Leu Leu Asp Leu Gln Met Ile
    10                  15                  20

Leu Asn Gly Ile Asn Asn Tyr Lys Asn Pro Lys Leu Thr Arg Met Leu
25                  30                  35                  40

Thr Phe Lys Phe Tyr Met Pro Lys Lys Ala Thr Glu Leu Lys His Leu
                45                  50                  55

Gln Cys Leu Glu Glu Glu Leu Lys Pro Leu Glu Glu Val Leu Asn Leu
            60                  65                  70

Ala Gln Ser Lys Asn Phe His Leu Arg Pro Arg Asp Leu Ile Ser Asn
        75                  80                  85

Ile Asn Val Ile Val Leu Glu Leu Lys Gly Ser Glu Thr Thr Phe Met
    90                  95                  100

Cys Glu Tyr Ala Asp Glu Thr Ala Thr Ile Val Glu Phe Leu Asn Arg
105                 110                 115                 120

Trp Ile Thr Phe Cys Gln Ser Ile Ile Ser Thr Leu Thr
                125                 130
```

SEQUENCE LISTING

<110> VICAL
Horton, Holly
Parker, Suezanne
Manthorpe, Marston
Felgner, Philip

<120> Treatment of Cancer Using Cytokine-Expressing Polynucleotides and Compositions Therefor

<130> 1530.006PC03

<140>
<141>

<150> US 60/067,087
<151> 1997-11-20

<150> US 60/079,914
<151> 1998-03-30

<150> US 60/100,820
<151> 1998-09-15

<160> 26

<170> PatentIn Ver. 2.0

<210> 1
<211> 5428
<212> DNA
<213> Homo sapiens

<400> 1

```
tcgcgcgttt cggtgatgac ggtgaaaacc tctgacacat gcagctcccg gagacggtca 60
cagcttgtct gtaagcggat gccgggagca gacaagcccg tcagggcgcg tcagcgggtg 120
ttggcgggtg tcggggctgg cttaactatg cggcatcaga gcagattgta ctgagagtgc 180
accatatgcg gtgtgaaata ccgcacagat gcgtaaggag aaaataccgc atcagattgg 240
ctattggcca ttgcatacgt tgtatccata tcataatatg tacatttata ttggctcatg 300
tccaacatta ccgccatgtt gacattgatt attgactagt tattaatagt aatcaattac 360
```

ggggtcatta gttcatagcc catatatgga gttccgcgtt acataactta cggtaaatgg 420

cccgcctggc tgaccgccca acgacccccg cccattgacg tcaataatga cgtatgttcc 480

catagtaacg ccaataggga ctttccattg acgtcaatgg gtggagtatt tacggtaaac 540

tgcccacttg gcagtacatc aagtgtatca tatgccaagt acgcccccta ttgacgtcaa 600

tgacggtaaa tggcccgcct ggcattatgc ccagtacatg accttatggg actttcctac 660

ttggcagtac atctacgtat tagtcatcgc tattaccatg gtgatgcggt tttggcagta 720

catcaatggg cgtggatagc ggtttgactc acggggattt ccaagtctcc accccattga 780

cgtcaatggg agtttgtttt ggcaccaaaa tcaacgggac tttccaaaat gtcgtaacaa 840

ctccgcccca ttgacgcaaa tgggcggtag gcgtgtacgg tgggaggtct atataagcag 900

agctcgttta gtgaaccgtc agatcgcctg gagacgccat ccacgctgtt ttgacctcca 960

tagaagacac cgggaccgat ccagcctccg cggccgggaa cggtgcattg gaacgcggat 1020

tccccgtgcc aagagtgacg taagtaccgc ctatagactc tataggcaca cccctttggc 1080

tcttatgcat gctatactgt ttttggcttg gggcctatac acccccgctt ccttatgcta 1140

taggtgatgg tatagcttag cctataggtg tgggttattg accattattg accactcccc 1200

tattggtgac gatactttcc attactaatc cataacatgg ctctttgcca caactatctc 1260

tattggctat atgccaatac tctgtccttc agagactgac acggactctg tatttttaca 1320

ggatggggtc ccatttatta tttacaaatt cacatataca acaacgccgt cccccgtgcc 1380

cgcagttttt attaaacata gcgtgggatc tccacgcgaa tctcgggtac gtgttccgga 1440

catgggctct tctccggtag cggcggagct tccacatccg agccctggtc ccatgcctcc 1500

agcggctcat ggtcgctcgg cagctccttg ctcctaacag tggaggccag acttaggcac 1560

agcacaatgc ccaccaccac cagtgtgccg cacaaggccg tggcggtagg gtatgtgtct 1620

gaaaatgagc gtggagattg ggctcgcacg gctgacgcag atggaagact taaggcagcg 1680

gcagaagaag atgcaggcag ctgagttgtt gtattctgat aagagtcaga ggtaactccc 1740

gttgcggtgc tgttaacggt ggagggcagt gtagtctgag cagtactcgt tgctgccgcg 1800

cgcgccacca gacataatag ctgacagact aacagactgt tcctttccat gggtcttttc 1860

tgcagatggc ctcgcccttt gctttactga tggtcctggt ggtgctcagc tgcaagtcaa 1920

gctgctctct gggctgtgat ctccctgaga cccacagcct ggataacagg aggaccttga 1980

tgctcctggc acaaatgagc agaatctctc cttcctcctg tctgatggac agacatgact 2040

ttggatttcc ccaggaggag tttgatggca accagttcca gaaggctcca gccatctctg 2100

tcctccatga gctgatccag cagatcttca acctctttac cacaaaagat tcatctgctg 2160

cttgggatga ggacctccta gacaaattct gcaccgaact ctaccagcag ctgaatgact 2220

tggaagcctg tgtgatgcag gaggagaggg tgggagaaac tcccctgatg aatgcggact 2280

ccatcttggc tgtgaagaaa tacttccgaa gaatcactct ctatctgaca gagaagaaat 2340

acagcccttg tgcctgggag gttgtcagag cagaaatcat gagatccctc tctttatcaa 2400

caaacttgca agaaagatta aggaggaagg aataaggatc cagatctgct gtgccttcta 2460

gttgccagcc atctgttgtt tgcccctccc ccgtgccttc cttgaccctg gaaggtgcca 2520

ctcccactgt cctttcctaa taaaatgagg aaattgcatc gcattgtctg agtaggtgtc 2580

attctattct ggggggtggg gtggggcagc acagcaaggg ggaggattgg gaagacaata 2640

gcaggcatgc tggggatgcg gtgggctcta tgggtaccca ggtgctgaag aattgacccg 2700

gttcctcctg ggccagaaag aagcaggcac atccccttct ctgtgacaca ccctgtccac 2760

gcccctggtt cttagttcca gccccactca taggacactc atagctcagg agggctccgc 2820

cttcaatccc acccgctaaa gtacttggag cggtctctcc ctccctcatc agcccaccaa 2880

accaaaccta gcctccaaga gtgggaagaa attaaagcaa gataggctat taagtgcaga 2940

gggagagaaa atgcctccaa catgtgagga agtaatgaga gaaatcatag aatttcttcc 3000

gcttcctcgc tcactgactc gctgcgctcg gtcgttcggc tgcggcgagc ggtatcagct 3060

cactcaaagg cggtaatacg gttatccaca gaatcagggg ataacgcagg aaagaacatg 3120

tgagcaaaag gccagcaaaa ggccaggaac cgtaaaaagg ccgcgttgct ggcgtttttc 3180

cataggctcc gcccccctga cgagcatcac aaaaatcgac gctcaagtca gaggtggcga 3240

aacccgacag gactataaag ataccaggcg tttccccctg gaagctccct cgtgcgctct 3300

cctgttccga ccctgccgct taccggatac ctgtccgcct ttctcccttc gggaagcgtg 3360

gcgctttctc aatgctcacg ctgtaggtat ctcagttcgg tgtaggtcgt tcgctccaag 3420

ctgggctgtg tgcacgaacc ccccgttcag cccgaccgct gcgccttatc cggtaactat 3480

cgtcttgagt ccaacccggt aagacacgac ttatcgccac tggcagcagc cactggtaac 3540

aggattagca gagcgaggta tgtaggcggt gctacagagt tcttgaagtg gtggcctaac 3600

tacggctaca ctagaaggac agtatttggt atctgcgctc tgctgaagcc agttaccttc 3660

ggaaaaagag ttggtagctc ttgatccggc aaacaaacca ccgctggtag cggtggtttt 3720

tttgtttgca agcagcagat tacgcgcaga aaaaaaggat ctcaagaaga tcctttgatc 3780

ttttctacgg ggtctgacgc tcagtggaac gaaaactcac gttaagggat tttggtcatg 3840

agattatcaa aaaggatctt cacctagatc cttttaaatt aaaaatgaag ttttaaatca 3900

atctaaagta tatatgagta aacttggtct gacagttacc aatgcttaat cagtgaggca 3960

cctatctcag cgatctgtct atttcgttca tccatagttg cctgactccg gggggggggg 4020

gcgctgaggt ctgcctcgtg aagaaggtgt tgctgactca taccaggcct gaatcgcccc 4080

atcatccagc cagaaagtga gggagccacg gttgatgaga gctttgttgt aggtggacca 4140

gttggtgatt ttgaactttt gctttgccac ggaacggtct gcgttgtcgg gaagatgcgt 4200

gatctgatcc ttcaactcag caaaagttcg atttattcaa caaagccgcc gtcccgtcaa 4260

gtcagcgtaa tgctctgcca gtgttacaac caattaacca attctgatta gaaaaactca 4320

tcgagcatca aatgaaactg caatttattc atatcaggat tatcaatacc atatttttga 4380

aaaagccgtt tctgtaatga aggagaaaac tcaccgaggc agttccatag gatggcaaga 4440

tcctggtatc ggtctgcgat tccgactcgt ccaacatcaa tacaacctat taatttcccc 4500

tcgtcaaaaa taaggttatc aagtgagaaa tcaccatgag tgacgactga atccggtgag 4560

aatggcaaaa gcttatgcat ttctttccag acttgttcaa caggccagcc attacgctcg 4620

tcatcaaaat cactcgcatc aaccaaaccg ttattcattc gtgattgcgc ctgagcgaga 4680

cgaaatacgc gatcgctgtt aaaaggacaa ttacaaacag gaatcgaatg caaccggcgc 4740

aggaacactg ccagcgcatc aacaatattt tcacctgaat caggatattc ttctaatacc 4800

tggaatgctg ttttcccggg gatcgcagtg gtgagtaacc atgcatcatc aggagtacgg 4860

ataaaatgct tgatggtcgg aagaggcata aattccgtca gccagtttag tctgaccatc 4920

tcatctgtaa catcattggc aacgctacct ttgccatgtt tcagaaacaa ctctggcgca 4980

tcgggcttcc catacaatcg atagattgtc gcacctgatt gcccgacatt atcgcgagcc 5040

catttatacc catataaatc agcatccatg ttggaattta atcgcggcct cgagcaagac 5100

gtttcccgtt gaatatggct cataacaccc cttgtattac tgtttatgta agcagacagt 5160

tttattgttc atgatgatat atttttatct tgtgcaatgt aacatcagag attttgagac 5220

acaacgtggc tttccccccc cccccattat tgaagcattt atcagggtta ttgtctcatg 5280

agcggataca tatttgaatg tatttagaaa aataaacaaa taggggttcc gcgcacattt 5340

ccccgaaaag tgccacctga cgtctaagaa accattatta tcatgacatt aacctataaa 5400

aataggcgta tcacgaggcc ctttcgtc 5428

<210> 2
<211> 5259
<212> DNA
<213> Homo sapiens

<400> 2

tcgcgcgttt cggtgatgac ggtgaaaacc tctgacacat gcagctcccg gagacggtca 60

cagcttgtct gtaagcggat gccgggagca gacaagcccg tcagggcgcg tcagcgggtg 120

ttggcgggtg tcggggctgg cttaactatg cggcatcaga gcagattgta ctgagagtgc 180

accatatgcg gtgtgaaata ccgcacagat gcgtaaggag aaaataccgc atcagattgg 240

ctattggcca ttgcatacgt tgtatccata tcataatatg tacatttata ttggctcatg 300

tccaacatta ccgccatgtt gacattgatt attgactagt tattaatagt aatcaattac 360

ggggtcatta gttcatagcc catatatgga gttccgcgtt acataactta cggtaaatgg 420

cccgcctggc tgaccgccca acgacccccg cccattgacg tcaataatga cgtatgttcc 480

catagtaacg ccaataggga ctttccattg acgtcaatgg gtggagtatt tacggtaaac 540

tgcccacttg gcagtacatc aagtgtatca tatgccaagt acgcccccta ttgacgtcaa 600

tgacggtaaa tggcccgcct ggcattatgc ccagtacatg accttatggg actttcctac 660

ttggcagtac atctacgtat tagtcatcgc tattaccatg gtgatgcggt tttggcagta 720

catcaatggg cgtggatagc ggtttgactc acggggattt ccaagtctcc accccattga 780

cgtcaatggg agtttgtttt ggcaccaaaa tcaacgggac tttccaaaat gtcgtaacaa 840

ctccgcccca ttgacgcaaa tgggcggtag gcgtgtacgg tgggaggtct atataagcag 900

agctcgttta gtgaaccgtc agatcgcctg gagacgccat ccacgctgtt ttgacctcca 960
tagaagacac cgggaccgat ccagcctccg cggccgggaa cggtgcattg gaacgcggat 1020
tccccgtgcc aagagtgacg taagtaccgc ctatagactc tataggcaca cccctttggc 1080
tcttatgcat gctatactgt ttttggcttg gggcctatac acccccgctt ccttatgcta 1140
taggtgatgg tatagcttag cctataggtg tgggttattg accattattg accactcccc 1200
tattggtgac gatactttcc attactaatc cataacatgg ctctttgcca caactatctc 1260
tattggctat atgccaatac tctgtccttc agagactgac acggactctg tatttttaca 1320
ggatggggtc ccatttatta tttacaaatt cacatataca acaacgccgt cccccgtgcc 1380
cgcagttttt attaaacata gcgtgggatc tccacgcgaa tctcgggtac gtgttccgga 1440
catgggctct tctccggtag cggcggagct tccacatccg agccctggtc ccatgcctcc 1500
agcggctcat ggtcgctcgg cagctccttg ctcctaacag tggaggccag acttaggcac 1560
agcacaatgc ccaccaccac cagtgtgccg cacaaggccg tggcggtagg gtatgtgtct 1620
gaaaatgagc gtggagattg ggctcgcacg gctgacgcag atggaagact taaggcagcg 1680
gcagaagaag atgcaggcag ctgagttgtt gtattctgat aagagtcaga ggtaactccc 1740
gttgcggtgc tgttaacggt ggagggcagt gtagtctgag cagtactcgt tgctgccgcg 1800
cgcgccacca gacataatag ctgacagact aacagactgt tcctttccat gggtcttttc 1860
tgcagatggc ctcgcccttt gctttactga tggtcctggt ggtgctcagc tgcaagtcaa 1920
gctgctctct gggctgtgat ctccctgaga cccacagcct ggataacagg aggaccttga 1980
tgctcctggc acaaatgagc agaatctctc cttcctcctg tctgatggac agacatgact 2040
ttggatttcc ccaggaggag tttgatggca accagttcca gaaggctcca gccatctctg 2100
tcctccatga gctgatccag cagatcttca acctctttac cacaaaagat tcatctgctg 2160
cttgggatga ggacctccta gacaaattct gcaccgaact ctaccagcag ctgaatgact 2220
tggaagcctg tgtgatgcag gaggagaggg tgggagaaac tcccctgatg aatgcggact 2280
ccatcttggc tgtgaagaaa tacttccgaa gaatcactct ctatctgaca gagaagaaat 2340
acagcccttg tgcctgggag gttgtcagag cagaaatcat gagatccctc tctttatcaa 2400
caaacttgca agaaagatta aggaggaagg aataaggatc cagatctact tctggctaat 2460

aaaagatcag agctctagag atctgtgtgt tggttttttg tgtggtaccc aggtgctgaa 2520

gaattgaccc ggttcctcct gggccagaaa gaagcaggca catccccttc tctgtgacac 2580

accctgtcca cgcccctggt tcttagttcc agccccactc ataggacact catagctcag 2640

gagggctccg ccttcaatcc cacccgctaa agtacttgga gcggtctctc cctccctcat 2700

cagcccacca aaccaaacct agcctccaag agtgggaaga aattaaagca agataggcta 2760

ttaagtgcag agggagagaa aatgcctcca acatgtgagg aagtaatgag agaaatcata 2820

gaatttcttc cgcttcctcg ctcactgact cgctgcgctc ggtcgttcgg ctgcggcgag 2880

cggtatcagc tcactcaaag gcggtaatac ggttatccac agaatcaggg gataacgcag 2940

gaaagaacat gtgagcaaaa ggccagcaaa aggccaggaa ccgtaaaaag gccgcgttgc 3000

tggcgttttt ccataggctc cgcccccctg acgagcatca caaaaatcga cgctcaagtc 3060

agaggtggcg aaacccgaca ggactataaa gataccaggc gtttccccct ggaagctccc 3120

tcgtgcgctc tcctgttccg accctgccgc ttaccggata cctgtccgcc tttctccctt 3180

cgggaagcgt ggcgctttct caatgctcac gctgtaggta tctcagttcg gtgtaggtcg 3240

ttcgctccaa gctgggctgt gtgcacgaac cccccgttca gcccgaccgc tgcgccttat 3300

ccggtaacta tcgtcttgag tccaacccgg taagacacga cttatcgcca ctggcagcag 3360

ccactggtaa caggattagc agagcgaggt atgtaggcgg tgctacagag ttcttgaagt 3420

ggtggcctaa ctacggctac actagaagga cagtatttgg tatctgcgct ctgctgaagc 3480

cagttacctt cggaaaaaga gttggtagct cttgatccgg caaacaaacc accgctggta 3540

gcggtggttt ttttgtttgc aagcagcaga ttacgcgcag aaaaaaagga tctcaagaag 3600

atcctttgat cttttctacg gggtctgacg ctcagtggaa cgaaaactca cgttaaggga 3660

ttttggtcat gagattatca aaaaggatct tcacctagat ccttttaaat taaaaatgaa 3720

gttttaaatc aatctaaagt atatatgagt aaacttggtc tgacagttac caatgcttaa 3780

tcagtgaggc acctatctca gcgatctgtc tatttcgttc atccatagtt gcctgactcc 3840

gggggggggg ggcgctgagg tctgcctcgt gaagaaggtg ttgctgactc ataccaggcc 3900

tgaatcgccc catcatccag ccagaaagtg agggagccac ggttgatgag agctttgttg 3960

taggtggacc agttggtgat tttgaacttt tgctttgcca cggaacggtc tgcgttgtcg 4020

```
ggaagatgcg tgatctgatc cttcaactca gcaaaagttc gatttattca acaaagccgc 4080
cgtcccgtca agtcagcgta atgctctgcc agtgttacaa ccaattaacc aattctgatt 4140
agaaaaactc atcgagcatc aaatgaaact gcaatttatt catatcagga ttatcaatac 4200
catatttttg aaaaagccgt ttctgtaatg aaggagaaaa ctcaccgagg cagttccata 4260
ggatggcaag atcctggtat cggtctgcga ttccgactcg tccaacatca atacaaccta 4320
ttaatttccc ctcgtcaaaa ataaggttat caagtgagaa atcaccatga gtgacgactg 4380
aatccggtga gaatggcaaa agcttatgca tttctttcca gacttgttca acaggccagc 4440
cattacgctc gtcatcaaaa tcactcgcat caaccaaacc gttattcatt cgtgattgcg 4500
cctgagcgag acgaaatacg cgatcgctgt taaaaggaca attacaaaca ggaatcgaat 4560
gcaaccggcg caggaacact gccagcgcat caacaatatt ttcacctgaa tcaggatatt 4620
cttctaatac ctggaatgct gttttcccgg ggatcgcagt ggtgagtaac catgcatcat 4680
caggagtacg gataaaatgc ttgatggtcg gaagaggcat aaattccgtc agccagttta 4740
gtctgaccat ctcatctgta acatcattgg caacgctacc tttgccatgt ttcagaaaca 4800
actctggcgc atcgggcttc ccatacaatc gatagattgt cgcacctgat tgcccgacat 4860
tatcgcgagc ccatttatac ccatataaat cagcatccat gttggaattt aatcgcggcc 4920
tcgagcaaga cgtttcccgt tgaatatggc tcataacacc ccttgtatta ctgtttatgt 4980
aagcagacag ttttattgtt catgatgata tatttttatc ttgtgcaatg taacatcaga 5040
gattttgaga cacaacgtgg ctttcccccc ccccccatta ttgaagcatt tatcagggtt 5100
attgtctcat gagcggatac atatttgaat gtatttagaa aaataaacaa ataggggttc 5160
cgcgcacatt tccccgaaaa gtgccacctg acgtctaaga aaccattatt atcatgacat 5220
taacctataa aaataggcgt atcacgaggc cctttcgtc                        5259
```

<210> 3
<211> 5480
<212> DNA
<213> Homo sapiens

<400> 3

```
tcgcgcgttt cggtgatgac ggtgaaaacc tctgacacat gcagctcccg gagacggtca 60
cagcttgtct gtaagcggat gccgggagca gacaagcccg tcagggcgcg tcagcgggtg 120
```

ttggcgggtg tcggggctgg cttaactatg cggcatcaga gcagattgta ctgagagtgc 180

accatatgcg gtgtgaaata ccgcacagat gcgtaaggag aaaataccgc atcagattgg 240

ctattggcca ttgcatacgt tgtatccata tcataatatg tacatttata ttggctcatg 300

tccaacatta ccgccatgtt gacattgatt attgactagt tattaatagt aatcaattac 360

ggggtcatta gttcatagcc catatatgga gttccgcgtt acataactta cggtaaatgg 420

cccgcctggc tgaccgccca acgacccccg cccattgacg tcaataatga cgtatgttcc 480

catagtaacg ccaataggga ctttccattg acgtcaatgg gtggagtatt tacggtaaac 540

tgcccacttg gcagtacatc aagtgtatca tatgccaagt acgcccccta ttgacgtcaa 600

tgacggtaaa tggcccgcct ggcattatgc ccagtacatg accttatggg actttcctac 660

ttggcagtac atctacgtat tagtcatcgc tattaccatg gtgatgcggt tttggcagta 720

catcaatggg cgtggatagc ggtttgactc acggggattt ccaagtctcc accccattga 780

cgtcaatggg agtttgtttt ggcaccaaaa tcaacgggac tttccaaaat gtcgtaacaa 840

ctccgcccca ttgacgcaaa tgggcggtag gcgtgtacgg tgggaggtct atataagcag 900

agctcgttta gtgaaccgtc agatcgcctg gagacgccat ccacgctgtt ttgacctcca 960

tagaagacac cgggaccgat ccagcctccg cggccgggaa cggtgcattg gaacgcggat 1020

tccccgtgcc aagagtgacg taagtaccgc ctatagactc tataggcaca cccctttggc 1080

tcttatgcat gctatactgt ttttggcttg gggcctatac acccccgctt ccttatgcta 1140

taggtgatgg tatagcttag cctataggtg tgggttattg accattattg accactcccc 1200

tattggtgac gatactttcc attactaatc cataacatgg ctctttgcca caactatctc 1260

tattggctat atgccaatac tctgtccttc agagactgac acggactctg tatttttaca 1320

ggatgggtc ccatttatta tttacaaatt cacatataca acaacgccgt cccccgtgcc 1380

cgcagttttt attaaacata gcgtgggatc tccacgcgaa tctcgggtac gtgttccgga 1440

catgggctct tctccggtag cggcggagct tccacatccg agccctgctc ccatgcctac 1500

agcggctcat ggtcgctcgg cagctccttg ctcctaacag tggaggccag acttaggcac 1560

agcacaatgc ccaccaccac cagtgtgccg cacaaggccg tggcggtagg gtatgtgtct 1620

gaaaatgagc gtggagattg ggctcgcacg gctgacgcag atggaagact taaggcagcg 1680

gcagaagaag atgcaggcag ctgagttgtt gtattctgat aagagtcaga ggtaactccc 1740

gttgcggtgc tgttaacggt ggagggcagt gtagtctgag cagtactcgt tgctgccgcg 1800

cgcgccacca gacataatag ctgacagact aacagactgt tcctttccat gggtcttttc 1860

tgcagtcacc gtcgtcgaca cgtgtgatca gatatcacca tggccctcct gttccctcta 1920

ctggcagccc tagtgatgac cagctatagc cctgttggat ctctggcctg tgatctgcct 1980

cagaaccatg gcctacttag caggaacacc ttggtgcttc tgcaccaaat gaggagaatc 2040

tcccctttct tgtgtctcaa ggacagaaga gacttcaggt tcccccagga gatggtaaaa 2100

gggagccagt tgcagaaggc ccatgtcatg tctgtcctcc atgagatgct gcagcagatc 2160

ttcagcctct tccacacaga gcgctcctct gctgcctgga acatgaccct cctagaccaa 2220

ctccacactg gacttcatca gcaactgcaa cacctggaga cctgcttgct gcaggtagtg 2280

ggagaaggag aatctgctgg ggcaattagc agccctgcac tgaccttgag gaggtacttc 2340

cagggaatcc gtgtctacct gaaagagaag aaatacagcg actgtgcctg ggaagttgtc 2400

agaatggaaa tcatgaaatc cttgttctta tcaacaaaca tgcaagaaag actgagaagt 2460

aaagatagag acctgggctc atcttgagga tccagatctg ctgtgccttc tagttgccag 2520

ccatctgttg tttgcccctc ccccgtgcct tccttgaccc tggaaggtgc cactcccact 2580

gtcctttcct aataaaatga ggaaattgca tcgcattgtc tgagtaggtg tcattctatt 2640

ctggggggtg gggtggggca gcacagcaag ggggaggatt gggaagacaa tagcaggcat 2700

gctggggatg cggtgggctc tatgggtacc caggtgctga agaattgacc cggttcctcc 2760

tgggccagaa agaagcaggc acatcccctt ctctgtgaca caccctgtcc acgcccctgg 2820

ttcttagttc cagccccact cataggacac tcatagctca ggagggctcc gccttcaatc 2880

ccacccgcta aagtacttgg agcggtctct ccctccctca tcagcccacc aaaccaaacc 2940

tagcctccaa gagtgggaag aaattaaagc aagataggct attaagtgca gagggagaga 3000

aaatgcctcc aacatgtgag gaagtaatga gagaaatcat agaatttctt ccgcttcctc 3060

gctcactgac tcgctgcgct cggtcgttcg gctgcggcga gcggtatcag ctcactcaaa 3120

ggcggtaata cggttatcca cagaatcagg ggataacgca ggaaagaaca tgtgagcaaa 3180

aggccagcaa aaggccagga accgtaaaaa ggccgcgttg ctggcgtttt tccataggct 3240

ccgccccccc gacgagcatc acaaaaatcg acgctcaagt cagaggtggc gaaacccgac 3300
aggactataa agataccagg cgtttccccc tggaagctcc ctcgtgcgct ctcctgttcc 3360
gaccctgccg cttaccggat acctgtccgc ctttctccct tcgggaagcg tggcgctttc 3420
tcaatgctca cgctgtaggt atctcagttc ggtgtaggtc gttcgctcca agctgggctg 3480
tgtgcacgaa ccccccgttc agcccgaccg ctgcgcctta tccggtaact atcgtcttga 3540
gtccaacccg gtaagacacg acttatcgcc actggcagca gccactggta acaggattag 3600
cagagcgagg tatgtaggcg gtgctacaga gttcttgaag tggtggccta actacggcta 3660
cactagaagg acagtatttg gtatctgcgc tctgctgaag ccagttacct tcggaaaaag 3720
agttggtagc tcttgatccg gcaaacaaac caccgctggt agcggtggtt tttttgtttg 3780
caagcagcag attacgcgca gaaaaaaagg atctcaagaa gatcctttga tcttttctac 3840
ggggtctgac gctcagtgga acgaaaactc acgttaaggg attttggtca tgagattatc 3900
aaaaaggatc ttcacctaga tccttttaaa ttaaaaatga agttttaaat caatctaaag 3960
tatatatgag taaacttggt ctgacagtta ccaatgctta atcagtgagg cacctatctc 4020
agcgatctgt ctatttcgtt catccatagt tgcctgactc cggggggggg gggcgctgag 4080
gtctgcctcg tgaagaaggt gttgctgact cataccaggc ctgaatcgcc ccatcatcca 4140
gccagaaagt gagggagcca cggttgatga gagctttgtt gtaggtggac cagttggtga 4200
ttttgaactt ttgctttgcc acggaacggt ctgcgttgtc gggaagatgc gtgatctgat 4260
ccttcaactc agcaaaagtt cgatttattc aacaaagccg ccgtcccgtc aagtcagcgt 4320
aatgctctgc cagtgttaca accaattaac caattctgat tagaaaaact catcgagcat 4380
caaatgaaac tgcaatttat tcatatcagg attatcaata ccatattttt gaaaaagccg 4440
tttctgtaat gaaggagaaa actcaccgag gcagttccat aggatggcaa gatcctggta 4500
tcggtctgcg attccgactc gtccaacatc aatacaacct attaatttcc cctcgtcaaa 4560
aataaggtta tcaagtgaga aatcaccatg agtgacgact gaatccggtg agaatggcaa 4620
aagcttatgc atttctttcc agacttgttc aacaggccag ccattacgct cgtcatcaaa 4680
atcactcgca tcaaccaaac cgttattcat tcgtgattgc gcctgagcga gacgaaatac 4740
gcgatcgctg ttaaaaggac aattacaaac aggaatcgaa tgcaaccggc gcaggaacac 4800

tgccagcgca tcaacaatat tttcacctga atcaggatat tcttctaata cctggaatgc 4860

tgttttcccg gggatcgcag tggtgagtaa ccatgcatca tcaggagtac ggataaaatg 4920

cttgatggtc ggaagaggca taaattccgt cagccagttt agtctgacca tctcatctgt 4980

aacatcattg gcaacgctac ctttgccatg tttcagaaac aactctggcg catcgggctt 5040

cccatacaat cgatagattg tcgcacctga ttgcccgaca ttatcgcgag cccatttata 5100

cccatataaa tcagcatcca tgttggaatt taatcgcggc ctcgagcaag acgtttcccg 5160

ttgaatatgg ctcataacac cccttgtatt actgtttatg taagcagaca gttttattgt 5220

tcatgatgat atatttttat cttgtgcaat gtaacatcag agattttgag acacaacgtg 5280

gctttccccc cccccccatt attgaagcat ttatcagggt tattgtctca tgagcggata 5340

catatttgaa tgtatttaga aaaataaaca aataggggtt ccgcgcacat ttccccgaaa 5400

agtgccacct gacgtctaag aaaccattat tatcatgaca ttaacctata aaaataggcg 5460

tatcacgagg ccctttcgtc 5480

<210> 4
<211> 5322
<212> DNA
<213> Homo sapiens

<400> 4

tcgcgcgttt cggtgatgac ggtgaaaacc tctgacacat gcagctcccg gagacggtca 60

cagcttgtct gtaagcggat gccgggagca gacaagcccg tcagggcgcg tcagcgggtg 120

ttggcgggtg tcggggctgg cttaactatg cggcatcaga gcagattgta ctgagagtgc 180

accatatgcg gtgtgaaata ccgcacagat gcgtaaggag aaaataccgc atcagattgg 240

ctattggcca ttgcatacgt tgtatccata tcataatatg tacatttata ttggctcatg 300

tccaacatta ccgccatgtt gacattgatt attgactagt tattaatagt aatcaattac 360

ggggtcatta gttcatagcc catatatgga gttccgcgtt acataactta cggtaaatgg 420

cccgcctggc tgaccgccca acgacccccg cccattgacg tcaataatga cgtatgttcc 480

catagtaacg ccaataggga ctttccattg acgtcaatgg gtggagtatt tacggtaaac 540

tgcccacttg gcagtacatc aagtgtatca tatgccaagt acgcccccta ttgacgtcaa 600

tgacggtaaa tggcccgcct ggcattatgc ccagtacatg accttatggg actttcctac 660

ttggcagtac atctacgtat tagtcatcgc tattaccatg gtgatgcggt tttggcagta 720

catcaatggg cgtggatagc ggtttgactc acggggattt ccaagtctcc accccattga 780

cgtcaatggg agtttgtttt ggcaccaaaa tcaacgggac tttccaaaat gtcgtaacaa 840

ctccgcccca ttgacgcaaa tgggcggtag gcgtgtacgg tgggaggtct atataagcag 900

agctcgttta gtgaaccgtc agatcgcctg gagacgccat ccacgctgtt ttgacctcca 960

tagaagacac cgggaccgat ccagcctccg cggccgggaa cggtgcattg gaacgcggat 1020

tccccgtgcc aagagtgacg taagtaccgc ctatagactc tataggcaca cccctttggc 1080

tcttatgcat gctatactgt ttttggcttg gggcctatac acccccgctt ccttatgcta 1140

taggtgatgg tatagcttag cctataggtg tgggttattg accattattg accactcccc 1200

tattggtgac gatactttcc attactaatc cataacatgg ctctttgcca caactatctc 1260

tattggctat atgccaatac tctgtccttc agagactgac acggactctg tatttttaca 1320

ggatggggtc ccatttatta tttacaaatt cacatataca acaacgccgt cccccgtgcc 1380

cgcagttttt attaaacata gcgtgggatc tccacgcgaa tctcgggtac gtgttccgga 1440

catgggctct tctccggtag cggcggagct tccacatccg agccctggtc ccatgcctcc 1500

agcggctcat ggtcgctcgg cagctccttg ctcctaacag tggaggccag acttaggcac 1560

agcacaatgc ccaccaccac cagtgtgccg cacaaggccg tggcggtagg gtatgtgtct 1620

gaaaatgagc gtggagattg ggctcgcacg gctgacgcag atggaagact taaggcagcg 1680

gcagaagaag atgcaggcag ctgagttgtt gtattctgat aagagtcaga ggtaactccc 1740

gttgcggtgc tgttaacggt ggagggcagt gtagtctgag cagtactcgt tgctgccgcg 1800

cgcgccacca gacataatag ctgacagact aacagactgt tcctttccat gggtcttttc 1860

tgcagtcacc gtcgtcgaca cgtgtgatca gatatcgcgg ccgctctaga atggccctcc 1920

tgttccctct actggcagcc ctagtgatga ccagctatag ccctgttgga tctctgggct 1980

gtgatctgcc tcagaaccat ggcctactta gcaggaacac cttggtgctt ctgcaccaaa 2040

tgaggagaat ctcccctttc ttgtgtctca aggacagaag agacttcagg ttcccccagg 2100

agatggtaaa agggagccag ttgcagaagg cccatgtcat gtctgtcctc catgagatgc 2160

tgcagcagat cttcagcctc ttccacacag agcgctcctc tgctgcctgg aacatgaccc 2220

tcctagacca actccacact ggacttcatc agcaactgca acacctggag acctgcttgc 2280

tgcaggtagt gggagaagga gaatctgctg gggcaattag cagccctgca ctgaccttga 2340

ggaggtactt ccagggaatc cgtgtctacc tgaaagagaa gaaatacagc gactgtgcct 2400

gggaagttgt cagaatggaa atcatgaaat ccttgttctt atcaacaaac atgcaagaaa 2460

gactgagaag taaagataga gacctgggct catcttgagg atccagatct acttctggct 2520

aataaaagat cagagctcta gagatctgtg tgttggtttt ttgtgtggta cccaggtgct 2580

gaagaattga cccggttcct cctgggccag aaagaagcag gcacatcccc ttctctgtga 2640

cacaccctgt ccacgcccct ggttcttagt tccagcccca ctcataggac actcatagct 2700

caggagggct ccgccttcaa tcccacccgc taaagtactt ggagcggtct ctccctccct 2760

catcagccca ccaaaccaaa cctagcctcc aagagtggga agaaattaaa gcaagatagg 2820

ctattaagtg cagagggaga gaaaatgcct ccaacatgtg aggaagtaat gagagaaatc 2880

atagaatttc ttccgcttcc tcgctcactg actcgctgcg ctcggtcgtt cggctgcggc 2940

gagcggtatc agctcactca aaggcggtaa tacggttatc cacagaatca ggggataacg 3000

caggaaagaa catgtgagca aaaggccagc aaaaggccag gaaccgtaaa aaggccgcgt 3060

tgctggcgtt tttccatagg ctccgccccc ctgacgagca tcacaaaaat cgacgctcaa 3120

gtcagaggtg gcgaaacccg acaggactat aaagatacca ggcgtttccc cctggaagct 3180

ccctcgtgcg ctctcctgtt ccgaccctgc cgcttaccgg atacctgtcc gcctttctcc 3240

cttcgggaag cgtggcgctt tctcaatgct cacgctgtag gtatctcagt tcggtgtagg 3300

tcgttcgctc caagctgggc tgtgtgcacg aaccccccgt tcagcccgac cgctgcgcct 3360

tatccggtaa ctatcgtctt gagtccaacc cggtaagaca cgacttatcg ccactggcag 3420

cagccactgg taacaggatt agcagagcga ggtatgtagg cggtgctaca gagttcttga 3480

agtggtggcc taactacggc tacactagaa ggacagtatt tggtatctgc gctctgctga 3540

agccagttac cttcggaaaa agagttggta gctcttgatc cggcaaacaa accaccgctg 3600

gtagcggtgg tttttttgtt tgcaagcagc agattacgcg cagaaaaaaa ggatctcaag 3660

aagatccttt gatcttttct acggggtctg acgctcagtg gaacgaaaac tcacgttaag 3720

ggatcttggt catgagatta tcaaaaagga tcttcaccta gatcctttta aattaaaaat 3780

gaagttttaa atcaatctaa agtatatatg agtaaacttg gtctgacagt taccaatgct 3840

taatcagtga ggcacctatc tcagcgatct gtctatttcg ttcatccata gttgcctgac 3900

tccggggggg gggggcgctg aggtctgcct cgtgaagaag gtgttgctga ctcataccag 3960

gcctgaatcg ccccatcatc cagccagaaa gtgagggagc cacggttgat gagagctttg 4020

ttgtaggtgg accagttggt gattttgaac ttttgctttg ccacggaacg gtctgcgttg 4080

tcgggaagat gcgtgatctg atccttcaac tcagcaaaag ttcgatttat tcaacaaagc 4140

cgccgtcccg tcaagtcagc gtaatgctct gccagtgtta caaccaatta accaattctg 4200

attagaaaaa ctcatcgagc atcaaatgaa actgcaattt attcatatca ggattatcaa 4260

taccatattt ttgaaaaagc cgtttctgta atgaaggaga aaactcaccg aggcagttcc 4320

ataggatggc aagatcctgg tatcggtctg cgattccgac tcgtccaaca tcaatacaac 4380

ctattaattt cccctcgtca aaaataaggt tatcaagtga gaaatcacca tgagtgacga 4440

ctgaatccgg tgagaatggc aaaagcttat gcatttcttt ccagacttgt tcaacaggcc 4500

agccattacg ctcgtcatca aaatcactcg catcaaccaa accgttattc attcgtgatt 4560

gcgcctgagc gagacgaaat acgcgatcgc tgttaaaagg acaattacaa acaggaatcg 4620

aatgcaaccg gcgcaggaac actgccagcg catcaacaat attttcacct gaatcaggat 4680

attcttctaa tacctggaat gctgttttcc cggggatcgc agtggtgagt aaccatgcat 4740

catcaggagt acggataaaa tgcttgatgg tcggaagagg cataaattcc gtcagccagt 4800

ttagtctgac catctcatct gtaacatcat tggcaacgct acctttgcca tgtttcagaa 4860

acaactctgg cgcatcgggc ttcccataca atcgatagat tgtcgcacct gattgcccga 4920

cattatcgcg agcccattta tacccatata aatcagcatc catgttggaa tttaatcgcg 4980

gcctcgagca agacgtttcc cgttgaatat ggctcataac accccttgta ttactgttta 5040

tgtaagcaga cagttttatt gttcatgatg atatattttt atcttgtgca atgtaacatc 5100

agagattttg agacacaacg tggctttccc cccccccca ttattgaagc atttatcagg 5160

gttattgtct catgagcgga tacatatttg aatgtattta gaaaaataaa caaatagggg 5220

ttccgcgcac atttccccga aaagtgccac ctgacgtcta agaaaccatt attatcatga 5280

cattaaccta taaaaatagg cgtatcacga ggccctttcg tc 5322

<210> 5
<211> 5422
<212> DNA
<213> Mus musculus

<400> 5

tcgcgcgttt cggtgatgac ggtgaaaacc tctgacacat gcagctcccg gagacggtca 60
cagcttgtct gtaagcggat gccgggagca gacaagcccg tcagggcgcg tcagcgggtg 120
ttggcgggtg tcggggctgg cttaactatg cggcatcaga gcagattgta ctgagagtgc 180
accatatgcg gtgtgaaata ccgcacagat gcgtaaggag aaaataccgc atcagattgg 240
ctattggcca ttgcatacgt tgtatccata tcataatatg tacatttata ttggctcatg 300
tccaacatta ccgccatgtt gacattgatt attgactagt tattaatagt aatcaattac 360
ggggtcatta gttcatagcc catatatgga gttccgcgtt acataactta cggtaaatgg 420
cccgcctggc tgaccgccca acgacccccg cccattgacg tcaataatga cgtatgttcc 480
catagtaacg ccaataggga ctttccattg acgtcaatgg gtggagtatt tacggtaaac 540
tgcccacttg gcagtacatc aagtgtatca tatgccaagt acgcccccta ttgacgtcaa 600
tgacggtaaa tggcccgcct ggcattatgc ccagtacatg accttatggg actttcctac 660
ttggcagtac atctacgtat tagtcatcgc tattaccatg gtgatgcggt tttggcagta 720
catcaatggg cgtggatagc ggtttgactc acggggattt ccaagtctcc accccattga 780
cgtcaatggg agtttgtttt ggcaccaaaa tcaacgggac tttccaaaat gtcgtaacaa 840
ctccgcccca ttgacgcaaa tgggcggtag gcgtgtacgg tgggaggtct atataagcag 900
agctcgttta gtgaaccgtc agatcgcctg gagacgccat ccacgctgtt ttgacctcca 960
tagaagacac cgggaccgat ccagcctccg cggccgggaa cggtgcattg gaacgcggat 1020
tccccgtgcc aagagtgacg taagtaccgc ctatagactc tataggcaca cccctttggc 1080
tcttatgcat gctatactgt ttttggcttg gggcctatac acccccgctt ccttatgcta 1140
taggtgatgg tatagcttag cctataggtg tgggttattg accattattg accactcccc 1200
tattggtgac gatactttcc attactaatc cataacatgg ctctttgcca caactatctc 1260
tattggctat atgccaatac tctgtccttc agagactgac acggactctg tatttttaca 1320

ggatggggtc ccatttatta tttacaaatt cacatataca acaacgccgt cccccgtgcc 1380

cgcagttttt attaaacata gcgtgggatc tccacgcgaa tctcgggtac gtgttccgga 1440

catgggctct tctccggtag cggcggagct tccacatccg agccctggtc ccatgcctcc 1500

agcggctcat ggtcgctcgg cagctccttg ctcctaacag tggaggccag acttaggcac 1560

agcacaatgc ccaccaccac cagtgtgccg cacaaggccg tggcggtagg gtatgtgtct 1620

gaaaatgagc gtggagattg ggctcgcacg gctgacgcag atggaagact taaggcagcg 1680

gcagaagaag atgcaggcag ctgagttgtt gtattctgat aagagtcaga ggtaactccc 1740

gttgcggtgc tgttaacggt ggagggcagt gtagtctgag cagtactcgt tgctgccgcg 1800

cgcgccacca gacataatag ctgacagact aacagactgt tcctttccat gggtcttttc 1860

tgcagatggc taggctctgt gctttcctga tggtcctggc ggtgatgagc tactggccaa 1920

cctgctctct aggatgtgac ctgcctcaga ctcataacct caggaacaag agagccttga 1980

cactcctggt acaaatgagg agactctccc ctctctcctg cctgaaggac aggaaggact 2040

ttggattccc gcaggagaag gtggatgccc agcagatcaa gaaggctcaa gccatccctg 2100

tcctgagtga gctgacccag cagatcctga acatcttcac atcaaaggac tcatctgctg 2160

cttggaatgc aaccctccta gactcattct gcaatgacct ccaccagcag ctcaatgacc 2220

tgcaaggttg tctgatgcag caggtggggg tgcaggaatt tcccctgacc caggaagatg 2280

ccctgctggc tgtgaggaaa tacttccaca ggatcactgt gtacctgaga gagaagaaac 2340

acagcccctg tgcctgggag gtggtcagag cagaagtctg gagagccctg tcttcctctg 2400

ccaatgtgct gggaagactg agagaagaga aatgaagatc tgctgtgcct tctagttgcc 2460

agccatctgt tgtttgcccc tcccccgtgc cttccttgac cctggaaggt gccactccca 2520

ctgtcctttc ctaataaaat gaggaaattg catcgcattg tctgagtagg tgtcattcta 2580

ttctgggggg tggggtgggg cagcacagca agggggagga ttgggaagac aatagcaggc 2640

atgctgggga tgcggtgggc tctatgggta cccaggtgct gaagaattga cccggttcct 2700

cctgggccag aaagaagcag gcacatcccc ttctctgtga cacaccctgt ccacgcccct 2760

ggttcttagt tccagcccca ctcataggac actcatagct caggagggct ccgccttcaa 2820

tcccacccgc taaagtactt ggagcggtct ctccctccct catcagccca ccaaaccaaa 2880

cctagcctcc aagagtggga agaaattaaa gcaagatagg ctattaagtg cagagggaga 2940

gaaaatgcct ccaacatgtg aggaagtaat gagagaaatc atagaatttc ttccgcttcc 3000

tcgctcactg actcgctgcg ctcggtcgtt cggctgcggc gagcggtatc agctcactca 3060

aaggcggtaa tacggttatc cacagaatca ggggataacg caggaaagaa catgtgagca 3120

aaaggccagc aaaaggccag gaaccgtaaa aaggccgcgt tgctggcgtt tttccatagg 3180

ctccgccccc ctgacgagca tcacaaaaat cgacgctcaa gtcagaggtg gcgaaacccg 3240

acaggactat aaagatacca ggcgtttccc cctggaagct ccctcgtgcg ctctcctgtt 3300

ccgaccctgc cgcttaccgg atacctgtcc gcctttctcc cttcgggaag cgtggcgctt 3360

tctcaatgct cacgctgtag gtatctcagt tcggtgtagg tcgttcgctc caagctgggc 3420

tgtgtgcacg aaccccccgt tcagcccgac cgctgcgcct tatccggtaa ctatcgtctt 3480

gagtccaacc cggtaagaca cgacttatcg ccactggcag cagccactgg taacaggatt 3540

agcagagcga ggtatgtagg cggtgctaca gagttcttga agtggtggcc taactacggc 3600

tacactagaa ggacagtatt tggtatctgc gctctgctga agccagttac cttcggaaaa 3660

agagttggta gctcttgatc cggcaaacaa accaccgctg gtagcggtgg tttttttgtt 3720

tgcaagcagc agattacgcg cagaaaaaaa ggatctcaag aagatccttt gatcttttct 3780

acggggtctg acgctcagtg gaacgaaaac tcacgttaag ggattttggt catgagatta 3840

tcaaaaagga tcttcaccta gatcctttta aattaaaaat gaagttttaa atcaatctaa 3900

agtatatatg agtaaacttg gtctgacagt taccaatgct taatcagtga ggcacctatc 3960

tcagcgatct gtctatttcg ttcatccata gttgcctgac tccggggggg gggggcgctg 4020

aggtctgcct cgtgaagaag gtgttgctga ctcataccag gcctgaatcg ccccatcatc 4080

cagccagaaa gtgagggagc cacggttgat gagagctttg ttgtaggtgg accagttggt 4140

gattttgaac ttttgctttg ccacggaacg gtctgcgttg tcgggaagat gcgtgatctg 4200

atccttcaac tcagcaaaag ttcgatttat tcaacaaagc cgccgtcccg tcaagtcagc 4260

gtaatgctct gccagtgtta caaccaatta accaattctg attagaaaaa ctcatcgagc 4320

atcaaatgaa actgcaattt attcatatca ggattatcaa taccatattt ttgaaaaagc 4380

cgtttctgta atgaaggaga aaactcaccg aggcagttcc ataggatggc aagatcctgg 4440

tatcggtctg cgattccgac tcgtccaaca tcaatacaac ctattaattt cccctcgtca 4500

aaaataaggt tatcaagtga gaaatcacca tgagtgacga ctgaatccgg tgagaatggc 4560

aaaagcttat gcatttcttt ccagacttgt tcaacaggcc agccattacg ctcgtcatca 4620

aaatcactcg catcaaccaa accgttattc attcgtgatt gcgcctgagc gagacgaaat 4680

acgcgatcgc tgttaaaagg acaattacaa acaggaatcg aatgcaaccg gcgcaggaac 4740

actgccagcg catcaacaat attttcacct gaatcaggat attcttctaa tacctggaat 4800

gctgttttcc cggggatcgc agtggtgagt aaccatgcat catcaggagt acggataaaa 4860

tgcttgatgg tcggaagagg cataaattcc gtcagccagt ttagtctgac catctcatct 4920

gtaacatcat tggcaacgct acctttgcca tgtttcagaa acaactctgg cgcatcgggc 4980

ttcccataca atcgatagat tgtcgcacct gattgcccga cattatcgcg agcccattta 5040

tacccatata aatcagcatc catgttggaa tttaatcgcg gcctcgagca agacgtttcc 5100

cgttgaatat ggctcataac accccttgta ttactgttta tgtaagcaga cagttttatt 5160

gttcatgatg atatattttt atcttgtgca atgtaacatc agagattttg agacacaacg 5220

tggctttccc cccccccca ttattgaagc atttatcagg gttattgtct catgagcgga 5280

tacatatttg aatgtattta gaaaaataaa caaatagggg ttccgcgcac atttccccga 5340

aaagtgccac ctgacgtcta agaaaccatt attatcatga cattaaccta taaaaatagg 5400

cgtatcacga ggccctttcg tc 5422

<210> 6
<211> 5259
<212> DNA
<213> Mus musculus

<400> 6

tcgcgcgttt cggtgatgac ggtgaaaacc tctgacacat gcagctcccg gagacggtca 60

cagcttgtct gtaagcggat gccgggagca gacaagcccg tcagggcgcg tcagcgggtg 120

ttggcgggtg tcggggctgg cttaactatg cggcatcaga gcagattgta ctgagagtgc 180

accatatgcg gtgtgaaata ccgcacagat gcgtaaggag aaaataccgc atcagattgg 240

ctattggcca ttgcatacgt tgtatccata tcataatatg tacatttata ttggctcatg 300

tccaacatta ccgccatgtt gacattgatt attgactagt tattaatagt aatcaattac 360
ggggtcatta gttcatagcc catatatgga gttccgcgtt acataactta cggtaaatgg 420
cccgcctggc tgaccgccca acgacccccg cccattgacg tcaataatga cgtatgttcc 480
catagtaacg ccaataggga ctttccattg acgtcaatgg gtggagtatt tacggtaaac 540
tgcccacttg gcagtacatc aagtgtatca tatgccaagt acgcccccta ttgacgtcaa 600
tgacggtaaa tggcccgcct ggcattatgc ccagtacatg accttatggg actttcctac 660
ttggcagtac atctacgtat tagtcatcgc tattaccatg gtgatgcggt tttggcagta 720
catcaatggg cgtggatagc ggtttgactc acggggattt ccaagtctcc accccattga 780
cgtcaatggg agtttgtttt ggcaccaaaa tcaacgggac tttccaaaat gtcgtaacaa 840
ctccgcccca ttgacgcaaa tgggcggtag gcgtgtacgg tgggaggtct atataagcag 900
agctcgttta gtgaaccgtc agatcgcctg gagacgccat ccacgctgtt ttgacctcca 960
tagaagacac cgggaccgat ccagcctccg cggccgggaa cggtgcattg gaacgcggat 1020
tccccgtgcc aagagtgacg taagtaccgc ctatagactc tataggcaca cccctttggc 1080
tcttatgcat gctatactgt ttttggcttg gggcctatac acccccgctt ccttatgcta 1140
taggtgatgg tatagcttag cctataggtg tgggttattg accattattg accactcccc 1200
tattggtgac gatactttcc attactaatc cataacatgg ctctttgcca caactatctc 1260
tattggctat atgccaatac tctgtccttc agagactgac acggactctg tatttttaca 1320
ggatggggtc ccatttatta tttacaaatt cacatataca acaacgccgt cccccgtgcc 1380
cgcagttttt attaaacata gcgtgggatc tccacgcgaa tctcgggtac gtgttccgga 1440
catgggctct tctccggtag cggcggagct tccacatccg agccctggtc ccatgcctcc 1500
agcggctcat ggtcgctcgg cagctccttg ctcctaacag tggaggccag acttaggcac 1560
agcacaatgc ccaccaccac cagtgtgccg cacaaggccg tggcggtagg gtatgtgtct 1620
gaaaatgagc gtggagattg ggctcgcacg gctgacgcag atggaagact taaggcagcg 1680
gcagaagaag atgcaggcag ctgagttgtt gtattctgat aagagtcaga ggtaactccc 1740
gttgcggtgc tgttaacggt ggagggcagt gtagtctgag cagtactcgt tgctgccgcg 1800
cgcgccacca gacataatag ctgacagact aacagactgt tcctttccat gggtcttttc 1860

tgcagatggc taggctctgt gctttcctga tggtcctggc ggtgatgagc tactggccaa 1920

cctgtctcct aggatgtgac ctgcctcaga ctcataacct caggaacaag agagccttga 1980

cactccaggt acaaatgagg agactctccc ctctctcctg cctgaaggac aggaaggact 2040

ttggatcccc gcaggagaag gtggatgccc agcagatcaa gaaggctcaa gccatccctg 2100

tcctgagtga gctgacccag cagatcctga acatcttcac atcaaaggac tcatctgctg 2160

cttggaatgc aaccctccta gactcattct gcaatgacct ccaccagcag ctcaatgacc 2220

tgcaaggttg tctgatgcag caggtggggg tgcaggaatt tcccctgacc caggaagatg 2280

ccctgcaggc tgtgaggaaa tacttccaca ggatcactgt gtacctgaga gagaagaaac 2340

acagcccctg tgcctgggag gtggtcagag cagaagtctg gagagccctg tcttcctctg 2400

ccaatgagct gggaagactg agagaagaga aatgaagatc cagatctact tctggctaat 2460

aaaagatcag agctctagag atctgtgtgt tggttttttg tgtggtaccc aggtgctgaa 2520

gaattgaccc ggttcctcct gggccagaaa gaagcaggca catccccttc tctgtgacac 2580

accctgtcca cgcccctggt tcttagttcc agccccactc ataggacact catagctcag 2640

gagggctccg ccttcaatcc cacccgctaa agtacttgga gcggtctctc cctccctcat 2700

cagcccacca aaccaaacct agcctccaag agtgggaaga aattaaagca agataggcta 2760

ttaagtgcag agggagagaa aatgcctcca acatgtgagg aagtaatcag agaaatcata 2820

gaatttcttc cgcttcctcg ctcactgact cgctgcgctc ggtcgttcgg ctgcggcgag 2880

cggtatcagc tcactcaaag gcggtaatac ggttatccac agaatcaggg gataacgcag 2940

gaaagaacat gtgagcaaaa ggccagcaaa aggccaggaa ccgtaaaaag gccgcgttgc 3000

tggcgttttt ccataggctc cgcccccctg acgagcatca caaaaatcga cgctcaagtc 3060

agaggtggcg aaacccgaca ggactataaa gataccaggc gtttccccct ggaagctccc 3120

tcgtgcgctc tcctgttccg accctgccgc ttaccggata cctgtccgcc tttctccctt 3180

cgggaagcgt ggcgctttct caatgctcac gctgtaggta tctcagttcg gtgtaggtcg 3240

ttcgctccaa gctgggctgt gtgcacgaac cccccgttca gcccgaccgc tgcgccttat 3300

ccggtaacta tcgtcttgag tccaacccgg taagacacga cttatcgcca ctggcagcag 3360

ccactggtaa caggattagc agagcgaggt atgtaggcgg tgctacagag ttcttgaagt 3420

ggtggcctaa ctacggctac actagaagga cagtatttgg tatctgcgct ctgctgaagc 3480
cagttacctt cggaaaaaga gttggtagct cttgatccgg caaacaaacc accgctggta 3540
gcggtggttt ttttgtttgc aagcagcaga ttacgcgcag aaaaaaagga tctcaagaag 3600
atcctttgat cttttctacg gggtctgacg ctcagtggaa cgaaaactca cgttaaggga 3660
ttttggtcat gagattatca aaaaggatct tcacctagat ccttttaaat taaaaatgaa 3720
gttttaaatc aatctaaagt atatatgagt aaacttggtc tgacagttac caatgcttaa 3780
tcagtgaggc acctatctca gcgatctgtc tatttcgttc atccatagtt gcctgactcc 3840
gggggggggg ggcgctgagg tctgcctcgt gaagaaggtg ttgctgactc ataccaggcc 3900
tgaatcgccc catcatccag ccagaaagtg agggagccac ggttgatgag agctttgttg 3960
taggtggacc agttggtgat tttgaacttt tgctttgcca cggaacggtc tgcgttgtcg 4020
ggaagatgcg tgatctgatc cttcaactca gcaaaagttc gatttattca acaaagccgc 4080
cgtcccgtca agtcagcgta atgctctgcc agtgttacaa ccaattaacc aattctgatt 4140
agaaaaactc atcgagcatc aaatgaaact gcaatttatt catatcagga ttatcaatac 4200
catatttttg aaaaagccgt ttctgtaatg aaggagaaaa ctcaccgagg cagttccata 4260
ggatggcaag atcctggtat cggtctgcga ttccgactcg tccaacatca atacaaccta 4320
ttaatttccc ctcgtcaaaa ataaggttat caagtgagaa atcaccatga gtgacgactg 4380
aatccggtga gaatggcaaa agcttatgca tttctttcca gacttgttca acaggccagc 4440
cattacgctc gtcatcaaaa tcactcgcat caaccaaacc gttattcatt cgtgattgcg 4500
cctgagcgag acgaaatacg cgatcgctgt taaaaggaca attacaaaca ggaatcgaat 4560
gcaaccggcg caggaacact gccagcgcat caacaatatt ttcacctgaa tcaggatatt 4620
cttctaatac ctggaatgct gttttcccgg ggatcgcagt ggtgagtaac catgcatcat 4680
caggagtacg gataaaatgc ttgatggtcg gaagaggcat aaattccgtc agccagttta 4740
gtctgaccat ctcatctgta acatcattgg caacgctacc tttgccatgt ttcagaaaca 4800
actctggcgc atcgggcttc ccatacaatc gatagattgt cgcacctgat tgcccgacat 4860
tatcgcgagc ccatttatac ccatataaat cagcatccat gttggaattt aatcgcggcc 4920
tcgagcaaga cgtttcccgt tgaatatggc tcataacacc ccttgtatta ctgtttatgt 4980

```
aagcacacag ttttattgtt catgatgata tatttttatc ttgtgcaatg taacatcaga 5040

gattttgaga cacaacgtgg ctttcccccc ccccccatta ttgaagcatt tatcagggtt 5100

attgtctcat gagcggatac atatttgaat gtatttagaa aaataaacaa ataggggttc 5160

cgcgcacatt tccccgaaaa gtgccacctg acgtctaaga aaccattatt atcatgacat 5220

taacctataa aaataggcgt atcacgaggc cctttcgtc                       5259
```

<210> 7
<211> 585
<212> DNA
<213> Homo sapiens

<220>
<221> CDS
<222> (1)..(585)

<220>
<221> sig_peptide
<222> (1)..(69)

<220>
<221> mat_peptide
<222> (70)..(585)

<400> 7

```
atg gcc ctc ctg ttc cct cta ctg gca gcc cta gtg atg acc agc tat    48
Met Ala Leu Leu Phe Pro Leu Leu Ala Ala Leu Val Met Thr Ser Tyr
            -20                 -15                 -10

agc cct gtt gga tct ctg ggc tgt gat ctg cct cag aac cat ggc cta    96
Ser Pro Val Gly Ser Leu Gly Cys Asp Leu Pro Gln Asn His Gly Leu
        -5                  -1  1               5

ctt agc agg aac acc ttg gtg ctt ctg cac caa atg agg aga atc tcc   144
Leu Ser Arg Asn Thr Leu Val Leu Leu His Gln Met Arg Arg Ile Ser
 10                  15                  20                  25

cct ttc ttg tgt ctc aag gac aga aga gac ttc agg ttc ccc cag gag   192
Pro Phe Leu Cys Leu Lys Asp Arg Arg Asp Phe Arg Phe Pro Gln Glu
                30                  35                  40

atg gta aaa ggg agc cag ttg cag aag gcc cat gtc atg tct gtc ctc   240
Met Val Lys Gly Ser Gln Leu Gln Lys Ala His Val Met Ser Val Leu
            45                  50                  55

cat gag atg ctg cag cag atc ttc agc ctc ttc cac aca gag cgc tcc   288
His Glu Met Leu Gln Gln Ile Phe Ser Leu Phe His Thr Glu Arg Ser
        60                  65                  70
```

tct gct gcc tgg aac atg acc ctc cta gac caa ctc cac act gga ctt 336
Ser Ala Ala Trp Asn Met Thr Leu Leu Asp Gln Leu His Thr Gly Leu
75 80 85

cat cag caa ctg caa cac ctg gag acc tgc ttg ctg cag gta gtg gga 384
His Gln Gln Leu Gln His Leu Glu Thr Cys Leu Leu Gln Val Val Gly
90 95 100 105

gaa gga gaa tct gct ggg gca att agc agc cct gca ctg acc ttg agg 432
Glu Gly Glu Ser Ala Gly Ala Ile Ser Ser Pro Ala Leu Thr Leu Arg
110 115 120

agg tac ttc cag gga atc cgt gtc tac ctg aaa gag aag aaa tac agc 480
Arg Tyr Phe Gln Gly Ile Arg Val Tyr Leu Lys Glu Lys Lys Tyr Ser
125 130 135

gac tgt gcc tgg gaa gtt gtc aga atg gaa atc atg aaa tcc ttg ttc 528
Asp Cys Ala Trp Glu Val Val Arg Met Glu Ile Met Lys Ser Leu Phe
140 145 150

tta tca aca aac atg caa gaa aga ctg aga agt aaa gat aga gac ctg 576
Leu Ser Thr Asn Met Gln Glu Arg Leu Arg Ser Lys Asp Arg Asp Leu
155 160 165

ggc tca tct 585
Gly Ser Ser
170

<210> 8
<211> 195
<212> PRT
<213> Homo sapiens

<400> 8

Met Ala Leu Leu Phe Pro Leu Leu Ala Ala Leu Val Met Thr Ser Tyr
-20 -15 -10

Ser Pro Val Gly Ser Leu Gly Cys Asp Leu Pro Gln Asn His Gly Leu
-5 -1 1 5

Leu Ser Arg Asn Thr Leu Val Leu Leu His Gln Met Arg Arg Ile Ser
10 15 20 25

Pro Phe Leu Cys Leu Lys Asp Arg Arg Asp Phe Arg Phe Pro Gln Glu
30 35 40

Met Val Lys Gly Ser Gln Leu Gln Lys Ala His Val Met Ser Val Leu
45 50 55

His Glu Met Leu Gln Gln Ile Phe Ser Leu Phe His Thr Glu Arg Ser

60 65 70

Ser Ala Ala Trp Asn Met Thr Leu Leu Asp Gln Leu His Thr Gly Leu
75 80 85

His Gln Gln Leu Gln His Leu Glu Thr Cys Leu Leu Gln Val Val Gly
90 95 100 105

Glu Gly Glu Ser Ala Gly Ala Ile Ser Ser Pro Ala Leu Thr Leu Arg
110 115 120

Arg Tyr Phe Gln Gly Ile Arg Val Tyr Leu Lys Glu Lys Lys Tyr Ser
125 130 135

Asp Cys Ala Trp Glu Val Val Arg Met Glu Ile Met Lys Ser Leu Phe
140 145 150

Leu Ser Thr Asn Met Gln Glu Arg Leu Arg Ser Lys Asp Arg Asp Leu
155 160 165

Gly Ser Ser
170

<210> 9
<211> 567
<212> DNA
<213> Homo sapiens

<220>
<221> CDS
<222> (1)..(567)

<220>
<221> sig_peptide
<222> (1)...(69)

<220>
<221> mat_peptide
<222> (70)..(567)

<400> 9

atg gcc tcg ccc ttt gct tta ctg atg gtc ctg gtg gtg ctc agc tgc 48
Met Ala Ser Pro Phe Ala Leu Leu Met Val Leu Val Val Leu Ser Cys
-20 -15 -10

aag tca agc tgc tct ctg ggc tgt gat ctc cct gag acc cac agc ctg 96
Lys Ser Ser Cys Ser Leu Gly Cys Asp Leu Pro Glu Thr His Ser Leu
-5 -1 1 5

gat aac agg agg acc ttg atg ctc ctg gca caa atg agc aga atc tct 144
Asp Asn Arg Arg Thr Leu Met Leu Leu Ala Gln Met Ser Arg Ile Ser

10 15 20 25

cct tcc tcc tgt ctg atg gac aga cat gac ttt gga ttt ccc cag gag 192
Pro Ser Ser Cys Leu Met Asp Arg His Asp Phe Gly Phe Pro Gln Glu
30 35 40

gag ttt gat ggc aac cag ttc cag aag gct cca gcc atc tct gtc ctc 240
Glu Phe Asp Gly Asn Gln Phe Gln Lys Ala Pro Ala Ile Ser Val Leu
45 50 55

cat gag ctg atc cag cag atc ttc aac ctc ttt acc aca aaa gat tca 288
His Glu Leu Ile Gln Gln Ile Phe Asn Leu Phe Thr Thr Lys Asp Ser
60 65 70

tct gct gct tgg gat gag gac ctc cta gac aaa ttc tgc acc gaa ctc 336
Ser Ala Ala Trp Asp Glu Asp Leu Leu Asp Lys Phe Cys Thr Glu Leu
75 80 85

tac cag cag ctg aat gac ttg gaa gcc tgt gtg atg cag gag gag agg 384
Tyr Gln Gln Leu Asn Asp Leu Glu Ala Cys Val Met Gln Glu Glu Arg
90 95 100 105

gtg gga gaa act ccc ctg atg aat gcg gac tcc atc ttg gct gtg aag 432
Val Gly Glu Thr Pro Leu Met Asn Ala Asp Ser Ile Leu Ala Val Lys
110 115 120

aaa tac ttc cga aga atc act ctc tat ctg aca gag aag aaa tac agc 480
Lys Tyr Phe Arg Arg Ile Thr Leu Tyr Leu Thr Glu Lys Lys Tyr Ser
125 130 135

cct tgt gcc tgg gag gtt gtc aga gca gaa atc atg aga tcc ctc tct 528
Pro Cys Ala Trp Glu Val Val Arg Ala Glu Ile Met Arg Ser Leu Ser
140 145 150

tta tca aca aac ttg caa gaa aga tta agg agg aag gaa 567
Leu Ser Thr Asn Leu Gln Glu Arg Leu Arg Arg Lys Glu
155 160 165

<210> 10
<211> 189
<212> PRT
<213> Homo sapiens

<400> 10

Met Ala Ser Pro Phe Ala Leu Leu Met Val Leu Val Val Leu Ser Cys
-20 -15 -10

Lys Ser Ser Cys Ser Leu Gly Cys Asp Leu Pro Glu Thr His Ser Leu
-5 -1 1 5

Asp Asn Arg Arg Thr Leu Met Leu Leu Ala Gln Met Ser Arg Ile Ser

```
10                  15                  20                  25

Pro Ser Ser Cys Leu Met Asp Arg His Asp Phe Gly Phe Pro Gln Glu
                30                  35                  40

Glu Phe Asp Gly Asn Gln Phe Gln Lys Ala Pro Ala Ile Ser Val Leu
            45                  50                  55

His Glu Leu Ile Gln Gln Ile Phe Asn Leu Phe Thr Thr Lys Asp Ser
        60                  65                  70

Ser Ala Ala Trp Asp Glu Asp Leu Leu Asp Lys Phe Cys Thr Glu Leu
    75                  80                  85

Tyr Gln Gln Leu Asn Asp Leu Glu Ala Cys Val Met Gln Glu Glu Arg
90                  95                  100                 105

Val Gly Glu Thr Pro Leu Met Asn Ala Asp Ser Ile Leu Ala Val Lys
                110                 115                 120

Lys Tyr Phe Arg Arg Ile Thr Leu Tyr Leu Thr Glu Lys Lys Tyr Ser
            125                 130                 135

Pro Cys Ala Trp Glu Val Val Arg Ala Glu Ile Met Arg Ser Leu Ser
        140                 145                 150

Leu Ser Thr Asn Leu Gln Glu Arg Leu Arg Arg Lys Glu
    155                 160                 165
```

<210> 11
<211> 567
<212> DNA
<213> Mus musculus

<220>
<221> CDS
<222> (1)..(567)

<220>
<221> sig_peptide
<222> (1)..(69)

<220>
<221> mat_peptide
<222> (70)..(567)

<400> 11

```
atg gct agg ctc tgt gct ttc ctg atg gtc ctg gcg gtg atg agc tac     48
Met Ala Arg Leu Cys Ala Phe Leu Met Val Leu Ala Val Met Ser Tyr
            -20                 -15                 -10
```

```
tgg cca acc tgc tct cta gga tgt gac ctg cct cag act cat aac ctc      96
Trp Pro Thr Cys Ser Leu Gly Cys Asp Leu Pro Gln Thr His Asn Leu
        -5                  -1   1                5

agg aac aag aga gcc ttg aca ctc ctg gta caa atg agg aga ctc tcc     144
Arg Asn Lys Arg Ala Leu Thr Leu Leu Val Gln Met Arg Arg Leu Ser
 10                  15                  20                  25

cct ctc tcc tgc ctg aag gac agg aag gac ttt gga ttc ccg cag gag     192
Pro Leu Ser Cys Leu Lys Asp Arg Lys Asp Phe Gly Phe Pro Gln Glu
                30                  35                  40

aag gtg gat gcc cag cag atc aag aag gct caa gcc atc cct gtc ctg     240
Lys Val Asp Ala Gln Gln Ile Lys Lys Ala Gln Ala Ile Pro Val Leu
            45                  50                  55

agt gag ctg acc cag cag atc ctg aac atc ttc aca tca aag gac tca     288
Ser Glu Leu Thr Gln Gln Ile Leu Asn Ile Phe Thr Ser Lys Asp Ser
        60                  65                  70

tct gct gct tgg aat gca acc ctc cta gac tca ttc tgc aat gac ctc     336
Ser Ala Ala Trp Asn Ala Thr Leu Leu Asp Ser Phe Cys Asn Asp Leu
     75                  80                  85

cac cag cag ctc aat gac ctg caa ggt tgt ctg atg cag cag gtg ggg     384
His Gln Gln Leu Asn Asp Leu Gln Gly Cys Leu Met Gln Gln Val Gly
 90                  95                 100                 105

gtg cag gaa ttt ccc ctg acc cag gaa gat gcc ctg ctg gct gtg agg     432
Val Gln Glu Phe Pro Leu Thr Gln Glu Asp Ala Leu Leu Ala Val Arg
                110                 115                 120

aaa tac ttc cac agg atc act gtg tac ctg aga gag aag aaa cac agc     480
Lys Tyr Phe His Arg Ile Thr Val Tyr Leu Arg Glu Lys Lys His Ser
            125                 130                 135

ccc tgt gcc tgg gag gtg gtc aga gca gaa gtc tgg aga gcc ctg tct     528
Pro Cys Ala Trp Glu Val Val Arg Ala Glu Val Trp Arg Ala Leu Ser
        140                 145                 150

tcc tct gcc aat gtg ctg gga aga ctg aga gaa gag aaa                 567
Ser Ser Ala Asn Val Leu Gly Arg Leu Arg Glu Glu Lys
    155                 160                 165
```

<210> 12
<211> 189
<212> PRT
<213> Mus musculus

<400> 12

```
Met Ala Arg Leu Cys Ala Phe Leu Met Val Leu Ala Val Met Ser Tyr
```

```
                -20                 -15                 -10

        Trp Pro Thr Cys Ser Leu Gly Cys Asp Leu Pro Gln Thr His Asn Leu
                -5              -1   1               5

        Arg Asn Lys Arg Ala Leu Thr Leu Leu Val Gln Met Arg Arg Leu Ser
         10                  15                  20                  25

        Pro Leu Ser Cys Leu Lys Asp Arg Lys Asp Phe Gly Phe Pro Gln Glu
                        30                  35                  40

        Lys Val Asp Ala Gln Gln Ile Lys Lys Ala Gln Ala Ile Pro Val Leu
                    45                  50                  55

        Ser Glu Leu Thr Gln Gln Ile Leu Asn Ile Phe Thr Ser Lys Asp Ser
                60                  65                  70

        Ser Ala Ala Trp Asn Ala Thr Leu Leu Asp Ser Phe Cys Asn Asp Leu
            75                  80                  85

        His Gln Gln Leu Asn Asp Leu Gln Gly Cys Leu Met Gln Gln Val Gly
         90                  95                 100                 105

        Val Gln Glu Phe Pro Leu Thr Gln Glu Asp Ala Leu Leu Ala Val Arg
                        110                 115                 120

        Lys Tyr Phe His Arg Ile Thr Val Tyr Leu Arg Glu Lys Lys His Ser
                    125                 130                 135

        Pro Cys Ala Trp Glu Val Val Arg Ala Glu Val Trp Arg Ala Leu Ser
                140                 145                 150

        Ser Ser Ala Asn Val Leu Gly Arg Leu Arg Glu Glu Lys
            155                 160                 165
```

<210> 13
<211> 459
<212> DNA
<213> Homo sapiens

<220>
<221> CDS
<222> (1)..(459)

<220>
<221> sig_peptide
<222> (1) (60)

<220>
<221> mat_peptide
<222> (61)..(459)

<400> 13

```
atg tac agg atg caa ctc ctg tct tgc att gca cta agc ctt gca ctt     48
Met Tyr Arg Met Gln Leu Leu Ser Cys Ile Ala Leu Ser Leu Ala Leu
-20                 -15                 -10                 -5

gtc aca aac agt gca cct act tca agt tct aca aag aaa aca cag cta     96
Val Thr Asn Ser Ala Pro Thr Ser Ser Ser Thr Lys Lys Thr Gln Leu
            -1   1                   5                  10

caa ctg gag cat tta ctt ctg gat tta cag atg att ttg aat gga att    144
Gln Leu Glu His Leu Leu Leu Asp Leu Gln Met Ile Leu Asn Gly Ile
        15                  20                  25

aat aat tac aag aat ccc aaa ctc acc agg atg ctc aca ttt aag ttt    192
Asn Asn Tyr Lys Asn Pro Lys Leu Thr Arg Met Leu Thr Phe Lys Phe
    30                  35                  40

tac atg ccc aag aag gcc aca gaa ctg aaa cat ctt cag tgt cta gaa    240
Tyr Met Pro Lys Lys Ala Thr Glu Leu Lys His Leu Gln Cys Leu Glu
 45                  50                  55                  60

gaa gaa ctc aaa cct ctg gag gaa gtg cta aat tta gct caa agc aaa    288
Glu Glu Leu Lys Pro Leu Glu Glu Val Leu Asn Leu Ala Gln Ser Lys
                65                  70                  75

aac ttt cac tta aga ccc agg gac tta atc agc aat atc aac gta ata    336
Asn Phe His Leu Arg Pro Arg Asp Leu Ile Ser Asn Ile Asn Val Ile
            80                  85                  90

gtt ctg gaa cta aag gga tct gaa aca aca ttc atg tgt gaa tat gct    384
Val Leu Glu Leu Lys Gly Ser Glu Thr Thr Phe Met Cys Glu Tyr Ala
        95                 100                 105

gat gag aca gca acc att gta gaa ttt ctg aac aga tgg att acc ttt    432
Asp Glu Thr Ala Thr Ile Val Glu Phe Leu Asn Arg Trp Ile Thr Phe
    110                 115                 120

tgt caa agc atc atc tca aca ctg act                                459
Cys Gln Ser Ile Ile Ser Thr Leu Thr
125                 130
```

<210> 14
<211> 153
<212> PRT
<213> Homo sapiens

<400> 14

```
Met Tyr Arg Met Gln Leu Leu Ser Cys Ile Ala Leu Ser Leu Ala Leu
-20                 -15                 -10                 -5
```

```
Val Thr Asn Ser Ala Pro Thr Ser Ser Ser Thr Lys Lys Thr Gln Leu
                -1   1                5                  10

Gln Leu Glu His Leu Leu Leu Asp Leu Gln Met Ile Leu Asn Gly Ile
        15                  20                  25

Asn Asn Tyr Lys Asn Pro Lys Leu Thr Arg Met Leu Thr Phe Lys Phe
    30                  35                  40

Tyr Met Pro Lys Lys Ala Thr Glu Leu Lys His Leu Gln Cys Leu Glu
 45                  50                  55                  60

Glu Glu Leu Lys Pro Leu Glu Glu Val Leu Asn Leu Ala Gln Ser Lys
                 65                  70                  75

Asn Phe His Leu Arg Pro Arg Asp Leu Ile Ser Asn Ile Asn Val Ile
             80                  85                  90

Val Leu Glu Leu Lys Gly Ser Glu Thr Thr Phe Met Cys Glu Tyr Ala
        95                  100                 105

Asp Glu Thr Ala Thr Ile Val Glu Phe Leu Asn Arg Trp Ile Thr Phe
    110                 115                 120

Cys Gln Ser Ile Ile Ser Thr Leu Thr
125                 130
```

<210> 15
<211> 26
<212> DNA
<213> Artificial Sequence

<220>
<223> Description of Artificial Sequence:PCR oligo

<400> 15
aactgcagat ggctaggctc tgtgct 26

<210> 16
<211> 26
<212> DNA
<213> Artificial Sequence

<220>
<223> Description of Artificial Sequence:PCR oligo

<400> 16
gaagatcttc atttctcttc tctcag 26

<210> 17
<211> 26
<212> DNA
<213> Artificial Sequence

<220>

<223> Description of Artificial Sequence:PCR oligo

<400> 17
aactgcagat ggcctcgccc tttgct 26

<210> 18
<211> 28
<212> DNA
<213> Artificial Sequence

<220>
<223> Description of Artificial Sequence:PCR oligo

<400> 18
cgggatcctt attccttcct ccttaatc 28

<210> 19
<211> 25
<212> DNA
<213> Artificial Sequence

<220>
<223> Description of Artificial Sequence:PCR oligo

<400> 19
gctctagatg gccctcctgt tccct 25

<210> 20
<211> 26
<212> DNA
<213> Artificial Sequence

<220>
<223> Description of Artificial Sequence:PCR oligo

<400> 20
gcggatcctc aagatgagcc caggtc 26

<210> 21
<211> 36
<212> DNA
<213> Artificial Sequence

<220>
<223> Description of Artificial Sequence: PCR oligo

<400> 21
acgcgtcgac atgtgtcctc agaagctaac catctc 36

<210> 22
<211> 34
<212> DNA
<213> Artificial Sequence

<220>
<223> Description of Artificial Sequence: PCR oligo

<400> 22

gcggatccct aggatcggac cctgcaggga acac 34

<210> 23
<211> 37
<212> DNA
<213> Artificial Sequence

<220>
<223> Description of Artificial Sequence: PCR oligo

<400> 23
catgccatgg gtcaatcacg ctacctcctc tttttgg 37

<210> 24
<211> 30
<222> DNA
<213> Artificial Sequence

<220>
<223> Description of Artificial Sequence: PCR oligo

<400> 24
gcggatcctc aggcggagct cagatagccc 30

<210> 25
<211> 5469
<212> DNA
<213> Mus musculus

<220>
<221> CDS
<222> (1923)..(2393)

<220>
<221> sig_peptide
<222> (1923)..(1994)

<220>
<221> mat_peptide
<222> (1995)..(2393)

<400> 25

tcgcgcgttt cggtgatgac ggtgaaaacc tctgacacat gcagctcccg gagacggtca 60

cagcttgtct gtaagcggat gccgggagca gacaagcccg tcagggcgcg tcagcgggtg 120

ttggcgggtg tcggggctgg cttaactatg cggcatcaga gcagattgta ctgagagtgc 180

accatatgcg gtgtgaaata ccgcacagat gcgtaaggag aaaataccgc atcagattgg 240

ctattggcca ttgcatacgt tgtatccata tcataatatg tacatttata ttggctcatg 300

tccaacatta ccgccatgtt gacattgatt attgactagt tattaatagt aatcaattac 360

ggggtcatta gttcatagcc catatatgga gttccgcgtt acataactta cggtaaatgg 420

cccgcctggc tgaccgccca acgacccccg cccattgacg tcaataatga cgtatgttcc 480

catagtaacg ccaataggga ctttccattg acgtcaatgg gtggagtatt tacggtaaac 540

tgcccacttg gcagtacatc aagtgtatca tatgccaagt acgcccccta ttgacgtcaa 600

tgacggtaaa tggcccgcct ggcattatgc ccagtacatg accttatggg actttcctac 660

ttggcagtac atctacgtat tagtcatcgc tattaccatg gtgatgcggt tttggcagta 720

catcaatggg cgtggatagc ggtttgactc acggggattt ccaagtctcc accccattga 780

cgtcaatggg agtttgtttt ggcaccaaaa tcaacgggac tttccaaaat gtcgtaacaa 840

ctccgcccca ttgacgcaaa tgggcggtag gcgtgtacgg tgggaggtct atataagcag 900

agctcgttta gtgaaccgtc agatcgcctg gagacgccat ccacgctgtt ttgacctcca 960

tagaagacac cgggaccgat ccagcctccg cggccgggaa cggtgcattg gaacgcggat 1020

tccccgtgcc aagagtgacg taagtaccgc ctatagagtc tataggccca cccccttggc 1080

ttcttatgca tgctatactg tttttggctt ggggtctata cacccccgct tcctcatgtt 1140

ataggtgatg gtatagctta gcctataggt gtgggttatt gaccattatt gaccactccc 1200

ctattggtga cgatactttc cattactaat ccataacatg gctctttgcc acaactctct 1260

ttattggcta tatgccaata cactgtcctt cagagactga cacggactct gtatttttac 1320

aggatggggt ctcatttatt atttacaaat tcacatatac aacaccaccg tccccagtgc 1380

```
cccgagtttt tattaaacat aacgtgggat ctccacgcga atctcgggta cgtgttccgg 1440
acatgggctc ttctccggta gcggcggagc ttctacatcc gagccctgct cccatgcctc 1500
cagcgactca tggtcgctcg gcagctcctt gctcctaaca gtggaggcca gacttaggca 1560
cagcacgatg cccaccacca ccagtgtgcc gcacaaggcc gtggcggtag ggtatgtgtc 1620
tgaaaatgag ctcggggagc gggcttgcac cgctgacgca tttggaagac ttaaggcagc 1680
ggcagaagaa gatgcaggca gctgagttgt tgtgttctga taagagtcag aggtaactcc 1740
cgttgcggtg ctgttaacgg tggagggcag tgtagtctga gcagtactcg ttgctgccgc 1800
gcgcgccacc agacataata gctgacagac taacagactg ttcctttcca tgggtctttt 1860
ctgcagtcac cgtccttaga tctagcctca accctgacta tcttccaggt cattgttcca 1920

ac atg gcc ctg tgg atc gac agg atg caa ctc ctg tct tgc att gca    1967
   Met Ala Leu Trp Ile Asp Arg Met Gln Leu Leu Ser Cys Ile Ala
                   -20                 -15                 -10

cta agt ctt gca ctt gtc aca aac agt gca cct act tca agt tct aca   2015
Leu Ser Leu Ala Leu Val Thr Asn Ser Ala Pro Thr Ser Ser Ser Thr
                -5                  -1   1               5

aag aaa aca cag cta caa ctg gag cat tta ctg ctg gat tta cag atg   2063
Lys Lys Thr Gln Leu Gln Leu Glu His Leu Leu Leu Asp Leu Gln Met
            10                  15                  20

att ttg aat gga att aat aat tac aag aat ccc aaa ctc acc agg atg   2111
Ile Leu Asn Gly Ile Asn Asn Tyr Lys Asn Pro Lys Leu Thr Arg Met
        25                  30                  35

ctc aca ttt aag ttt tac atg ccc aag aag gcc aca gaa ctg aaa cat   2159
Leu Thr Phe Lys Phe Tyr Met Pro Lys Lys Ala Thr Glu Leu Lys His
40                  45                  50                  55

ctt cag tgt cta gaa gaa gaa ctc aaa cct ctg gag gaa gtg cta aat   2207
Leu Gln Cys Leu Glu Glu Glu Leu Lys Pro Leu Glu Glu Val Leu Asn
                60                  65                  70

tta gct caa agc aaa aac ttt cac tta aga ccc agg gac tta atc agc   2255
Leu Ala Gln Ser Lys Asn Phe His Leu Arg Pro Arg Asp Leu Ile Ser
            75                  80                  85

aat atc aac gta ata gtt ctg gaa cta aag gga tct gaa aca aca ttc   2303
Asn Ile Asn Val Ile Val Leu Glu Leu Lys Gly Ser Glu Thr Thr Phe
        90                  95                  100

atg tgt gaa tat gct gat gag aca gca acc att gta gaa ttt ctg aac   2351
```

Met Cys Glu Tyr Ala Asp Glu Thr Ala Thr Ile Val Glu Phe Leu Asn
105 110 115

aga tgg att acc ttt tgt caa agc atc atc tca aca cta act 2393
Arg Trp Ile Thr Phe Cys Gln Ser Ile Ile Ser Thr Leu Thr
120 125 130

tgataattaa gtgcttccca cttaaaacat atcagggatc tcgactctag aggatcatcg 2453

cggccgctct agaccaggcg cctggatcca gatctgctgt gccttctagt tgccagccat 2513

ctgttgtttg cccctccccc gtgccttcct tgaccctgga aggtgccact cccactgtcc 2573

tttcctaata aaatgaggaa attgcatcgc attgtctgag taggtgtcat tctattctgg 2633

ggggtggggt ggggcagcac agcaaggggg aggattggga agacaatagc aggcatgctg 2693

gggatgcggt gggctctatg ggtacccagg tgctgaagaa ttgacccggt tcctcctggg 2753

ccagaaagaa gcaggcacat ccccttctct gtgacacacc ctgtccacgc ccctggttct 2813

tagttccagc cccactcata ggacactcat agctcaggag ggctccgcct tcaatcccac 2873

ccgctaaagt acttggagcg gtctctccct ccctcatcag cccaccaaac caaacctagc 2933

ctccaagagt gggaagaaat taaagcaaga taggctatta agtgcagagg gagagaaaat 2993

gcctccaaca tgtgaggaag taatgagaga aatcatagaa tttcttccgc ttcctcgctc 3053

actgactcgc tgcgctcggt cgttcggctg cggcgagcgg tatcagctca ctcaaaggcg 3113

gtaatacggt tatccacaga atcaggggat aacgcaggaa agaacatgtg agcaaaaggc 3173

cagcaaaagg ccaggaaccg taaaaaggcc gcgttgctgg cgtttttcca taggctccgc 3233

cccccctgacg agcatcacaa aaatcgacgc tcaagtcaga ggtggcgaaa cccgacagga 3293

ctataaagat accaggcgtt tccccctgga agctccctcg tgcgctctcc tgttccgacc 3353

ctgccgctta ccggatacct gtccgccttt ctcccttcgg gaagcgtggc gctttctcaa 3413

tgctcacgct gtaggtatct cagttcggtg taggtcgttc gctccaagct gggctgtgtg 3473

cacgaacccc ccgttcagcc cgaccgctgc gccttatccg gtaactatcg tcttgagtcc 3533

aacccggtaa gacacgactt atcgccactg gcagcagcca ctggtaacag gattagcaga 3593

gcgaggtatg taggcggtgc tacagagttc ttgaagtggt ggcctaacta cggctacact 3653

agaaggacag tatttggtat ctgcgctctg ctgaagccag ttaccttcgg aaaaagagtt 3713

ggtagctctt gatccggcaa acaaaccacc gctggtagcg gtggtttttt tgtttgcaag 3773

cagcagatta cgcgcagaaa aaaaggatct caagaagatc ctttgatctt ttctacgggg 3833

tctgacgctc agtggaacga aaactcacgt taagggattt tggtcatgag attatcaaaa 3893

aggatcttca cctagatcct tttaaattaa aaatgaagtt ttaaatcaat ctaaagtata 3953

tatgagtaaa cttggtctga cagttaccaa tgcttaatca gtgaggcacc tatctcagcg 4013

atctgtctat ttcgttcatc catagttgcc tgactccggg gggggggggc gctgaggtct 4073

gcctcgtgaa gaaggtgttg ctgactcata ccaggcctga atcgccccat catccagcca 4133

gaaagtgagg gagccacggt tgatgagagc tttgttgtag gtggaccagt tggtgatttt 4193

gaacttttgc tttgccacgg aacggtctgc gttgtcggga agatgcgtga tctgatcctt 4253

caactcagca aaagttcgat ttattcaaca aagccgccgt cccgtcaagt cagcgtaatg 4313

ctctgccagt gttacaacca attaaccaat tctgattaga aaaactcatc gagcatcaaa 4373

tgaaactgca atttattcat atcaggatta tcaataccat atttttgaaa aagccgtttc 4433

tgtaatgaag gagaaaactc accgaggcag ttccatagga tggcaagatc ctggtatcgg 4493

tctgcgattc cgactcgtcc aacatcaata caacctatta atttcccctc gtcaaaaata 4553

aggttatcaa gtgagaaatc accatgagtg acgactgaat ccggtgagaa tggcaaaagc 4613

ttatgcattt ctttccagac ttgttcaaca ggccagccat tacgctcgtc atcaaaatca 4673

ctcgcatcaa ccaaaccgtt attcattcgt gattgcgcct gagcgagacg aaatacgcga 4733

tcgctgttaa aaggacaatt acaaacagga atcgaatgca accggcgcag gaacactgcc 4793

agcgcatcaa caatattttc acctgaatca ggatattctt ctaatacctg gaatgctgtt 4853

ttcccgggga tcgcagtggt gagtaaccat gcatcatcag gagtacggat aaaatgcttg 4913

atggtcggaa gaggcataaa ttccgtcagc cagtttagtc tgaccatctc atctgtaaca 4973

tcattggcaa cgctaccttt gccatgtttc agaaacaact ctggcgcatc gggcttccca 5033

tacaatcgat agattgtcgc acctgattgc ccgacattat cgcgagccca tttataccca 5093

tataaatcag catccatgtt ggaatttaat cgcggcctcg agcaagacgt ttcccgttga 5153

atatggctca taacacccct tgtattactg tttatgtaag cagacagttt tattgttcat 5213

gatgatatat ttttatcttg tgcaatgtaa catcagagat tttgagacac aacgtggctt 5273

tccccccccc cccattattg aagcatttat cagggttatt gtctcatgag cggatacata 5333

```
tttgaatgta tttagaaaaa taaacaaata ggggttccgc gcacatttcc ccgaaaagtg 5393

ccacctgacg tctaagaaac cattattatc atgacattaa cctataaaaa taggcgtatc 5453

acgaggccct ttcgtc                                                 5469
```

<210> 26
<211> 157
<212> PRT
<213> Mus musculus

<400> 26

```
Met Ala Leu Trp Ile Asp Arg Met Gln Leu Leu Ser Cys Ile Ala Leu
                -20                 -15                 -10

Ser Leu Ala Leu Val Thr Asn Ser Ala Pro Thr Ser Ser Ser Thr Lys
            -5              -1   1               5

Lys Thr Gln Leu Gln Leu Glu His Leu Leu Leu Asp Leu Gln Met Ile
    10                  15                  20

Leu Asn Gly Ile Asn Asn Tyr Lys Asn Pro Lys Leu Thr Arg Met Leu
 25                  30                  35                  40

Thr Phe Lys Phe Tyr Met Pro Lys Lys Ala Thr Glu Leu Lys His Leu
                 45                  50                  55

Gln Cys Leu Glu Glu Glu Leu Lys Pro Leu Glu Glu Val Leu Asn Leu
             60                  65                  70

Ala Gln Ser Lys Asn Phe His Leu Arg Pro Arg Asp Leu Ile Ser Asn
         75                  80                  85

Ile Asn Val Ile Val Leu Glu Leu Lys Gly Ser Glu Thr Thr Phe Met
    90                  95                  100

Cys Glu Tyr Ala Asp Glu Thr Ala Thr Ile Val Glu Phe Leu Asn Arg
105                 110                 115                 120

Trp Ile Thr Phe Cys Gln Ser Ile Ile Ser Thr Leu Thr
                125                 130
```

## Description

*Background of the Invention*

*Field of the Invention*

The present invention relates to a non-infectious, non-integrating polynucleotide construct comprising a polynucleotide encoding a molecule which is a peptide, polypeptide or protein for use in treatment of cancer in mammals. Generally, the present invention makes available methods of treating cancer in a mammal by administering a polynucleotide construct comprising a polynucleotide encoding a cytokine.

*Related Art*

Cytokines have been demonstrated both in pre-clinical animal models as well as in humans to have potent anti-tumor effects. In particular IFN's have been tried for the treatment of a number of human concerns.

The interferons (IFNs) are a family of cytokines with potent anti-viral, antiproliferative, and immunomodulatory activities and play important roles in the body's defensive response to viruses, bacteria, and tumors (Baron, S. et al., JAMA 266:1375 (1991)). On the basis ofantigenicity, biochemical properties, and producer cell, the interferon's have been divided into two classes, type I interferon and type II interferon. IFNα, IFNβ, IFNω, and IFNτ are type I interferons, and bind to the same α/β receptor. IFNγ is a type II interferon, and binds to the γ receptor (Pestka, S., Ann. Rev. Biochem. 56:727 (1987). IFNα and IFNβ are naturally expressed in many cells upon viral infection. IFNγ is produced by activated T lymphocytes and natural killer (NK) cells. IFNτ is believed to possess hormone activity, and plays an important role in pregnancy in cattle, sheep, and related ruminants (Imakawa, K. et a/., Nature 330:377 (1987); Stewart, H.J. et al., J. Endocrinology 115:R13 (1987)). Due to the pleiotropic activities of IFNs, these cytokines have been studied for their therapeutic efficacy in a number of diseases, particularly cancers and viral infectious diseases.

IFNω was discovered independently by three different groups in 1985 (Capon, D.J., et al., Molec. Cell. Biol. 5: 768-779 (1985), Feinstein, S. et al., Molec. Cell. Biol 5:510 (1985); and Hauptmann and Swetly, Nucl. Acids Res. 13: 4739-4749 (1985)). Unlike IFNα, for which at least 14 different functional nonallelic genes have been identified in man, IFNω is encoded by a single functional gene. IFNω genes are believed to be present in most mammals, but have not been found in dogs, rats or mice. The mature IFNω polypeptide is 172 amino acids and shares 60% sequence homology with the human IFNα's. Due to the sequence similarity with IFNα, IFNω was originally considered to be a member or a subfamily of IFNα, and was originally termed IFNα-II. IFNω is a significant component (= 10%) of human leukocyte-derived interferon, the natural mixture of interferon produced after viral infection (Adolf, G. et al., Virology 175:410 (1990)). IFNω has been demonstrated to bind to the same α/β receptor as IFNα (Flores, I. et al., J. Biol. Chem. 266: 19875-19877 (1991)), and to share similar biological activities with IFNα, including anti-proliferative activity against tumor cells *in vitro* (Kubes, M. et al., J. Interferon Research 14:57 (1994) and immunomodulatory activity (Nieroda et al., Molec. Cell. Differentiation 4: 335-351 (1996)).

Recombinant IFNα polypeptide has been approved for use in humans for hairy cell leukemia, AIDS-related Kaposi's sarcoma, malignant melanoma, chronic hepatitis B and C, chronic myleogenous leukemia, and condylomata acuminata (Baron, S. et al., JAMA 266:1375 (1991)). However, for each of these indications, IFNα polypeptide must be administered repeatedly, often on a daily basis, for extended periods of time to maintain effective serum levels due to the short half-life (hours) of the polypeptide in the serum (Friedman, Interferons: A Primer, Academic Press, New York, pp. 104-107 (1981); Galvani and Cawley, Cytokine Therapy, Cambridge University Press; Cambridge, pp. 114-115 (1992)). Thus, in spite of producing clinical benefit for many disease conditions, the use of IFNα polypeptide is associated with acute and chronic side effects in most patients (Jones, Cancer 57: 1709-17 5 (1986); and Quesda et al., Blood 68: 493-497 (1986)). The severity of the adverse reaction correlates with peak serum interferon levels.

Viral or plasmid vectors containing IFNα genes have been used in *ex vivo* therapy to treat mouse tumors. For example, tumor cells were transfected *in vitro* with viral or plasmid vectors containing an IFNα gene, and the transfected tumor cells were injected into mice (Belldegrun, A., et al.., J. Natl. Cancer Inst. 85: 207-216 (1993); Ferrantini, M. et al., Cancer Research 53: 1107-1112 (1993); Ferrantini, M. et al., J. Immunology 153: 4604-4615 (1994); Kaido, T. et al., Int. J. Cancer 60: 221-229 (1995); Ogura, H. et al., Cancer Research 50: 5102-5100 (1990); Santodonato, L., et al., Human Gene Therapy 7:1-10 (1996); Santodonato, L., et al., Gene Therapy 4:1246-1255 (1997)). In another *ex vivo* study, cervical carcinoma and leukemia cells were transfected with a viral vector containing the interferon-consensus gene, and the transfected cells were injected into mice (Zhang, J.-F. et al., Cancer Gene Therapy 3: 31-38 (1996)). In all of these *ex vivo* studies, varying levels of anti-tumor efficacy, such as tumor regression and/or prolonged survival, have been observed.

Viral or plasmid vectors containing interferon genes have also been used in *in vivo* therapy for tumor-bearing mice. For example, a viral vector containing the interferon-consensus gene was injected into mice bearing transplanted MDA-MB-435 breast cancer, hamster melanoma, or K562 leukemia, and tumor regression was reported (Zhang, J.-F. et al., Proc. Natl. Acad. Sci. USA 93: 4513-4518 (1996)). In a similar study, a plasmid vector containing human IFNβ gene complexed with cationic lipid was injected intracranially into mice bearing a human glioma, and tumor regression was reported (Yagi, K. et al., Biochemistry and Molecular Biology International 32: 167-171 (1994)). In a murine model of renal cell carcinoma the direct intratumoral injection of an IL-2 plasmid DNA : lipid complex has been shown to result in complete tumor regression and a significant induction of a tumor specific CTL response increase in survival (Saffran et al., Cancer Gene Therapy 5: 321-330 (1998)).

Plasmid vectors containing cytokine genes have also been reported to result in systemic levels of the encoded cytokine and in some cases, biological effects characteristic of each cytokine in mice. For example, the intramuscular injection of plasmid DNA encoding either TGFβ, IL-2, IL-4, IL-5, or IFNα resulted in physiologically significant amounts in the systemic circulation of the corresponding cytokine polypeptide (Raz, E. et al., Proc. Natl. Acad. Sci. USA 90: 4523-4527 (1993); Raz, E. et al., Lupus 4: 266-292 (1995); Tokui, M. et al., Biochem. Biophys. Res. Comm. 233: 527-531 (1997); Lawson, C. et al., J. Interferon Cytokine Res. 17: 255-261 (1997); Yeow, W.-S. et al., J. Immunol. 160: 2932-2939 (1998)).

U.S. Patent No. 5,676,954 reports on the injection of genetic material, complexed with cationic liposomes carriers, into mice. U.S. Patent Nos. 4,897,355; 4,946,787; 5,049,386; 5,459,127; 5,589,466; 5,693,622; 5,580,859; 5,703,055; and International Patent Application No. PCT/US94/06069 (publication no. WO 94/29469) provide cationic lipids for use in transfecting DNA into cells and mammals. U.S. Patent Nos. 5,589,466, 5,693,622, 5,580,859, 5,703,055, and international patent application no. PCT/US94/06069 (publication no. WO 94/29469) provide methods for delivering DNA-cationic lipid complexes to mammals.

Even though some viral vectors used in *ex vivo* and *in vivo* cancer therapy in murine models showed anti-tumor efficacy, the use of viral vectors to deliver interferon-expressing genes in vivo could induce anti-viral immune responses or result in viral integration into host chromosomes, casing disruption of essential host genes or activation of oncogenes (Ross et al., Human Gene Therapy 7: 1781-1790 (1996)).

For treatment of multiple metastatic carcinomas of a body cavity are treated using laparoscopy (Childers et al, Gynecol. Oncol. 59: 25-33, (1995)), catheterization (Naumann et al, Gynecol. Oncol. 50: 291-3, (1993)) or other access devices (Almadrones et al, Semin. Oncol. Nurs. 11: 194-202, (1995)). Treatment is usually by surgical removal of primary and large metastatic tumors and postoperative chemotherapy (Kigwawa et al, Am. J. Clin. Oncol. 17: 230-3, (1994); Markman et al, J. Clin. Oncol. 10: 1485-91, (1992)) or radiotherapy (Fjeld et al, Acta. Obstet. Gynecol. Scand. Suppl. 155: 105-11, (1992)). Tumor recurrence is monitored by magnetic resonance imaging (Forstner et al, Radiology 196: 715-20, (1995)), ascites cytology (Clement, Am. J. Clin. Pathol. 103: 673-6, (1995); Forstner et al, Radiology 196: 715-20, 1995) and blood analyses (Forstner et al, Radiology 196: 715-20, (1995)). Many intraperitoneal (i.p.) cancers, such as ovarian cancer, eventually metastasize via the lymphatic system to the lungs or other vital organs, and the prognosis for the patient is very poor (Kataoka et al, Nippon Sanka Fujinka Gakkai Zasshi 46: 337-44, 1994; Hamilton, Curr. Probl. Cancer 16: 1-57, (1992)).

Human ovarian cancer is often diagnosed at an advanced stage when the effectiveness of surgery and chemotherapy are limited. The lack of effective treatment options for late-stage patients warrants the development of new treatment modalities for this disease. There have been several attempts to develop an effective immunotherapy for the treatment of ovarian cancer.

The early work in this area involved mouse studies in which bacteria-derived immunostimulants, such as *Bacillus Calmette-Guerin* (BCG) and *Corynebacterium parvum,* were injected i.p. as non-specific activators of the immune system. (Knapp and Berkowitz, Am. J. Obstet. Gynecol., 128: 782-786, (1977); Bast et al., J. Immunol., 123: 1945-1951, (1979); Vanhaelen, et al. Cancer Research, 41 : 980-983, (1981); and Berek, et al., Cancer Research, 44, 1871-1875, (1984)). These studies generally resulted in a non-specific immune response that often did not prevent the growth of later tumors. In addition, if the bacterial antigens were injected more than 24 hours after tumor cell inoculation, there was minimal antitumor response, suggesting that treatment of late-stage ovarian cancer patients with this type of therapy would not be effective.

More recent studies in both.mice and humans have involved the i.p. or intravenous (i.v.) administration of cytokine proteins as more specific activators of the immune response (Adachi, et al, Cancer Immunol. Immunother. 37: 1-6, (1993); Lissoni, et al, Tumori. 78: 118-20, (1992)). Treating murine ovarian tumors with a combination of recombinant IL-2 and GM-CSF proteins had some beneficial effect in inhibiting ascites production; however, IL-2 was only effective if it was combined with GM-CSF (Kikuchi, et al., Cancer Immunol. Immunother., 43: 257-261, (1996)). Similarly, a combination of IL-2 and lymphokine-activated killer (LAK) cells was able to reduce i.p. sarcomas in mice, while IL-2 protein alone was not as effective (Ottow, et al., Cellular Immunology, 104: 366-376, (1987)). Human clinical trials evaluating IL-2 protein therapy of ovarian cancer patients indicated some antitumor effects (Chapman et al., Investigational New Drugs, 6:179-188, (1988);West et al., N. Engl. J. Med. 316:898-905, 1987; Lotze et al., Arch. Surg. 121: 1373-1379, 1986; Benedetti Panici et al., Cancer Treatment Review, 16A:123-127, 1989; Beller et al., Gynecol. Oncol., 34:407-412, 1989; Urba et al., J. Natl. Cancer Inst., 81:602-611, 1989; Stewart et al., Cancer Res., 50:6302-6310, 1990;

Steis et al., J. Clin. Oncol., 8:1618-1629, 1990; Lissoni et al., Tumori, 78:118-120, 1992; Sparano et al., J. of Immunotherapy, 16:216-223, 1994; Freedman et al., J. of Immunotherapy, 16:198-210, 1994; Edwards et al., J. Clin. Oncol., 15:3399-3407, 1997).

Recent studies in mice have involved the injection of DNA constructs encoding "suicide" genes followed by treatment with prodrugs. This approach has successfully caused regression of some small tumors but has been less successful on larger tumor masses. (Szala, et al. Gene Therapy 3: 1025-1031, 1996; Sugaya, et al. Hum Gene Ther 7: 223-30 (1996)). In another study, liposome-mediated *E1A* gene therapy for mice bearing ovarian cancers that overexpress HER-2/*neu* resulted in reduced mortality among these tumor bearing mice. (Yu, et al. Oncogene, 11: 1383-1385 (1995)). Similarly, the successful treatment of murine ovarian carcinoma (MOT) has been demonstrated using cisplatin-induced gene transfer of DNA constructs encoding IFNγ via i.p. injection. (Son, Cancer Gene Therapy 4: 391-396 (1997)). However, this study demonstrated that tumors were poorly responsive to either the IFNγ gene or cisplatin alone, suggesting that the effectiveness of the cisplatin-based gene therapy protocol was mainly due to enhanced sensitization of cisplatin-exposed tumor cells to transfection by the IFNγ gene. (Son, Cancer Gene Therapy 4: 391-396, 1997).

Clearly, there is a need for superior therapeutic compositions and methods for treating mammalian cancer. Further, there is a need for an *in vivo* delivery system for IFNω. The present invention .provides a simple and safe yet effective compositions for treatment of mammalian cancer.

The present invention also solves the problems inherent in prior attempts to treat body cavity malignancies. The inventors show herein that the malignant cell dissemination into body cavities, such as into the peritoneal cavity during late stage ovarian cancer, can be suppressed simply by administering as few as two to six doses of a polynucleotide formulation directly into the body cavity. This treatment results in selective transfection of malignant cells, and subsequent long-term local production of an effective amount of therapeutic molecules.

Kinoshita *et* al (1997) investigated whether systemic gene expression could be produced by intravenous or intraperitoneal injection of IL-2 and IFNγ DNA complexed with cationic liposome in mice. The group of mice injected intravenously with IFN-Y DNA-liposome complexes had significantly higher mean IFNγ levels of lung metastatic tumor cells and intraperitoneal cells than the group with IL-2 DNA-liposome complexes or liposome alone. However, with regard to IL-2 levels, there were no significant differences among all groups. On vaccination study (intraperitoneal injection), three IFNγ DNA-liposome complex administrations showed the effectiveness as a tumor vaccine, although in mice treated with IL-2 DNA-liposome complexes or single administration, the tumor was established and rapidly grew. (Kinoshita et al. Biotherapy 11 (3) 448-450. Hofland and Huang reported that liposome-plasmid DNA complexes specifically inhibited proliferation of human ovarian carcinoma cells *in vitro* and *in vivo.* It was thought that this inhibiting activity was likely to be sequence independent, since it was observed using several different plasmids. The degree of growth inhibition appeared dependent on both the lipid to DNA ratio and the total dose of liposome-plasmid DNA complex given to the cells.

*Summary of the Invention*

In a first aspect the invention relates to use of a non-infectious, non-integrating polynucleotide construct comprising a polynucleotide encoding a molecule which is a peptide, polypeptide or protein in the manufacture of a medicament for treatment of cancer in a mammal, wherein the treatment comprises administering the medicament into a body cavity of said mammal such that said polynucleotide is incorporated into tumor or malignant cells within said body cavity and said molecule is expressed in said cells in an amount effective to treat cancer, wherein the construct is administered as a complex of said construct and one or more cationic compounds selected from the group consisting of cationic lipids, cationic peptides, cationic proteins, cationic polymers, and mixtures thereof, wherein said molecule is a cytokine or an active fragment thereof, and wherein said cytokine is selected from the group consisting of IFNω, IFNα, IFNτ, IFNβ, IL-2, an active fragment of any of thereof, and a combination of any of thereof.

In a further aspect the invention relates to a non-infectious, non-integrating polynucleotide construct comprising a polynucleotide encoding a molecule which is a peptide, polypeptide or protein, for use in treatment of cancer in a mammal, wherein the treatment comprises administering the medicament into a body cavity of said mammal such that said polynucleotide is incorporated into tumor or malignant cells within said body cavity and said molecule is expressed in said cells in an amount effective to treat cancer, wherein the construct is administered as a complex of said construct and one or more cationic compounds selected from the group consisting of cationic lipids, cationic peptides, cationic proteins, cationic polymers, and mixtures thereof, wherein said molecule is a cytokine or an active fragment thereof, and wherein said cytokine is selected from the group consisting of IFNω, IFNα, $IFN_T$, IFNβ, IL-2, an active fragment of any of thereof, and a combination of any of thereof.

Further aspects of the present invention are set out in the claims.

Compared to injection of recombinant cytokine polypeptides, the uses described herein have several important advantages. The present invention shows that *in vivo* transfection of cells with encoding polynucleotide, such as an IL-2 or IFNω, results in serum levels of the corresponding cytokine that have therapeutic effects, and yet are lower than the maximal serum levels typically required when cytokine polypeptides are injected. Further, injecting frequent high doses of cytokine polypeptides can produce debilitating side effects. The uses of the present invention provide cytokine therapy requiring less frequent injections of cytokine-encoding nucleic acids. The injection of polynucleotide constructs encoding cytokines produces sustained, low levels of biologically active cytokines that have beneficial effects, while minimizing adverse side effects.

Compared to the delivery of cytokine genes via a viral gene delivery vectors, the polynucleotide construct for use in the present method also has important advantages. Injection of non-viral vectors of the present method does not induce significant toxicity or pathological immune responses, as described, for example, in mice, pigs or monkeys (Parker, et al., Human Gene Therapy 6: 575-590 (1995); and San, et al., Human Gene Therapy 4: 781-788 (1993)). Thus, a non-viral vector is safer and can be repeatedly injected.

*Brief Description of the Figures*

A more complete appreciation of the invention and many of the attendant advantages thereof will be readily obtained as the same become better understood by reference to the following detailed description when considered in connection with the accompanying figures.

FIG. 1 shows the plasmid map of VR4151 (SEQ ID No. 4). The cytomegalovirus immediate-early gene promoter enhancer and 5' untranslated sequences (5'UTR + intron A) drive the expression of the human interferon ω coding sequence. The transcriptional terminator region includes polyadenylation and termination signals derived from the rabbit β-globin gene.

FIG. 2 shows the pharmacokinetics of hIFNω in the serum of C57BL/6 mice (FIG. 2A) and nude mice (FIG. 2B) after a single intramuscular (i.m.) injection of hIFNω plasmid DNA (VR4151). Mice were injected i.m. with 100 μg of VR4151. Following the intramuscular injection, mice were bled daily, and serum was collected and assayed for hIFNω polypeptide using an ELISA. Each point represents an average of four mice. In C57BL/6 mice, the single i.m. injection resulted in peak serum levels of 254 pg/ml on day 6 after injection, and serum levels were still detectable 14 days after injection (50 pg/ml) (FIG. 2A). In nude mice, the single i.m. injection resulted in peak serum levels of 648 pg/ml on day 7, and serum levels were still detectable 14 days after injection (134 pg/ml) (FIG. 2B).

FIG. 3 shows that systemic mIFNα treatment reduces tumor volume (FIGs. 3A, 3C, and 3E) and increases survival (FIGs. 3B. 3D, and 3F) in three murine tumor models. C57BL/6 mice bearing subcutaneous B16F10 melanoma (FIGs. 3A and 3B), subcutaneous glioma 261 (FIGs. 3C and 3D), or DBA/2 mice bearing subcutaneous Cloudman melanoma (FIGs. 3E and 3F) were injected with 100μg either of VR4111 (mIFNα plasmid) or VR1055 (control plasmid), twice per week for three weeks, beginning on day 4 after tumor cell injection (n=8-10 mice per group).

FIG. 4 shows that systemic mIFNα, mIL-2 or mIL-12 plasmid DNA treatment reduces tumor volume (FIG. 4A) and mIFNα or mIL-12 plasmid DNA treatment increases survival (FIG.4B) in the subcutaneous B16F10 melanoma model. C57BL/6 mice bearing subcutaneous B16F10 melanoma were injected with 100 μg of VR4111 (mIFNα), VR4001 (mIL-12), VR1110 (mIL-2), or VR1012 (control plasmid) (n = 15-16 mice per group) twice per week for three weeks.

FIG. 5 shows that i.m. administration of hIFNω pDNA reduces tumor volume (FIG. 5A) and increases survival (FIG. 5B) in nude mice bearing human A431 epidermoid carcinoma tumors. Mice bearing human A431 tumors between 30-80 $mm^3$ were injected i.m. with 200 μg of either VR4151 (hIFNω plasmid) or VR1055 (control plasmid) twice per week for three weeks (n= 15)

FIG. 6 shows that i.m. administration of mIFNα pDNA reduces B16F10 melanoma lung metastases in C57BL/6 mice. Mice bearing lung metastases of B16F10 melanoma were injected i.m. with 100 μg of either VR4111 or VR1055 twice per week for three weeks, beginning on day 4 after tumor cell injection (n=10 mice per group). "TNTC" means too numerous to count as seen in the control group.

FIG. 7 shows that i.m. administration of mIFNα pDNA reduces intradermal M5076 primary tumor growth (FIG. 7A) as well as liver metastases (FIG. 7B) in C57BL/6 mice bearing murine M5076 reticulum cell sarcoma cells. Mice bearing M5076 tumors were injected i.m. with 100 μg of either VR4111 or VR1055 twice per week for three weeks, beginning on day 4 after tumor cell inoculation (n=10-13 mice per group).

FIG. 8 shows a comparison of different dosages and frequencies of mIFNα pDNA administration in the subcutaneous B16F10 melanoma model. C57BL/6 mice bearing subcutaneous B16F10 melanoma were injected i.m. with 50 μg or 100 μg of either VR4111 or VR1055 twice a week for 3 weeks beginning 4 days after tumor cell inoculation (n=10 mice per group). All groups treated with 100 μg of VR4111 showed significant reduction in tumor growth by day 21 (p=0.002) and significant enhancement in survival (p<0.008) with all treatments tested (FIGs. 8A and 8B). In mice treated with 50 μg VR4111, tumor growth was significantly reduced by day 21 (p=0.005), and survival was significantly increased (p<0.003) in the groups of mice that were injected twice per week or once per week. The group injected every other week with 50 μg VR4111 was not significantly different from the mice that received the control plasmid (FIGs. 8C and 8D).

FIG. 9 shows the results of experiments performed to determine the role of NK and T cells in the antitumor response induced by mIFNα plasmid DNA. Nude mice (T cell deficient) (FIGS. 9A and 9B), and beige-nude mice (NK and T cell deficient) (FIGS. 9C and 9D) bearing subcutaneous B16F10 melanoma tumors were injected i.m. with 100 μg of either VR4111 or VR1055 twice per week for three weeks, beginning on day 4 after tumor cell injection (n=15 mice per group). No significant reduction in tumor volume or increase in survival was found for nude or nude-beige mice treated with VR4111, suggesting that T cells are involved in the mIFNα antitumor response.

FIG. 10 shows the results of experiments performed to evaluate the role of $CD4^+$ and $CD8^+$ T cells in the mIFNα DNA antitumor response. For depletion of $CD4^+$ and $CD8^+$ T cells, C57BL/6 mice bearing subcutaneous B16F10 melanoma tumors were injected i.p. with 500 μg of either the anti-CD4 mAb (clone GK1.5, rat IgG) (ATCC, Rockville, MD) or anti-CD8 mAb (clone 2.43, rat IgG) (ATCC, Rockville, MD) one day after each i.m. injection of 100 μg of either VR4111 or VR1055 twice per week for three weeks (n = 10 mice per group). The mIFNα plasmid DNA therapy significantly reduced tumor growth ($p \leq 0.002$) and enhanced survival ($p \leq 0.008$) of both normal mice and mice depleted of $CD4^+$ T cells, suggesting that $CD4^+$ T cells were not required for the response. In contrast, mice depleted of $CD8^+$ T cells and injected with VR4111 had tumor volumes and survival that were not significantly different from mice treated with the control plasmid DNA, indicating a requirement for $CD8^+$ T cells in the antitumor response.

FIG. 11 shows that intratumoral hIFNω (VR4151) and hIFNα (VR4112) treatment reduces tumor volume in the human A375 melanoma model (FIG. 11A) and human NIH-OVCAR3 (FIG. 11B) in nude mice. Mice bearing subcutaneous tumor received direct intratumoral injections of a complex of DNA:DMRIE/DOPE (1:1 DNA:lipid mass ratio, 100 μg of plasmid DNA) for 6 consecutive days followed by an additional 5 treatments every other day for a total of I injections (A375 melanoma model), or for every other day for a total of 11 injections (NIH-OVCAR3 ovarian cancer model).

FIG. 12 shows that intratumoral mIFNα (VR4101) plasmid DNA treatment reduces tumor volume (FIG. 12A) and increases survival (FIG. 12B) in the subcutaneous B16F10 melanoma model in C57BL/6 mice. Mice received a subcutaneous implantation of $10^4$ B16F10 cells into the flank. Beginning at day 12 post tumor implant, mice received six consecutive intratumoral injections of a complex of pDNA:DMRIE/DOPE (1:1 DNA:DMRIE mass ratio, 100 μg of plasmid DNA).

FIG. 13 shows luciferase activity in peritoneal tissues and MOT ascites in mice after i.p. injection of luciferase DNA: lipid complex. The results show high levels of reporter gene expression in ascites but low levels in peritoneal tissue. MOT tumor-bearing C3H/HeN mice received i.p. injections of a complex of pDNA:DMRIE/DOPE (1:1 DNA:DMRIE mass ratio, 100 μg of plasmid DNA) on days 5 and 6 after tumor cell implant. Tissues were collected I day (FIG. 13A) or 3 days (FIG.13B) following the DNA:lipid injection.

FIG. 14 shows serum levels of IL-2 after i.p. injection of either IL-2 pDNA or protein in MOT tumor bearing mice. The serum levels of IL-2 were much lower than levels in ascites. Ascites and serum were collected at 4 hours and days 1, 2, 3, 6 and 10 post DNA or protein injection (5 mice for each time point), and analyzed for mIL-2 polypeptide using an ELISA.

FIG. 15 shows a significant reduction in MOT tumor growth (p=0.01) (FIG. 15A) and increased survival (p=0.04) (FIG. 15B) of mice treated with i.p. injection of IL-2 pDNA:lipid on days 5-10 after tumor cell injection. The DNA was complexed at either a 1:1 (15A and 15B) or 5:1 (FIGS. 15C and 15D) DNA:DMRIE mass ratio (100 μg pDNA). Plasmid DNA without lipid was not effective (FIGs. 15E and 15F)

FIG. 16 shows that i.p. mIL-2 plasmid DNA (VR1110):lipid treatment inhibits tumor growth (FIG. 16A) and enhances survival (FIG. 16B) in the MOT tumor model in C3H/HeN mice. MOT tumor-bearing mice received three alternative-day i.p. injections of a complex of pDNA:DMRIE/DOPE (1:1 DNA:DMRIE mass ratio, 100 μg of plasmid DNA).

FIG. 17 shows a significant reduction in MOT tumor growth and increased survival of mice treated with i.p. injection of IL-2 DNA:lipid followed by debulking of tumor ascites. MOT tumor-bearing mice received six consecutive intraperitoneal injections of a complex of pDNA:DMRIE/DOPE (1:1 DNA:DMRIE mass ratio, 100 μg of pDNA) and debulked of 5 ml of tumor ascites 4 days after the last DNA:lipid injection (n=10).

FIG. 18 shows dose-response of mIL-2 pDNA (VR1110):lipid treatment in the MOT tumor model. C3H/HeN mice bearing MOT tumor were injected with 25, 50 or 100 μg of VR1110:DMRIE/DOPE on days 5, 8 and 11 after MOT tumor cell injection. In mice treated with 50 or 100μg of VR1110, tumor growth was significantly reduced (p=0.002) and survival significantly enhanced (p=0.01) by day 15 post tumor cell inoculation compared to the control. Tumor-bearing mice treated with 25 μg of VR1110:lipid were not significantly different from the control mice for either tumor volume or survival (n=15).

FIG. 19 shows the cytokine profile of ovarian tumor ascites in C3H/HeN mice MOT tumor model following mIL-2 pDNA (VR1110):lipid treatment. Mice received i.p. injections of a complex of pDNA/DMRIE/DOPE (1:1 DNA/DMRIE mass ratio, 100 μg of plasmid DNA) on days 5, 8 and 11 after tumor cell implant. Two days after each injection, mice were sacrificed (5 mice for each time point), and the ascites were collected and analyzed for cytokine concentration. The level of IL-2 (days 7, 10 and 13) as well as IFNγ and GM-CSF (days 10 and 13) were markedly elevated suggesting that IL-2 upregulates IFNγ and GM-CSF production.

FIG. 20 shows that i.p. mIFNα pDNA (VR4111):lipid treatment enhances survival (FIG. 20B) in the MOT tumor model in C3H/HeN mice. MOT tumor-bearing mice received three alternative-day i.p. injections of a complex of pDNA:DMRIE/DOPE (1:1 DNA:DMRIE mass ratio, 100 μg of plasmid DNA).

*Detailed Description of the Preferred Embodiments*

The polynucleotide construct for use is administered as a complex with one or more cationic compounds selected from the group consisting of cationic lipids, cationic peptides, cationic proteins, cationic polymers, and mixtures thereof. Preferably, the polynucleotide construct is complexed with one or more cationic lipids. More preferably, the polynucleotide construct is complexed with one or more cationic lipids and one or more neutral lipids. Still more preferably, the cationic. lipid is (±)-N-(2-hydroxyethyl)-N,N-dimethyl-2,3-bis(tetradecyloxy)-1-propaniminium bromide (DMRIE) and the neutral lipid is 1,2-dioleoyl-sn-glycero-3-phosphatidylethanolamine (DOPE) such that the mass ratio of polynucleotide construct to lipid is from about 10:1 and about 0.5:1. More preferably, the mass ratio of polynucleotide construct to lipid is from about 5:1 and about 1:1. Still more preferably, the mass ratio of polynucleotide construct to lipid is about 5:1.

Cytokine-encoding plasmids discussed herein include VR4102 (hIFNα in the VR1012 vector) (SEQ ID No. 1), VR4112 (hIFNα in the VR1055 vector) (SEQ ID No. 2), VR4150 (hIFNω in the VR1012 vector) (SEQ ID No. 3), VR4151 (hIFNω in the VR1055 vector) (SEQ ID No. 4), VR4101 (mIFNα in the VR1012 vector) (SEQ ID No. 5), VR4111 (mIFNα in the VR1055 vector) (SEQ ID No. 6), and VR1110 (mIL-2 in the VR1012 vector), VR1103 (hIL-2 in the VR1012 vector) (SEQ ID No: 25), VR4001 (mIL-12 in the VR1033 vector), and VR1700 (mGM-CSF in the VR1012 vector).

Cytokine-encoding cDNAs discussed herein include the cDNA for hIFNω (SEQ ID No. 7), the cDNA for hIFNα (SEQ ID No. 9), the cDNA for mTFNα (SEQ ID No. 11), the cDNA for hIL-2 (SEQ ID No. 13 and the coding portion of SEQ ID No. 25), the cDNA for mLL-2 (for example, as disclosed in Kashima et al., Nature 313:402-404 (1985), the cDNA for mIL-12 (for example, as disclosed in Tone et al., Eur. J. Immunol. 26:1222-1227(1996), and the cDNA for mGM-CSF (for example, as disclosed in Gough et al., EMBO J. 4:645-653 (1985). Cytokine polypeptides discussed herein include hIFNα (SEQ ID No. 8), hIFNα (SEQ ID No. 10), mIFNα (SEQ ID No. 12), and hIL-2 (SEQ ID No. 14 and SEQ ID No. 26).

By "stringent conditions" is intended a hybridization by overnight incubation at 42°C in a solution comprising: 50% formamide, 5x SSC (750 mM NaCl, 15 mM trisodium citrate), 50 mM sodium phosphate (pH 7.6), 5x Denhardt's solution, 10% dextran sulfate, and 20 μg/ml denatured, sheared salmon sperm DNA, followed by repeatedly washing the filters (at least three times) in 0.1x SSC and 0.1% sodium dodecyl sulfate (w/v) for 20 minutes at about 65°C.

By "active fragment" is intended a fragment of a cytokine that displays the antiproliferative activity of the mature or full length cytokine. For example, a full length hIFNω is set forth in amino acids -23 to 172 of SEQ ID No. 8. The corresponding mature hIFNω is set forth in amino acids 1 to 172 of SEQ ID No. 8. Active fragments of hIFNω include, but are not limited to a polypeptide comprising amino acids 86-172 in SEQ ID No. 8, a polypeptide comprising amino acids 61-172 in SEQ ID No. 8, a polypeptide comprising amino acids 41-172 in SEQ ID No. 8, and a polypeptide comprising amino acids 21-172 in SEQ ID No. 8. A full length hIFNα is set forth in amipo acids -23 to 166 of SEQ ID No. 10. The corresponding mature hIFNα is set forth in amino acids 1 to 166 of SEQ ID No. 10. Active fragments of hIFNα include, but are not limited to a polypeptide comprising amino acids 83-166 in SEQ ID No. 10, a polypeptide comprising amino acids 61-166 in SEQ ID No. 10, a polypeptide comprising amino acids 41-166 in SEQ ID No. 10, and a polypeptide comprising amino acids 21-166 in SEQ ID No. 10. Full length hIL-2 is set forth in amino acids -20 to 133 of SEQ ID No. 14. The corresponding mature hIL-2 is set forth in amino acids 1 to 133 of SEQ ID No. 14. Active fragments of hIL-2 include, but are not limited to a polypeptide comprising amino acids 58 to 105 in SEQ ID No. 14, and a polypeptide comprising amino acids 20 to 126 in SEQ ID No. 14.

Assays of antiproliferative activity *in vitro* are well known to those of ordinary skill in the art. For example, one antiproliferation assay that can be used is to treat cultured cells, such as human ovarian NIH-OVCAR3 cells (ATCC, Rockville, MD), with supernatants from human melanoma UM449 cells transfected with the polynucleotide construct containing a polynucleotide encoding an IFNω or an active fragment thereof. In this antiproliferation assay, NIH-OVCAR3 cells are cultured and plated in 96 well-tissue culture plates. The plates are incubated for 24 hours at 37°C in a humidified 5% $CO_2$ atmosphere. Twenty μl of tissue culture supernatants from transfected UM449 cells are added to duplicate wells. An interferon reference standard (*e.g.*, human leukocyte interferon, Sigma Chemical Co., St. Louis, MO) is included in each assay. The cells are incubated with the test samples or the interferon standard for an additional 72 hours at 37°C. To quantitate the effects on cell proliferation, 50 μl of XTT/ECR substrate (Cell Proliferation Kit, Boehringer Mannheim, Indianapolis, IN) is added to each well and the plates are incubated for an additional 24 hours at 37°C prior to measurement of the $OD_{490}$. Other cell lines can be used in the antiproliferation assay. For example, any of the cells listed on Table 1 can be used. Another antiproliferation assay that can be used is provided in Nieroda, et al ( Mol. Cell. Differentiation 4: 335-351 (1996)).

For treatment of cancer, a polynucleotide construct comprising a polynucleotide encoding a cytokine for use according to the present invention can be delivered intra-cavity.

By "naked" is meant that the polynucleotide construct is free from association with any delivery vehicle known in the art that can act to facilitate entry into cells, for example, from transfection-facilitating proteins, viral particles, liposomes, cationic lipids, and calcium phosphate precipitating agents.

As used herein, "*ex vivo*" cells are cells into which the polynucleotide construct is introduced, for example, by transfection, lipofection, electroporation, bombardment, or microinjection. The cells containing the polynucleotide construct are then administered *in vivo* into mammalian tissue. Such *ex vivo* polynucleotide constructs are well-known to those of ordinary skill in the art. For example, see Belldegrun, A., et al., J. Natl. Cancer Inst. 85: 207-216 (1993); Ferrantini, M. et al., Cancer Research 53: 1107-1112 (1993); Ferrantini, M. el al., J. Immunology 153 : 4604-4615 (1994); Kaido, T., et al., Int, J. Cancer 60: 221-229 (1995); Ogura, H., et al., Cancer Research 50: 5102-5106 (1990); Santodonato, L., et al., Human Gene Therapy 7:1-10 (1996); Santodonato, L., et al., Gene Therapy 4:1246-1255 (1997); and Zhang, J.-F. et al., Cancer Gene Therapy 3: 31-38 (1996).

The polynucleotide construct is administered in a "cell-free" fashion when it is administered independently, i.e., free of *ex vivo* cells or *ex vivo* cellular material.

In the "intra-cavity delivery" embodiment, a method of selectively transfecting malignant cells in a tumor-bearing body cavity of a mammal by introducing a polynucleotide construct is made available, into the body cavity, is made available, wherein the polynucleotide is incorporated into tumor cells and the tumor cells subsequently express the protein encoded by the polynucleotide in an amount effective to treat cancer. The polynucleotide construct is administered free from *ex vivo* cells and free from *ex vivo* cellular material.

A cavity is a space within the body that can confine a fluid volume for some period of time. The cavity can either be present in a normal animal, or it can be produced as a result of disease, surgery or trauma. Cavities in the normal animal include the peritoneum, the cerebrospinal fluid space, the ventricles of the brain, the pleural space around lung, the bronchiolar airways, the nasal sinus, the bladder, the vagina, the ear, the synovium of various joints (knee, hip etc.), the internal network of salivary gland tissue, and the gastrointestinal tract including stomach. Surgical removal of tumor tissue can also produce a space which fits the definition of a cavity. An open wound produced by trauma or surgery and closed by suture can be defined as a cavity, and the area under a blister produced by an infection, abrasion or a burn also fits the definition.

There are special bioavailability considerations when a gene delivery system is administered into a cavity. First the fluid volume in the cavity can be substantially comprised of the vehicle in which the delivery system is suspended. Second, the delivery system can have particular access to cells that are either suspended in the cavity, or that are lining the surface of the cavity. Third, in some cases normally differentiated cells that are lining the cavity may be embedded in an extracellular matrix and, may not be accessible to the delivery system. Thus, the delivery system may preferentially transfect cells that are growing outside the normal extracellular matrix and avoid the cells that are growing within the extracellular matrix, conferring a kind of cell selectivity to the delivery system.

With respect to the first point, body fluids such as serum, have been shown to inhibit gene delivery systems. For example, the transfection activities of Lipofectin and LipofectAMINE are inhibited by serum. It is thought that serum factors bind to cationic lipid/DNA complexes and block their uptake into cells. In cavity models the endogenous fluid volume can be removed, the cavity can be washed, and the delivery system can be administered into the cavity in a vehicle that is compatible with optimal gene delivery efficacy. Thus the cavity model allows the investigator to create a fluid environment which allows for optimal gene delivery potency.

With respect to the second point, cells that are either floating in the cavity or are lining the surface of the cavity have preferential access to the delivery system and can be preferentially transfected relative to other cells in the body. Since the delivery system is confined within the cavity, peripheral cells in the body outside of the cavity will not be transfected. Thus, there is tissue targeting to the cells within the cavity. For example, gene delivery systems administered into the peritoneal cavity will have access to metastatic tumor cells derived from colon or ovarian cancers that are floating in the peritoneum or are attached to the surfaces of the peritoneum. Delivery systems administered into the pleural space should transfect cancer cells in the plural effusion. Delivery systems administered into the cerebral spinal fluid should have access to metastatic cancer cells present there.

With respect to the third point, differentiated cells that are present in normal tissues are often embedded into an extracellular matrix. This matrix can be difficult to penetrate with large particulate delivery systems. Some cells, such as poorly differentiated tumor cells, that are present in cavities can grow outside of the normal extracellular matrix and are therefore more accessible to gene delivery systems. In this way the delivery system can preferentially transfect those cells that are growing outside of the extracellular matrix and not transfect those cells that are growing within the extracellular matrix. This is another form of in vivo, cell type specific targeting. Examples of normal cells that are not embedded in an extracellular matrix and are therefore more accessible to gene delivery systems are, bronchial airway cells, lung cells in the plural space, and ependimal cells lining the surface of the ventricles of the brain. Normal bladder cells that line the surface of the bladder are embedded in a tight extracellular matrix and are therefore not readily accessible to a gene delivery system delivered into the bladder, but tumor cells which grow up and out of the extracellular matrix into the bladder vesicle are accessible to gene delivery systems administered into the bladder vesicle. Thus normal bladder tissue would be expected to resist transfection whereas, bladder tumor would be expected to be transfectable.

A preferred application of the intra-cavity delivery is in the treatment of peritoneally disseminated cancers. More specifically, a mammal bearing peritoneal tumor may be injected i.p. with an effective amount of a polynucleotide complexed with a lipid in a physiologically acceptable diluent in a total volume sufficient to access the entire body cavity. The mammal may have tumor ascites in the peritoneal cavity as in an ovarian cancer. In the most preferred application, this methodology may be used in treating ovarian cancer of a human.

Debulking of tumor ascites is commonly performed on human ovarian cancer patients. Debulking involves removal of tumor ascites from the peritoneal cavity. In humans bearing ovarian tumor ascites, the ascites fluid would be debulked by insertion of a catheter i.p. followed by periodic draining of ascites fluid. It is contemplated that the tumor ascites would be debulked before and/or after the i.p. administration of the polynucleotide formulation of the present invention.

Transfection efficacy of the intra-cavity delivery embodiment may be determined by collecting the tumor ascites and serum at various times after the injection and performing diagnostic assays appropriate for the encoded molecule (s). Naturally, other means of determining tumor mass, growth, and viability may also be used to assess the effectiveness of the present invention.

For treatment of cancer by any of the above disclosed embodiments, any polynucleotide encoding an IFNω, or an active fragment thereof, can be used. For example, the polynucleotide construct can be a construct comprising a polynucleotide that hybridizes under stringent conditions to the nucleotide sequence of SEQ ID No. 7 or the complement thereof, wherein the polynucleotide sequence encodes a polypeptide that has antiproliferative activity when added to NIH-OVCAR3 cells *in vitro,* and one or more cationic compounds selected from the group consisting of cationic lipids, cationic peptides, cationic proteins, cationic polymers, and mixtures thereof. Alternatively, the construct can be a polynucleotide construct comprising a polynucleotide that encodes a polypeptide comprising an amino acid sequence which, except for at least one but not more than 20 amino acid substitutions, deletions, or insertions, is identical to amino acids -23 to 172 or 1 to 172 in SEQ ID No. 8, wherein the polypeptide has antiproliferative activity when added to NIH-OVCAR3 cells *in vitro,* and one or more cationic compounds selected from the group consisting of cationic lipids. cationic peptides, cationic proteins, cationic polymers, and mixtures thereof Alternatively, the construct can be a polynucleotide construct comprising a polynucleotide that encodes a polypeptide comprising amino acids 86-172 in SEQ ID No. 8, wherein the polypeptide has antiproliferative activity when added to NIH-OVCAR3 cells *in vitro*; and one or more cationic compounds selected from the group consisting of cationic lipids, cationic peptides, cationic proteins, cationic polymers, and mixtures thereof. Preferably, IFNω is encoded by nucleotides 1 to 585 in SEQ ID No. 7 (corresponding to amino acids -23 to 172 in SEQ ID No. 8), or by nucleotides 70 to 585 in (SEQ ID No. 7 (corresponding to amino acids 1 to 172 in SEQ ID No. 8). More preferably, the polynucleotide construct is VR4151.

For treatment of cancer, any polynucleotide encoding IFNα, or active fragment thereof, can also be used. For example, the polynucleotide construct can be a construct comprising a polynucleotide that hybridizes under stringent conditions to the nucleotide sequence of SEQ ID No. 9 or the complement thereof, wherein the polynucleotide sequence encodes a polypeptide that has antiproliferative activity when added to NIH-OVCAR3 cells *in vitro,* and one or more cationic compounds selected from the group consisting of cationic lipids, cationic peptides, cationic proteins, cationic polymers, and mixtures thereof Alternatively, the construct can be a polynucleotide construct comprising a polynucleotide that encodes a polypeptide comprising an amino acid sequence which, except for at least one but not more than 20 amino acid substitutions, deletions, or insertions, is identical to amino acids -23 to 166 or 1 to 166 in SEQ ID No. 10, wherein the polypeptide has antiproliferative activity when added to NIH-OVCAR3 cells *in vitro,* and one or more cationic compounds selected from the group consisting of cationic lipids, cationic peptides, cationic proteins, cationic polymers, and mixtures thereof. Alternatively, the construct can be a polynucleotide construct comprising a polynucleotide that encodes a polypeptide comprising amino acids 83-166 in SEQ ID No. 10, wherein the polypeptide has antiproliferative activity when added to NIH-OVCAR3 cells *in vitro*; and one or more cationic compounds selected from the group consisting of cationic lipids, cationic peptides, cationic proteins, cationic polymers, and mixtures thereof. Preferably, IFNα is encoded by nucleotides 1 to 567 in SEQ ID No. 9 (corresponding to amino acids -23 to 166 in (SEQ ID No. 10), or by nucleotides 1 to 567 in SEQ ID No. 9 (corresponding to amino acids 1 to 166 in SEQ ID No. 10). Preferably, the polynucleotide construct is VR4112.

For polynucleotide constructs that do not contain a polynucleotide encoding IFNω, the polynucleotide construct is preferably a cell-free construct. For polynucleotide constructs that contain a polynucleotide encoding IFNω, the polynucleotide construct can be administered either within *ex vivo* cells or free of *ex vivo* cells or *ex vivo* cellular material. Preferably, the polynucleotide construct is administered free of *ex vivo* cells or *ex vivo* cellular material.

In the "intra-cavity delivery" the polynucleotide construct is complexed with one or more cationic compounds. More preferably, the polynucleotide construct is complexed with one or more cationic lipids by ionic interaction. Generally, the complex then contacts the cell membrane and is transfected into the cell. This transfection mechanism is referred to as "lipofection," and is a highly efficient transfection procedure (Felgner, et al., Proc. Natl. Acad Sci. USA 84:7413-7417, 1987); and Felgner, et al, Nature 337:387-388, 1989). Still more preferably, the polynucleotide construct is complexed with one or more cationic lipids and one or more neutral lipids.

For purposes of the present invention, lipid refers to a synthetic or naturally occurring compound that possesses both a lipophilic region and a polar region, commonly referred to as a head group. Preferred cationic compounds are cationic lipids. Cationic lipids are described in U.S. Pat. Nos. 4,897,355; 4,946,787; 5,049,386; 5,264,618; 5,279,833; 5,334,761; 5,429,127; 5,459,127; 5,589,466; 5,676,954; 5,693,622: 5,580,859; 5,703,055; and 5,578,475; and international publications WO 04/9469, WO 95/14381, 95/14651, 95/17373, 96/18372, 96/26179, 96/40962, 96/40963, 96/41873, and 97/00241, and documents cited therein. As illustrated in the above-cited patents and patent applications, cationic lipids comprise structural features that may be present in a variety of core molecular classes.

Examples of cationic lipids are 5-carboxyspermylglycine dioctadecylamide (DOGS) and dipalmitoyl-phophatidylethanolamine-5-carboxyspermylamide (DPPES). Cationic cholesterol derivatives are also useful, including {3β-[N-N',N'-dimethylamino)ethane]-carbomoyl}-cholesterol (DC-Chol). Dimethyldioctdecyl-ammonium bromide (DDAB), N-(3-aminopropyl)-N,N-(*bis*-(2-tetradecyloxylthyl))-N-methyl-ammonium bromide (PA-DEMO), N-(3-aminopropyl)-N, N-(*bis*-(2-dodecyloxyethyl))-N-methyl-ammonium bromide (PA-DELO), N,N,N-*tris*-(2-dodecyloxy)ethyl-N-(3-amino)propyl-ammonium bromide (PA-TELO), and N'-(3-aminopropyl)((2-dodecyloxy)ethyl)-$N^2$-(2-dodecyloxy)ethyl-1-piperazinaminium bromide (GA-LOE-BP) can also be employed in the present invention.

Non-diether cationic lipids, such as DL-1,2-dioleoyl-3-dimethylaminopropyl-β-hydroxyethylammonium (DORI diester), 1-O-oleyl-2-oleoyl-3-dimethylaminopropyl-β-hydroxyethylammonium (DORI ester/ether), and their salts promote *in vivo* gene delivery. Preferred cationic lipids comprise groups attached via a heteroatom attached to the quaternary ammonium moiety in the head group. A glycyl spacer can connect the linker to the hydroxyl group.

Preferred cationic lipids are 3,5-(N,N-dilysyl)-diaminobenzoyl-3-(DL-1,2-dioleoyl-dimethylaminopropyl-β-hydroxyethylamine) (DLYS-DABA-DORI diester), 3,5-(N,N-di-lysyl)diamino-benzoylglycyl-3-(DL-1,2-dioleoyldimethylaminopropyl-β-hydroxyethylamine) (DLYS-DABA-GLY-DORI diester), and (±)-N-(2-hydroxyethyl)-N,N-dimethyl-2,3-bis (tetradecyloxy)-1-propaniminium bromide (DMRIE).

Also preferred are (±)-N,N-dimethyl-N-[2-(sperminecarboxamido)ethyl]-2,3-bis(dioleyloxy)-1-propaniminium pentahydrochloride (DOSPA), (±)-N-(2-aminoethyl)-N,N-dimethyl-2,3-bis(tetradecyloxy)-l-propaniminium bromide (β-aminoethyl-DMRIE or βAE-DMRIE) (Wheeler, et al. Biochim. Biophys. Acta 1280:1-11 (1996)), and (±)-N-(3-aminopropyl)-N,N-dimethyl-2,3-bis(dodecyloxy)-1-propaniminium bromide (GAP-DLRIE) (Wheeler, et al., Proc. Natl. Acad. Sci. USA 93:11454-11459 (1996)), which have been developed from DMRIE.

Other examples of DMRIE-derived cationic lipids that are useful for the present invention are (±)-N-(3-aminopropyl)-N,N-dimethyl-2,3-(*bis*-decyloxy)-1-propanaminium bromide (GAP-DDRIE), (±)-N-(3-aminopropyl)-N,N-dimethyl-2,3-(*bis*-tetradecyloxy)-1-propanaminium bromide (GAP-DMRIE), (±)-N-((N"-methyl)-N'-ureyl)propyl-N,N-dimethyl-2,3-bis(tetradecyloxy)-1-propanaminium bromide (GMU-DMRIE), (±)-N-(2-hydroxyethyl)-N,N-dimethyl-2,3-bis(dodecyloxy)-1-propanaminium bromide (DLRIE), and (±)-N-(2-hydroxyethyl)-N,N-dimethyl-2,3-*bis*-([Z]-9-octadecenyloxy) propyl-1- propaniminium bromide (HP-DORIE).

The lipids of the lipid-containing formulation can comprise a cationic lipid alone, or further comprise a neutral lipid such as cardiolipin, phosphatidylcholine, phosphatidylethanolamine, dioleoylphosphatidylcholine, dioleoylphosphatidyl-ethanolamine,1,2-dioleoyl-sn-glycero-3-phosphatidylethanolamine (DOPE), sphingomyelin, and mono-, di- or tri- acylglycerol. Other additives, such as cholesterol, fatty acid, ganglioside, glycolipid, neobee, niosome, prostaglandin, sphingolipid, and any other natural or synthetic amphiphiles, can also be used. A preferred molar ratio of cationic lipid to neutral lipid in these lipid-containing formulations is from about 9:1 to about 1:9; an equimolar ratio is particularly preferred. The lipid-containing formulation can further comprise a lyso lipid (*e.g.*, lysophosphatidylcholine, lysophosphatidyl-ethanolamine, or a lyso form of a cationic lipid).

More preferably, the cationic lipid is (±)-N-(2-hydroxyethyl)-N,N-dimethyl-2,3-bis(tetradeecyloxy)-1-propaniminium bromide (DMRIE) and the neutral lipid is 1,2-dioleoyl-sn-glycero-3-phosphatidylethanolamine (DOPE) such that the mass ratio of polynucleotide construct to lipid is from about 10:1 and about 0.5:1. Still more preferably, the mass ratio of polynucleotide construct to lipid is from about 5:1 and about 1:1. Still more preferably, the mass ratio of polynucleotide construct to lipid is about 5:1.

Lipid-containing pharmaceutical composition for administration in a complex with the polynucleotide construct for use in a method of treatment according to the invention can also comprise cationic lipid together with an effective amount of a lysophosphatide. The lysophosphatide can have a neutral or a negative head group. Lysophosphatidylcholine and lysophosphatidyl-ethanolamine are preferred, and 1-oleoyl lysophosphatidylcholine is particularly preferred. Lysophosphatide lipids are advantageously present in the lipid-containing formulation in a 1:2 ratio of lysolipid to cationic lipid. Lyso forms of a cationic lipid can also be used to promote polynucleotide delivery. These lyso forms are advantageously present in effective amounts up to about one-third of the total cationic lipid in the lipid-containing formulations.

In a formulation for preparing DNA: lipid complexes, the cationic lipid can be present at a concentration of between about 0.1 mole % and about 100 mole %, preferably about 5 mole % and 100 mole %, and most preferably between about 20 mole % and 100 mole %, relative to other formulation components present in the formulation. The neutral lipid can be present in a concentration of between zero and about 99:9 mole %, preferably zero and about 95 mole %, and most preferably zero and about 80 mole %. In order to produce lipid vesicles having a net positive charge, the quantity of the positively charged component must exceed that of the negatively charged component. The negatively charged lipid can be present at between zero and about 49 mole %, and preferably between zero and about 40 mole %. Cholesterol or a similar sterol can be present at between zero to about 80 mole %, and preferably zero and about 50 mole %.

The polynucleotide construct for use in the present invention can be solubilized in a buffer prior to mixing with lipid vesicles. Suitable buffers include, for example, phosphate buffered saline (PBS), normal saline, Tris buffer, and sodium phosphate vehicle (100-150 mM preferred). Insoluble polynucleotides can be solubilized in a weak acid or base, and then diluted to the desired volume with a neutral buffer such as PBS. The pH of the buffer is suitably adjusted, and moreover, a pharmaceutically acceptable additive can be used in the buffer to provide an appropriate osmolarity within the lipid vesicle.

A lipid solution comprising at least one amphipathic lipid can spontaneously assemble to form primary lipid vesicles, heterogeneous in size. Therefore, according to a preferred method, the lipids of the lipid-containing formulation, comprising at least one cationic lipid, are prepared by dissolution in a solvent such as chloroform and the mixture is evaporated to dryness as a film on the inner surface of a glass vessel. On suspension in an aqueous solvent, the amphipathic lipid molecules assemble themselves into primary lipid vesicles. These primary lipid vesicles may be reduced to a selected mean diameter by means of a freeze-thaw procedure. Vesicles of uniform size can be formed prior to drug delivery according to methods for vesicle production known to those in the art; for example, the sonication of a lipid solution as described by Felgner, et al (Proc. Natl. Acad. Sci. USA 84:7413-7417 (1987)) and U.S. Pat. No. 5,264,618.

The term "mammal," is intended to encompass a singular "mammal" and plural "mammals," and includes, but is not limited to humans; primate mammals such as apes, monkeys, orangutans, and chimpanzees; canine mammals such as dogs and wolves; feline mammals such as cats, lions, and tigers; equine mammals such as horses, donkeys, deer, zebra, and giraffe; and bears. Preferably, the mammal is a human subject.

Tumor cell formation and growth, also known as "transformation," describes the formation and proliferation of cells that have lost their ability to control cellular division; that is the cells are cancerous. "Malignant cells" are defined as cells that have lost the ability to control the cell division cycle, leading to a transformed or cancerous phenotype.

The medicament or polynucleotide construct for use in the present invention can be used to treat a variety of mammalian cancers or tumors. Types of mammalian cancers and tumors that can be treated using the pharmaceutical composition and methods of the present invention include, but are not limited to all solid tumors, cutaneous tumors, melanoma, malignant melanoma, renal cell carcinoma, colorectal carcinoma, colon cancer, hepatic metastases of advanced colorectal carcinoma, lymphomas (including glandular lymphoma), malignant lymphoma, Kaposi's sarcoma, prostate cancer, kidney cancer, ovarian cancer, lung cancer, head and neck cancer, pancreatic cancer, mesenteric cancer, gastric cancer, rectal cancer, stomach cancer, bladder cancer, leukemia (including hairy cell leukemia and chronic myelogenous leukemia), breast cancer, non-melanoma skin cancer (including squamous cell carcinoma and basal cell carcinoma), hemangioma multiple myeloma, and glioma. Preferably, the cancer is melanoma, ovarian cancer, or metastases thereof.

By "treatment" is meant reduction in tumor size, a reduction in the rate of metastasis, and/or a slowing of tumor growth, and/or no worsening in disease over a specified period of time.

For tumors involving body cavity of a mammal, delivery For tumors involving body cavity of a mammal, "intra-cavity" delivery is preferred. In particular, the use of this methodology is envisioned in treating cancers involving (i) the peritoneal cavity--pancreatic cancer, gastric cancer, ovarian cancer, mesenteric cancer, glandular lymphoma and metastatic melanoma; (ii) the thoracic cavity--lung cancer and glandular lymphoma; (iii) the rectal cavity--rectal cancer; (iv) the stomach cavity--stomach cancer; and (v) the urinary bladder vesicle--bladder cancer. When advantageous, systemic, local; and/or intra-cavity delivery can be combined, especially in a mammal having a primary site of tumor and one or more metastases.

An additional embodiment of the present invention is directed to uses combining any of the uses of the present invention with one or more additional cancer therapies including, but not limited to bone marrow transplant, cord blood cell transplant, surgery, chemotherapy, radiation therapy, and immunotherapy. The polynucleotide construct or medicament for use in the present invention can be administered prior to the commencement of one or more of the additional cancer therapies, during the practice of one or more of the additional cancer therapies, and after the end of one or more of the additional cancer therapies.

Types of bone marrow transplant include, but are not limited to autologous bone marrow transplant and heterologous (i.e., from a donor) bone marrow transplant.

Types of surgery include, but are not limited to surgery for breast cancer, prostate cancer, colon cancer, brain cancer, and head and neck cancer.

Chemotherapeutic agents include, but are not limited to alkylating agents, including mechlorethamine, cyclophosphamide, ifosfamide, melphalan, chlorambucil, dicarbazine, streptazocine, carmustine, lomustine, semustine, chlorozotocin, busulfan, triethylenemelamine, thiotepa, hexamethylmelamine; antimetabolites, including methotrexate; pyrimidine analogs, including fluorouracil, 5-fluorouracil, floxuridine (5'-fluoro-2'-deoxyuridine), idoxuridine, cytarabine, -phosphonoacetyl-L-aspartate, 5-azacytidine, azaribine, 6-azauridine, pyrazofuran, 3-deazauridine, acivicin; purine analogs, including thioguanine, mercaptopurine, azathioprine, pentostatin, erythrohydroxynonyladenine; vinca alkaloids, including vincristine and vinblastine; epipodophyllotoxins, including etoposide and teniposide; antibiotics, including dactinomycin, daunorubicin, doxorubicin, bleomycin sulfate, plicamycin, mitomycin; enzymes, including L-asparaginase; platinum coordination complexes, including cisplatin, carboplatin; hydroxyurea, procarbazine, mitotane; and hormones or related agents, including adrenocorticosteroids such as prednisone and prednisolone; aminoglutethimide; progestins such as hydroxyprogesterone caproate, medroxyprogesterone acetate, megesterol acetate, estrogens and androgens such as diethylstilbestrol, fluoxymesterone, ethynyl estradiol, antiestrogens such as tamoxifen, and gonadotropin-releasing hormone analogs such as leuprolide.

A "polypeptide" refers to any translation product of a polynucleotide, regardless of the size of the translation product, and regardless of whether the translation product is post-translationally modified (*e.g.*, glycosylated) or not.

The polynucleotide construct for use in the present invention, can be administered by any suitable route of administration, including intramuscularly, subcutaneously, intravenously, transdermally, intranasally, by inhalation, or transmucosally (*i.e.*, across a mucous membrane). Similarly, the medicament of the present invention can by administered by any suitable route, including intramuscularly, into or near a tumor, into a cavity (*e.g.*, intraperitoneally), subcutaneously, intravenously, transdermally, intranasally, by inhalation, or transmucosally (*i.e.*, across a mucous membrane).

Any mode of administration can be used so long as the mode results in the expression of one or more cytokines in an amount sufficient to decrease the tumorigenicity of the cancer bearing mammal. This includes needle injection, catheter infusion, biolistic injectors, particle accelerators (*i.e.*, "gene guns"), pneumatic "needleless" injectors (*e.g.*, MedEJet, PedoJet, Bioject), gelfoam sponge depots, other commercially available depot materials, osmotic pumps (*e.g.*, Alza minipumps), oral or suppositorial solid (tablet or pill) pharmaceutical formulations, and decanting or topical applications during surgery. Preferred methods include needle injection and catheter infusion.

A "polynucleotide construct" is a polynucleotide molecule that carries genetic information for encoding one or more molecules, preferably, cytokines. The polynucleotide material delivered to the cells *in vivo* can take any number of forms. It can contain the entire sequence or only a functionally active fragment of a cytokine gene.

The polynucleotide construct comprises at least one polynucleotide (*e.g.*, DNA, RNA, ribozyme, phosphorothioate, or other modified nucleic acid) encoding one or more molecules. Preferred molecules are cytokines. The polynucleotide can be provided in linear, circular (*e.g.* plasmid), or branched form; and double-stranded or single-stranded form. The polynucleotide can involve a conventional phosphodiester bond or a nonconventional bond (*e.g.*, an amide bond as in peptide nucleic acid (PNA)). The choice of polynucleotide encoding a cytokine will depend on the desired kinetics and duration of expression. When long term delivery of the polynucleotide construct is desired, the preferred polynucleotide is DNA. Alternatively, when short term delivery is desired, the preferred polynucleotide is mRNA. RNA will be rapidly translated into polypeptide, but will be degraded by the target cell more quickly than DNA. In general, because of the greater resistance of circular DNA molecules to nucleases, circular DNA molecules will persist longer than linear polynucleotides, and they will be less likely to cause insertional mutation by integrating into the target genome.

In one embodiment, the polynucleotide sequence encoding one or more cytokines is RNA. Most preferably, the RNA is messenger RNA (mRNA). Methods for introducing RNA sequences into mammalian cells is described in U.S. patent No. 5,580,859. A viral alphavector, a non-infectious vector useful for administering RNA, may be used to introduce RNA into mammalian cells. Methods for the *in vivo* introduction of alphaviral vectors to mammalian tissues are described in Altman-Hamamdzic, S., et al., Gene Therapy 4: 815-822 (1997). Preferably, the polynucleotide sequence encoding one or more cytokines is DNA. In a DNA construct, a promoter is preferably operably linked to the polynucleotide encoding a cytokine. The promoter may be a cell-specific promoter that directs substantial transcription of the DNA only in predetermine cells. Other transcription control elements, besides a promoter, can be included in the polynucleotide construct to direct cell-specific transcription of the DNA.

An operable linkage is a linkage in which a polynucleotide sequence encoding a cytokine is connected to one or more regulatory sequence in such a way as to place expression of the cytokine sequence under the influence or control of the regulatory sequence(s). Two DNA sequences (such as a coding sequence and a promoter region sequence linked to the 5' end of the coding sequence) are operably linked if induction of promoter function results in the transcription of mRNA encoding the desired polypeptide and if the nature of the linkage between the two DNA sequences does not (1) result in the introduction of a frame-shift mutation, (2) interfere with the ability of the expression regulatory sequences to direct the expression of the polypeptide, antisense RNA, or (3) interfere with the ability of the DNA template to be transcribed. Thus, a promoter region would be operably linked to a DNA sequence if the promoter was capable of affecting transcription of that DNA sequence.

Preferably, the polynucleotide construct is a circular or linearized plasmid containing non-infectious nucleotide sequence. A linearized plasmid is a plasmid that was previously circular but has been linearized, for example, by digestion with a restriction endonuclease. The polynucleotide sequence encoding a cytokine may comprise a sequence which directs the secretion of the polypeptide.

"Non-infectious" means that the polynucleotide construct does not infect mammalian cells. Thus, the polynucleotide construct can contain functional sequences from non-mammalian (*e.g.*, viral or bacterial) species, but the construct does not contain non-mammalian nucleotide sequences that facilitate infection of the construct into mammalian cells.

"Non-integrating" means that the polynucleotide construct does not integrate into the genome of mammalian cells. The construct can be a non-replicating DNA sequence, .or specific replicating sequences genetically engineered to lack the ability to integrate into the genome. The polynucleotide construct does not contain functional sequences that facilitate integration of the cytokine-encoding polynucleotide sequence into the genome of mammalian cells.

The polynucleotide construct is assembled out of components where different selectable genes, origins, promoters, introns, 5' untranslated (UT) sequence, terminators, polyadenylation signals, 3' UT sequence, and leader peptides, etc. are put together to make the desired vector. The precise nature of the regulatory regions needed for gene expression can vary between species or cell types, but shall in general include, as necessary, 5' non-transcribing and 5' non-translating (non-coding) sequences involved with initiation of transcription and translation respectively, such as the TATA box, capping sequence, CAAT sequence, and the like, with those elements necessary for the promoter sequence being provided by the promoters of the invention. Such transcriptional control sequences can also include enhancer sequences or upstream activator sequences, as desired.

Secretion of a cytokine from a cell can be facilitated by a leader or secretory signal sequence. In a preferred embodiment, either the native leader sequence of a cytokine is used, or a functional derivative of that sequence that retains the ability to direct the secretion of the peptide that is operably linked to it. Alternatively, a heterologous mammalian leader sequence, or a functional derivative thereof, may be used. For example, the wild-type leader sequence may be substituted with the leader sequence of human tissue plasminogen activator or mouse β-glucuronidase.

The polynucleotide and amino acid sequences encoding an IFNω include the sequences for the complete IFNω and the mature IFNω set forth in U.S. Patent No. 4.917,887; European Patent Publication No. 0 170 204 B1; and Capon, D.J., et al., Molec. Cell. Biol. 5: 768-779 (1985); Hauptmann, R. and P. Swetly, Nucl. Acids Res. 13: 4739-4749 (1985); Adolf, G.R., et al., Biochim. Biophys. Acta 1089: 167-174 (1991); Mege, D., et al., J. Interf. Res. 11: 341-350 (1991); Charlier, M., et al., J. Interf. Res. 13 313-322 (1993); Hughes, A.L., J. Mol. Evol. 41: 539-548 (1995); and Roberts, R.M., et al., Prog. Nucl. Acid Res. Molec. Biol. 56:287-325, edited by W.E. Cohn, Academic Press (1997).

The polynucleotide and amino acid sequences encoding IFNα include the sequences for the complete TFNα and the mature IFNα set forth in U.S. Patent Nos. 4,530,901; 4,695,543; 4,695,623; 4,748,233; 4,892,743; 4,897,471; 4,973,479; 4,975,276; and 5,098,703; and in Pestka, S., Methods Enzymol. 119: 3-14 (1986); Hughes, A.L., J. Mol. Evol. 41: 539-548 (1995); and Roberts, R.M., et al., Prog. Nucl. Acid Res. Molec. Biol. 56:287-325, edited by W.E. Cohn, Academic Press (1997).

The polynucleotide and amino acid sequences encoding an IL-2 include the sequences for the complete human IL-2 and mature IL-2 set forth in Lupker, J. et al., EP 0307285-A3 (1989), U.S. Patent No. 5,641,665, Maeda et al., Biochem. Biophys. Res. Commun. 115:1040-1047 (1983), Mita et al., Biochem. Biophys. Res. Commun. 117:114-121. (1983), Taniguchi et al., Nature 302:305-310 (1983), Devos et al., Nucleic Acid Res. 11:4307-4323 (1983), Fujita et al., Proc. Natl. Acad. Sci. USA 80:74347-7441 (1983), Clark et al., Proc. Natl. Acad Sci. USA 81:2543-2547 (1984) and Cullen, DNA 7:645-650 (1988).

The polynucleotide sequences encoding an IFNω, an IFNα, and an IL-2 also include sequences that encode the complete polypeptide encoded by the nucleotide sequences set forth in SEQ ID Nos. 7, 9 and 13, respectively, and the mature polypeptides encoded by nucleotide sequences set forth in SEQ ID Nos. 7, 9 and 13, respectively. The polynucleotide sequences encoding IL-2 further includes the sequence that encodes the complete IL-2 polypeptide encoded by the nucleotide sequence set forth in SEQ ID No. 25, shown as SEQ ID No. 26.

Thus, a polynucleotide sequence encoding a polypeptide for use in the present invention can encode a polypeptide having one to twenty amino acid substitutions, deletions or insertions, either from natural mutations or human manipulation, relative to the full length or mature IFNα, IFNω, or IL-2. Preferably, no more than one to fifteen substitutions, deletions or insertions are present, relative to the full length or mature IFNα, IFNω, or IL-2 (excluding the signal sequence). More preferably, no more than one to ten substitutions, deletions or insertions are present. Still more preferably, no more than one to five substitutions, deletions or insertions are present.

Determining an effective amount of substance to be delivered can depend upon a number of factors including, for example, the chemical structure and biological activity of the substance, the age and weight of the mammal, the precise condition requiring treatment and its severity, and the route of administration. The precise amount, number of doses, and timing of doses will be determined by the attending physician or veterinarian.

If the polynucleotide construct for use in the present invention is administered as a medicament the medicament can be formulated according to known methods for preparing pharmaceutical compositions, whereby the substance to be delivered is combined with a pharmaceutically acceptable carrier vehicle. Suitable vehicles and their preparation are described, for example, in Remington's Pharmaceutical Sciences, 16th Edition, A. Osol, Ed., Mack Publishing Co., Easton, PA (1980), and Remington's Pharmaceutical Sciences, 19th Edition, A.R. Gennaro, Ed., Mack Publishing Co., Easton, PA (1995).

The medicament can be in the form of an emulsion, gel, solution, suspension, or other form known in the art. Optionally, it can contain one or more lipids as described above. In addition, the medicament can also contain pharmaceutically acceptable additives including, for example, diluents, binders, stabilizers, and preservatives. Administration of pharmaceutically acceptable salts of the polynucleotides described herein is preferred. Such salts can be prepared from pharmaceutically acceptable non-toxic bases including organic bases and inorganic bases. Salts derived from inorganic bases include sodium, potassium, lithium, ammonium, calcium, magnesium, and the like. Salts derived from pharmaceutically acceptable organic non-toxic bases include salts of primary, secondary, and tertiary amines, basic amino acids, and the like.

For aqueous medicaments used *in vivo,* sterile pyrogen-free water is preferred. Such formulations will contain an effective amount of the substance together with a suitable amount of vehicle in order to prepare pharmaceutically acceptable compositions suitable for administration to a human or animal.

A medicament can be in solution form, or alternatively, in lyophilized form for reconstitution with a suitable vehicle, such as sterile, pyrogen-free water. Both liquid and lyophilized forms will comprise one or more agents, preferably buffers, in amounts necessary to suitably adjust the pH of the injected solution.

The container in which the medicament is packaged prior to use can comprise a hermetically sealed container enclosing an amount of the lyophilized formulation or a solution containing the formulation suitable for a pharmaceutically effective dose thereof, or multiples of an effective dose. The medicament is packaged in a sterile container, and the hermetically sealed container is designed to preserve sterility of the pharmaceutical formulation until use. Optionally, the container can be associated with administration means and or instruction for use.

Having now generally described the invention, the same will become more readily understood by reference to the following specific examples which are included herein for purposes of illustration only and are not intended to be limiting unless otherwise specified.

Described herein are: 1) the *in vitro* characterization of biological activities of and IFNs delivered by plasmid DNA (anti-proliferative activity and anti-viral activity *in vitro*); 2) *in vivo* expression of cytokines following *in vivo* administration of cytokine-expressing pDNA; and 3) the *in vivo* characterization of anti-tumor activity of cytokines in murine models of solid and metastatic tumors following intratumoral, intramuscular or intra-cavity administration of cytokine-encoding pDNA.

The cytokine-encoding polynucleotide constructs have potent anti-proliferative activity *in vitro.* Moreover, the *in vivo* anti-tumor activities of IFNω, IFNα, IL-2, and IL-12 are herein demonstrated in multiple murine tumor models including nude mice bearing subcutaneous human tumors, or in immunocompetent mice bearing murine solid and metastatic tumors. Intratumoral, intramuscular, or intraperitoneal injection of the cytokine-encoding plasmids is shown to result in a statistically significant slowing of tumor growth and/or a statistically significant increase in survival. In addition to the potent antitumor effects of the cytokine plasmids delivered via intratumoral or intramuscular injection, this is the first *in vivo* demonstration of anti-tumor activity for human interferon-ω. Moreover, the *in vivo* antitumor activity of IL-2 in the treatment of peritoneally disseminated cancers, such as ovarian metastatic melanoma is also demonstrated.

Examples 4 and 5 are provided for comparison

***Example I***

***Construction of Expression Vectors***

Three basic eukaryotic expression plasmid vectors, termed VR1012, VR1055 and VR1033 were used in the construction of all plasmids used in the following examples. The blank plasmids, VR1012 and VR1055 differ only in transcriptional termination sequences. The backbone of both plasmids is derived from pUC19, with the beta-lactamase (ampicillin resistance) gene replaced by the aminoglycoside acetyltransferase (kanamycin resistance) gene from pET9a (Novagen, Madison, WI). Both plasmids direct eukaryotic gene expression from a cassette containing the human cytomegalovirus immediate early I (CMV IE) gene promoter/enhancer, CMV IE 5' untranslated (UT) sequence, and intron A. Following these regulatory elements is a cloning polylinker for insertion of polypeptide coding sequences. Following the polylinker in VR1012 is the 3' UT sequence from the bovine growth hormone gene for polyadenylation and transcriptional termination. In VR1055, the transcriptional terminator region includes a polyadenylation and termination signals derived from the rabbit b-globin gene. VR1033 is identical to VR1012, except that it contains a cap-independent translational enhancer from the encephalomyocarditis virus within the cloning polylinker sequence. This sequence allows the production of two different polypeptides from a single expressed mRNA.

Plasmid VR4101 (murine interferon α (mIFNα)) was constructed by cloning the murine interferon α cDNA into the vector VR1012 vector. The cDNA was obtained by amplifying the coding sequence from the plasmid RSV-"1 (Kelly, K.A. and P.M. Pitha, Nucl. Acids Res. 13: 805-823 (1985); Kelly, K.A. and P.M. Pitha, Nucl. Acids Res. 13: 825-839 (1985)), which was provided by Dr. Paula Pitha-Rowe of Johns Hopkins University. Plasmid VR4111 was constructed by transferring the coding sequences from VR4101 to the VR1055 cloning vector. The oligonucleotide primers used for polymerase chain reaction (PCR) were 5'-AACTGCAGATGGCTAGGCTCTGTGCT-3' (SEQ ID No. 15) and 5'-GAAG-ATCTTCATTTCTCTTCTC-TCAG-3' (SEQ ID No. 16). Reaction conditions were 30 cycles of 94°C for 1 minute (denaturing), 58°C for 2 minutes (annealing), and 72°C for 1 minute (extension).

Plasmid VR4102 (human interferon α (hIFNα)) was constructed by cloning the human interferon α cDNA into the VR1012 vector. The cDNA was obtained by amplifying the coding sequence from human genomic DNA prepared from a fresh blood sample. Plasmid VR4112 was constructed by transferring the coding sequence sequences from VR4102 to the VR1055 cloning vector. Genomic DNA was isolated using the QIAamp Blood Kit (Qiagen, Inc.). The oligonucleotide primers used for PCR were 5'-AACTGCAGATGGCCTC-GCCCTTTGCT-3' (SEQ ID No. 17) and 5'-CGGGATCCTTATTCCTTC-CTCCTTAATC-3' (SEQ ID No. 18). Reaction conditions were 30 cycles of 94$^{B}$C for 1 minute (denaturing); 58°C for 2 minutes (annealing), and 72°C for 1 minute (extension).

Plasmid VR4150 (human interferon ω (hIFNω)) was constructed by cloning the human IFNω cDNA into the VR1012 cloning vector. The cDNA was obtained by amplifying the coding sequence from human genomic DNA prepared from a fresh blood sample. Plasmid VR4151 (SEQ ID No. 1) was constructed by transferring the coding sequences from VR4150 to the VR1055 cloning vector. The oligonucleotide primers used for PCR were 5'-GCTCTAGATGGCCCTCCTGTTCCCT-3' (SEQ ID No. 19) and 5'-GCGG-ATCCTCAAGATGAGCCCAGGTC-3' (SEQ ID No. 20). Reaction conditions were 30 cycles of 94°C for 1 minute (denaturing), 58°C for 2 minutes (annealing), and 72°C for 1 minute (extension).

Plasmid VR1110 (murine interleukin-2 (mIL-2)) was constructed by cloning modified murine IL-2 cDNA into the VR1012 vector. The 5' UT sequence and the two amino acids of the leader peptide were replaced with the rat insulin II gene 5' UT sequence and coding region of the first six amino acids of the rat preproinsulin leader peptide. The IL-2 cDNA was then cloned into the BamHI site of VR1012.

Plasmid VR1103 (human interleukin-2 (hIL-2)) is identical to VR1110 with the exception that the murine IL-2 cDNA was replaced with the cDNA for human IL-2 (Parker et. al. 1996).

Plasmid VR4001 (murine interleukin-12 (mIL-12)) was constructed by cloning the cDNA's encoding the two murine subunits p35 and p40 into the VR1033 vector. Both cDNA's were obtained by amplifying the coding sequences from plasmids provided by Dr. Thomas Gajewski of The University of Chicago (J. Immun.,154:5637; J. Immun., 156: 1095). The oligonucleotides used for PCR of p35 were 5'- CAT GCC ATG GGT CAA TCA CGC TAC CTC CTC TTT TTG G-3' (SEQ ID No. 23) and 5'- GCG GAT CCT CAG GCG GAG CTC AGA TAG CCC-3' (SEQ ID No. 24). The oligonucleotides used for PCR of p40 were 5'- ACG CGT CGA CAT GTG TCC TCA GAA GCT AAC CAT CTC-3' (SEQ ID No. 21) and 5'- GCG GAT CCC TAG GAT CGG ACC CTG CAG GGA ACA C-3' (SEQ ID No. 22). Reaction conditions were 30 cycles of 94°C for 1 minute (denaturing), 58°C for 2 minutes (annealing), and 72°C for 1 minute (extension).

Plasmids VR1223 (luciferase) was constructed by cloning cytoplasmic luciferase gene into the VR1012 vector (Hartikka et al., Hum. Gene Ther. 7:1205-1217, 1996). The source of the cytoplasmic luciferase gene found in VR1223 was the plasmid termed pSP-luc+ which was purchased from Promega. An Avr II - Xba I restriction fragment encoding the luciferase cDNA was transferred from pSP-luc+ to VR1012 to make VR1223.

Plasmid VR1412 (β-galactosidase) was constructed by cloning cytoplasmic β-gal gene into the VR1012 vector (Doh et al., Gene Ther. 4:648-663).

Plasmid VR1332 was constructed by inserting a *Sal*I-*Bam*HI fragment encoding chloramphenicol acetyltransferase (CAT) from pBS-CAT (Promega) into *Sal*I/*Bam*HI-cut VR1012 vector (Hartikka et al., Hum. Gene Ther. 7: 1205-1217 (1996)).

***Example 2***

*Purification of pDNA*

pDNA was transformed into *Escherichia coli* DH10B-competent cells and grown in Terrific Broth (Sambrook, J. et al., in: Molecular Cloning: A laboratory manual, Cold Spring Harbor Laboratory Press, Cold Spring Harbor, NY, p. A.2 (1989)) complemented with 50 μg/ml kanamycin in a 1 Liter shaker flask. Cells were harvested by centrifugation at the end of the exponential growth phase (approximately 16 hr), typically yielding 10 grams of biomass net weight per liter. Covalently closed circular pDNA was isolated by a modified lysis procedure-(Horn, N.A. et al., Human Gene Therapy 6: 565-573 (1995)) followed by standard double CsCl-ethidium bromide gradient ultracentrifugation with an average yield of approximately 5 mg per liter. Plasmids were ethanol precipitated and resolubilized in saline at 4°C and dialyzed against saline. Endotoxin content was determined by the *Limulus* Amebocyte Lysate assay (Associates of Cape Cod, Inc., Falmouth, MA). All plasmid preparations were free of detectable RNA. Endotoxin levels were less than 0.6 Endotoxin Units/μg of plasmid DNA. The spectrophotometric A260/A280 ratios were between 1.75 and 2.0.

***Example 3***

***In Vitro Evaluation of Biological Activity of IFNω and IFNα***

To assure that the interferon plasmid DNA used in the following examples encoded biologically active interferon, cell proliferation and antiviral assays were performed. All culture medium used in this and following examples was obtained from Life Technologies (Gaithersburg, MD), and all serum was obtained from HyClone (Logan, Utah).

UM449 cells (American Type Culture Collection, Rockville, MD) were plated at a concentration of 2 x $10^5$ cells per well in a 6 well plate and incubated for 24 hours. Plasmid DNA and the lipid, DMRIE/DOPE (1:1) were each diluted to a concentration of 1 mg in 0.5 ml Optimem medium (Life Technologies, Gaithersburg, MD). The lipid DMRIE/DOPE consists of the cationic lipid (±)-N-(2-hydroxyethyl)-N,N-dimethyl-2,3-bis(tetradecyloxy)-1-propanaminium bromide (DMRIE) and the neutral lipid dioleoylphosphatidylethanolamine (DOPE) at a 1:1 mol:mol ratio (Felgner et al., J. Biol. Chem. 269:2550-2561, 1994). DMRIE/DOPE has been shown to be effective for both *in vitro* (Felgner et al, J. Biol. Chem. 269: 2550-2561, 1994) and *in vivo* transfection (Stopeck et al., J. Clin. Oncol. 15:341-349, 1997 and Rubin et al., Gene Ther. 4:419-425, 1997). The lipid mixture and the DNA mixture were then gently mixed. Medium was removed from the cells which were rinsed gently with PBS, followed by addition of the DNA:lipid mixture (1 ml/well). After incubating the cells for 4-5 h at 37°C, one ml of Optimem with 30% fetal calf serum (FCS) was added to each well. Following an overnight incubation at 37°C, one ml of Optimem with 10% FCS was added to each well. Tissue culture supernatants were collected 48 h after the start of the *in vitro* transfection.

***a Antiproliferative activity***

To evaluate the antiproliferative and hence, anti-tumor activity of IFNω and IFNα, supernatants from the above described UM449 cells transfected with the interferon or control plasmid DNA were tested in a cell proliferation assay of murine or human tumor cell lines (ATCC, Rockville,MD) using the Boehringer Mannheim (Indianapolis, IN) Cell Proliferation Kit II (XTT). Murine or human tumor cells were plated in 96 well plates at the desired concentration (cell concentration varied with each cell line evaluated, for example, at a concentration of 5 x $10^3$ cells/ml for B16F10 cells and 5 x $10^4$ cells/ml for the Cloudman S91 and glioma 261 cells). The plates were incubated at 37°C for 24 hours followed by addition of tissue culture supernatants from UM449 cells *in vitro* transfected with either interferon plasmid DNA or control plasmid DNA. As a positive control for mIFNα plasmid DNA, mIFNα protein (ICN Pharmaceuticals Inc., Costa Mesa, CA) was serially diluted and added to the wells. For the hIFN plasmid DNA, an interferon reference standard (human leukocyte interferon, Sigma Chemical Co., St. Louis, MO) was included in each assay. Following a 24-72 hour incubation, at 37°C, 50 μl of XTT/ECR substrate was added to each well. Plates were incubated for 6-24 hours at 37°C and the optical density (OD) at 490 nm was determined. Increasing amounts of interferon result in inhibition of cell proliferation and a reduction in the $OD_{490}$. The percent reduction in cell proliferation due to addition of the supernatants was determined by the formula:

$$1 - \frac{OD_{490} \text{ of cells incubated with interferon plasmid DNA supernatants}}{OD_{490} \text{ of cells incubated with control plasmid DNA supernatants}} \times 100$$

As shown in Table 1, both human interferons displayed the characteristic potent anti-proliferative activity against a wide variety of human tumor cell lines, with the most sensitive line being the NIH-OVCAR3 ovarian line and the least sensitive being the SK-OV-3 ovarian line. Also, the supernatants from the mIFNα pDNA (VR4111)-transfected UM449 cells inhibited the proliferation of murine B16F10 melanoma (generous gift from Dr. Suzuki at the University of Texas, Galveston, Texas), murine Cloudman melanoma S91 (American Type Culture Collection, Rockville, MD), and murine glioma 261 cell lines (Division of Cancer Treatment Tumor Repository, National Cancer Institute, Frederick Cancer Research and Development Center, Frederick, MD) by 40, 42 and 17%, respectively.

**Table 1. IFNω (VR4150) and IFNα (VR4102) *in vitro* biological assay: anti-proliferation activity against human tumor cell lines**

| | % reduction in cell proliferation (compared to control plasmid DNA supernatants) | |
|---|---|---|
| Cell line (tissue type) | Interferon ω | Interferon α |
| NIH-OVCAR3 (ovarian) | 60 | 43 |

(continued)

| | % reduction in cell proliferation (compared to control plasmid DNA supernatants) | |
|---|---|---|
| Cell line (tissue type) | Interferon ω | Interferon α |
| SCC-4 (squamous) | 36 | 36 |
| ACHN (renal) | 41 | 35 |
| A431 (epidermoid) | 24 | 19 |
| SCC-15 (squamous) | 29 | 29 |
| U87MG (glioblastoma) | 36 | 30 |
| A375 (melanoma) | 24 | 21 |
| PC3 (prostate) | 20 | 22 |
| UMUC3 (bladder) | 14 | 6 |
| A549 (lung) | 17 | 15 |
| MCF7 (breast) | 18 | 18 |
| SK-OV-3 (ovarian) | <10 | < 10 |

***b. Antiviral Activity***

An antiviral assay was performed to evaluate the ability of the supernatants from the interferon plasmid DNA-transfected cells to protect murine L929 cells or human A549 cells from infection by murine encephalomyocarditis (EMC) virus (Assay performed at IIT Institute, Chicago, IL). *In vitro* transfections were performed as described above and supernatants were collected from cells transfected with either VR4151 (hIFNT), VR4112 (hIFNα), VR4111 (mIFNα) or VR1055 (control). Antiviral activity of the supernatants was performed by IIT Research Institute (Chicago. IL). The antiviral assay evaluated the degree of protection of either human A549 or murine L929 cells from infection with murine encephalomyocarditis (EMC) virus. Briefly, 2.5 x $10^4$ L929 cells were plated into 96-well plates and incubated for 24 h. Tissue culture supernatants were serially diluted and added to the L929 cells which were incubated for another 24 h. Supernatants were then removed from the wells, the cells were washed and murine EMC virus was added to each well at a multiplicity of infection of 0.04. Assay plates were incubated further for 24 h followed by removal of supernatants, washing of wells, fixation with 5% formalin and staining with 1% crystal violet. Samples with interferon activity protected the cells from virus infection, resulting in darkly stained cell monolayers.

Supernatants from UM449 cells transfected with VR4151, VR4112, or VR4111 had antiviral activity of 30,000, 3,000 or 30 Units/ml, respectively, on human A549 cells. When evaluated for antiviral activity on the murine L929 cell line, supernatants from UM449 cells transfected with VR4151, VR4112, or VR4111 had antiviral activity of 300, 1000 and 30,000 Units/ml, respectively (Table 2) showing species specificity of the hIFNs for human cells and mIFNs for mouse cells.

**Table 2. Antiviral Activity of interferon Plasmid DNA**

| | Interferon (Units/ml) | |
|---|---|---|
| Plasmid | Human cell line | Murine cell line |
| VR4151 (hIFNω) | 30,000 | 300 |
| VR4112 (hIFNα) | 3,000 | 1,000 |
| VR4111 (mIFNα) | 30 | 30,000 |

***Example 4***

***Systemic Interferon Therapy; Intramuscular Administration of Cytokine-Expressing Plasmids***

***Cell lines and tumor models***

Murine B16F10 cells were grown in RPMI-1640 (GibcoBRL) and 5% fetal bovine serum (FBS). Murine Cloudman S91 cells were grown in Ham's F-10 medium with 25 mM Hepes, 0.1 mM, non-essential amino acids, 1 mM sodium pyruvate, 0.05 mM β-mercaptoethanol, 2.5% FBS and 12.5% horse serum. Human melanoma UM449 cells were grown in RPMI 1640 with 10% FBS.

Murine glioma 261 tumor fragments and M5076 reticulum cell sarcoma cells were obtained from the Division of Cancer Treatment Tumor Repository (National Cancer Institute, Frederick Cancer Research and Development Center, Frederick, MD). The glioma 261 tumor fragments ($2mm^3$) were initially implanted into the inguinal region of C57BL/6 mice using a 13 g trocar (Popper Sons, Inc., New Hyde Park, NY). Tumors which grew in the mice were used to establish a tumorigenic cell line. Minced tumor fragments were placed in Iscove's tissue culture medium with 10% FBS. Glioma 261 tumor cells began to attach to the flasks after several days, and the cells were propagated using standard tissue culture techniques. The M5076 cells were grown as ascites in C57BL/6 mice and frozen in liquid nitrogen. Human A431 cells were obtained from the American Type Culture Collection and were grown in DMEM and 10% FBS.

C57BL/6, DBA/2, nude (*nu*/*nu*) and beige-nude *(bg*/*nu*/*xid)* female mice between the ages of 6-8 weeks were obtained from Harlan Sprague Dawley (San Diego, CA). All animal experiments in this and the following examples were conducted in accordance with Vical's Institutional Animal Care and Use Committee as well as the standards set forth in the National Research Council guidelines concerning animal care and use.

To establish subcutaneous B16F10 melanoma tumors, C57BL/6, nude or nude-beige mice were injected subcutaneously on the flank with $10^4$ B16F10 cells. The Cloudman melanoma model was established by subcutaneous injection of $10^5$ Cloudman S91 cells on the flank of DBA/2 mice and the glioma 261 model was established by subcutaneous injection of $5 \times 10^4$ glioma 261 cells on the flank of C57BL/6 mice. To establish human epidermoid carcinomas, nude mice were injected subcutaneously on the flank with $5 \times 10^3$ A431 cells.

To establish intradermal M5076 tumors and liver metastases thereof, C57BL/6 mice were injected intradermally with $10^5$ M5076 reticulum cell sarcoma cells. In this model, primary intradermal tumors spontaneously metastasize to the liver. On day 29 after tumor cell injection, mice were sacrificed, livers removed and fixed in 10% buffered formalin (Fisher Scientific, Pittsburgh, PA), and the liver nodules were counted.

To establish lung metastases of B16F10 melanoma, C57BL/6 mice were injected intravenously with $2 \times 10^4$ B16F10 cells. On day 25 after tumor cell injection, the mice were sacrificed, lungs removed and fixed in 10% buffered formalin, and the lung nodules were counted.

To monitor the primary tumor growth, tumor sizes were determined 2-3 times per week by measuring with calipers ($1 \times w \times h$), and tumor volumes were determined using the formula: tumor volume ($mm^3$) = 0.52 ($1 \times w \times h$).

Tumor volume was analyzed using the Mann-Whitney U non-parametric Statistical Test to identify groups having significantly different mean weights. Mouse survival was analyzed using a Kaplan-Meier survival plot followed by a Logrank (Mantel-Cox) test to identify significant differences in survival between groups. Differences were considered statistically significant when the p value was less than or equal to 0.05.

***Intramuscular injections***

Fifty to 100 μg of plasmid DNA in 50 μl of saline was injected into the rectus femoris muscle of each hind leg for a total DNA dose of 100 to 200 mg. The muscle injections were performed using a 300 ml sterile tuberculin syringe fitted with a 28G ½ needle (Becton Dickenson) and a plastic collar cut from a 200 ml micropipette tip. The collar length was adjusted to limit the needle from penetrating further than 2 mm into the rectus femoris muscle.

***Serum levels of interferon following intramuscular injection of interferon plasmid DNA***

Serum samples from C57BL/6 mice injected intramuscularly with either VR4111 (mIFNα plasmid) or VR1055 (control plasmid DNA) were analyzed in a mIFNα ELISA (n=10). For the ELISA, 96-well plates (Immulon 4HBX high binding plates from Dynex Technologies, Chantilly, VA) were coated with rat anti-mouse IFNα monoclonal antibody (mAb) from Caltag Laboratories (Burlingame. CA) at a concentration of 5 μg/ml in 100 mM sodium carbonate buffer, pH 9.5 (50 μl per well). Plates were incubated with the coating mAb for 16 hours at 4°C. The plates were then washed 3 times with a wash buffer (phosphate-buffered saline (PBS)), pH 7.2 and 0.05% Tween-20 (Sigma, St. Louis, MO)). The plates were blocked in PBS containing 3% bovine serum albumin (BSA, Sigma) and 0.05% Tween-20 (400μl per well) and incubated for 24 hours at 4EC followed by washing three times with wash buffer.

Serum samples (10 μl) from mice injected intramuscularly with VR4111 (mIFNα) were mixed with 40 μl of assay buffer (PBS, 1% BSA, 0.05% Tween-20) and the mixture was added to each assay well. The positive control was mIFNα polypeptide (Biosource International, Camarillo, CA), which was serially diluted in assay buffer and 50 μl was added to the positive control wells. The negative control was serum from mice injected intramuscularly with VR1055. After adding the test samples and controls, the plates were incubated for 16 hours at 4°C. The plates were then washed 6 times with wash buffer, followed by addition of a sheep anti-mouse IFNα polyclonal antibody (pAb) (Biosource International, Camarillo, CA). The PAb was added at a 1:500 dilution in assay buffer (50 μl per well) and the plates were incubated for 5 hours at room temperature.

Following incubation with the pAb, the plates were washed six times with wash buffer followed by addition of anti-sheep IgG conjugated with peroxidase (Sigma) at a 1:5000 dilution in assay buffer (50 μl per well) and incubated for I hour at room temperature. The plates were washed 6 times with wash buffer and 200 μl of 3,3',5,5'-tetramethylbenzidine liquid substrate (TMB) (Sigma Chemical Co.) was added per well. Plates were incubated at room temperature for 30 minutes, followed by determination of the optical density of the wells at 650 nm. A standard curve was generated by plotting the ng/ml of mIFNα polypeptide versus the optical density at 650 nm. The concentration of mIFNα in the test serum samples was determined from the linear portion of the mIFNα standard curve. The sensitivity of the mIFNα ELISA was 50 ng/ml.

C57BL/6 mice injected intramuscularly with VR4111 had detectable serum levels of mIFNα after 5 intramuscular injections of 100 μg VR4111 (twice a week for two weeks, followed by one injection the next week). The average serum level of mIFNα after 5 intramuscular injections of VR4111 was 1465 ng/ml (average of 16 mice). At the time of this study, no commercial mIFNα ELISA kit had been developed. Since sensitivity of the in-hose mIFNα ELISA is 50 ng/ml, lower serum levels of mIFNα could exist in the mice at earlier timepoints, but we were unable to detect this in our ELISA.

To determine the serum levels of hIFNω, C57BL/6 or nude mice received a single intramuscular injection of 100 μg of VR4151 (hIFNω plasmid DNA) or VR1055 (control plasmid DNA) (50 μg per leg bilaterally) in the rectus femoris. Serum samples were collected daily over a two week period and analyzed in the hIFNω ELISA kit (Alexis, San Diego, CA) which was sensitive to 2 pg/m!. Serum samples were collected from 4-5 mice per day. In the C57BL/6 mice, measurable serum levels of hIFNω were detected as early as one day after injection (69 pg/ml) (Figure 2A). In these mice, peak serum levels were found six days after injection (254 pg/ml) and expression continued out to day 14 (50 pg/ml), the final timepoint of the study.

In nude mice, serum levels of IFNω were found as early as one day after injection (133 pg/ml). Peak serum levels were found on day 7 (648 pg/ml) and expression continued out to day 14 (134 pgml), the final time point of the study (FIG. 2B). Thus, interferon could be detected in the serum after a single intramuscular injection of an interferon-encoding plasmid DNA.

***Systemic interferon treatment inhibits primary tumor growth***

As shown in FIGs. 3-5 and FIG. 7A, mice bearing different tumors were found to significantly benefit from intramuscular injection of different cytokines. To test the efficacy of IFNα plasmid, C57BL/6 mice bearing subcutaneous B16F10 melanoma, subcutaneous glioma 261, or intradermal M5076 tumors, or DBA/2 mice bearing subcutaneous Cloudman melanoma were injected with 100 μg either of VR4111 (mIFNα) or VR1055 (control), twice per week for three weeks, beginning on day 4 after tumor cell injection (n = 8-10 mice per group). In all three subcutaneous tumor models, the mice treated intramuscularly with VR4111 had a significant reduction in tumor volume ($p<0.05$) (FIGs. 3A, 3C, and 3E), and a significant enhancement of survival ($p<0.02$) compared to the mice that received the control plasmid (FIGs. 3B, 3D, and 3F). In the intradermal tumor model, mice treated with intramuscular VR4111 had a significant reduction in primary tumor volume ($p<0.001$) compared to the mice that received the control plasmid (FIG. 7A).

To compare the efficacy of IL-2, IL-12 to IFNα plasmids, C57BL/6 mice bearing subcutaneous B16F10 melanoma were injected with 100 μg of VR4111 (mIFNα), VR4001 (mIL-12), VR1110 (mIL-2), or VR1012 (control) (n = 15-16 mice per group) twice per week for three weeks. Mice receiving intramuscular injections of VR4111 had a significant reduction in tumor growth ($p < 0.0002$) (FIG. 4A) by day 17 as well as a significant increase in survival ($p = 0.00001$) (FIG. 4B). By day 28 of the study, 100% of the VR4111-treated mice were still alive, compared to only 20% of the VR1012-treated mice. Mice treated with VR1110 had a modest reduction in tumor growth by day 17 ($p < 0.02$) (FIG. 4A) but did not have an increase in survival compared to the VR1012-treated mice (FIG. 4B). Mice treated with VR4001 also had a modest reduction in tumor growth by day 17 ($p < 0.03$) (FIG. 4A) as well as a significant increase in survival ($p = 0.02$) (FIG. 4B). By day 28, 55% of the mice treated with VR4001 were alive, compared to 20% of the VR 1012-treated mice.

To test the efficacy of IFNω, mice bearing human A431 tumors between 30-80 $mm^3$ were injected intramuscularly with 200 μg of either VR4151 (hIFNω) or VR1055 (control) twice per week for three weeks (n=15). Mice bearing subcutaneous A431 tumors and injected intramuscularly with VR4151 had a significant reduction in tumor volume ($p < 0.05$) (FIG. 5A) and a significant increase in survival ($p < 0.05$), compared to the mice that received the control plasmid (FIG. 5B).

***Systemic mIFNα plasmid DNA treatment inhibits the growth of tumor metastases***

As shown in FIG. 6 and FIG. 7, mice bearing different tumor metastases were found to significantly benefit from intramuscular injection of IFNα. C57BL/6 mice bearing lung metastases of B16F10 melanoma were injected intramuscularly with 100 μg of either VR4111 (mIFNα) or VR1055 (control) twice per week for three weeks, beginning on day 4 after tumor cell injection (n=10). On day 25 after tumor cell injection, the mice were sacrificed, lungs removed and fixed in 10% buffered formalin (Fisher Scientific, Pittsburgh, PA) followed by counting of lung nodules.

While 70% of the control plasmid-treated mice had lung nodules that were too numerous to count, 80% of the mice treated with mIFNα plasmid DNA had 10 or fewer nodules (FIG. 6). TNTC denotes lungs with nodules that were too numerous to count.

In the liver metastases model, C57BL/6 mice bearing intradermal M5076 murine reticulum sarcoma were injected intramuscularly with 100 μg of either VR4111 or VR1055 twice per week for three weeks, beginning on day 4 after tumor cell injection (n=10-13 mice per group). On day 29 after tumor cell injection, the mice were sacrificed, livers removed and fixed in 10% buffered formalin (Fisher Scientific, Pittsburgh, PA) followed by counting of liver nodules.

While the control plasmid-treated mice had a mean of 190 hepatic tumor nodules or had nodules that were too numerous to count, mIFNα plasmid DNA-treated mice had a mean of 35 hepatic tumor nodules (FIG. 7B).

These results demonstrate that intramuscular injection of mIFNα plasmid DNA can effectively inhibit the growth of both primary and metastatic lesions. Thus, for patients with metastatic disease, intramuscular administration of therapeutic plasmid DNAs would be advantageous for the treatment of undiagnosed or inaccessible metastatic lesions.

***Regimen optimization of mIFNα therapy in the B16F10 melanoma model***

A regimen optimization study was conducted to evaluate the antitumor efficacy of fewer injections and/or a lower dose of VR4111 (mIFNα) in the subcutaneous B16F10 melanoma model. C57BL/6 mice were injected with either 100 or 50 μg of VR4111 or VR1055 over a 6 week period (n=10). Mice received intramuscular injections either twice per week, once per week or once every other week. All intramuscular injections began four days after the initial subcutaneous B16F10 tumor cell injection. Mice which received intramuscular injections of 100 μg of VR4111 at any of the time courses had a significant reduction in tumor volume ($p \leq 0.005$) and a significant increase in survival ($p=0.007$) (FIGs. 8A and 8B). The mice which received the 100 μg dose of VR4111 once every other week for 6 weeks had a total of only three intramuscular injections with significant antitumor efficacy. In contrast, mice receiving the 50 μg dose of VR4111 revealed a dose response based on the frequency of injection. While mice injected with 50 μg of VR4111 once or twice per week had a significant reduction in tumor volume ($p \leq 0.03$), and a significant increase in survival ($p=0.002$), mice injected only once every other week did not have a significant antitumor response for either tumor growth or survival (FIGs. 8C and 8D).

***Mechanism of mIFNα Antitumor Effect***

To investigate the role of natural killer (NK) and T cells in mediating the antitumor effect of systemically delivered mIFNα, VR4111 or VR1055 was administered intramuscularly to nude mice (which are T cell deficient) and to beige-nude mice (which are NK and T cell deficient) bearing subcutaneous B16F10 melanoma tumors. Beginning on day 4 after injection of $10^4$ B16F10 cells, 50 μg of plasmid DNA in 50 μl of saline was injected into the rectus femoris muscle of each hind leg for a total DNA dose of 100 μg twice per week for three weeks (n=15 mice per group).

There was neither a significant reduction in tumor volume nor enhancement of survival in the nude mice (FIGs. 9A and 9B) or beige-nude mice (FIGs. 9C and 9D). These results suggest that T cells may be required for the mIFNα antitumor response. NK cells appeared not to be required for the antitumor effect since nude mice ($NK^+$) did not have a greater antitumor response compared to the beige-nude mice ($NK^-$).

To further explore the role of T cells in the antitumor effect of mIFNα DNA therapy, C57BL/6 mice bearing subcutaneous B16F10 tumors were injected with depleting doses of monoclonal antibodies (mAbs) specific for either $CD4^+$ or $CD8^+$ T cells. For depletion of T cell subsets, anti-CD4 (clone Sk1.5, rat IgG) and anti-CD8 (clone 2.43, rat IgG) hybridomas (American Type Culture Collection, Rockville, MD) were used to generate the corresponding mAb. The anti-CD8 hybridoma was grown as ascites in nude mice and the mAbs were purified from ascites using ion exchange chromatography (Harlan Bioproducts for Science, San Diego, CA). Thp anti-CD4 hybridoma was grown *in vitro* with Dulbecco's Modified Eagle Medium, 10% fetal bovine serum and low IgG. The anti-CD4 mAb was purified from tissue culture supernatant by ammonium sulfate precipitation to 30%. The protein pellet was resolubilized and extensively dialyzed in Dulbecco's $Ca^{2+}/Mg^{2+}$-free phosphate-buffered saline (Zymed Laboratories Inc., San Francisco, CA).

Beginning on day 4 after subcutaneous injection with $10^4$ B16F10 cells, mice were injected intramuscularly with 100 μg of either VR4111 or VR1055 twice per week for three weeks. For depletion of $CD4^+$ and $CD8^+$ T cells, mice were injected intraperitoneally with 500 μg of either the anti-CD4 mAb (clone GK1.5, rat IgG) or anti-CD8 mAb (clone 2,43, rat IgG) one day prior to each intramuscular DNA injection (n = 10 mice per group). Control tumor-bearing mice were injected intraperitoneally with 500 μg of normal rat IgG (Simga Chemical Co., St. Louis, MO) (n=10). To assure complete depletion, sentinel mice were injected according to the same regimen, and once per week, spleens were collected, dissociated and assessed for the presence of $CD4^+$ and $CD8^+$ T cells. Spleen cells were stained with FITC-conjugated anti-CD4 and PE-conjugated anti-CD8 mAbs (Pharmingen, San Diego, CA) and analyzed by flow cytometry (Cytometry Research Services, San Diego, CA). The depletion of $CD4^+$ and $CD8^+$ T cells was consistently greater than 98%, as determined by cytometry.

The mIFNα DNA therapy significantly reduced tumor growth ($p \leq 0.002$) and enhanced survival ($p \leq 0.008$) of both normal mice and mice depleted of $CD4^+$ T cells, compared to mice injected with control plasmid and treated with normal IgG (FIGs. 10A and 10B). These results suggest that $CD4^+$ T cells are not required for the mIFNα antitumor effect. In contrast, mice depleted of $CD8^+$ T cells and injected with mIFNα DNA displayed tumor volumes and survival profiles that were not significantly different from mice treated with the control plasmid (FIGS. 10A and 10B). This result suggests that $CD8^+$ cells are involved in the mIFNα antitumor response.

*Example 5* A reference example.

*Local Interferon Therapy: Intratumoral Administration of interferon Plasmids*

The anti-tumor activity of IFNω and IFNα was evaluated *in vivo* in nude mice bearing subcutaneous human ovarian (NIH-OVCAR3) or human melanoma (A375) (nude/human/xenograft model), or in C57BL/6 mice bearing murine melanoma (B16F10) tumors following intratumoral administration of DNA complexed with a cationic lipid.

*Cell Lines and Tumor Models*

Athymic nude *(nu/nu)* and C57BL/6 mice between the ages of 6-10 weeks were obtained from Harlan Sprague Dawley (San Diego; CA).

Human A375 melanoma cells and human NIH-OVCAR3 ovarian carcinoma cells were:obtained from the American Type Culture Collection (Rockville, MD), and grown in Dulbecco's modified Eagle's medium (GibcoBRL, Gaithersburg, MD) supplemented with 10% FBS. B16F10 cells were a generous gift from Dr. Suzuki at the University of Texas (Galveston, Texas). Cells were grown in RPMI-1640 (GibcoBRL) and 5% fetal bovine serum (FBS).

To establish subcutaneous A375 melanoma tumors and subcutaneous NIH-OVCAR3 ovarian tumors, athymic nude/nude mice (10 mice/group) were injected subcutaneously with 5 x $10^6$ A375 cells and 5 x $10^7$ NIH-OVCAR3 cells respectively. To establish subcubaneous murine B16F10 melanoma tumors, C57BL/6 mice were injected subcutaneously with $10^4$ B16F10 cells.

Mice were monitored for tumor growth and survival. Tumor sizes were determined 3 times per week by measuring with calipers (I x w x h) and tumor volumes were determined using the formula: tumor volume ($mm^3$)= 0.52 (1 x w x h). Statistical analysis was done as described in Example 4.

***Local Interferon Plasmid DNA Inhibits Tumor Growth***

Established subcutaneous A375 human melanoma and NIH-OVCAR3 human ovarian carcinoma tumors in nude mice were transfected *in vivo* by intratumoral administration of pDNA/DMRIE-/DOPE (DNA: lipid) complexes (n=10). When tumors became palpable (80-300 $mm^3$, at day 27 post tumor cell implant for the A375 cells, and at day 41 post tumor cell implant for the NIH-OVCAR3 cells), mice were injected intratumorally with 100 μg of VR4112 (hIFN"), VR4151 (hIFNT), VR1055 (control) or VR1012 (control) complexed with DMRIE/DOPE (1:1 DNA:DMRIE mass ratio). Tumor bearing animals were treated intratumorally with DNA: lipid for 6 consecutive days followed by 5 treatments every other day for a total of 11 treatments (A375 melanoma model), or every other day for a total of 11treatments (NIH-OVCAR3 ovarian cancer model).

As shown in FIG. 11A, in the A375 model of melanoma, the direct intratumoral injection of VR4151: liquid complex for 6 consecutive days, followed by 5 additional injections every other day (100 μg plasmid DNA/injection, total of 11 injections), resulted in a statistically significant slowing of tumor growth, as compared to the control ($p < 0.03$, days 40-44).

As shown in FIG. 2B, in the NIH-OVCAR3 model of ovarian cancer, the direct intratumoral injection of VR4151: lipid complex every other day for a total of 11 injections (100 μg plasmid DNA/injection), resulted in sustained and statistically significant reduction in tumor growth as compared to the control plasmid control (p< 0.001 - 0.05, Days 45-65). A similar treatment regimen with VR4112: lipid was found to have a moderate effect on tumor growth which only reached statistical significance vs. the control plasmid at two time-points during the study ($p < 0.05$, days 53 and 57).

As shown in FIG. 12A, in the B16F10 melanoma model, direct intratumoral injection of mIFNα resulted in decrease in tumor volume. Once palpable tumors were established (80-300$mm^3$) on day 12 after tumor cell injection in C57B/6 mice bearing subcutaneous B16F10 melanoma tumors, mice were injected intratumorally with 100 μg of either VR4101 (mIFNα) or VR1012 (control) complexed with the cationic lipid, DMRIE/DOPE at a DNA/DMRIE mass ratio of 1:1 in a 100 μl volume. Mice received six consecutive intratumoral injections of either VR4101 or VR1012 (n=10 mice per group). As shown in FIG. 12A, although not statistically significant, intratumoral injection of VR4101 resulted in a 54% reduction in tumor volume by day 19 of the study. As shown in FIG. 12B, a significant increase in survival ($p = 0.02$) was found for the VR4101-treated mice, compared to the mice that received VR1012.

***Example 6***

***Local Cytokine Therapy: Intraperitoneal Administration of Cytokine-expressing Plasmids***

The goal of this example is to show that the present invention provides an effective method of treating malignant tumors of murine ovarian carcinoma via intraperitoneal (i.p.) injection of cytokine-expressing plasmid DNA. Since late-stage ovarian carcinoma is usually limited to the peritoneal cavity, it was envisioned that continuous secretion of a cytokine in this cavity would produce beneficial anti-tumor immune response. In particular, the present example clearly shows that the ovarian cancer therapy by intraperitoneal injection of a cytokine-expressing plasmid DNA:lipid complexes (1) results in sustained levels of the cytokine in the ascites, avoiding the need for frequent injections of the protein (in contrast to intraperitoneal injection of recombinant cytokine wherein the cytokine level declines shortly after injection), (2) targets tumor ascites, rather than peritoneal tissues (suggesting that systemic cytokine side effects should be reduced using this method), (3) inhibits tumor growth and enhances survival, and (4) can be combined with debulking of tumor ascites to enhance the antitumor effect.

***Cell Lines and Tumor Models***

As a model for human ovarian cancer, the murine ovarian teratocarcinoma (MOT) model in C3H/HeN mice was used. MOT exhibits many of the characteristics of late-stage human ovarian cancer including peritoneal spread, production of tumor ascites and tumor cell blockage of lymphatics (Ozols et al., 1979 and Fekete et al., 1952).

Murine ovarian teratocarcinoma (MOT) cells were obtained from Dr. Robert Knapp and Dr. Robert C. Bast at the Dana-Farber Cancer Center (Boston, MA). The MOT cells ($10^5$) were grown by serial intraperitoneal (i.p) transplantation in C3H/HeN mice and a stock of the cells was frozen in liquid nitrogen.

CTLL-2 cells were obtained from the American Type Culture Collection (ATCC, Rockville,MD) and were grown in RPMI 1640 with glutamine, 1% sodium pyruvate, 1% penicillin-streptomycin (Life Technologies, Gaithersburg, MD), 10% fetal bovine serum (HyClone, Logan, Utah) and 10 U/ml murine IL-2 (Boehringer Mannheim, Indianapolis, IN).

C3H/HeN and nude (*nu*/*nu*) female mice between the ages of 6-10 weeks were obtained from Harlan Sprague Dawley (San Diego, CA). All animal experiments were conducted in accordance with Vical's Institutional Animal Care and Use Committee as well as the standards set forth in the National Research Council guidelines concerning animal care and use.

To establish i.p. MOT tumors, C3H/HeN mice were injected i.p. with $10^5$ MOT cells in 100 ul of medium. In the MOT tumor model, tumor growth is typically monitored by weighing the mice which reflects the increase in volume of tumor ascites (Berek et al., Cancer Res., 44:1871-1875, 1984). The nude mouse study were performed in the same manner as the studies in C3H/HeN mice with injection i.p. of $10^5$ MOT cells and monitoring the weight of the mice. Statistical analysis on mouse weight and survival was done as described in Example 4.

***Preparation of plasmid DNA:lipid complexes and intraperitoneal injection***

To yield a pDNA:DMRIE mass ratio of 1:1, 100 $\mu$g of VR1110 (mIL-2) was diluted in 500 ul 0.9% saline. (Radix Labs, Eau Claire. WI), DMRIE/DOPE lipid (100 $\mu$g DMRIE) was diluted in 500 ul of 0.9% saline in a separate vial, and the pDNA and cationic lipid were combined and vortexed for 5 seconds. To yield a pDNA:DMRIE mass ratio of 5:1, 500 $\mu$g of mIL-2 pDNA was diluted in 500 ul 0.9% saline (Radix Labs, Eau Claire, WI); DMRIE/DOPE lipid (100 $\mu$g DMRIE) was diluted in 500 ul of 0.9% saline in a separate vial, and the pDNA and cationic lipid were combined and vortexed for 5 seconds.

The 1 ml pDNA:DMRIE/DOPE (DNA: lipid) complex was injected i.p. into mice bearing i.p. MOT tumors on various days after tumor cell implant. Control MOT tumor-bearing mice received i.p. injections of VR1012 (control): lipid at the same ratio (1:1 pDNA:DMRIE, 100 $\mu$g pDNA) and were injected i.p. on the same days as the cytokine-expressing or reporter gene treatment groups.

***Intraperitoneal injection of plasmid DNA:lipid results in targeted expression in tumor ascites***

The pDNA:lipid therapy was evaluated for the ability to target malignant cells within a cavity. C3H/HeN mice were injected i.p. with $10^5$ MOT cells followed by i.p. injection of 100 $\mu$g of VR1223 (luciferase): lipid (1:1 pDNA:DMRIE mass ratio) on days 5 and 6 after tumor cell implant. Control MOT tumor-bearing mice were injected i.p. with 100 $\mu$g of either VR1012: lipid or VR1223 without lipid on days 5 and 6 after MOT tumor cell injection. An additional group of control mice did not receive MOT tumor cells and were injected i.p. with VR1223: lipid on the same days as the other treatment groups (n=3). Three days later the mice were euthanized and tumor ascites and tissues (liver, kidney, spleen, diaphragm, intestine and ovary) were collected. Luciferase was extracted from the tissues by freeze-thawing and grinding of the samples in cell lysis reagent (Promega, Madison, WI) as previously described (Hartikka et al., Hum. Gene Ther., 7: 1205-1217, 1996). The tumor ascites was diluted 1:5 in cell lysis reagent followed by three cycles of freeze-thaw and collection of supernatant from the cell lysate. Samples were read in a microplate luminometer (Dynatech, Chantilly, VA) following addition of luciferase substrate (Promega, Madison, WI). The relative light units (RLU) of the samples were determined from a standard curve using purified firefly luciferase (Analytical Luminescence Laboratory, Sparks, MD). The protein concentration of each sample was determined using the BCA protein assay kit (Pierce Chemical Company; Rockford, IL). Luciferase levels were expressed as RLU per mg of protein. There may be a decrease in IL-2 pDNA expression from day 1 to day 3 post DNA injection.

On day three, tumor ascites had 900,000 RLU of luciferase/mg, while diaphragm and ovary tissue had only 327 and 16 RLU/mg (FIG. 13). Kidney, liver, spleen and intestinal tissue had no detectable luciferase activity. These results suggest that i.p. injection of pDNA: lipid complexes appears to target the tumor ascites in the peritoneal cavity with limited or negligible transfection of surrounding tissues. Luciferase detected in the diaphragm and ovary tissue was only found in MOT tumor-bearing mice injected with VR1223: lipid. When naive non-tumor bearing mice were injected with the same DNA: lipid complex, no luciferase activity was found in any of the tissues (data not shown). These results suggest that the low levels of luciferase in the diaphragm and ovary in MOT tumor-bearing mice may reflect metastases of tumor cells to these tissues. Tumor-bearing mice injected with luciferase pDNA without cationic lipid had no luciferase activity in either tumor ascites or surrounding tissues, indicating that lipid is required for optimal *in vivo* transfection of ovarian tumor ascites. Mice injected with VR1012: lipid had no detectable luciferase activity in either tumor ascites or tissues.

A follow-up study investigated the specific cell type in the ovarian tumor ascites that was transfected after i.p. injection of a reporter gene pDNA:DMRIE/DOPE complex. On days 5 and 6 after tumor cell implant ($10^5$ cells), C3H/HeN mice were injected i.p. with 100 μg of VR1412 (β-galactosidase (β-gal)): lipid, VR1012: lipid (1:1 DNA:DMRIE mass ratio), or with VR1412 without cationic lipid (n=3 mice per group). One day later the mice were sacrificed and the tumor ascites was collected. The ascites was spun at 2500 rpm for 2 minutes to pellet the cells, and the supernatant was removed. The tumor cells were fixed in 10% buffered formalin (Fisher Scientific, Pittsburgh, PA), placed in a cryomold containing OCT embedding medium (VWR, S. Plainfield, NJ), frozen in isopentane and then stored at - 70°C. The embedded and frozen samples were then cryostat sectioned (5 um), further fixed (0.5% glutaraldehyde in PBS), washed (PBS), stained with X-gal reagent (1 mg/ml X-gal diluted in PBS containing 5 mM potassium ferricyanide, 5 mM potassium ferrocyanide, and 1 mM magnesium chloride), washed again (PBS), and counterstained with hematoxylin and eosin. (The samples were cryostat sectioned and stained by Pathology Associates (Frederick, MD).)

The ascites from mice treated with either VR1012 or VR1412 without lipid had no β-gal activity in the samples. In contrast, the tumor ascites from mice injected with VR1412: lipid had β-gal staining primarily in the tumor cells (data not shown). In a few slides, several macrophages and lymphocytes were also positive for β-gal while neutrophils were negative for β-gal.

***Intraperitoneal injection of IL-2 pDNA:lipid results in sustained expression of IL-2 in tumor ascites***

A time-course study was done to determine the length of time that IL-2 could be detected after multiple i.p. injections of IL-2 pDNA: lipid or a single i.p. injection of either IL-2 protein or IL-2 pDNA: lipid in mice bearing i.p. ovarian tumor ascites. Beginning on day 5 after tumor cell injection, mice were injected multiple times with VR1110 (mIL-2): lipid or a single time with VR1110: lipid or IL-2 protein. Mice were sacrificed at various times and ascites and serum were analyzed for IL-2 levels. Ascites was collected from the sacrificed mice, the samples were spun at 14,000 rpm for 2 minuts and the supernatant was harvested. Blood was collected from the mice on the same day as the ascites collection and the serum was separated from blood cellls by allowing the blood to clot in serum separator tubes (Microtainer, Becton Dickinson, Franklin Lakes, NJ) followed by centrifugation at 14,000 rpm for 10 minutes and collection of the serum supernatant. IL-2 concentration (pg/ml) in the ascites and serum samples was determined using a murine IL-2 ELISA (R & D Systems, Minneapolis, MN). Since the volume of tumor ascites increases over time, the volume of ascites was also determined for each mouse. The total concentration of IL-2 in ascites was determined using the formula: IL-2 pg/ml x ml of ascites = pg IL-2/ total ascites. Serum IL-2 concentrations were reported as pg/ml.

*IL-2 in serum and ascites after multiple injections of IL-2 pDNA: lipid.*

Beginning on day 5 after tumor cell injection, mice were injected with VR1 110: lipid for either 2, 4 or 6 consecutive days or with control VR1012: lipid for 6 consecutive days (100 μl of plasmid DNA complexed with 100 μl DMRIE/DOPE at 1:1 mass ratio in a total volume of 1 ml). An additional group of mice received VR1110 that was not complexed with DMRIE/DOPE. Every two days for up to 17 days after the DNA:lipid injections, 3-4 mice were sacrificed per treatment group and ascites and serum were collected and analyzed using mIL-2 ELISA assay as described above.

Two injections of VR1110: lipid (100 μl DNA per day, 1:1 DNA:cationic lipid mass ratio) into mice bearing i.p.

MOT ovarian tumors yielded high levels of IL-2 protein in the tumor ascites. One day after VR11 10: lipid i.p. injection, 28,000 pg/ml of IL-2 was measured in the tumor ascites (Table 3). IL-2 expression in the ascites continued for over two weeks after DNA:lipid injection with 750 g/ml detected 17 days after the last pDNA:lipid injection. Mice injected with either four or six consecutive injections of VR1110: lipid also had high levels of IL-2 in the tumor ascites; however, due to the very high expression levels after more frequent VR1110: lipid injections, IL-2-mediated side effects were noted in the mice which did not survive beyond day 9 or 13 after DNA injection. In contrast, two consecutive injections of VR1110- plus DMRIE/DOPE did not cause observable IL-2 side effects yet IL-2 expression levels remained high for over 2 weeks. The IL-2 expressed after i.p. DNA:lipid injection of MOT-bearing mice appeared to remain localized in the peritoneal cavity as the IL-2 serum levels after VR1110: lipid i.p. injection were always less than 10% of the levels in the tumor ascites. Injection of VR1110 without lipid yielded very low levels of IL-2 in ascites and serum (0-32 pg/ml). Injection of the control vector, VR1012, resulted in only background levels of IL-2.

Table 3

mIL-2 concentration in ascites (pg/ml)

| Treatment | Days post tumor cell injection | | | | | | | | |
|---|---|---|---|---|---|---|---|---|---|
| | 7 | 9 | 11 | 13 | 15 | 17 | 19 | 21 | 23 |
| VR1110 (mIL-2) without DM/DP. 6 injs. | 28 | 12 | 0 | 1 | 0 | 1 | 0 | | |
| VR1012 (control) + DM/DP, 6 injs. | 0 | 0 | 8 | 0 | 0 | 1 | 43 | | |
| VR1110 (mIL-2) + DM/DP, 6 injs. | | | 7196 | 7716 | 7824 | 3895 | 3200 | | |
| VR1110 (mIL-2) + DM/DP, 4 injs. | | 3524 | 4187 | 5968 | 3050 | 3984 | 2392 | 441 | |
| VR1110 (mIL-2) + DM/DP, 2 injs. | 28882 | 4750 | 11725 | 8047 | 1246 | 1445 | 1407 | 774 | 753 |

mIL-2 concentration in serum (pg/ml)

| Treatment | Days post tumor cell injection | | | | | | | | |
|---|---|---|---|---|---|---|---|---|---|
| | 7 | 9 | 11 | 13 | 15 | 17 | 19 | 21 | 23 |
| VR1110 (mIL-2) without DM/DP. 6 injs. | 0 | 4 | 0 | 32 | 0 | 0 | 0 | | |
| VR1012 (control) + DM/DP, 6 injs. | 0 | 0 | 0 | 0 | 0 | 0 | 62 | | |
| VR1110 (mIL-2) + DM/DP, 6 injs. | | | 66 | 85 | 34 | 5 | 4 | | |
| VR1110 (mIL-2) + DM/DP, 4 injs. | | 114 | 81 | 40 | 10 | 3 | 2 | 0 | |
| VR1110 (mIL-2) + DM/DP, 2 injs. | 625 | 418 | 18 | 76 | 6 | 0 | 1 | 0 | 0 |

*IL-2 in serum and ascites after single injection of pDNA:lipid injection compared to protein injection.*

Five days after i.p. injection of $10^5$ MOT tumor cells, C3H/HeN mice were injected with 100 μg of either VR1110: lipid or VR1012: lipid (1:1 DNA:DMRIE mass ratio) or with 100 μg of VR1110 without lipid. For the IL-2 protein-treated group, mice were injected with 1 μg recombinant murine IL-2 protein (R & D Systems, Minneapolis, MN). The pDNA: lipid, pDNA alone, or recombinant protein was injected i.p. in a total volume of 1 ml saline per mouse. Five mice from each group were sacrificed beginning at 4 hours and continuing on days 1, 2, 3, 6 and 10 post DNA or protein injection. Ascites and serum were collected and analyzed using mIL-2 ELISA assay as described above.

Mice injected i.p. with IL-2 protein had peak levels of IL-2 in ascites (10 ng) at 4 hours after injection of IL-2 and a 1000-fold reduction in IL-2 one day.later (0.009 ng) (FIG. 14A). In contrast, mice injected with IL-2 pDNA: lipid had peak IL-2 levels in ascites 2 days after injection (64 ng) and only a 2.6 fold reduction in IL-2 by 10 days after injection (25 ng) (FIG. 14A). These results indicate that mice receiving IL-2 pDNA: lipid have more sustained levels of IL-2 in the ascites compared to mice receiving IL-2 protein. Tumor-bearing mice injected i.p. with either VR1012: lipid or VR1110 without cationic lipid had no IL-2 in the tumor ascites. In a related study, MOT tumor-bearing mice injected i.p. with 10-fold less VR1110: lipid (10 μg DNA) still had detectable IL-2 in the tumor ascites 11 days after the VR1110: lipid injection (data not shown).

Serum levels of IL-2 after either i.p. protein or DNA injection reflected a similar pattern as that found in tumor ascites; however, the serum IL-2 levels were markedly reduced compared to the ascites IL-2 levels. Four hours after protein injection, IL-2 in the serum was 2.4 ng/ml and negligible by ohe day after protein injection. Serum levels of IL-2 one day after VR1110: lipid injection was 1 ng/ml and undetectable by 6 days after the injection (FIG. 14B). These results suggest that the majority of the IL-2 after either IL-2 protein or pDNA: lipid delivery remains in the peritoneal cavity.

***Intraperitoneal injection of IL-2 plasmid DNA:lipid inhibits tumor growth and enhances survival***

*Six consecutive-day treatments.* The plasmid DNA:lipid therapy was evaluated for the ability to reduce tumor growth and to increase survival of mice with MOT tumors. On day 5 after MOT tumor cell injection, mice were injected i.p. with 100 μg of either VR1110 or VR1012, both complexed witch DMRIE/DOPE. The plasmid DNA was complexed at either a 5:1 or 1:1 DNA:DMRIE mass ratio. An additional treatment group received VR1110 that was not complexed with lipid. A total volume of 1 ml DNA:lipid or DNA alone in physiological saline was injected i.p. The DNA treatments occurred over 6 consecutive days, beginning on day 5 (days 5-10). MOT tumor growth was measured by weighing the mice. All treatment groups consisted of 10 mice per group.

The high IL-2 expression level in ascites was accompanied by significant antitumor effects. Mice treated with VR1110: lipid on days 5-10 after tumor cell injection had a significant reduction in MOT tumor growth compared to the mice treated with the VR1012: lipid (p=0.01) (FIG. 15A). A significant enhancement in survival was also found for mice injected i.p. with VR1110: lipid (p=0.05) (FIG. 15B).

Complexing the pDNA with a cationic lipid seemed necessary for the antitumor effect as treatment of tumor-bearing mice with VR1110 without lipid was not effective (FIGs. 15E and 15F). Furthermore, a 1:1 DNA:cationic lipid mass ratio was found to be more affective at reducing tumor burden and increasing survival than a 5:1 DNA:cationic lipid mass ratio (FIGs. 15C and 15D).

*Three alternative-day treatments.* C3H/HeN mice bearing i.p. MOT tumor ascites were injected i.p. with VR1110; lipid or with VR1012: lipid (100 μg DNA) on days 5, 8 and 11 after tumor cell implant. By day 14 post tumor cell injection, mice treated with VR1110: lipid had a significant reduction in mean weight (p=0.001) compared to the mice treated with the control pDNA: lipid (FIG. 16A). In addition, a significant increase in survival (p=0.008) was found for the VR1110: lipid-treated mice compared to the mice treated with the VR1012: lipid (FIG. 16B). By day 26 post .tumor cell injection, none of the mice treated with the VR1012 were still alive, while 50% of the mice treated with VR1110: lipid remained alive. By day 55 post tumor cell injection, 20% of the mice treated with VR1110: lipid appeared to be tumor-free.

Whether the IL-2 pDNA: lipid antitumor effect required T cells was investigated by implanting nude mice with i.p. MOT tumors followed by same VR1110: lipid regimen used in the C3H/HeN tumor-bearing mice (DNA treatment on days 5, 8 and 11 after tumor cell implant). No significant antitumor effect was found for the nude mice treated with VR1110: lipid suggesting that T cells may be required for the antitumor effect (data not shown).

***IL-2 plasmid DNA:lipid antitumor effect enhanced by debulking of tumor ascites,***

Debulking of tumor ascites is commonly performed on human ovarian cancer patients. A similar procedure was performed in the mice bearing MOT tumors and treated with VR1110: lipid. Mice bearing MOT tumors and injected i.p. with 100 ul DNA:lipid on days 5-10 as described above (1:1 DNA:lipid mass ratio), were also debulked of tumor ascites on day 14 after tumor cell injection (4 days after the last DNA:lipid injection). Mice were debulked of 5 ml of tumor ascites by insertion of a 22 G needle attached to a 5 ml syringe and removal of 5 ml of fluid. The mice were anesthetized with methoxyflurane during the debulking procedure. All treatment groups in this experiment consisted of 8-10 mice per group.

Debulking of ovarian tumor ascites in mice previously treated with IL-2 plasmid DNA:lipid further enhanced the efficacy of the treatment resulting in a significant reduction in tumor growth (p=0.01) and an increase in survival. Forty four percent of the IL-2 plasmid DNA:lipid-treated and debulked mice were alive on day 57 vs. 17% of the plasmid control-treated and debulked mice (FIG. 17). These results show that plasmid-mediated gene therapy in combination with conventional procedures such as debulking of tumor ascites may hold promise for future treatment of human ovarian cancer.

*Dose-response of IL-2 pDNA: lipid*

A dose-response study was initiated to determine the minimum dose of VR1110: lipid that could still result in a significant antitumor effect. C3H/HeN mice were injected with 25, 50 or 100 μg of IL-2 pDNA: lipid on days 5, 8 and 11 after MOT tumor cell injection. A control group of MOT tumor-bearing mice were injected with 100 μg of VR1012 complexed with lipid. By day 15 post tumor cell injection, mice treated with either the 50 or 100 μg dose of VR1110 complexed with lipid had a significant inhibition of tumor growth (p=0.002) compared to the mice treated with the VR1012: lipid (FIG. 18A). A significant increase in survival (p=0.01) was also found for the mice treated with either the 50 or 100 μg dose of VR1110: lipid (FIG. 18B). On day 25, none of the mice treated with the VR1012: lipid survived, while the mice injected with 50 or 100 μg of VR1110: lipid had 27 and 33% survival, respectively. By day 37, mice treated with 50 or 100 μg of VR1110: lipid had 20 and 27% survival, respectively. Tumor-bearing mice treated with 25 μg of VR1110 complexed with lipid were not significantly different from the control mice for either tumor volume or survival.

*Cytokine profile of ovarian tumor ascites*

Since i.p. injection of IL-2 pDNA: lipid into mice bearing i.p. MOT tumors resulted in high levels of IL-2 expression in the ascites, it was of interest to determine whether the IL-2 therapy initiated a cytokine cascade in the tumor ascites. C3H/HeN mice were injected i.p. with $10^5$ MOT cells. On days 5, 8 and 11 after tumor cell implant, the mice were injected i.p. with either VR1110: lipid or VR1012: lipid (1:1 pDNA:DMRIE mass ratio) or received no treatment after the MOT tumor cell injection. Two days after each injection of pDNA: lipid (days 7, 10 and 13 after tumor cell implant), 5 mice per group were sacrificed and the tumor ascites was collected. The total volume of ascites was determined per mouse. The ascites samples were spun at 14,000 rpm for 2 minutes followed by collection of the supernatants. The ascites supernatants were assayed for the concentration of the cytokines: IL-2, IL-4, IL-6, IL-10, IL-12, granulocyte-macrophage colony stimulating factor (GM-CSF), interferon gamma (IFN$\gamma$) and tumor necrosis factor alpha (TNF$\alpha$) using ELISA (R & D Systems, Minneapolis, MN). The concentration of transforming growth factor beta (TGF$\beta$) in the ascites was assayed using the TGF$\beta_2$ Emax Immunoassay System (Promega, Madison, WI). The amount of cytokine in the tumor ascites was calculated using the formula: cytokine concentration in pg/ml x ml of total ascites = pg of cytokine/total ascites.

As expected, tumor-bearing mice injected i.p. with VR1110: lipid had a marked increase in IL-2 levels with negligible levels in untreated tumor-bearing mice or mice injected with the VR1012: lipid (FIG. 19A). The levels of IFN$\gamma$ and GM-CSF were also markedly elevated in the mice treated with VR1110: lipid (FIGs. 19B and 19C). The IFN$\gamma$ and GM-CSF levels in these mice increased on days 10 and 13 after tumor cell injection but not on the day 7 timepoint suggesting that this could be due to IL-2 secretion. Some non-specific increase in IFN$\gamma$ was also found in the ascites of rumor-bearing mice after injection of the VR1012: lipid;however, by day 13, the levels of IFN$\gamma$ in the tumor-bearing mice treated with VR1110: lipid were 6-fold higher than in the mice treated with VR1012: lipid suggesting that expression of IL-2 upregulates IFN$\gamma$ production.

Levels of IL-6, TNF$\alpha$ and IL-10 were increased in both the IL-2 pDNA: lipid group as well as the control pDNA: lipid group suggesting that pDNA: lipid complexes may non-specifically stimulate production of these particular cytokines in the tumor ascites (FIGs. 19D, 19E, and 19F). No differences were found for IL-4, IL-12 or TGF$\beta$ in the ascites from any of the groups and levels of these cytokines were low (0-300 pg/ml for IL-4 and IL-12 and 0-2000 pg/ml for TGF$\beta$, data not shown). For all of the cytokines evaluated, mice treated with control pDNA without lipid, IL-2 pDNA without lipid or with lipid alone had similar cytokine levels as the untreated mice.

***Intraperitoneal injection of IFN$\alpha$pDNA:lipid enhances survival***

C3H/HeN mice were injected i.p. with $10^5$ MOT cells to establish ovarian i.p. tumors. The mice then received i.p. injections of 100 $\mu$g of VR44111 (mIFN$\alpha$) or VR1012 (control) complexed with DMRIE:DOPE cationic lipid at a 1:1 DNA:DMRIE mass ratio in a total volume of I ml saline. The mice received the i.p. injections of pDNA: lipid on days 5, 8 and 11 after tumor cell injection. The mice were weighed 3-6 times per week. Fifteen mice were included in each treatment group.

Mice bearing i.p. MOT tumors and treated with i.p. VR4111: lipid had a significant increase in survival ($p<0.006$) compared to the mice receiving the control plasmid (FIG. 20B). No significant reduction in tumor volume was found for the VR4111: lipid-treated mice (FIG. 20A).

***Example 7***

***Selective Transfection of Malignant Cells in Murine Intraperitoneal Melanoma Tumor Model***

The anti-tumor effect of DNA formulations with or without lipids in mouse i.p. melanoma model have been evaluated in the present example.

***Cell line and tumor model***

B16F10 mouse melanoma cells were grown *in vitro* in DMEM and 10% FCS. Two hundred thousand B16F10 mouse melanoma cells were implanted i.p. in C57BL/6 mice in 1-3 ml saline using a 28 G 1/2 needle and without puncturing internal organs.

*Preparation of plasmid DNA:lipid complexes*

The plasmid DNA-cationic lipid formulations were prepared just prior to use. Equal volumes of DNA and DMRIE : DOPE (1:1) were mixed by swirling to achieve a target concentration of 0.5 mg DNA/ml, 100 $\mu$g DMRIE/ml and 0.12 mg DOPE/ml as previously described (Parker et al, 1996; Saffran et al, 1998). The other cationic lipids were mixed similarly so that the mass ratio of DNA to cationic lipid was 5:1 and the cationic lipid to DOPE molar ratio was 1:1. The formulated material was then vortexed at high speed for 30 seconds and kept at room temperature until dose administration.

*CAT assay*

Tumor or other tissues were collected, immediately frozen in liquid nitrogen and ground into a powder using a reversible drill as described (Manthorpe,M., Hartikka, J., Vahlsing, H.L. and Sawdey, M. Quantification of plasmid DNA transfection in vivo. In Gene Quantification. F. Ferre, ed. Birkhauser, Boston, Ma., 1998 in press.). The dry frozen powder was thawed and extracted in lysis buffer and high speed supernates assayed for CAT activity using a two-phase partition assay as described (Sankaran, L., Analytical Biochemistry 200: 180-186 (1992)).

***Cationic lipids enhances tumor transfection***

C57BL/6 mice were injected i.p. with 200,000 B16F10 murine melanoma cells, and seven days later, injected i.p. with CAT pDNA (VR1332):DMRIE/DOPE. Two days later, tumor tissues were collected, extracted, and assayed for CAT activty.

As shown in Table 4. DNA alone transfected tumor, but DMRIE:DOPE increased transfection by 78 fold (from 2,326 to 182,052).

**Table 4**

| | pgs CAT per gm of tumor tissue collected (n = 4 to 6 mice as indicated); saline values subtracted | | |
|---|---|---|---|
| Mouse # | DNA only | DNA:DMRIE | DMRIE, no DNA |
| 1 | 0 | 82,600 | 0 |
| 2 | 307 | 137,551 | 0 |
| 3 | 706, | 220,600 | 0 |
| 4 | 791 | 287,458 | 0 |
| 5 | 5,892 | | 0 |
| 6 | 6,258 | | |
| Average | 2,326 | 182,052 | 0 |
| Std Error | 1,306 | 52,140 | 0 |
| Fold higher | 1 | 78 | 0 |

*Selective transfection of tumor cells*

*Normal animal.* Normal, non-tumor bearing BALB/c mice were injected i.p. with Img/2ml VR1332, with or without a cationic lipid, and with or without the neutral lipid, DOPE. Two days later, selected i.p. tissues (liver, lung, kidney, spleen, mesentery) were collected, extracted, and assayed for CAT activity.

As shown in Table 5, normal intraperitoneal organs were not at all transfected or transfected very little with a variety of cationic lipids: There was a low level transfection of mesentery tissues, and a moderate transfection of 1 of 5 spleens (the one transfected spleen may have been punctured by the injection needle).

**Table 5**

| | Average pgs CAT per gm of tumor tissue (avg., n = 5 mice) Saline background values have been subtracted | | | |
|---|---|---|---|---|
| ORGANS | DNA only | + DMRIE | + βAE-DMRIE | + GAP-DLRIE |
| Liver | 0 | 0 | 0 | 0 |
| Lung | 0 | 0 | 0 | 0 |
| Kidney | 0 | 0 | 0 | 0 |
| Spleen | 0 | 2,563(0)* | 0 | 0 |
| Mesentery | 370 | 1,549 | 1,866 | 1,127 |

(continued)

| ORGANS | + DOSPA | + DMRIE, no DOPE | + βAE-DMRIE, no DOPE |
|---|---|---|---|
| Liver | 0 | 0 | 0 |
| Lung | 0 | 0 | 0 |
| Kidney | 0 | 0 | 0 |
| Spleen | 0 | 0 | 0 |
| Mesentery | 600 | 1,091 | 3.604 |
| *one mouse with a CAT value; the rest = 0 | | | |

*Tumor-bearing animal compared to normal animal.* C57BL/6 mice were injected i.p. with 200,000 B16F10 murine melanoma cells, and seven days later, injected i.p. with VR1332 with or without cationic lipid/DOPE. Two days later, selected i.p. tissues (liver, lung, kidney, spleen, mesentery) were collected, extracted, and assayed for CAT activity.

As shown in Table 6, tumor tissues were transfected at high levels with pDNA complexed with cationic lipid/ DOPE. Normal i.p. organs were not transfected well compared to i.p. tumor tissues.

**Table 6**

Average pgs CAT per gm of tumor tissue (n = 5 mice); saline values subtracted

| ORGANS | DNA only | DNA + DMRIE:DOPE | DNA + DMRIE:DOPE normal mice no tumors |
|---|---|---|---|
| Tumor | 0 | 200,195 | n/a |
| Liver | 0 | 0 | 0 |
| Lung | 0 | 0 | 0 |
| Kidney | 0 | 0 | 0 |
| Spleen | 493 | 0 | 0 |
| Mesentery | 6,795 | nd | 2,562 |
| Ovary | nd | nd | 0 |

*Dose Response*

C57BL/6 mice were injected i.p. with 200,000 B16F10 murine melanoma cells, and seven days later, injected i.p. with 0.15 or 1.5 mgs of VR1332 with or without cationic lipid/DOPE in 3 ml saline. Two days later, tumor tissues were collected, extracted, and assayed for CAT activity.

As shown in Table 7, a higher dose of DNA transfected tumor tissues better than a lower dose. Also, DMRIE transfected better than the two other cationic lipids tested.

**Table 7**

Average pgs CAT per gm of tumor tissue (n = 5 mice); saline values subtracted

| DNA DOSE | DNA only | + DMRIE | + GAP-DIMRIE | + PA-DELO |
|---|---|---|---|---|
| 1.5 mgs | 16,592 | 1,270,466 | 524,006 | 581,616 |
| 0.15 mgs | 1,937 | 131,089 | 47,866 | 99,797 |

***Testing a variety of cationic lipids***

C57BL/6 mice were injected i.p. with 200,000 B16F10 murine melanoma cells, and seven days later, injected i.p. with 1 mg of VR1332 with or without cationic lipid/DOPE in 3 ml saline. Two days later, tumor tissues were collected, extracted, and assayed for CAT activiy.

As shown in Table 8, all cationic lipids tested increased transfection level, and DMRIE was one of three preferred cationic lipids.

Table 8

| CL:DOPE | Average pgs CAT per gm of tumor tissue (n = 5 mice); saline values have been subtracted Run 1 | Run 2 | Average |
|---|---|---|---|
| None | 6,247 | not done | 6,247 |
| GAP-DDRIE | 20,816 | 7,027 | 13,922 |
| GMU-DMRIE | 9,355 | 52,888 | 31,122 |
| HP-DORIE | 36,615 | 26,795 | 31,705 |
| DOSPA | 33,494 | 57,026 | 45,260 |
| PA-TELO | 63,326 | 32,681 | 48,004 |
| GA-LOE-BP | 59,729 | 63,528 | 61,629 |
| GAP-DMRIE | 80,760 | 63,002 | 71,881 |
| PA-DELO | 73,692 | 82,265 | 77,979 |
| GAP-DLRIE | 77,553 | 107,629 | 92,591 |
| DMRIE | 77,128 | 122,225 | 99,677 |
| DLRIE | 122,999 | 128,225 | 125,612 |
| PA-DEMO | 155,369 | 154,884 | 155,127 |

In sum, the success of the intra-cavity delivery embodiment of the present invention is also exemplified in the murine melanoma tumor model. Following i.p. injection of a polynucleotide, transfection occurs predominantly in tumor tissues, and normal intraperitoneal organs, such as liver, lung, and kidney are poorly transfected, if at all, with the polynucleotide formulation.

## Claims

1. Use of a non-infectious, non-integrating polynucleotide construct comprising a polynucleotide encoding a molecule which is a peptide, polypeptide or protein in the manufacture of a medicament for treatment of cancer in a mammal, wherein
the treatment comprises administering the medicament into a body cavity of said mammal such that said polynucleotide is incorporated into tumor or malignant cells within said body cavity and said molecule is expressed in said cells in an amount effective to treat cancer, wherein
the construct is to be administered as a complex of said construct and one or more cationic compounds selected from the group consisting of cationic lipids, cationic peptides, cationic proteins, cationic polymers, and mixtures thereof, wherein
said molecule is a cytokine or an active fragment thereof, and wherein
said cytokine is selected from the group consisting of IFNω, IFNα, $IFN_T$, IFNβ, IL-2, an active fragment of any of thereof, and a combination of any of thereof.

**2.** A non-infectious, non-integrating polynucleotide construct comprising a polynucleotide encoding a molecule which is a peptide, polypeptide or protein, for use in treatment of cancer in a mammal, wherein
the treatment comprises administering the medicament into a body cavity of said mammal such that said polynucleotide is incorporated into tumor or malignant cells within said body cavity and said molecule is expressed in said cells in an amount effective to treat cancer, wherein
the construct is to be administered as a complex of said construct and one or more cationic compounds selected from the group consisting of cationic lipids, cationic peptides, cationic proteins, cationic polymers, and mixtures thereof, wherein
said molecule is a cytokine or an active fragment thereof, and wherein
said cytokine is selected from the group consisting of IFNω, IFNα, $IFN_{T,}$ IFNβ, IL-2, an active fragment of any of thereof, and a combination of any of thereof.

**3.** Use according to claim 1 or a construct for use according to claim 2, wherein said cytokine is an interferon ω or an interferon α, or an interleukin-2.

**4.** Use according to claim 1 or a construct for use according to claim 2, wherein said cytokine is selected from the group consisting of:

(a) an interferon ω comprising amino acids 1 to 172 in SEQ ID NO: 8;
(b) an interferon ω comprising amino acids -23 to 172 in SEQ ID NO: 8;
(c) an interferon α comprising amino acids 1 to 166 in SEQ ID NO: 10;
(d) an interferon α comprising amino acids -23 to 166 in SEQ ID NO: 10;
(e) an interleukin-2 comprising amino acids 58 to 105 of SEQ ID NO: 14;
(f) an interleukin-2 comprising amino acids 20 to 126 of SEQ ID NO: 14.

**5.** Use according to claim 1 or a construct for use according to claim 2, wherein the polynucleotide construct is selected from the group consisting of VR4151 (SEQ ID NO: 4), VR4112 (SEQ ID NO: 2), and VR1103 (SEQ ID NO: 25).

**6.** Use or a construct for use according to any one of claims 1 to 5, wherein said polynucleotide construct is administered free from *ex vivo* cells or *ex vivo* cellular material.

**7.** Use or a construct for use according to any one of claims 1 to 6, wherein the body cavity is selected from the group consisting of peritoneal cavity, thoracic cavity, pleural cavity, rectal cavity, stomach cavity, and urinary bladder vesicle or; wherein said cancer is selected from the group consisting of renal cell carcinoma, colorectal carcinoma, lymphoma, melanoma, prostate cancer, ovarian cancer, lung cancer, stomach cancer, glandular lymphoma, bladder cancer, uterine cancer, mesenteric cancer, pancreatic cancer, gastric cancer, metastases thereof, and combinations thereof.

**8.** Use or a construct for use according to any of claims 1 to 7, wherein said one or more cationic compounds are one or more cationic lipids and/or wherein said complex further comprises one or more neutral lipids.

**9.** Use or a construct for use according to any one of claims 1 to 8, wherein the mass ratio of said polynucleotide construct to lipid is about 5:1 to about 1:1.

**10.** Use or a construct for use according to any one of claims 1 to 9, wherein said polynucleotide is (i) DNA operably linked to a promoter, or (ii) RNA.

**11.** Use or a construct for use according to any one of claims 1 to 10, wherein said polynucleotide construct further comprises a polynucleotide encoding one or more additional molecules.

**12.** Use or a construct for use according to claim 11, wherein said polynucleotide construct further comprises a polynucleotide encoding one or more additional cytokines.

**13.** Use or a construct for use according to any one of claims 1 to 12, wherein said polynucleotide construct comprises a region regulating gene expression and preferably, wherein said region regulating gene expression is cell specific.

**14.** Use or a construct for use according to any one of claims 1 to 13, wherein said treatment comprises administering the medicament in combination with one or more additional cancer treatment methods selected from the group consisting of surgery, radiation therapy, chemotherapy, immunotherapy, and gene therapy.

**15.** Use or a construct for use according to claim 14, wherein said treatment comprises administering the medicament at a time selected from the group consisting of:

(a) prior to the commencement of said one or more additional cancer treatments;
(b) during the practice of said one or more additional cancer treatments;
(c) after the end of said one or more additional cancer treatments.

**Patentansprüche**

**1.** Verwendung eines nichtinfektiösen, nichtintegrierenden Polynucleotidkonstrukts, das ein für ein Molekül, das ein Peptid, Polypeptid oder Protein ist, kodierendes Polynucleotid umfasst, bei der Herstellung eines Medikaments zur Behandlung von Krebs in einem Säugetier, wobei
die Behandlung die Verabreichung des Medikaments in einen Körperhohlraum des Säugetiers umfasst, sodass das Polynucleotid in einen Tumor oder in maligne Zellen innerhalb des Körperhohlraums inkorporiert wird und das Molekül in den Zellen in einer Menge exprimiert wird, die wirksam ist, um Krebs zu behandeln, wobei
das Konstrukt als Komplex aus dem Konstrukt und einer oder mehreren kationischen Verbindungen zu verabreichen ist, die aus der aus kationischen Lipiden, kationischen Peptiden, kationischen Proteinen, kationischen Polymeren und Gemischen davon bestehenden Gruppe ausgewählt sind, wobei
das Molekül ein Cytokin oder ein aktives Fragment davon ist und wobei
das Cytokin aus der aus IFNω, IFNα, $IFN_T$, IFNβ, IL-2 und einem aktiven Fragment beliebiger davon sowie Kombinationen beliebiger davon bestehenden Gruppe ausgewählt ist.

**2.** Nichtinfektiöses, nichtintegrierendes Polynucleotidkonstrukt, das ein für ein Molekül, das ein Peptid, Polypeptid oder Protein ist, kodierendes Polynucleotid umfasst, zur Verwendung bei der Behandlung von Krebs in einem Säugetier, wobei die Behandlung die Verabreichung des Medikaments in einen Körperhohlraum des Säugetiers umfasst, sodass das Polynucleotid in einen Tumor oder in maligne Zellen innerhalb des Körperhohlraums inkorporiert wird und das Molekül in den Zellen in einer Menge exprimiert wird, die wirksam ist, um Krebs zu behandeln, wobei
das Konstrukt als Komplex aus dem Konstrukt und einer oder mehreren kationischen Verbindungen zu verabreichen ist, die aus der aus kationischen Lipiden, kationischen Peptiden, kationischen Proteinen, kationischen Polymeren und Gemischen davon bestehenden Gruppe ausgewählt sind, wobei
das Molekül ein Cytokin oder ein aktives Fragment davon ist und wobei das Cytokin aus der aus IFNω, IFNα, $IFN_T$, IFNβ, IL-2 und einem aktiven Fragment beliebiger davon sowie Kombinationen beliebiger davon bestehenden Gruppe ausgewählt ist.

**3.** Verwendung nach Anspruch 1 oder Konstrukt zur Verwendung nach Anspruch 2, wobei das Cytokin ein Interferon-ω oder ein Interferon-α oder in Interleukin-2 ist.

**4.** Verwendung nach Anspruch 1 oder Konstrukt zur Verwendung nach Anspruch 2, wobei das Cytokin aus folgender Gruppe ausgewählt ist:

(a) Interferon-ω, das die Aminosäuren 1 bis 172 in Seq.-ID Nr. 8 umfasst;
(b) Interferon-ω, das die Aminosäuren -23 bis 172 in Seq.-ID Nr. 8 umfasst;
(c) Interferon-α, das die Aminosäuren 1 bis 166 in Seq.-ID Nr. 10 umfasst;
(d) Interferon-α, das die Aminosäuren -23 bis 166 in Seq.-ID Nr. 10 umfasst;
(e) Interleukin-2, das die Aminosäuren 58 bis 105 in Seq.-ID Nr. 14 umfasst;
(f) Interleukin-2, das die Aminosäuren 20 bis 126 in Seq.-ID Nr. 14 umfasst.

**5.** Verwendung nach Anspruch 1 oder Konstrukt zur Verwendung nach Anspruch 2, wobei das Polynucleotidkonstrukt aus der aus VR4151 (Seq.-ID Nr. 4), VR4112 (Seq.-ID Nr. 2) und VR1103 (Seq.-ID Nr. 25) bestehenden Gruppe ausgewählt ist.

**6.** Verwendung oder Konstrukt zur Verwendung nach einem der Ansprüche 1 bis 5, wobei das Polynucleotidkonstrukt frei von Ex-vivo-Zellen oder Ex-vivo-Zellmaterial verabreicht wird.

**7.** Verwendung oder Konstrukt zur Verwendung nach einem der Ansprüche 1 bis 6, wobei der Körperhohlraum aus der aus Peritonealhöhle, Brusthöhle, Pleurahöhle, Rektumhöhle, Magenhöhle und Harnblase bestehenden Gruppe ausgewählt ist; oder
wobei der Krebs aus der aus Nierenzellkarzinomen, kolorektalen Karzinomen, Lymphomen, Melanomen, Prostatakrebs, Ovarialkrebs, Lungenkrebs, Magenkrebs, Adenolymphomen, Blasenkrebs, Uteruskrebs, Mesenteriumkrebs, Pankreaskrebs, Magenkrebs, Metastasen davon und Kombinationen daraus ausgewählt ist.

**8.** Verwendung oder Konstrukt zur Verwendung nach einem der Ansprüche 1 bis 7, wobei die eine oder mehreren kationischen Verbindungen ein oder mehrere kationische Lipide ist/sind und/oder wobei der Komplex weiters ein oder mehrere neutrale Lipide umfasst.

**9.** Verwendung oder Konstrukt zur Verwendung nach einem der Ansprüche 1 bis 8, wobei das Massenverhältnis zwischen dem Polynucleotidkonstrukt und dem Lipid etwa 5:1 bis etwa 1:1 beträgt.

**10.** Verwendung oder Konstrukt zur Verwendung nach einem der Ansprüche 1 bis 9, wobei das Polynucleotid (i) operabel an einen Promotor gebundene DNA oder (ii) RNA ist.

**11.** Verwendung oder Konstrukt zur Verwendung nach einem der Ansprüche 1 bis 10, wobei das Polynucleotidkonstrukt weiters ein Polynucleotid umfasst, das für ein oder mehrere weitere Moleküle kodiert.

**12.** Verwendung oder Konstrukt zur Verwendung nach Anspruch 11, wobei das Polynucleotidkonstrukt weiters ein Polynucleotid umfasst, das für ein oder mehrere weitere Cytokine kodiert.

**13.** Verwendung oder Konstrukt zur Verwendung nach einem der Ansprüche 1 bis 12, wobei das Polynucleotidkonstrukt eine Region zur Regulierung der Genexpression umfasst und diese Region zur Regulierung der Genexpression vorzugsweise zellspezifisch ist.

**14.** Verwendung oder Konstrukt zur Verwendung nach einem der Ansprüche 1 bis 13, wobei die Behandlung die Verabreichung eines Medikaments in Kombination mit einem oder mehreren weiteren Krebsbehandlungsverfahren umfasst, die aus der aus Operation, Strahlentherapie, Chemotherapie, Immuntherapie und Gentherapie ausgewählt sind.

**15.** Verwendung oder Konstrukt zur Verwendung nach Anspruch 14, wobei die Behandlung die Verabreichung des Medikaments zu einem Zeitpunkt umfasst, der aus folgender Gruppe ausgewählt ist:

(a) vor Beginn der einen oder mehreren weiteren Krebsbehandlungen;
(b) während der Durchführung der einen oder mehreren weiteren Krebsbehandlungen;
(c) nach Beendigung der einen oder mehreren weiteren Krebsbehandlungen.

## Revendications

**1.** Utilisation d'une construction de polynucléotide non infectieuse, non intégrante, comprenant un polynucléotide codant pour une molécule qui est un peptide, un polypeptide ou une protéine dans la fabrication d'un médicament pour le traitement du cancer chez un mammifère, où
le traitement comprend l'administration du médicament dans une cavité corporelle dudit mammifère de sorte que ledit polynucléotide est incorporé dans la tumeur ou les cellules malignes dans ladite cavité corporelle, et ladite molécule est exprimée dans lesdites cellules en une quantité efficace pour traiter le cancer, où
la construction doit être administrée comme un complexe de ladite construction et d'un ou de plusieurs composés cationiques sélectionnés dans le groupe consistant en lipides cationiques, peptides cationiques, protéines cationiques, polymères cationiques et leurs mélanges, où
ladite molécule est une cytokine ou un fragment actif de celle-ci, et où
ladite cytokine est sélectionnée dans le groupe consistant en IFNω, IFNα, $IFN_T$, IFNβ, IL-2, un fragment actif de l'un quelconque de ceux-ci et une combinaison d'un quelconque de ceux-ci.

2. Construction de polynucléotide non infectieuse, non intégrante comprenant un polynucléotide codant pour une molécule qui est un peptide, un polypeptide ou une protéine, pour utilisation dans le traitement du cancer chez un mammifère, où
le traitement comprend l'administration du médicament dans une cavité corporelle dudit mammifère de sorte que ledit polynucléotide est incorporé dans la tumeur ou les cellules malignes dans ladite cavité corporelle, et ladite molécule est exprimée dans lesdites cellules en une quantité efficace pour traiter le cancer, où
la construction doit être administrée comme un complexe de ladite construction et d'un ou de plusieurs composés cationiques sélectionnés dans le groupe consistant en lipides cationiques, peptides cationiques, protéines cationiques, polymères cationiques et leurs mélanges, où
ladite molécule est une cytokine ou un fragment actif de celle-ci, et où
ladite cytokine est sélectionnée dans le groupe consistant en IFNω, IFNα, $IFN_T$, IFNβ, IL-2, un fragment actif d'un quelconque de ceux-ci et une combinaison d'un quelconque de ceux-ci.

3. Utilisation selon la revendication 1 ou construction pour utilisation selon la revendication 2, où ladite cytokine est un interféron ω ou un interféron α, ou une interleukine-2.

4. Utilisation selon la revendication 1 ou construction pour utilisation selon la revendication 2, où ladite cytokine est sélectionnée dans le groupe consistant en:

(a) un interféron ω comprenant les acides aminés 1 à 172 dans la SEQ ID NO: 8;
(b) un interféron ω comprenant les acides aminés -23 à 172 dans la SEQ ID NO: 8;
(c) un interféron α comprenant les acides aminés 1 à 166 dans la SEQ ID NO: 10;
(d) un interféron α comprenant les acides aminés -23 à 166 dans la SEQ ID NO: 10;
(e) une interleukine-2 comprenant les acides aminés 58 à 105 de la SEQ ID NO: 14;
(f) une interleukine -2 comprenant les acides aminés 20 à 126 de la SEQ ID NO: 14.

5. Utilisation selon la revendication 1 ou construction pour utilisation selon la revendication 2, où la construction de polynucléotide est sélectionnée dans le groupe consistant en VR4151 1 (SEQ ID NO: 4), VR4112 (SEQ ID NO: 2) et VR1103 (SEQ ID NO: 25).

6. Utilisation ou construction pour utilisation selon l'une quelconque des revendications 1 à 5, où ladite construction de polynucléotide est administrée d'une manière exempte de cellules *ex vivo* ou d'un matériau cellulaire *ex vivo.*

7. Utilisation ou construction pour utilisation selon l'une quelconque des revendications 1 à 6, où la cavité corporelle est sélectionnée dans le groupe consistant en cavité péritonéale, cavité thoracique, cavité pleurale, cavité rectale, cavité de l'estomac et vésicule de vessie urinaire;
ou bien où ledit cancer est sélectionné dans le groupe consistant en carcinome des cellules rénales, carcinome colorectal, lymphome, mélanome, cancer de la prostate, cancer des ovaires, cancer des poumons, cancer de l'estomac, lymphome glandulaire, cancer de la vessie, cancer de l'utérus, cancer mésentère, cancer pancréatique, cancer gastrique, et leurs métastases et des combinaisons de ceux-ci.

8. Utilisation ou construction pour utilisation selon l'une quelconque des revendications 1 à 7, où un ou plusieurs composés cationiques précités sont un ou plusieurs lipides cationiques et/ou où ledit complexe comprend en outre un ou plusieurs lipides neutres.

9. Utilisation ou construction pour utilisation selon l'une quelconque des revendications 1 à 8, où le rapport massique de ladite construction de polynucléotide au lipide est d'environ 5:1 à environ 1:1.

10. Utilisation ou construction pour utilisation selon l'une quelconque des revendications 1 à 9, où ledit polynucléotide est (i) l'ADN fonctionnellement lié à un promoteur, ou (ii) l'ARN.

11. Utilisation ou construction pour utilisation selon l'une quelconque des revendications 1 à 10, où ladite construction de polynucléotide comprend en outre un polynucléotide codant pour une ou plusieurs molécules additionnelles.

12. Utilisation ou construction pour utilisation selon la revendication 11, où ladite construction de polynucléotide comprend en outre un polynucléotide codant pour une ou plusieurs cytokines additionnelles.

13. Utilisation ou construction pour utilisation selon l'une quelconque des revendications 1 à 12, où ladite construction de polynucléotide comprend une région régulant l'expression de gènes et de préférence, où ladite région régulant l'expression de gènes est spécifique aux cellules.

**14.** Utilisation ou construction pour utilisation selon l'une quelconque des revendications 1 à 13, où ledit traitement comprend l'administration du médicament en combinaison avec une ou plusieurs méthodes de traitement additionnelles du cancer sélectionnées dans le groupe consistant en chirurgie, radiothérapie, chimiothérapie, immunothérapie et thérapie génique.

**15.** Utilisation ou construction pour utilisation selon la revendication 14, où ledit traitement comprend l'administration du médicament à un moment sélectionné dans le groupe consistant en:

(a) avant le commencement dudit au moins un ou plusieurs traitements de cancer additionnels;
(b) durant la pratique dudit un ou plusieurs traitements de cancer additionnels;
(c) à la fin d'un ou de plusieurs traitements de cancer additionnels précités.

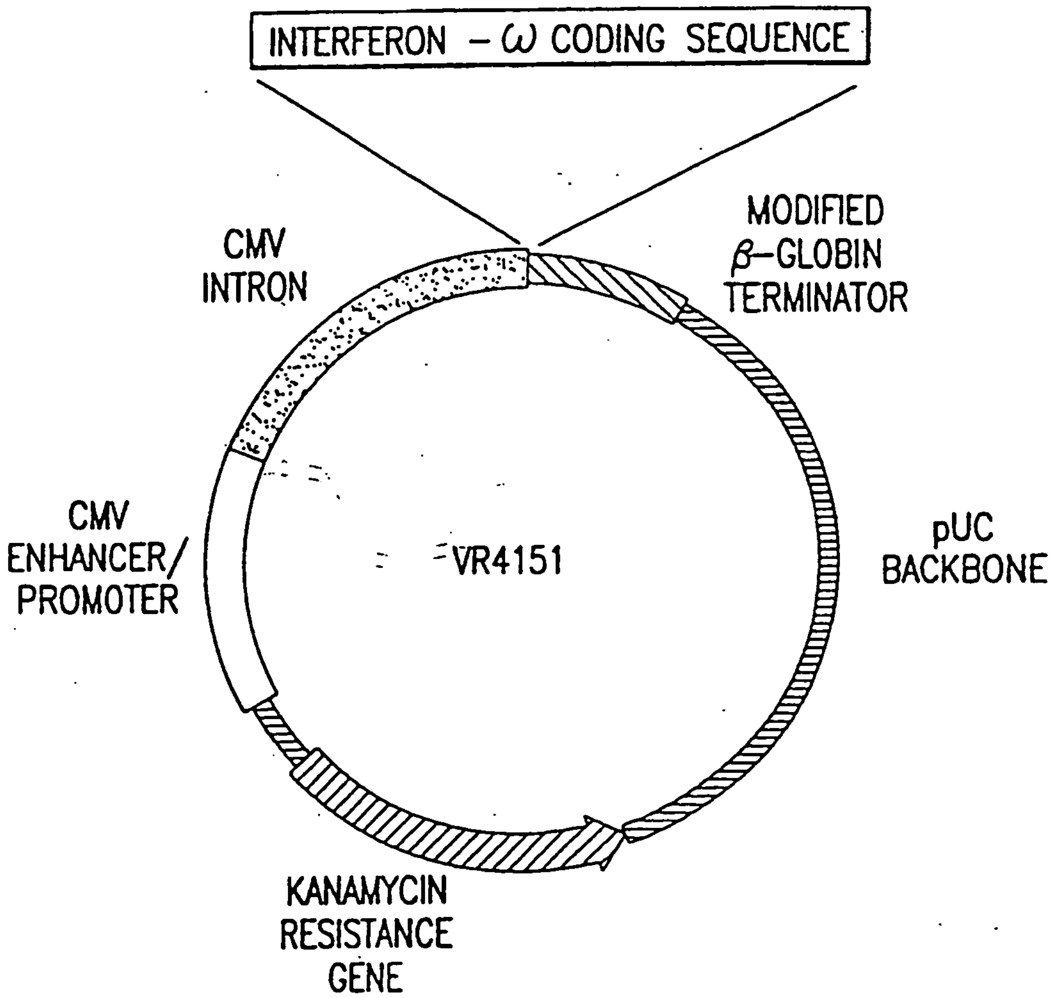
INTERFERON – ω CODING SEQUENCE
CMV INTRON
MODIFIED β–GLOBIN TERMINATOR
CMV ENHANCER/ PROMOTER
VR4151
pUC BACKBONE
KANAMYCIN RESISTANCE GENE

FIG.1

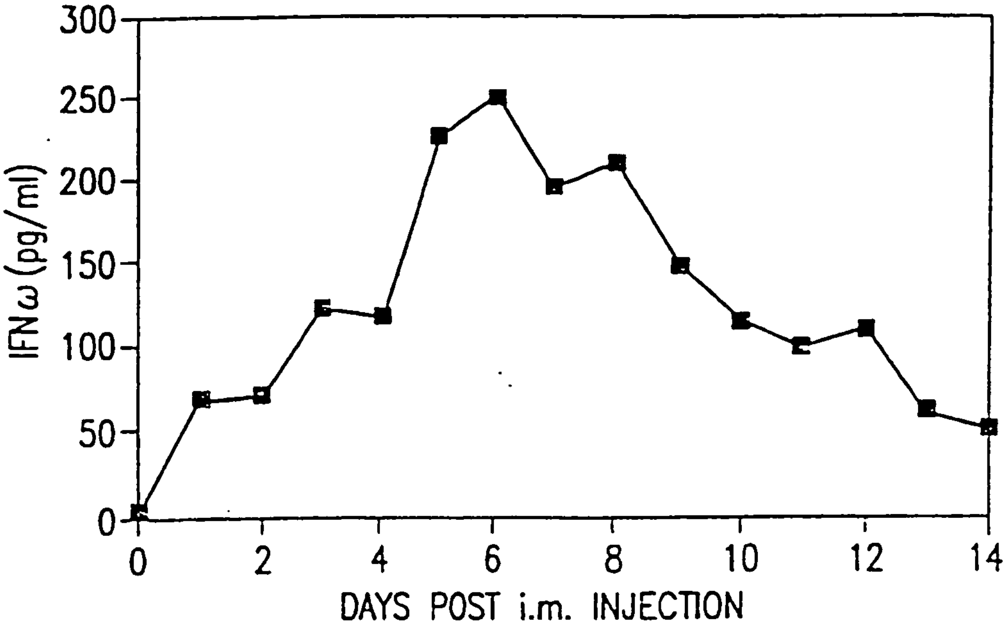
IFNω (pg/ml)
300
250
200
150
100
50
0
0
2
4
6
8
10
12
14
DAYS POST i.m. INJECTION

FIG.2A

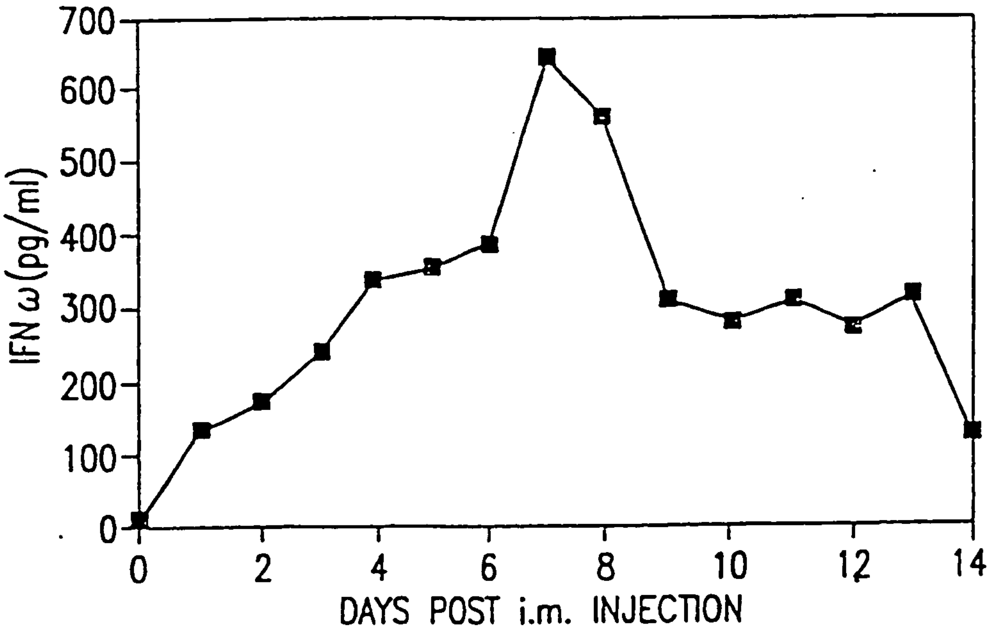
IFNω (pg/ml)
700
600
500
400
300
200
100
0
0
2
4
6
8
10
12
14
DAYS POST i.m. INJECTION

FIG.2B

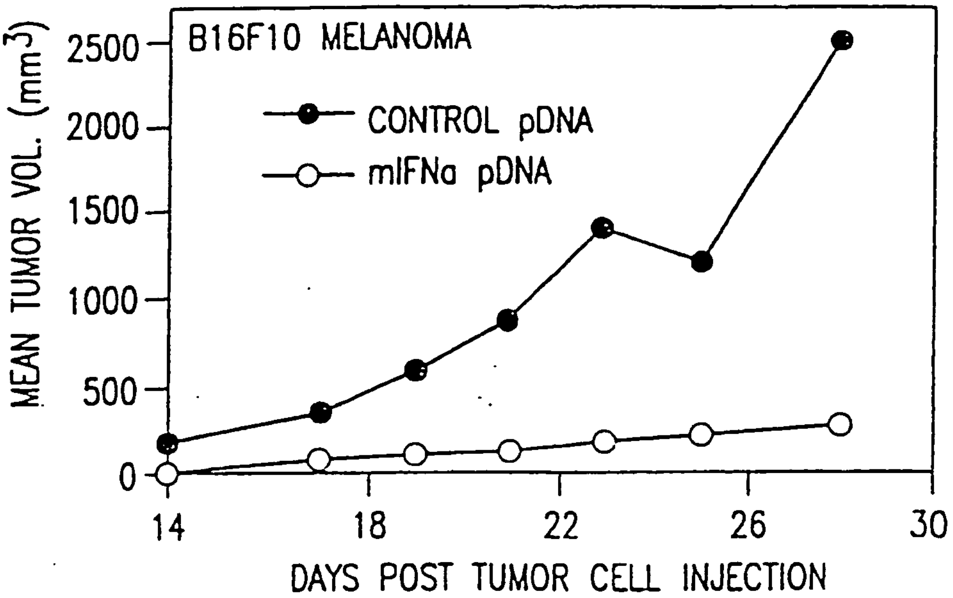
B16F10 MELANOMA
CONTROL pDNA
mIFNα pDNA
MEAN TUMOR VOL. (mm3)
2500
2000
1500
1000
500
0
14
18
22
26
30
DAYS POST TUMOR CELL INJECTION

FIG.3A

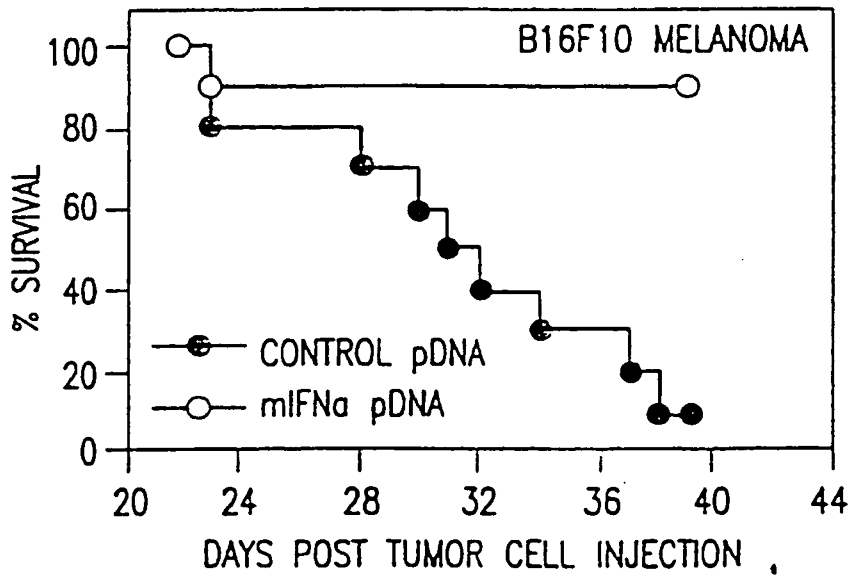
B16F10 MELANOMA
CONTROL pDNA
mIFNα pDNA
% SURVIVAL
100
80
60
40
20
0
20
24
28
32
36
40
44
DAYS POST TUMOR CELL INJECTION

FIG.3B

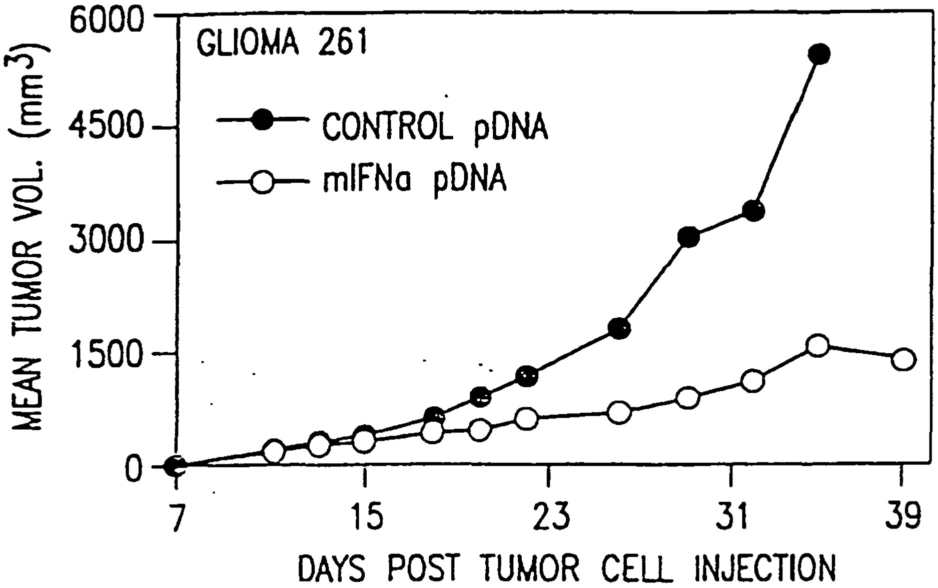
GLIOMA 261
CONTROL pDNA
mIFNa pDNA
MEAN TUMOR VOL. (mm3)
6000
4500
3000
1500
0
7
15
23
31
39
DAYS POST TUMOR CELL INJECTION

FIG.3C

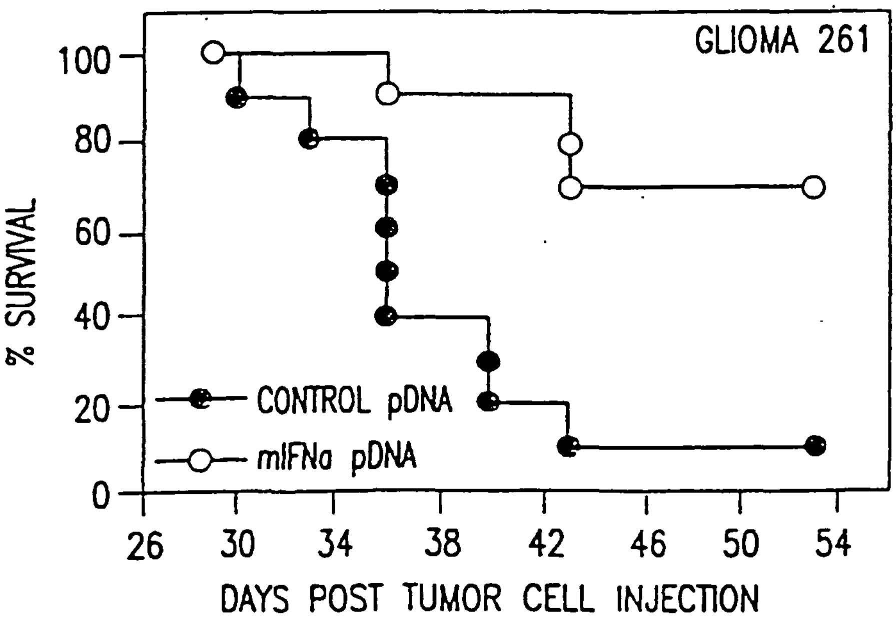
GLIOMA 261
CONTROL pDNA
mIFNa pDNA
% SURVIVAL
100
80
60
40
20
0
26
30
34
38
42
46
50
54
DAYS POST TUMOR CELL INJECTION

FIG.3D

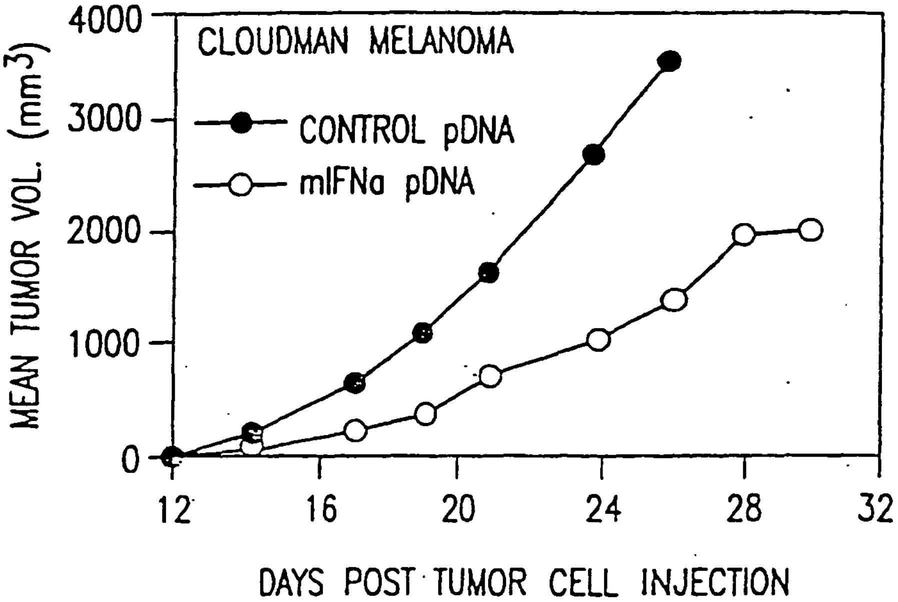
CLOUDMAN MELANOMA
CONTROL pDNA
mIFNa pDNA
MEAN TUMOR VOL. (mm3)
4000
3000
2000
1000
0
12
16
20
24
28
32
DAYS POST TUMOR CELL INJECTION

FIG.3E

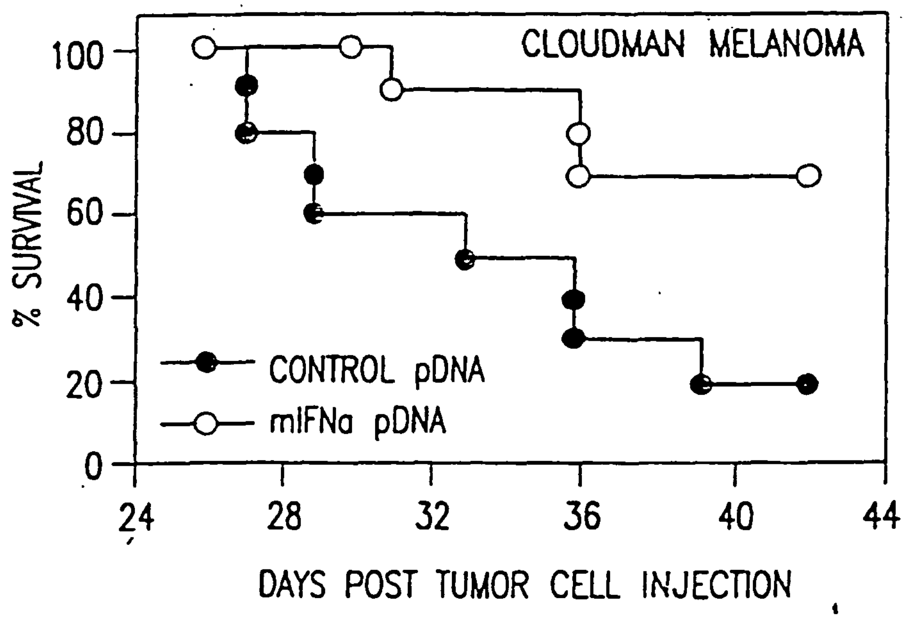
CLOUDMAN MELANOMA
CONTROL pDNA
mIFNa pDNA
% SURVIVAL
100
80
60
40
20
0
24
28
32
36
40
44
DAYS POST TUMOR CELL INJECTION

FIG.3F

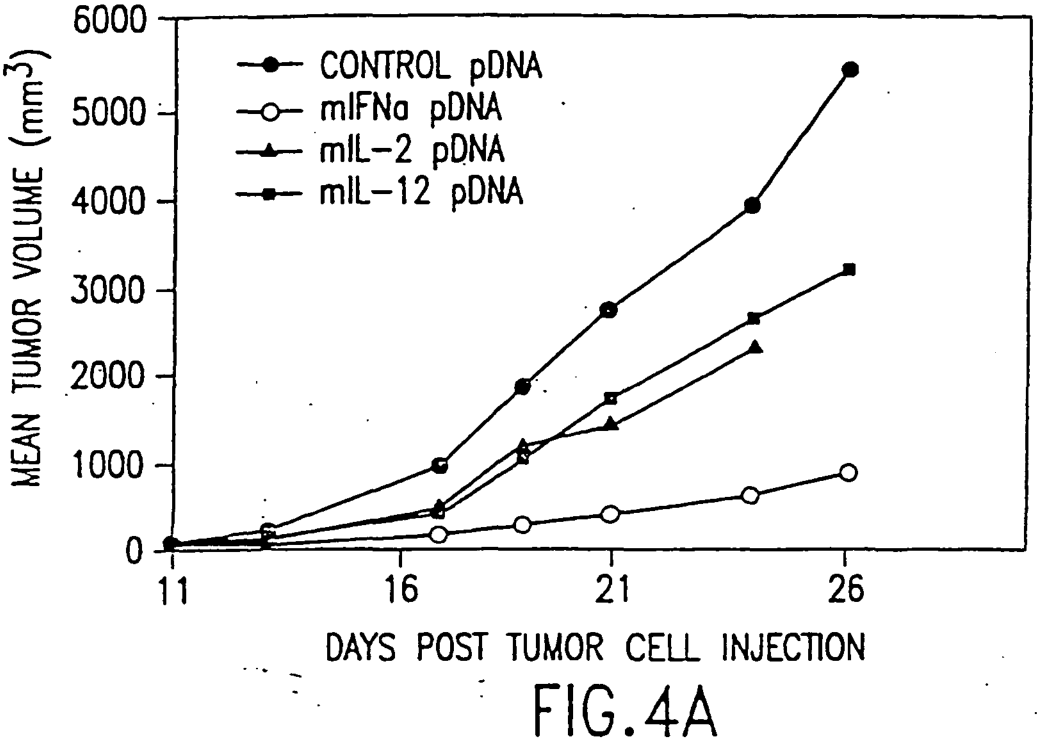
6000
5000
4000
3000
2000
1000
0
MEAN TUMOR VOLUME (mm3)
CONTROL pDNA
mIFNα pDNA
mIL-2 pDNA
mIL-12 pDNA
11
16
21
26
DAYS POST TUMOR CELL INJECTION

FIG.4A

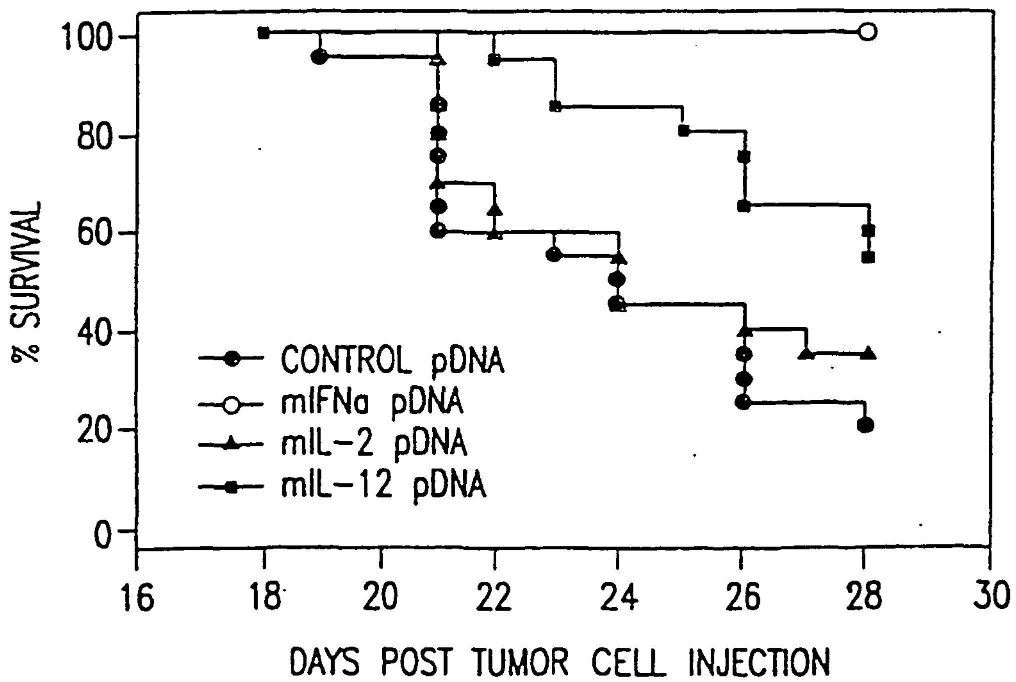
100
80
60
40
20
0
% SURVIVAL
CONTROL pDNA
mIFNα pDNA
mIL-2 pDNA
mIL-12 pDNA
16
18
20
22
24
26
28
30
DAYS POST TUMOR CELL INJECTION

FIG.4B

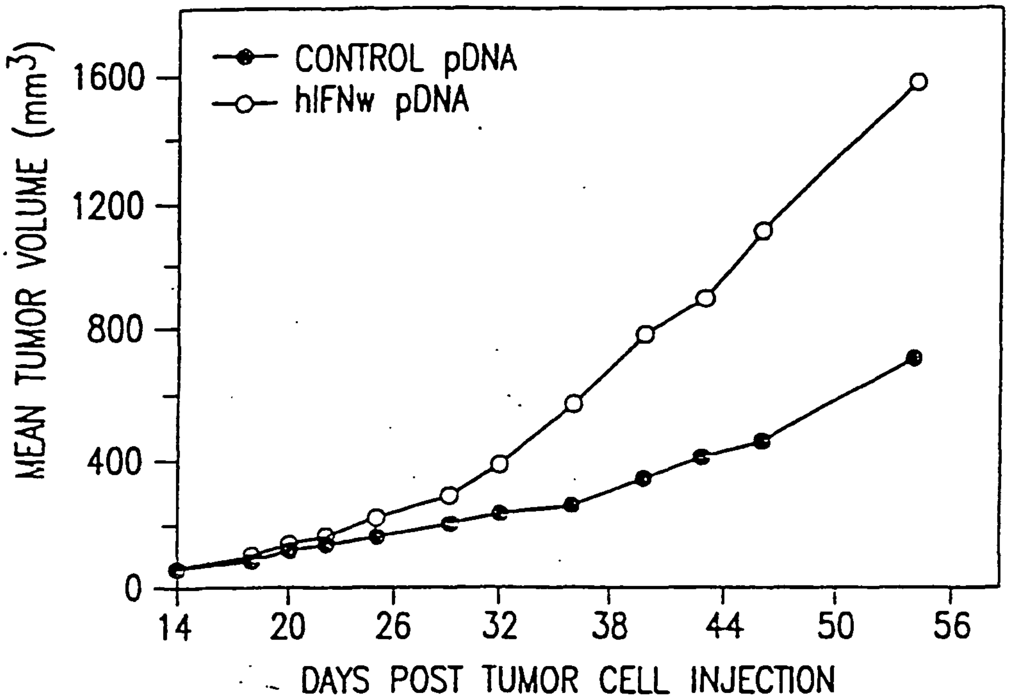
CONTROL pDNA
hIFNw pDNA
MEAN TUMOR VOLUME (mm3)
1600
1200
800
400
0
14
20
26
32
38
44
50
56
DAYS POST TUMOR CELL INJECTION

FIG.5A

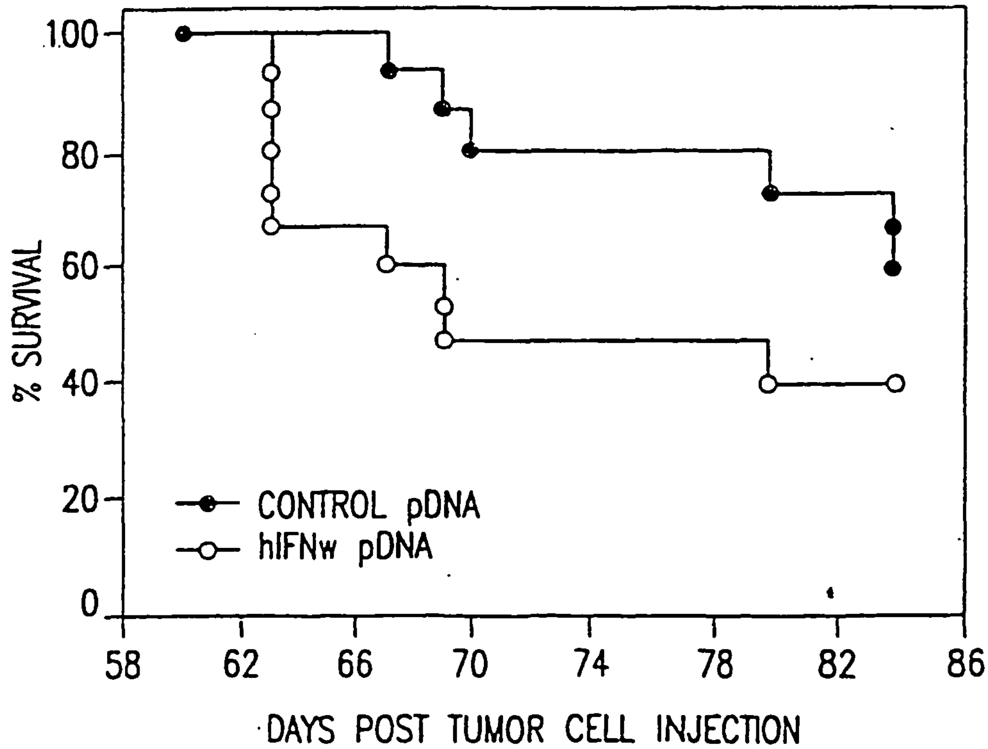
% SURVIVAL
100
80
60
40
20
0
CONTROL pDNA
hIFNw pDNA
58
62
66
70
74
78
82
86
DAYS POST TUMOR CELL INJECTION

FIG.5B

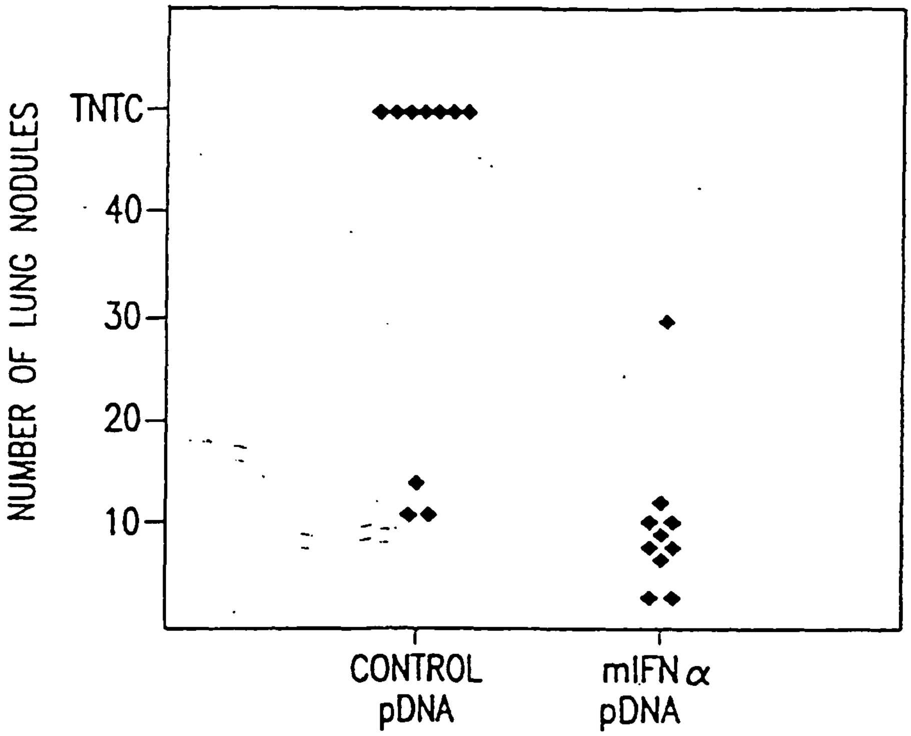
NUMBER OF LUNG NODULES
TNTC
40
30
20
10
CONTROL pDNA
mIFN α pDNA

FIG.6

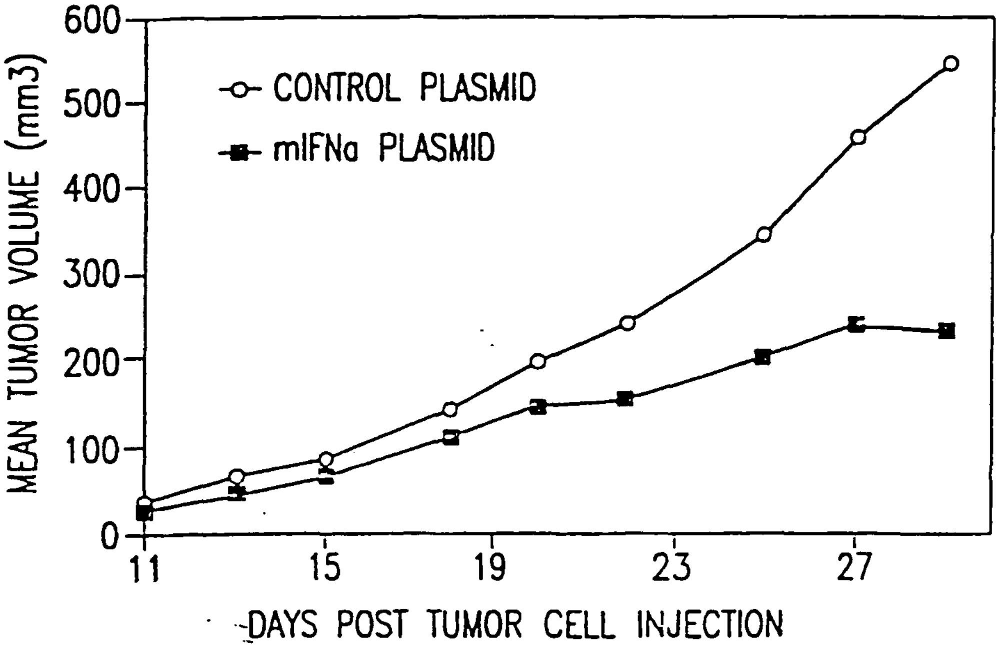
600
500
400
300
200
100
0
MEAN TUMOR VOLUME (mm3)
CONTROL PLASMID
mIFNα PLASMID
11
15
19
23
27
DAYS POST TUMOR CELL INJECTION

FIG.7A

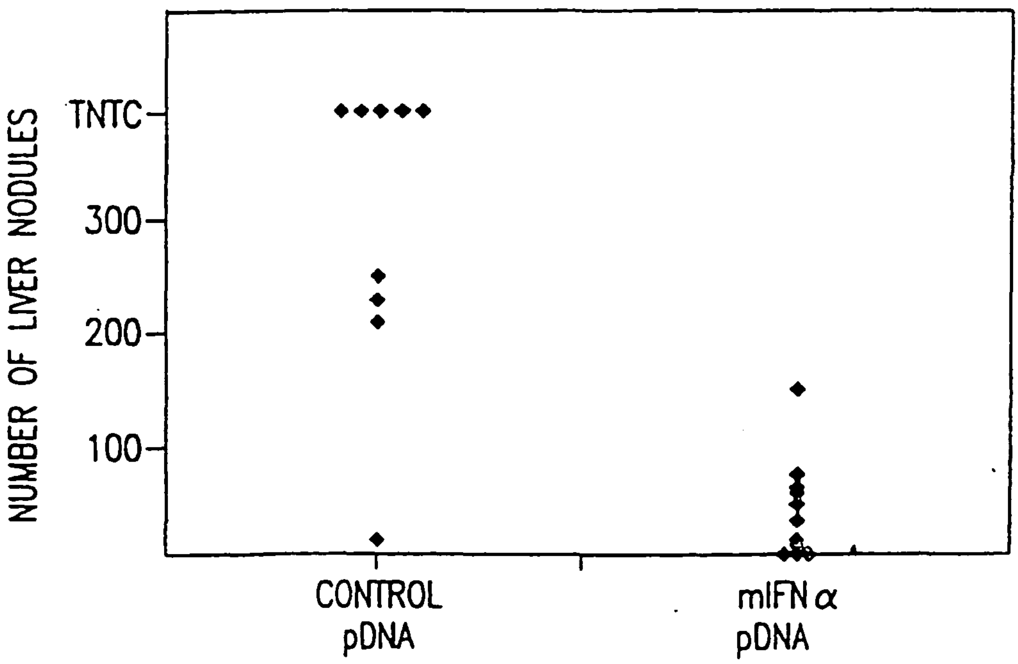
TNTC
300
200
100
NUMBER OF LIVER NODULES
CONTROL
pDNA
mIFNα
pDNA

FIG.7B

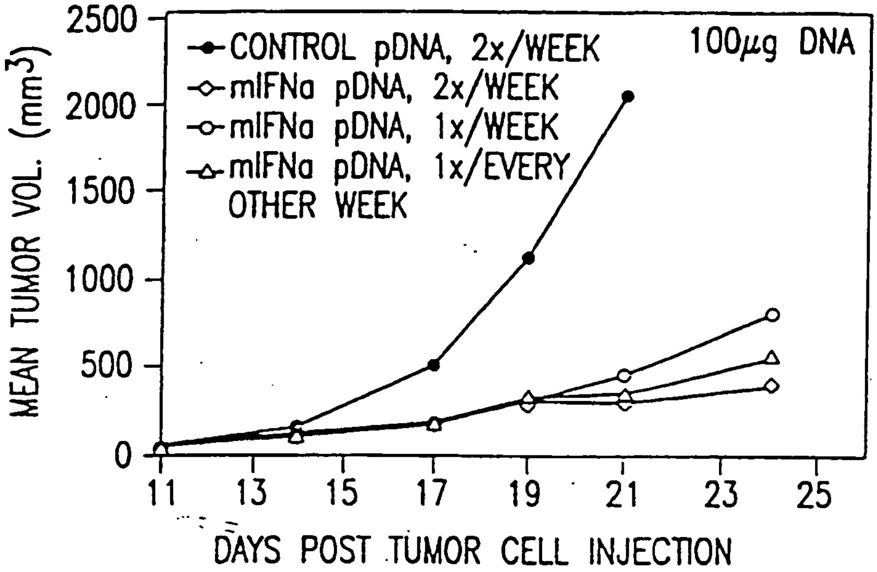
2500
2000
1500
1000
500
0
MEAN TUMOR VOL. (mm3)
CONTROL pDNA, 2x/WEEK
mIFNa pDNA, 2x/WEEK
mIFNa pDNA, 1x/WEEK
mIFNa pDNA, 1x/EVERY OTHER WEEK
100μg DNA
11
13
15
17
19
21
23
25
DAYS POST TUMOR CELL INJECTION

FIG.8A

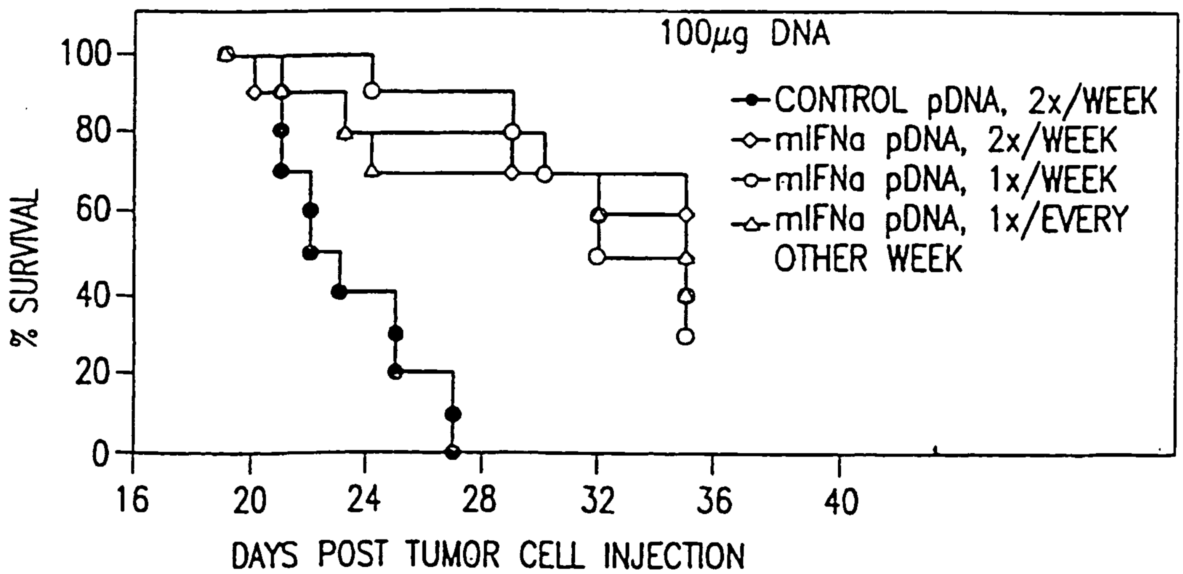
100
80
60
40
20
0
% SURVIVAL
100μg DNA
CONTROL pDNA, 2x/WEEK
mIFNa pDNA, 2x/WEEK
mIFNa pDNA, 1x/WEEK
mIFNa pDNA, 1x/EVERY OTHER WEEK
16
20
24
28
32
36
40
DAYS POST TUMOR CELL INJECTION

FIG.8B

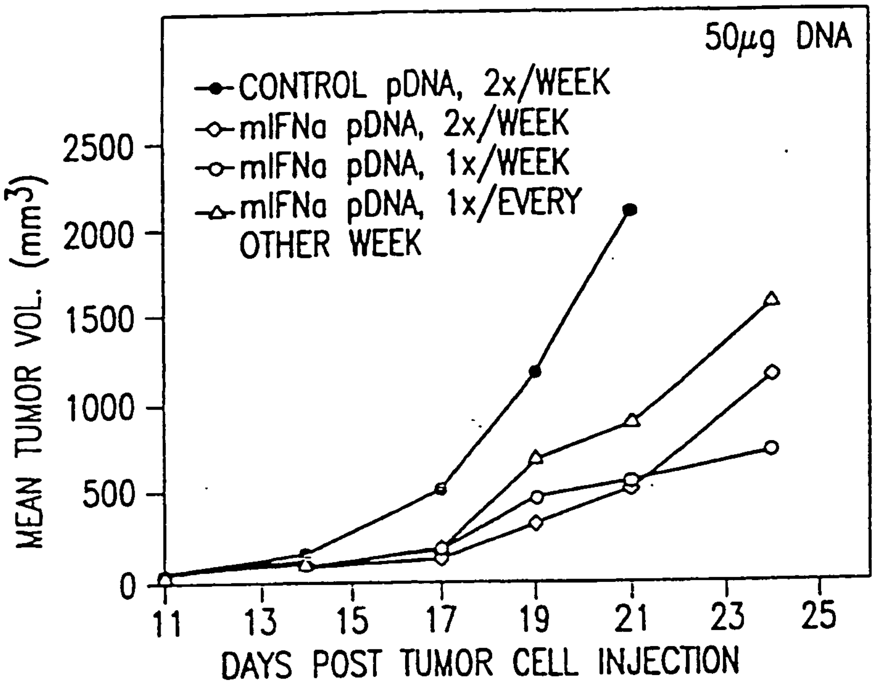
50μg DNA
CONTROL pDNA, 2x/WEEK
mIFNα pDNA, 2x/WEEK
mIFNα pDNA, 1x/WEEK
mIFNα pDNA, 1x/EVERY OTHER WEEK
MEAN TUMOR VOL. (mm3)
0
500
1000
1500
2000
2500
11
13
15
17
19
21
23
25
DAYS POST TUMOR CELL INJECTION

FIG.8C

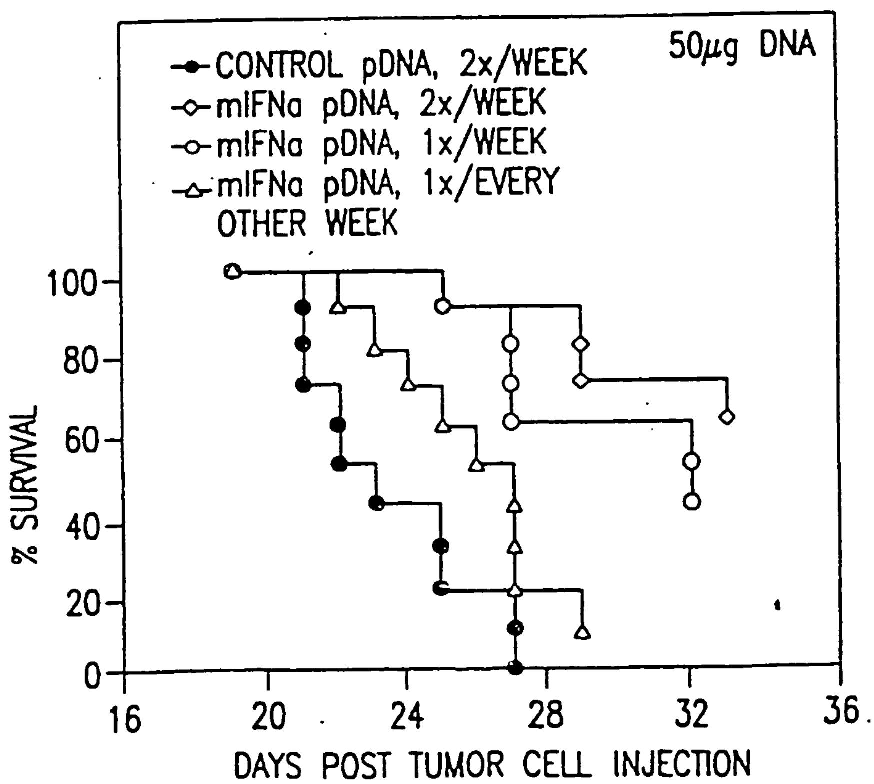
50μg DNA
CONTROL pDNA, 2x/WEEK
mIFNα pDNA, 2x/WEEK
mIFNα pDNA, 1x/WEEK
mIFNα pDNA, 1x/EVERY OTHER WEEK
% SURVIVAL
0
20
40
60
80
100
16
20
24
28
32
36
DAYS POST TUMOR CELL INJECTION

FIG.8D

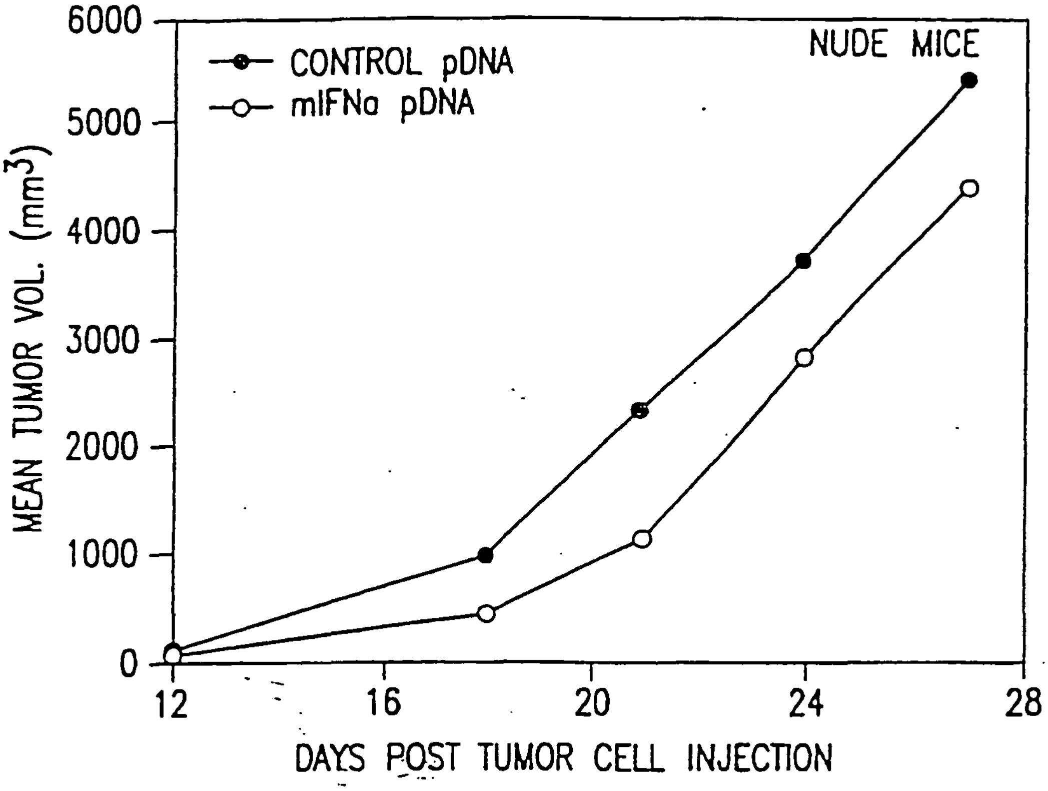
6000
5000
4000
3000
2000
1000
0
MEAN TUMOR VOL. (mm³)
CONTROL pDNA
mIFNα pDNA
NUDE MICE
12
16
20
24
28
DAYS POST TUMOR CELL INJECTION

FIG.9A

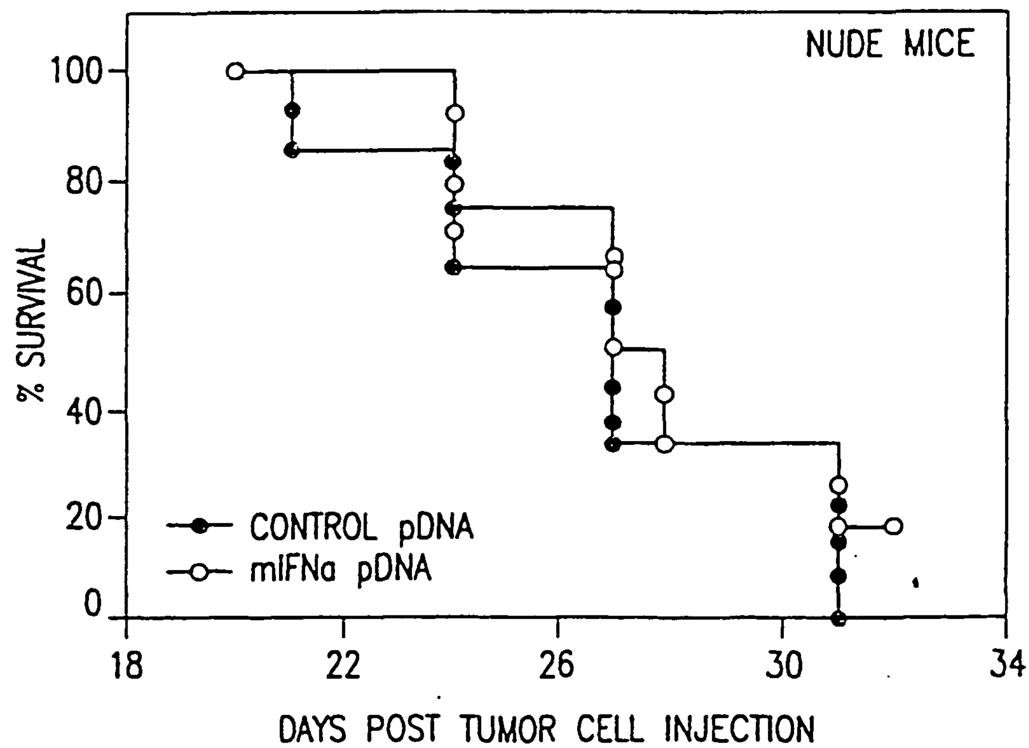
100
80
60
40
20
0
% SURVIVAL
NUDE MICE
CONTROL pDNA
mIFNα pDNA
18
22
26
30
34
DAYS POST TUMOR CELL INJECTION

FIG.9B

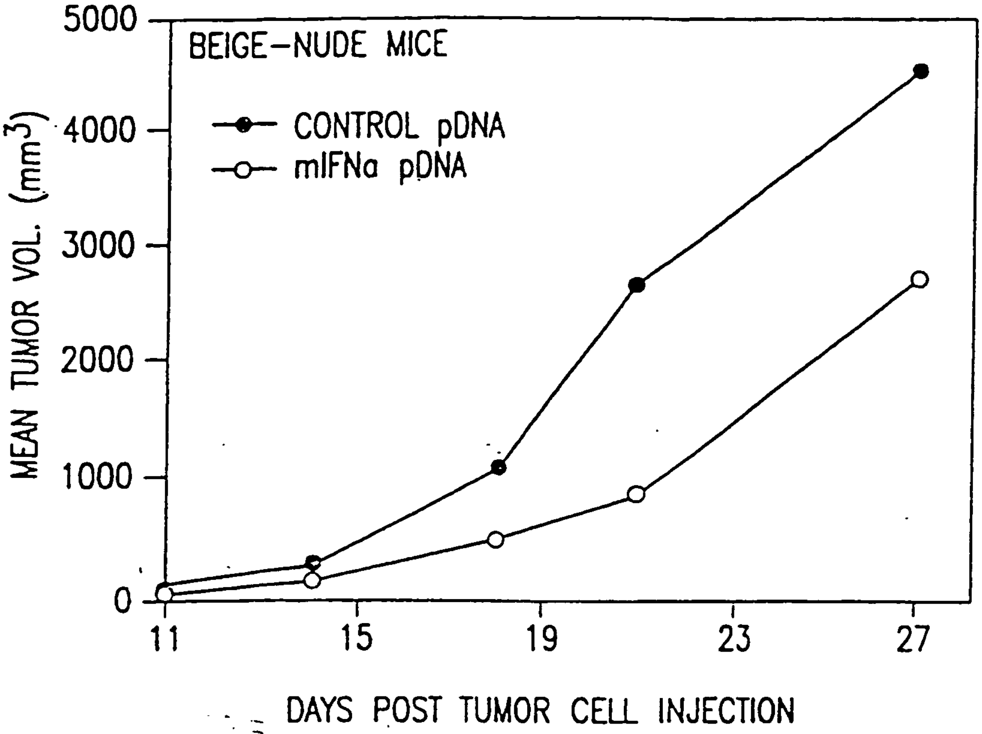
BEIGE-NUDE MICE
CONTROL pDNA
mIFNa pDNA
MEAN TUMOR VOL. (mm3)
5000
4000
3000
2000
1000
0
11
15
19
23
27
DAYS POST TUMOR CELL INJECTION

FIG.9C

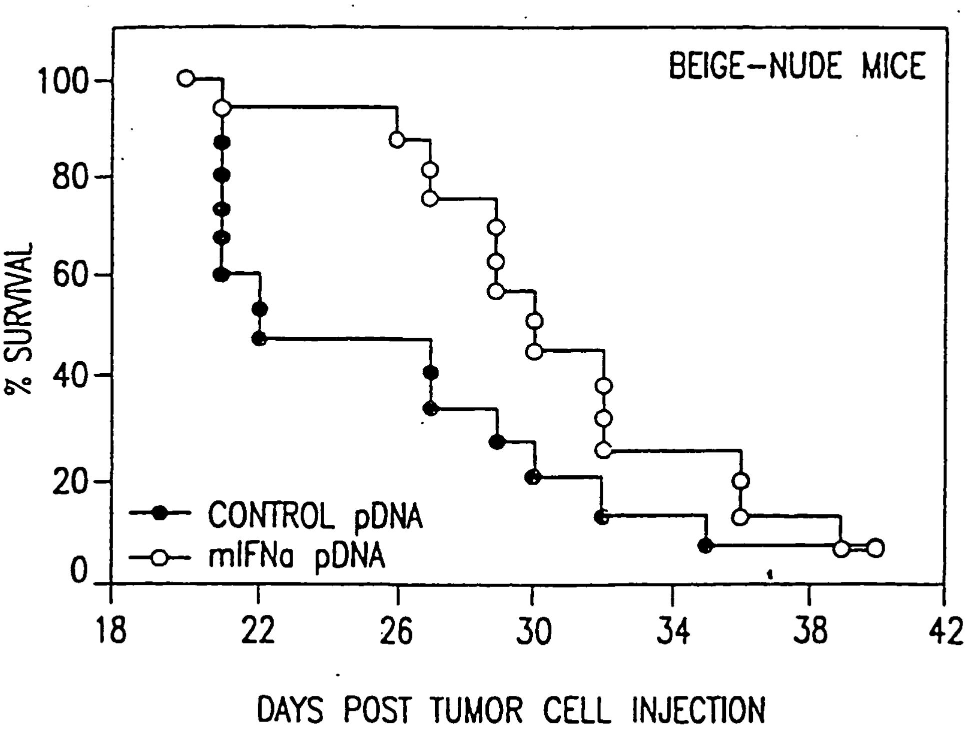
BEIGE-NUDE MICE
CONTROL pDNA
mIFNa pDNA
% SURVIVAL
100
80
60
40
20
0
18
22
26
30
34
38
42
DAYS POST TUMOR CELL INJECTION

FIG.9D

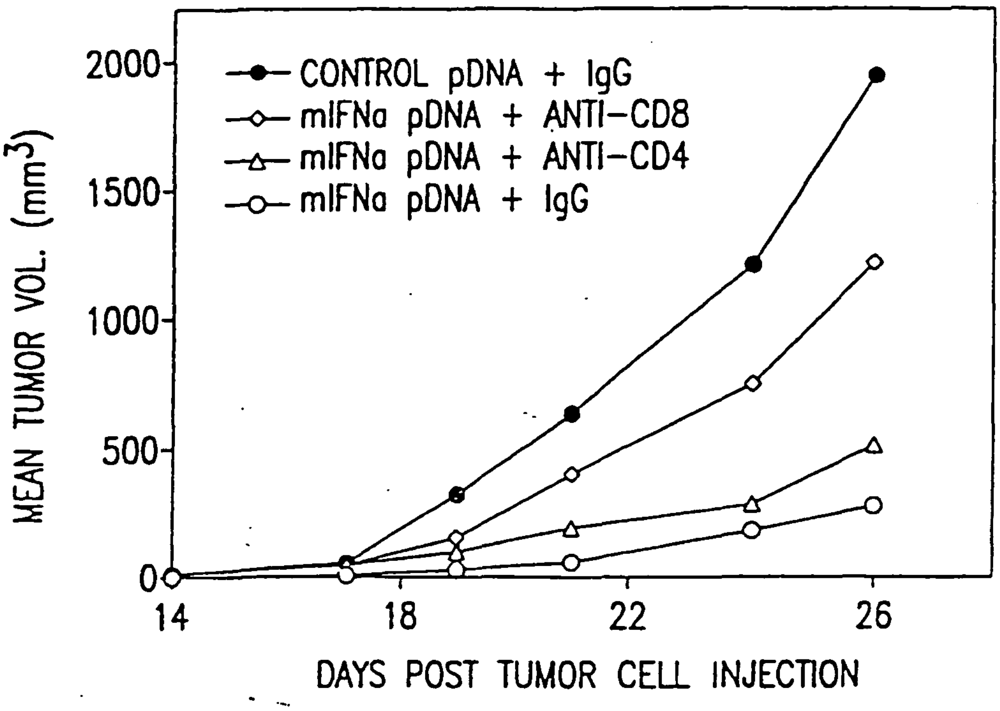


FIG. 10A

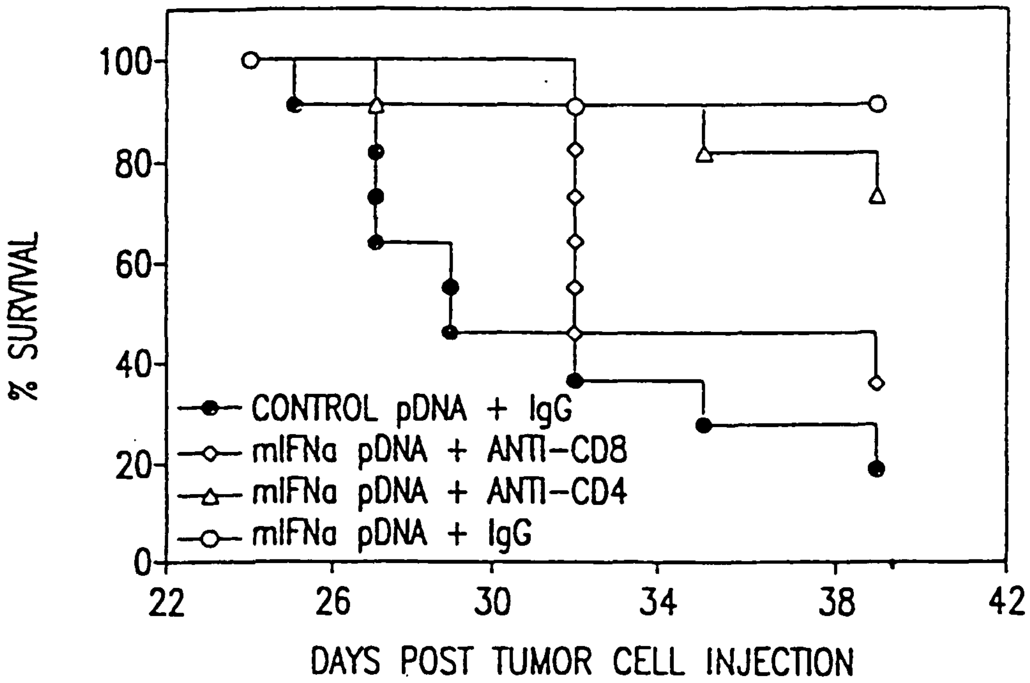


FIG. 10B

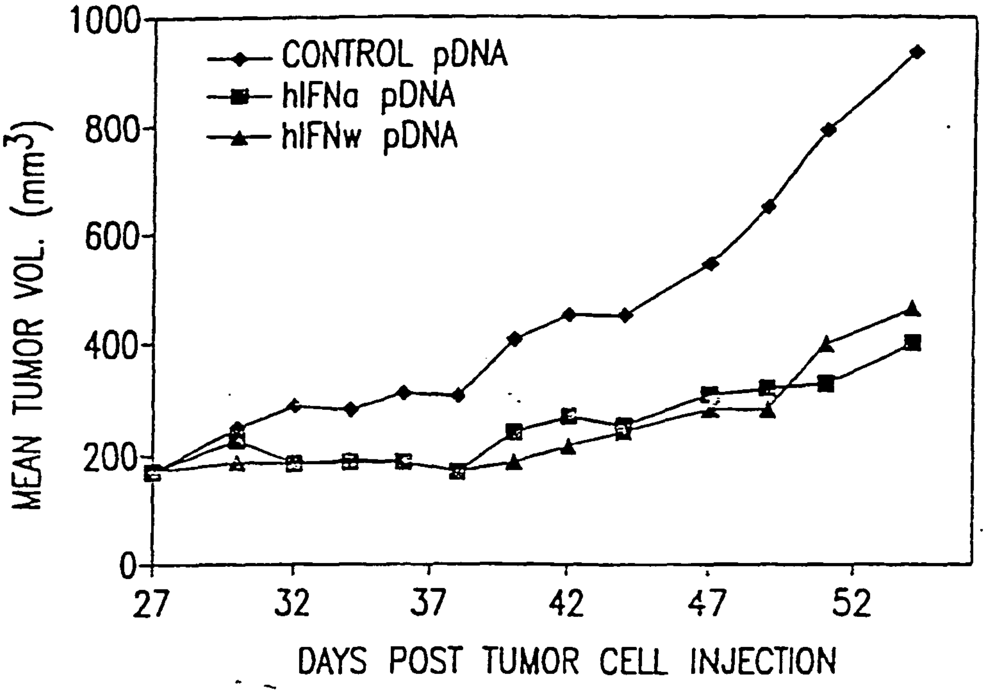


FIG.11A

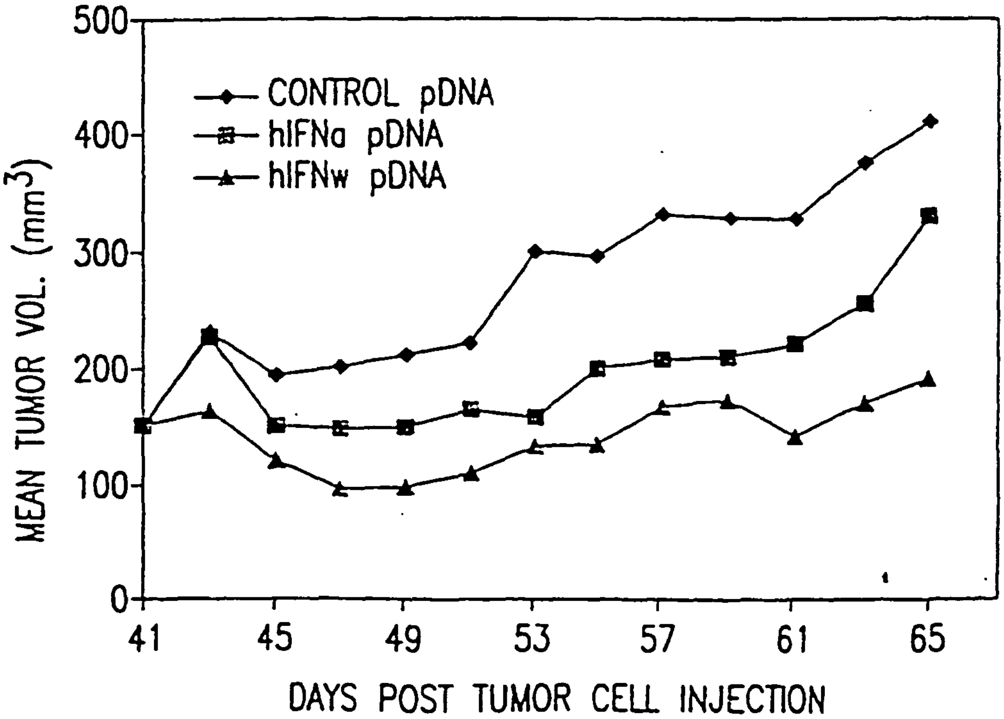


FIG.11B

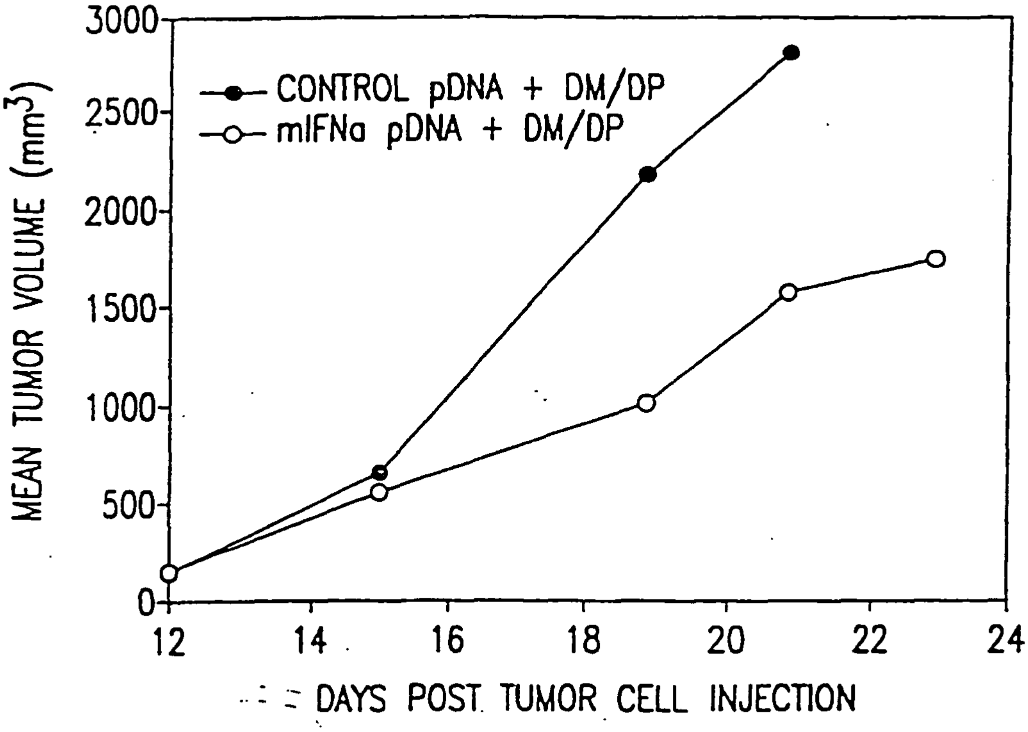


FIG.12A

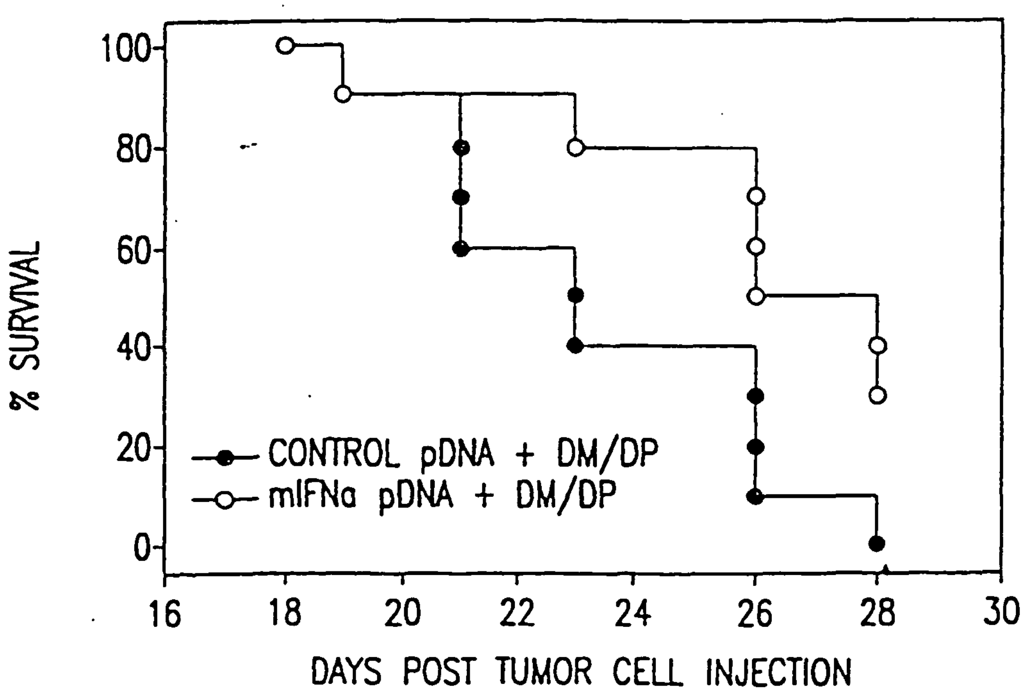


FIG.12B

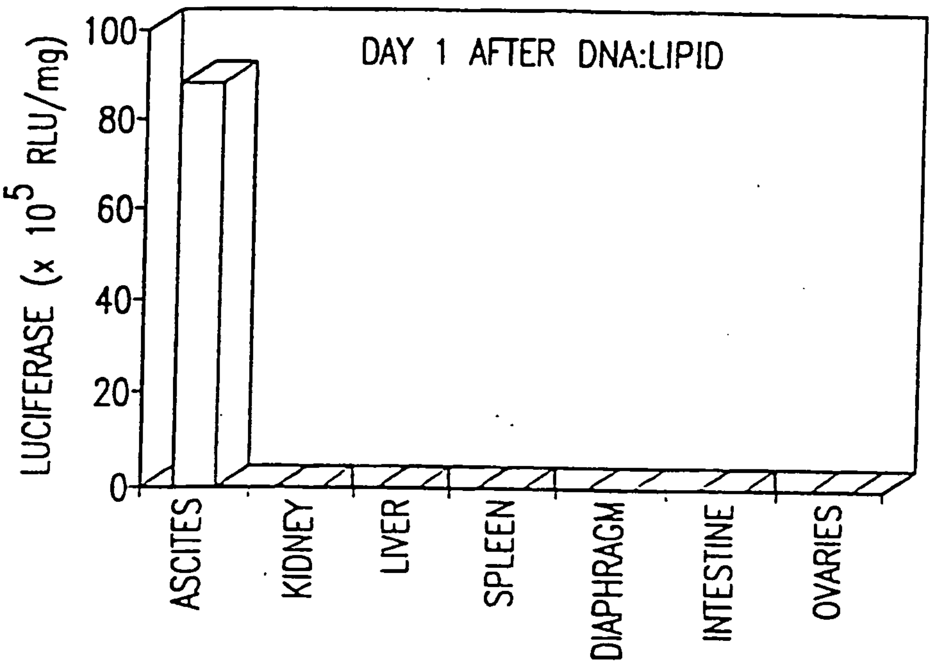


FIG. 13A

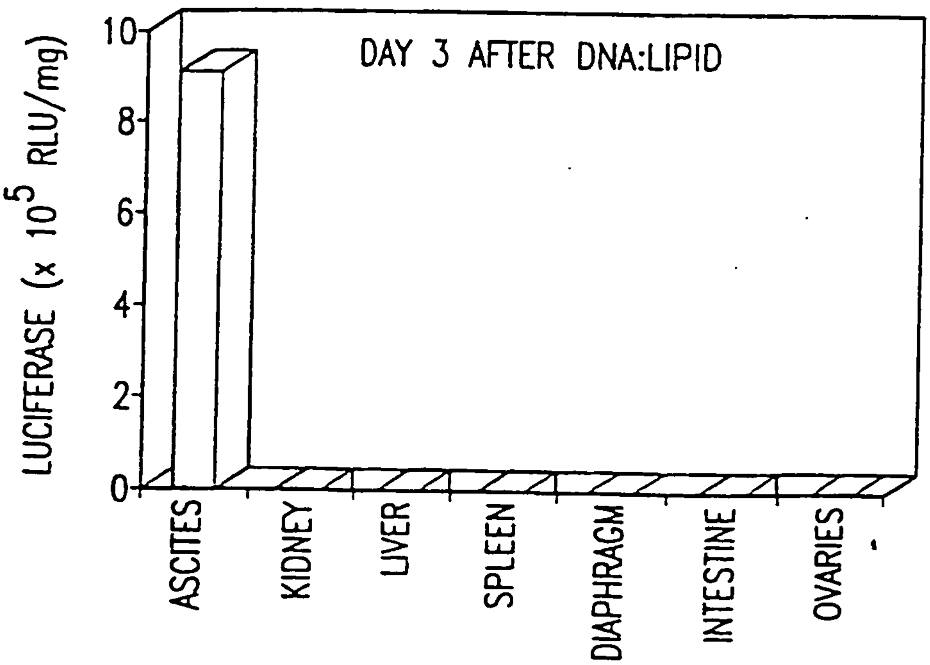


FIG.13B

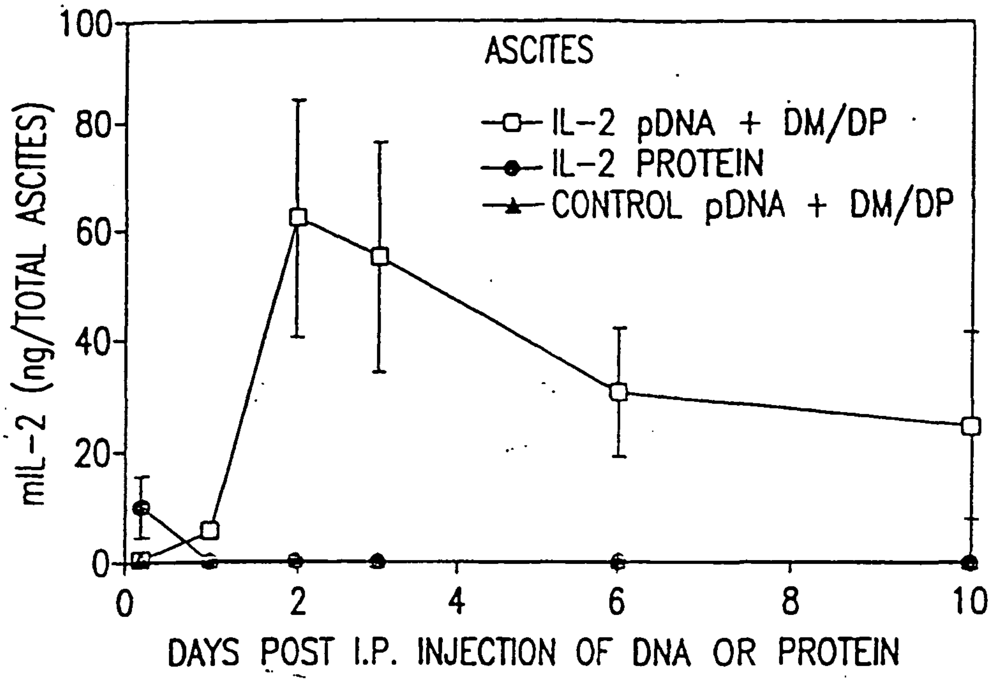


FIG.14A

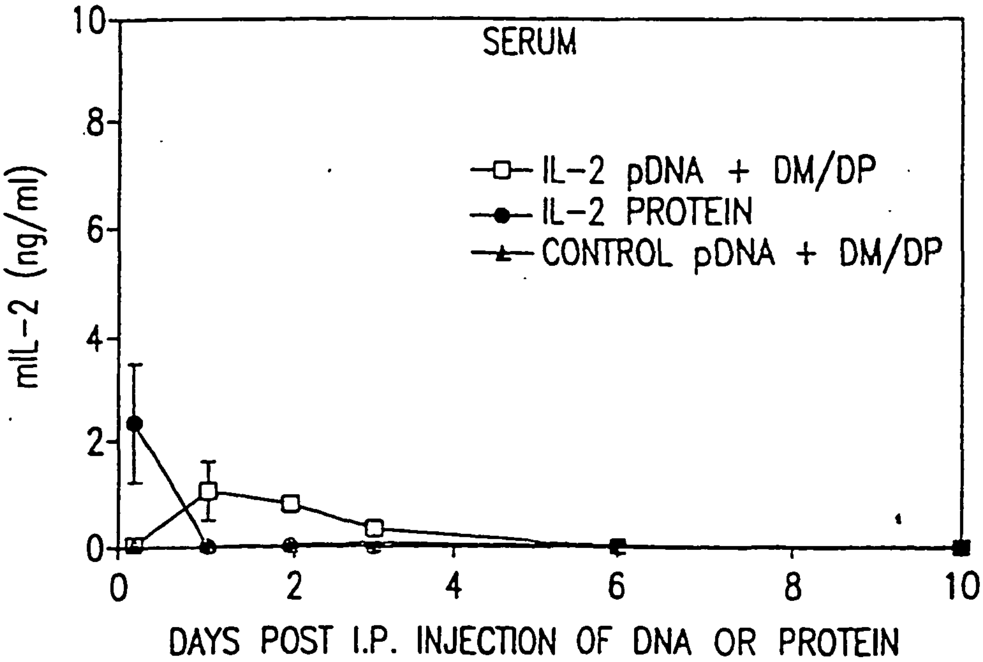


FIG.14B

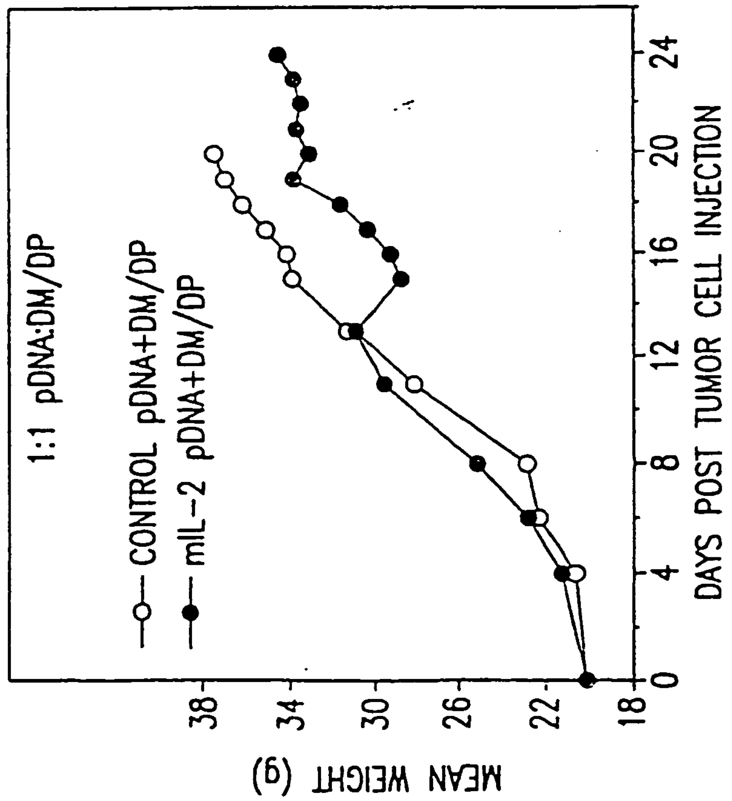


FIG. 15A

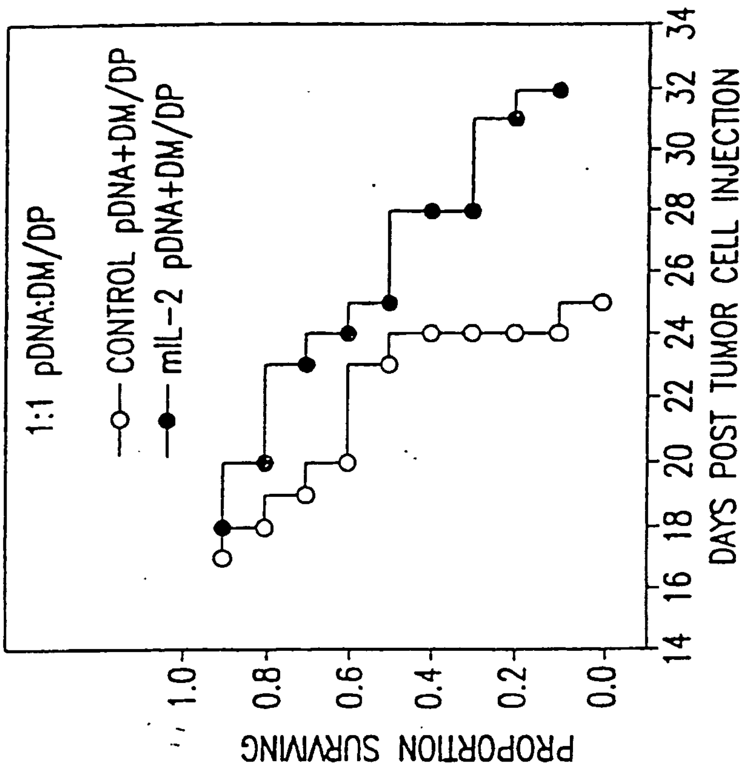


FIG. 15B

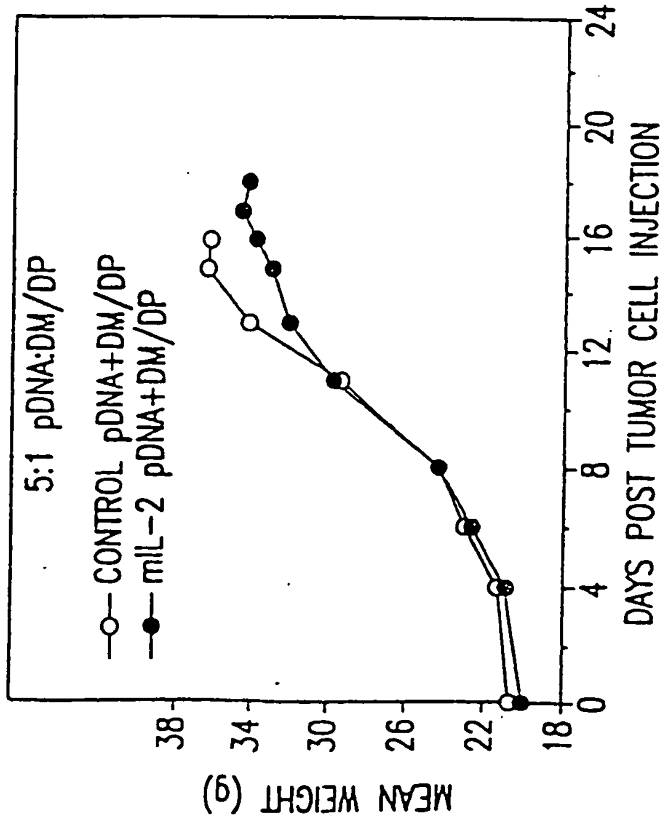


FIG.15C

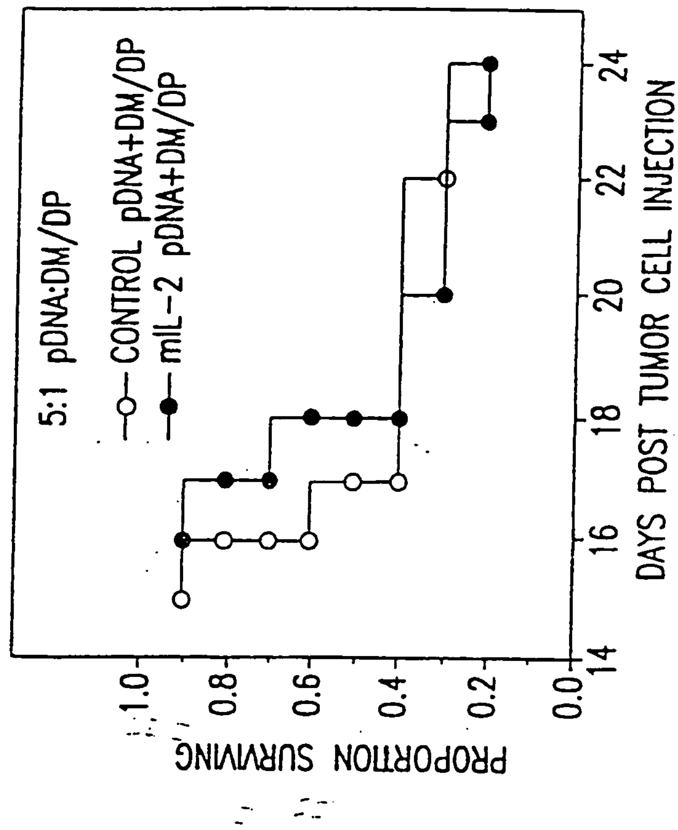


FIG.15D

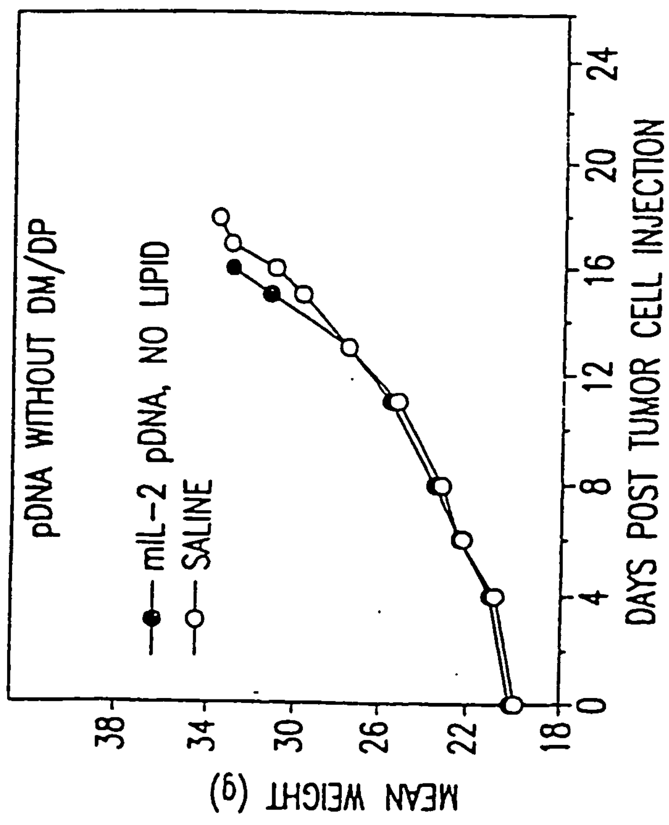


FIG.15E

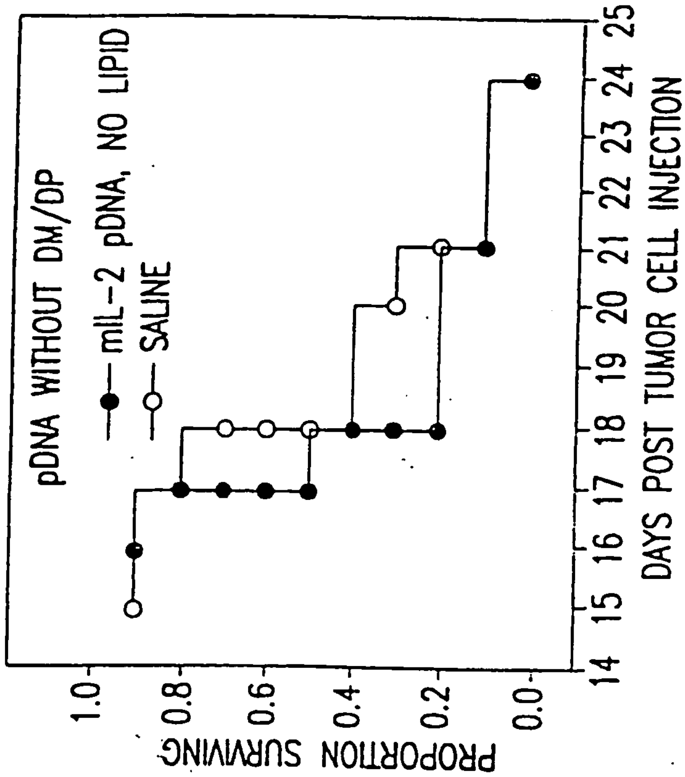


FIG.15F

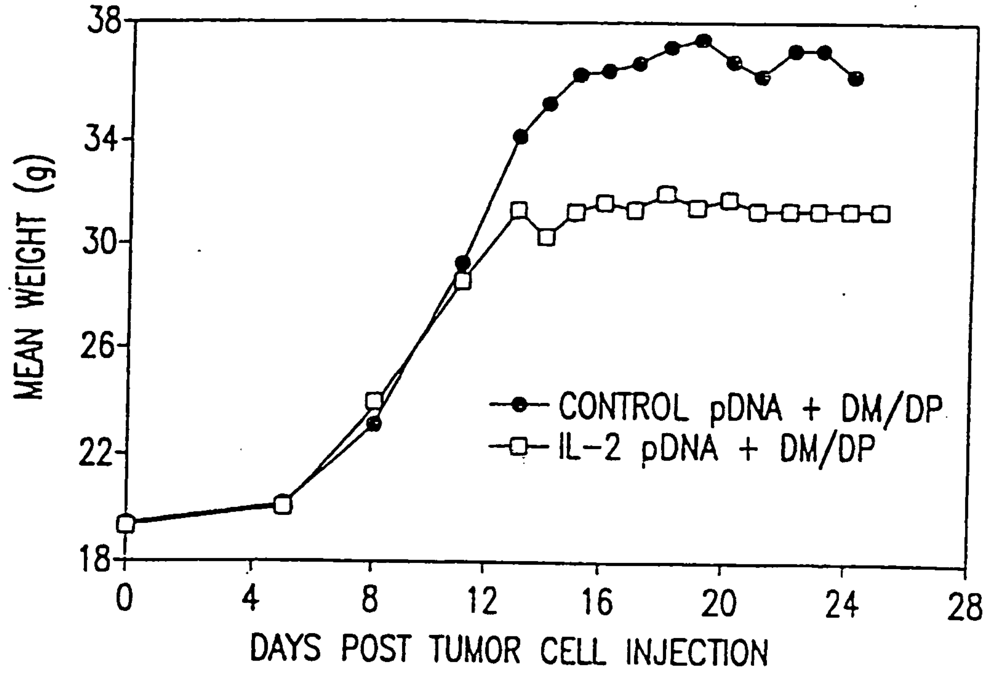


FIG.16A

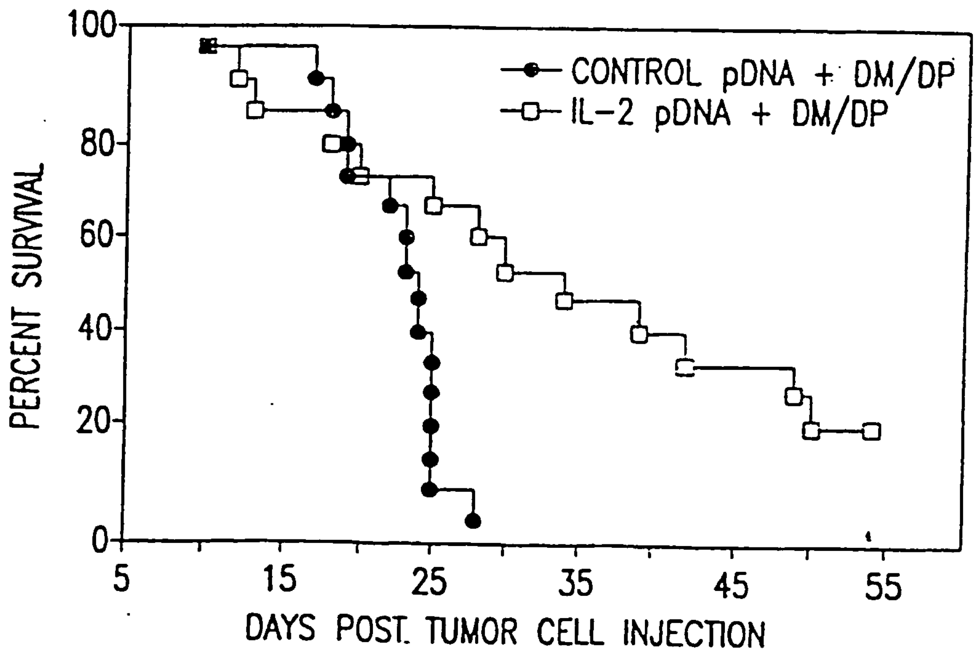


FIG.16B

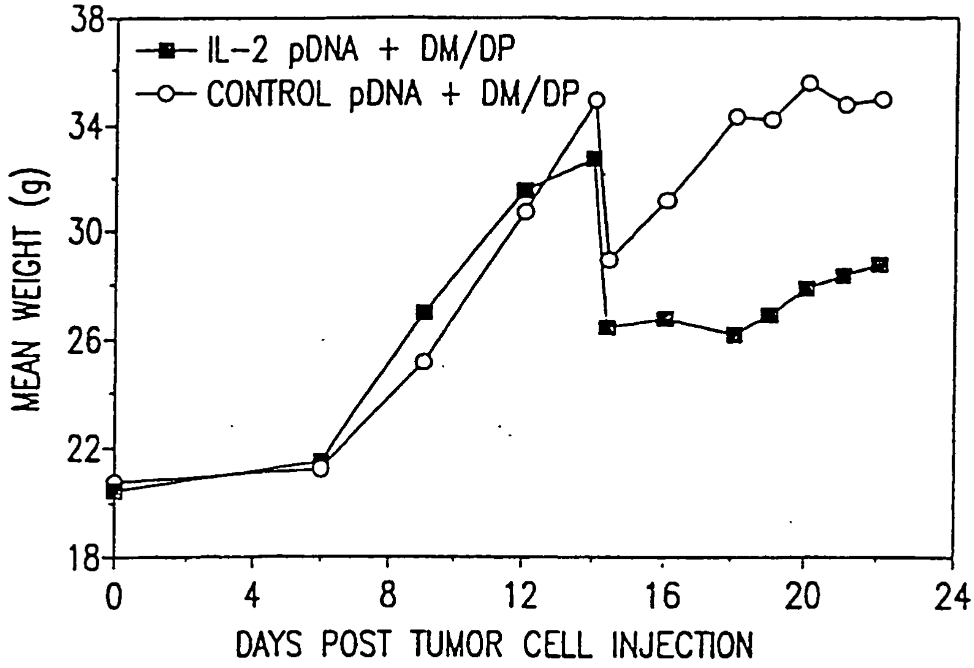


FIG.17A

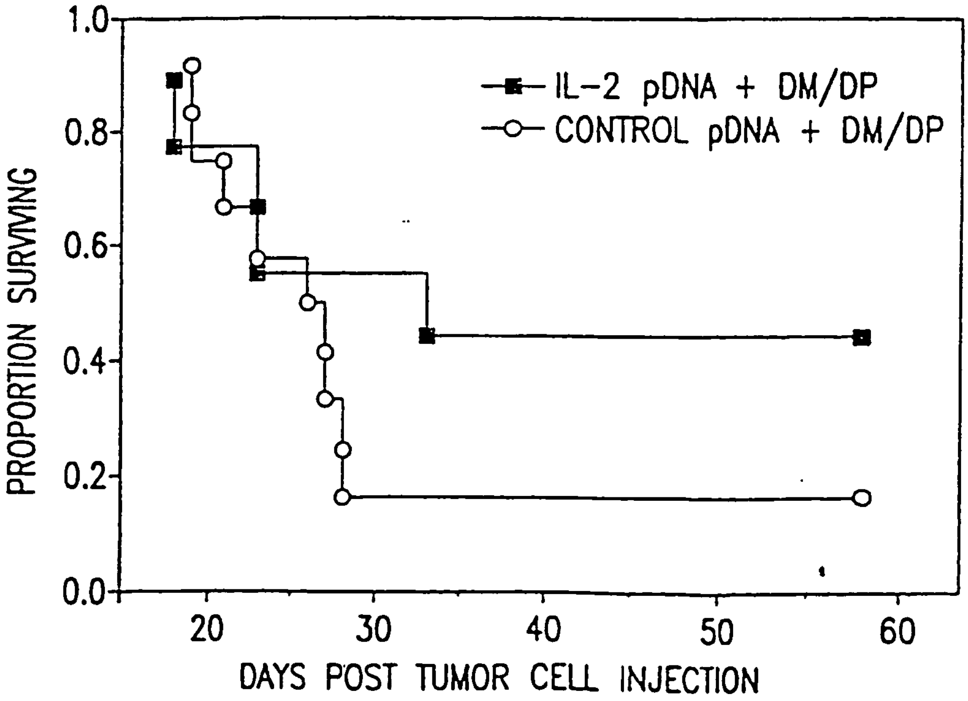


FIG.17B

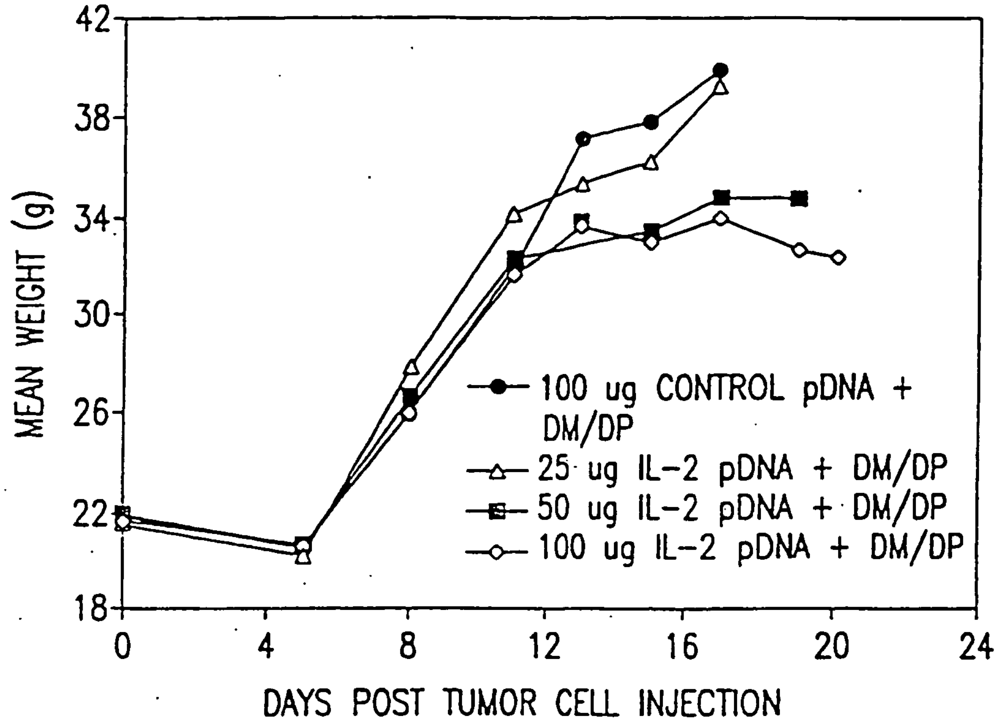


FIG.18A

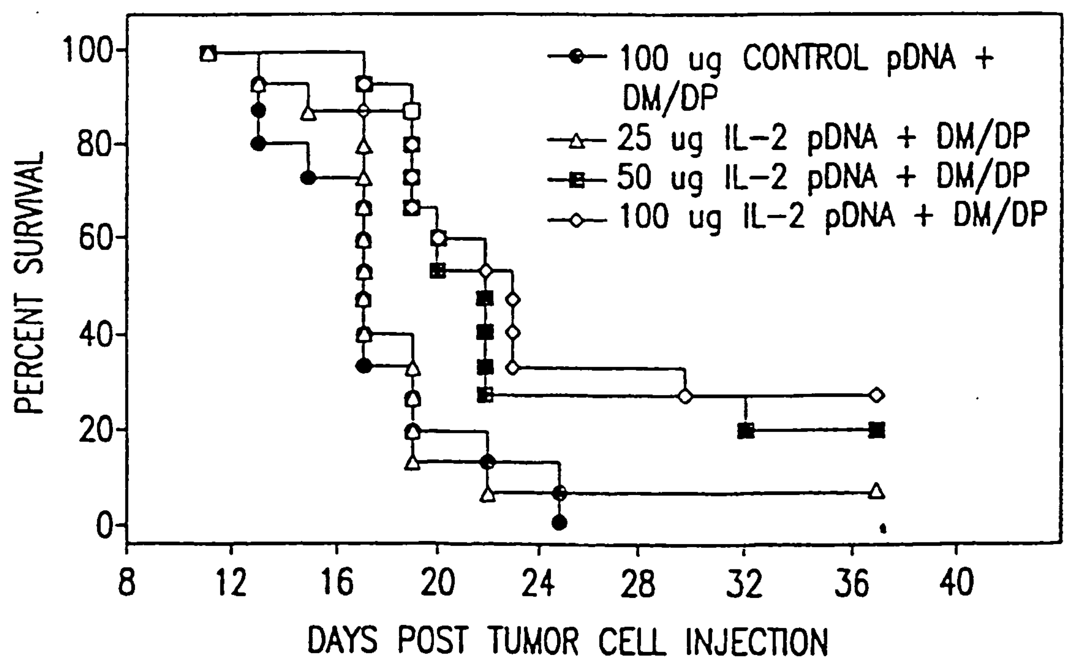


FIG.18B

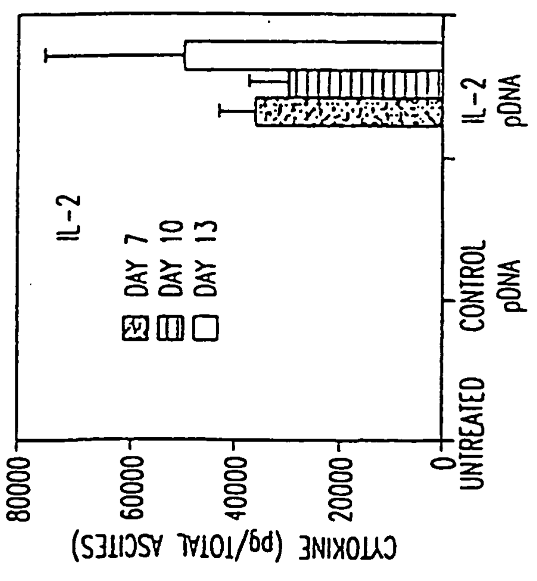


FIG. 19A

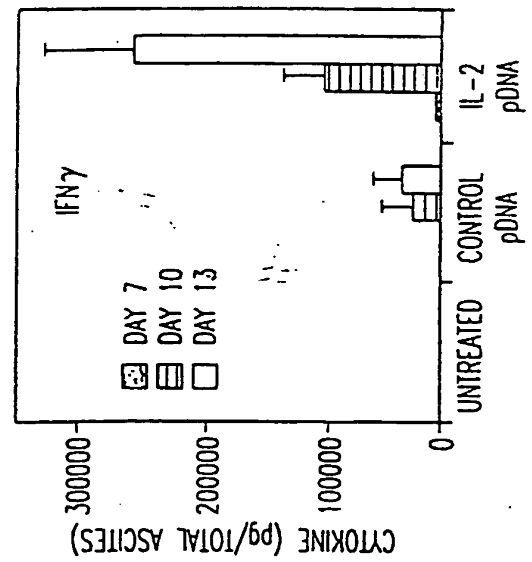


FIG.19B

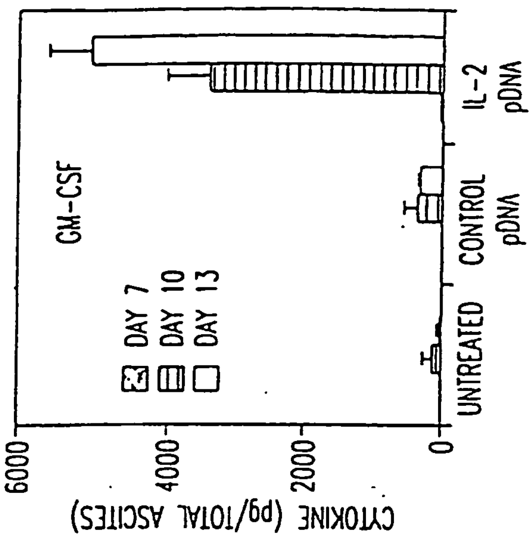


FIG.19C

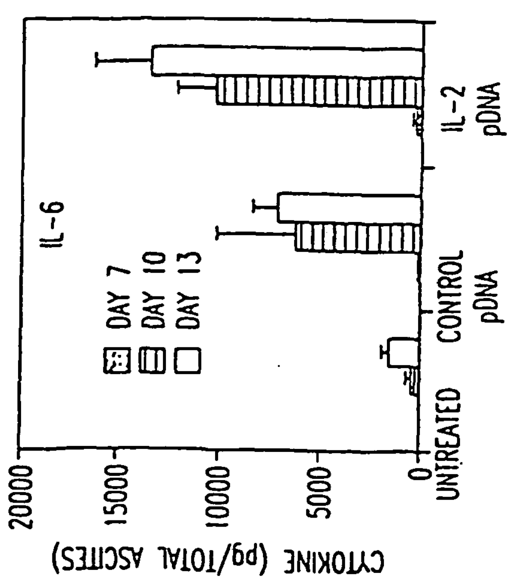


FIG.19D

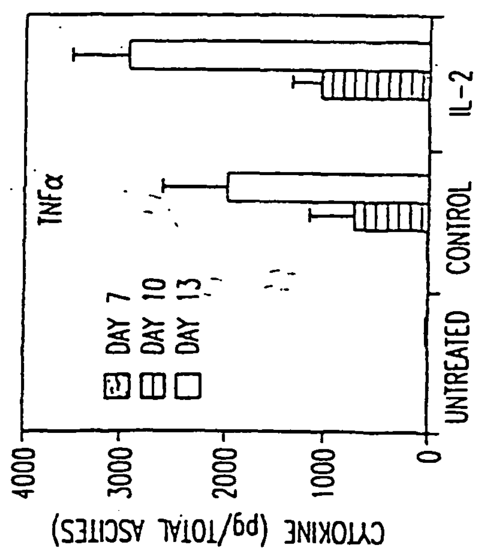


FIG.19E

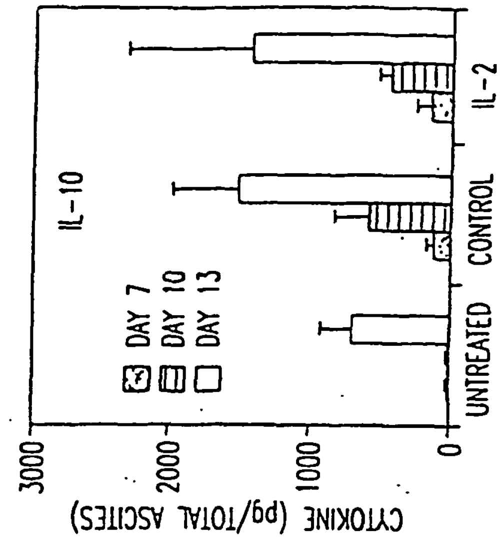


FIG.19F

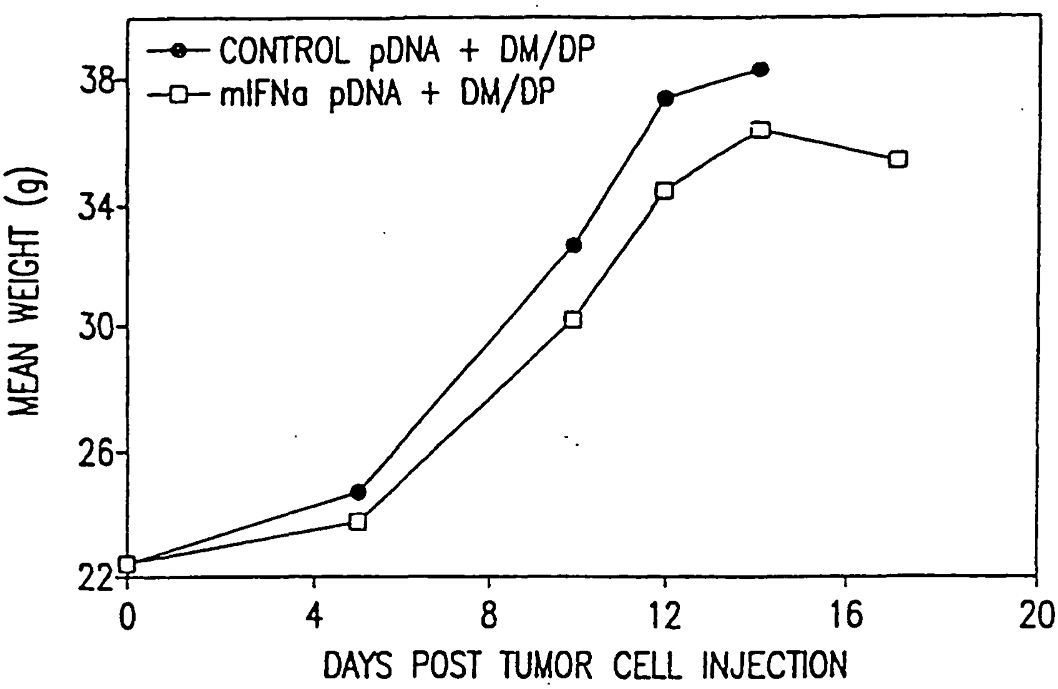


FIG.20A

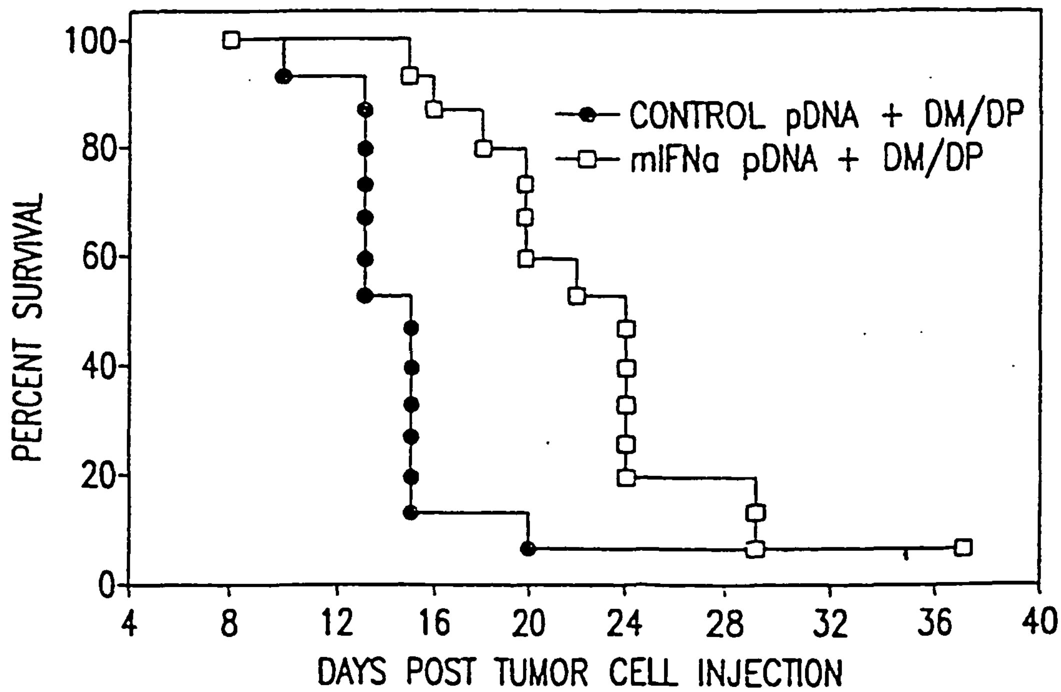


FIG.20B

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 5676954 A
- US 4897355 A
- US 4946787 A
- US 5049386 A
- US 5459127 A
- US 5589466 A
- US 5693622 A
- US 5580859 A
- US 5703055 A
- US 9406069 W
- WO 9429469 A
- US 5264618 A
- US 5279833 A
- US 5334761 A
- US 5429127 A
- US 5578475 A
- WO 049469 A
- WO 9514381 A
- WO 9514651 A
- WO 9517373 A
- WO 9618372 A
- WO 9626179 A
- WO 9640962 A
- WO 9640963 A
- WO 9641873 A
- WO 9700241 A
- US 4917887 A
- EP 0170204 B1
- US 4530901 A
- US 4695543 A
- US 4695623 A
- US 4748233 A
- US 4892743 A
- US 4897471 A
- US 4973479 A
- US 4975276 A
- US 5098703 A
- EP 0307285 A3, Lupker, J.
- US 5641665 A
- US 60067087 B
- US 60079914 B
- US 60100820 B


### Non-patent literature cited in the description


- **Baron, S. et al.** *JAMA,* 1991, vol. 266, 1375
- **Pestka, S.** *Ann. Rev. Biochem.,* 1987, vol. 56, 727
- **Imakawa, K.** *Nature,* 1987, vol. 330, 377
- **Stewart, H.J. et al.** *J. Endocrinology,* 1987, vol. 115, R13
- **Capon, D.J. et al.** *Molec. Cell. Biol.,* 1985, vol. 5, 768-779
- **Feinstein, S. et al.** *Molec. Cell. Biol,* 1985, vol. 5, 510
- **Hauptmann ; Swetly.** *Nucl. Acids Res.,* 1985, vol. 13, 4739-4749
- **Adolf, G. et al.** *Virology,* 1990, vol. 175, 410
- **Flores, I. et al.** *J. Biol. Chem.,* 1991, vol. 266, 19875-19877
- **Kubes, M. et al.** *J. Interferon Research,* 1994, vol. 14, 57
- **Nieroda et al.** *Molec. Cell. Differentiation,* 1996, vol. 4, 335-351
- **Friedman.** Interferons: A Primer. Academic Press, 1981, 104-107
- **Galvani ; Cawley.** Cytokine Therapy. Cambridge University Press, 1992, 114-115
- **Jones.** *Cancer,* 1986, vol. 57, 1709-17 5
- **Quesda et al.** *Blood,* 1986, vol. 68, 493-497
- **Belldegrun, A. et al.** *J. Natl. Cancer Inst.,* 1993, vol. 85, 207-216
- **Ferrantini, M. et al.** *Cancer Research,* 1993, vol. 53, 1107-1112
- **Ferrantini, M. et al.** *J. Immunology,* 1994, vol. 153, 4604-4615
- **Kaido, T. et al.** *Int. J. Cancer,* 1995, vol. 60, 221-229
- **Ogura, H. et al.** *Cancer Research,* 1990, vol. 50, 5102-5100
- **Santodonato, L. et al.** *Human Gene Therapy,* 1996, vol. 7, 1-10
- **Santodonato, L. et al.** *Gene Therapy,* 1997, vol. 4, 1246-1255
- **Zhang, J.-F. et al.** *Cancer Gene Therapy,* 1996, vol. 3, 31-38
- **Zhang, J.-F. et al.** *Proc. Natl. Acad. Sci. USA,* 1996, vol. 93, 4513-4518
- **Yagi, K. et al.** *Biochemistry and Molecular Biology International,* 1994, vol. 32, 167-171
- **Saffran et al.** *Cancer Gene Therapy,* 1998, vol. 5, 321-330

- **Raz, E. et al.** *Proc. Natl. Acad. Sci. USA,* 1993, vol. 90, 4523-4527
- **Raz, E. et al.** *Lupus,* 1995, vol. 4, 266-292
- **Tokui, M. et al.** *Biochem. Biophys. Res. Comm.,* 1997, vol. 233, 527-531
- **Lawson, C. et al.** *J. Interferon Cytokine Res.,* 1997, vol. 17, 255-261
- **Yeow, W.-S. et al.** *J. Immunol.,* 1998, vol. 160, 2932-2939
- **Ross et al.** *Human Gene Therapy,* 1996, vol. 7, 1781-1790
- **Childers et al.** *Gynecol. Oncol.,* 1995, vol. 59, 25-33
- **Naumann et al.** *Gynecol. Oncol.,* 1993, vol. 50, 291-3
- **Almadrones et al.** *Semin. Oncol. Nurs.,* 1995, vol. 11, 194-202
- **Kigwawa et al.** *Am. J. Clin. Oncol.,* 1994, vol. 17, 230-3
- **Markman et al.** *J. Clin. Oncol.,* 1992, vol. 10, 1485-91
- **Fjeld et al.** *Acta. Obstet. Gynecol. Scand. Suppl.,* 1992, vol. 155, 105-11
- **Forstner et al.** *Radiology,* 1995, vol. 196, 715-20
- **Clement.** *Am. J. Clin. Pathol.,* 1995, vol. 103, 673-6
- **Kataoka et al.** *Nippon Sanka Fujinka Gakkai Zasshi,* 1994, vol. 46, 337-44
- **Hamilton.** *Curr. Probl. Cancer,* 1992, vol. 16, 1-57
- **Knapp ; Berkowitz.** *Am. J. Obstet. Gynecol.,* 1977, vol. 128, 782-786
- **Bast et al.** *J. Immunol.,* 1979, vol. 123, 1945-1951
- **Vanhaelen et al.** *Cancer Research,* 1981, vol. 41, 980-983
- **Berek et al.** *Cancer Research,* 1984, vol. 44, 1871-1875
- **Adachi et al.** *Cancer Immunol. Immunother.,* 1993, vol. 37, 1-6
- **Lissoni et al.** *Tumori.,* 1992, vol. 78, 118-20
- **Kikuchi et al.** *Cancer Immunol. Immunother.,* 1996, vol. 43, 257-261
- **Ottow et al.** *Cellular Immunology,* 1987, vol. 104, 366-376
- **Chapman et al.** *Investigational New Drugs,* 1988, vol. 6, 179-188
- **West et al.** *N. Engl. J. Med.,* 1987, vol. 316, 898-905
- **Lotze et al.** *Arch. Surg.,* 1986, vol. 121, 1373-1379
- **Benedetti Panici et al.** *Cancer Treatment Review,* 1989, vol. 16A, 123-127
- **Beller et al.** *Gynecol. Oncol.,* 1989, vol. 34, 407-412
- **Urba et al.** *J. Natl. Cancer Inst.,* 1989, vol. 81, 602-611
- **Stewart et al.** *Cancer Res.,* 1990, vol. 50, 6302-6310
- **Steis et al.** *J. Clin. Oncol.,* 1990, vol. 8, 1618-1629
- **Lissoni et al.** *Tumori,* 1992, vol. 78, 118-120
- **Sparano et al.** *J. of Immunotherapy,* 1994, vol. 16, 216-223
- **Freedman et al.** *J. of Immunotherapy,* 1994, vol. 16, 198-210
- **Edwards et al.** *J. Clin. Oncol.,* 1997, vol. 15, 3399-3407
- **Szala et al.** *Gene Therapy,* 1996, vol. 3, 1025-1031
- **Sugaya et al.** *Hum Gene Ther,* 1996, vol. 7, 223-30
- **Yu et al.** *Oncogene,* 1995, vol. 11, 1383-1385
- **Son.** *Cancer Gene Therapy,* 1997, vol. 4, 391-396
- **Kinoshita et al.** *Biotherapy,* vol. 11 (3), 448-450
- **Parker et al.** *Human Gene Therapy,* 1995, vol. 6, 575-590
- **San et al.** *Human Gene Therapy,* 1993, vol. 4, 781-788
- **Kashima et al.** *Nature,* 1985, vol. 313, 402-404
- **Tone et al.** *Eur. J. Immunol.,* 1996, vol. 26, 1222-1227
- **Gough et al.** *EMBO J.,* 1985, vol. 4, 645-653
- **Nieroda et al.** *Mol. Cell. Differentiation,* 1996, vol. 4, 335-351
- **Ferrantini, M.** *J. Immunology,* 1994, vol. 153, 4604-4615
- **Kaido, T. et al.** *Int, J. Cancer,* 1995, vol. 60, 221-229
- **Ogura, H. et al.** *Cancer Research,* 1990, vol. 50, 5102-5106
- **Felgner et al.** *Proc. Natl. Acad Sci. USA,* 1987, vol. 84, 7413-7417
- **Felgner et al.** *Nature,* 1989, vol. 337, 387-388
- **Wheeler et al.** *Biochim. Biophys. Acta,* 1996, vol. 1280, 1-11
- **Wheeler et al.** *Proc. Natl. Acad. Sci. USA,* 1996, vol. 93, 11454-11459
- **Felgner et al.** *Proc. Natl. Acad. Sci. USA,* 1987, vol. 84, 7413-7417
- **Altman-Hamamdzic, S. et al.** *Gene Therapy,* 1997, vol. 4, 815-822
- **Hauptmann, R. ; P. Swetly.** *Nucl. Acids Res.,* 1985, vol. 13, 4739-4749
- **Adolf, G.R. et al.** *Biochim. Biophys. Acta,* 1991, vol. 1089, 167-174
- **Mege, D. et al.** *J. Interf. Res.,* 1991, vol. 11, 341-350
- **Charlier, M. et al.** *J. Interf. Res.,* 1993, vol. 13, 313-322
- **Hughes, A.L.** *J. Mol. Evol.,* 1995, vol. 41, 539-548

- **Roberts, R.M. et al.** Prog. Nucl. Acid Res. Molec. Biol. Academic Press, 1997, vol. 56, 287-325
- **Pestka, S.** *Methods Enzymol.,* 1986, vol. 119, 3-14
- **Maeda et al.** *Biochem. Biophys. Res. Commun.,* 1983, vol. 115, 1040-1047
- **Mita et al.** *Biochem. Biophys. Res. Commun.,* 1983, vol. 117, 114-121
- **Taniguchi et al.** *Nature,* 1983, vol. 302, 305-310
- **Devos et al.** *Nucleic Acid Res.,* 1983, vol. 11, 4307-4323
- **Fujita et al.** *Proc. Natl. Acad. Sci. USA,* 1983, vol. 80, 74347-7441
- **Clark et al.** *Proc. Natl. Acad Sci. USA,* 1984, vol. 81, 2543-2547
- **Cullen.** *DNA,* 1988, vol. 7, 645-650
- Remington’s Pharmaceutical Sciences. Mack Publishing Co, 1980
- Remington’s Pharmaceutical Sciences. Mack Publishing Co, 1995
- **Kelly, K.A. ; P.M. Pitha.** *Nucl. Acids Res.,* 1985, vol. 13, 805-823
- **Kelly, K.A. ; P.M. Pitha.** *Nucl. Acids Res.,* 1985, vol. 13, 825-839
- *J. Immun.,* vol. 154, 5637
- *J. Immun.,* vol. 156, 1095
- **Hartikka et al.** *Hum. Gene Ther.,* 1996, vol. 7, 1205-1217
- **Doh et al.** *Gene Ther.,* vol. 4, 648-663
- **Sambrook, J. et al.** Molecular Cloning: A laboratory manual. Cold Spring Harbor Laboratory Press, 1989, A.2
- **Horn, N.A. et al.** *Human Gene Therapy,* 1995, vol. 6, 565-573
- **Felgner et al.** *J. Biol. Chem.,* 1994, vol. 269, 2550-2561
- **Stopeck et al.** *J. Clin. Oncol.,* 1997, vol. 15, 341-349
- **Rubin et al.** *Gene Ther.,* 1997, vol. 4, 419-425
- **Berek et al.** *Cancer Res.,* 1984, vol. 44, 1871-1875
- Quantification of plasmid DNA transfection in vivo. **Manthorpe,M. ; Hartikka, J. ; Vahlsing, H.L. ; Sawdey, M.** Gene Quantification. Birkhauser, 1998
- **Sankaran, L.** *Analytical Biochemistry,* 1992, vol. 200, 180-186